(19) Europäisches Patentamt / European Patent Office / Office européen des brevets

(11) EP 2 441 769 B1

(12) **EUROPEAN PATENT SPECIFICATION**

(45) Date of publication and mention of the grant of the patent: **07.05.2014 Bulletin 2014/19**

(51) Int Cl.: ***C07H 21/00*** *(2006.01)* ***B01J 19/00*** *(2006.01)*

(21) Application number: **11161698.3**

(22) Date of filing: **04.05.2005**

(54) **Design, synthesis and assembly of synthetic nucleic acids**

Design, Synthese und Anordnung von synthetischen Nukleinsäuren

Conception, synthèse et assemblage d'acides nucléiques synthétiques

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **04.05.2004 US 567460 P**
**31.03.2005 US 666909 P**

(43) Date of publication of application:
**18.04.2012 Bulletin 2012/16**

(62) Document number(s) of the earlier application(s) in accordance with Art. 76 EPC:
**05780055.9 / 1 771 579**

(73) Proprietor: **REPLIGEN CORPORATION**
**Waltham, MA 02453 (US)**

(72) Inventors:
- **Govindarajan, Sridhar**
  **Redwood City, CA 94065 (US)**
- **Kulikov, Nicolay V.**
  **Redwood City, CA 94061 (US)**
- **Minshull, Jeremy S.**
  **Los Altos, CA 94022 (US)**
- **Ness, Jon E.**
  **Redwood City, CA 94063 (US)**

(74) Representative: **Peterreins, Frank**
**Fish & Richardson P.C.**
**Highlight Business Towers**
**Mies-van-der-Rohe-Strasse 8**
**80807 München (DE)**

(56) References cited:
**US-A1- 2004 029 781**

- **UHLEN M ET AL: "Complete sequence of the staphylococcal gene encoding protein A. A gene evolved through multiple duplications", JOURNAL OF BIOLOGICAL CHEMISTRY, THE AMERICAN SOCIETY OF BIOLOGICAL CHEMISTS, INC, US, vol. 259, no. 3, 1 January 1984 (1984-01-01), pages 1695-1702, XP008092291, ISSN: 0021-9258**
- **SHUTTLEWORTH H L ET AL: "Nucleotide sequence analysis of the gene for protein A from Staphylococcus aureus Cowan 1 (NCTC8530) and its enhanced expression in Escherichia coli", GENE, ELSEVIER, AMSTERDAM, NL, vol. 58, no. 2-3, 1 January 1987 (1987-01-01), pages 283-295, XP023544339, ISSN: 0378-1119, DOI: 10.1016/0378-1119(87)90383-0 [retrieved on 1987-01-01]**
- **JING G ET AL: "High-level expression of staphylococcal nuclease R gene in Escherichia coli", JOURNAL OF BIOTECHNOLOGY, ELSEVIER SCIENCE PUBLISHERS, AMSTERDAM, NL, vol. 22, no. 3, 1 February 1992 (1992-02-01), pages 271-282, XP023705250, ISSN: 0168-1656, DOI: 10.1016/0168-1656(92)90145-Y [retrieved on 1992-02-01]**
- **WELCH MARK ET AL: "Design Parameters to Control Synthetic Gene Expression in Escherichia coli", PLOS ONE, vol. 4, no. 9, September 2009 (2009-09), XP002670364, ISSN: 1932-6203**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> DNA TWOPOINTO INC.

<120> DESIGN, SYNTHESIS AND ASSEMBLY OF SYNTHETIC NUCLEIC ACIDS

<130> 00231-0142EP1

<140> Divisional Application based on EP05780055.9
<141> 2005-05-04

<150> EP05780055.9
<151> 2005-05-04

<150> PCT/US2005/015593
<151> 2005-05-04

<150> 60/666,909
<151> 2005-03-31

<150> 60/567,460
<151> 2004-05-04

<160> 158

<170> FastSEQ for Windows version 4.0

<210> 1
<211> 23
<212> DNA
<213> Artificial Sequence

<220>
<223> Synthetic Nucleotide

<220>
<221> misc_feature
<222> 1, 2, 3, 4, 5, 6, 7, 8, 9, 10, 12, 14, 16
<223> n = A,T,C or G

<400> 1
nnnnnnnnnn anbncngaag agc 23

<210> 2
<211> 23

<212> DNA
<213> Artificial Sequence

<220>
<223> Synthetic Nucleotide

<220>
<221> misc_feature
<222> 8, 9, 11, 13, 15, 16, 17, 18, 19, 20, 21, 22, 23
<223> n = A,T,C or G

<400> 2
gctcttcnna nbncnnnnnn nnn 23

<210> 3
<211> 22
<212> DNA
<213> Artificial Sequence

<220>
<223> Synthetic Nucleotide

<220>
<221> misc_feature
<222> 1, 2, 3, 4, 5, 6, 7, 8, 9, 10, 12, 14, 16
<223> n = A,T,C or G

<400> 3
nnnnnnnnnn anbncngaag ag 22

<210> 4
<211> 22
<212> DNA
<213> Artificial Sequence

<220>
<223> Synthetic Nucleotide

<220>
<221> misc_feature
<222> 7, 8, 10, 12, 14, 15, 16, 17, 18, 19, 20, 21, 22
<223> n = A,T,C or G

<400> 4
ctcttcnnan bncnnnnnnn nn 22

<210> 5
<211> 25
<212> DNA
<213> Artificial Sequence

<220>
<223> Synthetic Nucleotide

<220>
<221> misc_feature
<222> 1, 2, 3, 4, 5, 6, 7, 8, 9, 10, 12, 14, 16, 18, 19
<223> n = A,T,C or G

<400> 5
nnnnnnnnnn anbncndnng tcttc 25

<210> 6
<211> 25
<212> DNA
<213> Artificial Sequence

<220>
<223> Synthetic Nucleotide

<220>
<221> misc_feature
<222> 7, 8, 9, 11, 13, 15, 17, 18, 19, 20, 21, 22, 23, 24, 25
<223> n = A,T,C or G

<400> 6
gaagacnnna nbncndnnnn nnnnn 25

<210> 7
<211> 24
<212> DNA
<213> Artificial Sequence

<220>
<223> oligonucleotide

<220>
<221> misc_feature
<222> 1, 2, 3, 4, 5, 6, 7, 8, 9, 10, 12, 14, 16, 18
<223> n = A,T,C or G

<400> 7
nnnnnnnnnn anbncndnga gacc 24

<210> 8
<211> 24
<212> DNA
<213> Artificial Sequence

<220>
<223> oligonucleotide

<220>
<221> misc_feature
<222> 7, 8, 10, 12, 14, 16, 17, 18, 19, 20, 21, 22, 23, 24
<223> n = A,T,C or G

<400> 8
ggtctcnnan bncndnnnnn nnnn 24

<210> 9
<211> 24
<212> DNA
<213> Artificial Sequence

<220>
<223> Synthetic Nucleotide

<220>
<221> misc_feature
<222> 1, 2, 3, 4, 5, 6, 7, 8, 9, 10, 12, 14, 16, 18
<223> n = A,T,C or G

<400> 9
nnnnnnnnnn anbncndnga gacg 24

<210> 10
<211> 24
<212> DNA
<213> Artificial Sequence

<220>
<223> oligonucleotide

<220>
<221> misc_feature
<222> 7, 8, 10, 12, 14, 16, 17, 18, 19, 20, 21, 22, 23, 24
<223> n = A,T,C or G

<400> 10
cgtctcnnan bncndnnnnn nnnn 24

<210> 11
<211> 27
<212> DNA
<213> Artificial Sequence

<220>
<223> oligonucleotide

<220>
<221> misc_feature
<222> 1, 2, 3, 4, 5, 6, 7, 8, 9, 10, 12, 14, 16, 18, 19, 20, 21
<223> n = A,T,C or G

<400> 11
nnnnnnnnnn anbncndnnn ngcaggt 27

<210> 12
<211> 27
<212> DNA
<213> Artificial Sequence

<220>
<223> oligonucleotide

<220>
<221> misc_feature
<222> 7, 8, 9, 10, 11, 13, 15, 17, 19, 20, 21, 22, 23, 24, 25,
26, 27

<223> n = A,T,C or G

<400> 12
acctgcnnnn nanbncndnn nnnnnnn 27

<210> 13
<211> 30
<212> DNA
<213> Artificial Sequence

<220>
<223> oligonucleotide

<220>
<221> misc_feature
<222> 1, 2, 3, 4, 5, 6, 7, 8, 9, 10, 12, 14, 16, 18, 19, 20, 21, 22, 23, 24, 25

<223> n = A,T,C or G

<400> 13
nnnnnnnnnn anbncndnnn nnnnngctgc 30

<210> 14
<211> 30
<212> DNA
<213> Artificial Sequence

<220>
<223> Oligonucleotide

<220>
<221> misc_feature
<222> 6, 7, 8, 9, 10, 11, 12, 13, 14, 16, 18, 20, 22, 23, 24, 25, 26, 27, 28, 29, 30

<223> n = A,T,C or G

<400> 14
gcagcnnnnn nnnnanbncn dnnnnnnnnn 30

<210> 15
<211> 23
<212> DNA
<213> Artificial Sequence

<220>
<223> Oligonucleotide

<220>
<221> misc_feature
<222> 1, 2, 3, 4, 5, 6, 7, 8, 9, 10, 12, 14, 16, 18
<223> n = A,T,C or G

<400> 15
nnnnnnnnnn anbncndnga gac 23

<210> 16
<211> 23
<212> DNA
<213> Artificial Sequence

<220>

<223> Oligonucleotide

<220>
<221> misc_feature
<222> 6, 7, 9, 11, 13, 15, 16, 17, 18, 19, 20, 21, 22, 23
<223> n = A,T,C or G

<400> 16
gtctcnnanb ncndnnnnnn nnn 23

<210> 17
<211> 33
<212> DNA
<213> Artificial Sequence

<220>
<223> Oligonucleotide

<220>
<221> misc_feature
<222> 1, 2, 3, 4, 5, 6, 7, 8, 9, 10, 11, 13, 15, 17, 19, 20, 21, 22, 23, 24, 25, 26, 27, 28

<223> n = A,T,C or G

<400> 17
nnnnnnnnnn nanbncndnn nnnnnnnngt ccc 33

<210> 18
<211> 33
<212> DNA
<213> Artificial Sequence

<220>
<223> Oligonucleotide

<220>
<221> misc_feature
<222> 6, 7, 8, 9, 10, 11, 12, 13, 14, 15, 16, 18, 20, 22, 24, 25, 26, 27, 28, 29, 30, 31, 32, 33

<223> n = A,T,C or G

<400> 18
gggacnnnnn nnnnnnanbn cndnnnnnnn nnn 33

<210> 19
<211> 24
<212> DNA
<213> Artificial Sequence

<220>
<223> Oligonucleotide

<220>
<221> misc_feature
<222> 1, 2, 3, 4, 5, 6, 7, 8, 9, 10, 12, 14, 16, 18
<223> n = A,T,C or G

<400> 19
nnnnnnnnnn anbncndnga gacg 24

<210> 20
<211> 24
<212> DNA
<213> Artificial Sequence

<220>
<223> Oligonucleotide

<220>
<221> misc_feature
<222> 7, 8, 10, 12, 14, 16, 17, 18, 19, 20, 21, 22, 23, 24
<223> n = A,T,C or G

<400> 20
cgtctcnnan bncndnnnnn nnnn 24

<210> 21
<211> 31
<212> DNA
<213> Artificial Sequence

<220>
<223> Oligonucleotide

<220>
<221> misc_feature
<222> 1, 2, 3, 4, 5, 6, 7, 8, 9, 10, 12, 14, 16, 18, 19, 20, 21, 22, 23, 24, 25, 26

<223> n = A,T,C or G

<400> 21
nnnnnnnnnn anbncndnnn nnnnnncatc c 31

<210> 22
<211> 31
<212> DNA
<213> Artificial Sequence

<220>
<223> Oligonucleotide

<220>
<221> misc_feature
<222> 6, 7, 8, 9, 10, 11, 12, 13, 14, 15, 17, 19, 21, 23, 24, 25, 26, 27, 28, 29, 30, 31

<223> n = A,T,C or G

<400> 22
ggatgnnnnn nnnnnanbnc ndnnnnnnnn n 31

<210> 23
<211> 27
<212> DNA

<213> Artificial Sequence

<220>
<223> Oligonucleotide

<220>
<221> misc_feature
<222> 1, 2, 3, 4, 5, 6, 7, 8, 9, 10, 12, 14, 16, 18, 19, 20, 21, 22

<223> n = A,T,C or G

<400> 23
nnnnnnnnnn anbncndnnn nngatgc 27

<210> 24
<211> 27
<212> DNA
<213> Artificial Sequence

<220>
<223> Oligonucleotide

<220>
<221> misc_feature
<222> 6, 7, 8, 9, 10, 11, 13, 15, 17, 19, 20, 21, 22, 23, 24, 25, 26, 27

<223> n = A,T,C or G

<400> 24
gcatcnnnnn nanbncndnn nnnnnnn 27

<210> 25
<211> 28
<212> DNA
<213> Artificial Sequence

<220>
<223> Oligonucleotide

<220>
<221> misc_feature
<222> 1, 2, 3, 4, 5, 6, 7, 8, 9, 10, 12, 14, 16, 18, 19, 20, 21, 22, 23

<223> n = A,T,C or G

<400> 25
nnnnnnnnnn anbncndnnn nnngcgtc 28

<210> 26
<211> 28
<212> DNA
<213> Artificial Sequence

<220>
<223> Oligonucleotide

<220>
<221> misc_feature
<222> 6, 7, 8, 9, 10, 11, 13, 15, 17, 19, 20, 21, 22, 23, 24, 25, 26, 27, 28
<223> n = A,T,C or G

<400> 26
gacgcnnnnn nanbncndnn nnnnnnnn 28

<210> 27
<211> 22
<212> DNA
<213> Artificial Sequence

<220>
<223> Oligonucleotide

<220>
<221> misc_feature
<222> 1, 2, 3, 4, 5, 6, 7, 8, 9, 10, 11, 13, 14, 15, 16
<223> n = A,T,C or G

<400> 27
nnnnnnnnnn nannnnccca gt 22

<210> 28
<211> 22
<212> DNA
<213> Artificial Sequence

<220>
<223> Oligonucleotide

<220>
<221> misc_feature
<222> 7, 8, 9, 10, 11, 13, 14, 15, 16, 17, 18, 19, 20, 21, 22
<223> n = A,T,C or G

<400> 28
actgggnnnn nannnnnnnn nn 22

<210> 29
<211> 33
<212> DNA
<213> Artificial Sequence

<220>
<223> Oligonucleotide

<220>
<221> misc_feature
<222> 1, 2, 3, 4, 5, 6, 7, 8, 9, 10, 12, 14, 15, 16, 17, 18, 19, 20, 21, 22, 23, 24, 25, 26, 27
<223> n = A,T,C or G

<400> 29

nnnnnnnnnn anbnnnnnnn nnnnnnnctc cag 33

<210> 30
<211> 34
<212> DNA
<213> Artificial Sequence

<220>
<223> Oligonucleotide.

<220>
<221> misc_feature
<222> 7, 8, 9, 10, 11, 12, 13, 14, 15, 16, 17, 18, 19, 20, 21, 23, 25, 26, 27, 28, 29, 30, 31, 32, 33, 34

<223> n = A,T,C or G

<400> 30
ctggagnnnn nnnnnnnnnn nanbnnnnnn nnnn 34

<210> 31
<211> 27
<212> DNA
<213> Artificial Sequence

<220>
<223> Oligonucleotide

<220>
<221> misc_feature
<222> 1, 2, 3, 4, 5, 6, 7, 8, 9, 10, 12, 14, 15, 16, 17, 18, 19, 20, 21

<223> n = A,T,C or G

<400> 31
nnnnnnnnnn anbnnnnnnn nctccag 27

<210> 32
<211> 28
<212> DNA
<213> Artificial Sequence

<220>
<223> Oligonucleotide

<220>
<221> misc_feature
<222> 7, 8, 9, 10, 11, 12, 13, 14, 15, 17, 19, 20, 21, 22, 23, 24, 25, 26, 27, 28

<223> n = A,T,C or G

<400> 32
ctggagnnnn nnnnnanbnn nnnnnnnn 28

<210> 33
<211> 17

<212> DNA
<213> Artificial Sequence

<220>
<223> Oligonucleotide

<220>
<221> misc_feature
<222> 1, 2, 3, 4, 5, 6, 7, 8, 9, 10
<223> n = A,T,C or G

<400> 33
nnnnnnnnnn agcattc 17

<210> 34
<211> 18
<212> DNA
<213> Artificial Sequence

<220>
<223> Oligonucleotide

<220>
<221> misc-feature
<222> 7, 9, 10, 11, 12, 13, 14, 15, 16, 17, 18
<223> n = A,T,C or G

<400> 34
gaatgcnbnn nnnnnnnn 18

<210> 35
<211> 16
<212> DNA
<213> Artificial Sequence

<220>
<223> Oligonucleotide

<220>
<221> misc_feature
<222> 1, 2, 3, 4, 5, 6, 7, 8, 9, 10
<223> n = A,T,C or G

<400> 35
nnnnnnnnnn accagt 16

<210> 36
<211> 17
<212> DNA
<213> Artificial Sequence

<220>
<223> Oligonucleotide

<220>
<221> misc_feature
<222> 6, 8, 9, 10, 11, 12, 13, 14, 15, 16, 17
<223> n = A,T,C or G

<400> 36
actggnbnnn nnnnnnn 17

<210> 37
<211> 19
<212> DNA
<213> Artificial Sequence

<220>
<223> Oligonucleotide

<220>
<221> misc_feature
<222> 1, 2, 3, 4, 5, 6, 7, 8, 9, 10, 12
<223> n = A,T,C or G

<400> 37
nnnnnnnnnn anbcattgc 19

<210> 38
<211> 20
<212> DNA
<213> Artificial Sequence

<220>
<223> Oligonucleotide

<220>
<221> misc_feature
<222> 7, 9, 11, 12, 13, 14, 15, 16, 17, 18, 19, 20
<223> n = A,T,C or G

<400> 38
gcaatgnanb nnnnnnnnnn 20

<210> 39
<211> 2031
<212> DNA
<213> Artificial Sequence

<220>
<223> vector 1

<400> 39

```
gaggaagcgg aaggcgagag tagggaactg ccaggcatca aactaagcag aaggcccctg 60
acggatggcc tttttgcgtt tctacaaact ctttctgtgt tgtaaaacga cggccagtct 120
taagctcggg cctcaaataa tgattttaga tatcgccatc cagctgatat tccctatagt 180
gcatggtcat agctgtttcc tggcagctct ggcccgtgtc tcaaaatctc tgatgttaca 240
ttgtacaaga taaaataata tcatcatgaa caataaaact gtctgcttac ataaacagta 300
atacaagggg tgttatgagc catattcaac gggaaacgtc gaggccgcga ttaaattcca 360
acatggatgc tgatttatat gggtataaat gggctcgcga taatgtcggg caatcaggtg 420
cgacaatcta tcgcttgtat gggaagcccg atgcgccaga gttgtttctg aaacatggca 480
aaggtagcgt tgccaatgat gttacagatg agatggtcag actaaactgg ctgacggaat 540
```

```
ttatgccact tccgaccatc aagcatttta tccgtactcc tgatgatgca tggttactca 600
ccactgcgat ccccggaaaa acagcgttcc aggtattaga agaatatcct gattcaggtg 660
aaaatattgt tgatgcgctg gcagtgttcc tgcgccggtt gcactcgatt cctgtttgta 720
attgtccttt taacagcgat cgcgtatttc gcctcgctca ggcgcaatca cgaatgaata 780
acggtttggt tgatgcgagt gattttgatg acgagcgtaa tggctggcct gttgaacaag 840
tctggaaaga aatgcataaa cttttgccat tctcaccgga ttcagtcgtc actcatggtg 900
atttctcact tgataacctt atttttgacg aggggaaatt aataggttgt attgatgttg 960
gacgagtcgg aatcgcagac cgataccagg atcttgccat cctatggaac tgcctcggtg 1020
agttttctcc ttcattacag aaacggcttt ttcaaaaata tggtattgat aatcctgata 1080
tgaataaatt gcagtttcat ttgatgctcg atgagttttt ctaatcagaa ttggttaatt 1140
ggttgtaaca ctggcagagc attacgctga cttgacggga cggcgcaagc tcatgaccaa 1200
aatcccttaa cgtgagttac gcgcgcgtcg ttccactgag cgtcagaccc cgtagaaaag 1260
atcaaaggat cttcttgaga tccttttttt ctgcgcgtaa tctgctgctt gcaaacaaaa 1320
aaaccaccgc taccagcggt ggtttgtttg ccggatcaag agctaccaac tctttttccg 1380
aaggtaactg gcttcagcag agcgcagata ccaaatactg ttcttctagt gtagccgtag 1440
ttagcccacc acttcaagaa ctctgtagca ccgcctacat acctcgctct gctaatcctg 1500
ttaccagtgg ctgctgccag tggcgataag tcgtgtctta ccgggttgga ctcaagacga 1560
tagttaccgg ataaggcgca gcggtcgggc tgaacggggg gttcgtgcac acagcccagc 1620
ttggagcgaa cgacctacac cgaactgaga tacctacagc gtgagctatg agaaagcgcc 1680
acgcttcccg aagggagaaa ggcggacagg tatccggtaa gcggcagggt cggaacagga 1740
gagcgcacga gggagcttcc agggggaaac gcctggtatc tttatagtcc tgtcgggttt 1800
cgccacctct gacttgagcg tcgatttttg tgatgctcgt caggggggcg gagcctatgg 1860
aaaaacgcca gcaacgcggc ctttttacgg ttcctggcct tttgctggcc ttttgctcac 1920
atgttctttc ctgcgttatc ccctgattct gtggataacc gtattaccgc ctttgagtga 1980
gctgataccg ctcgccgcag ccgaacgacc gagcgcagcg agtcagtgag c          2031
```

<210> 40

<211> 4420

<212> DNA

<213> Artificial Sequence

<220>

<223> vector

<400> 40

```
gaggaagcgg aaggcgagag tagggaactg ccaggcatca aactaagcag aaggcccctg 60
acggatggcc tttttgcgtt tctacaaact ctttctgtgt tgtaaaacga cggccagtct 120
taagctcggg ccccaaataa tgattttatt ttgactgata gtgacctgtt cgttgcaaca 180
cattgatgag caatgctttt ttataatgcc aactttgtac aaaaaagctg aacgagaaac 240
gtaaaatgat ataaatatca atatattaaa ttagattttg cataaaaaac agactacata 300
atactgtaaa acacaacata tccagtcact atgaatcaac tacttagatg gtattagtga 360
cctgtagtcg accgacagcc ttccaaatgt tcttcgggtg atgctgccaa cttagtcgac 420
cgacagcctt ccaaatgttc ttctcaaacg gaatcgtcgt atccagccta ctcgctattg 480
tcctcaatgc cgtattaaat cataaaaaga aataagaaaa agaggtgcga gcctcttttt 540
tgtgtgacaa aataaaaaca tctacctatt catatacgct agtgtcatag tcctgaaaat 600
catctgcatc aagaacaatt tcacaactct tatacttttc tcttacaagt cgttcggctt 660
catctggatt ttcagcctct atacttacta aacgtgataa agtttctgta atttctactg 720
tatcgacctg cagactggct gtgtataagg gagcctgaca tttatattcc ccagaacatc 780
aggttaatgg cgtttttgat gtcattttcg cggtggctga gatcagccac ttcttccccg 840
ataacggaga ccggcacact ggccatatcg gtggtcatca tgcgccagct ttcatccccg 900
atatgcacca ccgggtaaag ttcacgggag actttatctg acagcagacg tgcactggcc 960
agggggatca ccatccgtcg cccgggcgtg tcaataatat cactctgtac atccacaaac 1020
agacgataac ggctctctct tttataggtg taaaccttaa actgcatttc accagcccct 1080
gttctcgtca gcaaaagagc cgttcatttc aataaaccgg gcgacctcag ccatcccttc 1140
ctgattttcc gctttccagc gttcggcacg cagacgacgg gcttcattct gcatggttgt 1200
gcttaccaga ccggagatat tgacatcata tatgccttga gcaactgata gctgtcgctg 1260
tcaactgtca ctgtaatacg ctgcttcata gcatacctct tttgacata cttcgggtat 1320
acatatcagt atatattctt ataccgcaaa aatcagcgcg caaatacgca tactgttatc 1380
tggcttttag taagccggat ccacgcggcg tttacgcccc gccctgccac tcatcgcagt 1440
actgttgtaa ttcattaagc attctgccga catggaagcc atcacagacg gcatgatgaa 1500
cctgaatcgc cagcggcatc agcaccttgt cgccttgcgt ataatatttg cccatggtga 1560
aaacgggggc gaagaagttg tccatattgg ccacgtttaa atcaaaactg gtgaaactca 1620
cccagggatt ggctgagacg aaaaacatat tctcaataaa ccctttaggg aaataggcca 1680
```

```
ggttttcacc gtaacacgcc acatcttgcg aatatatgtg tagaaactgc cggaaatcgt 1740
cgtggtattc actccagagc gatgaaaacg tttcagtttg ctcatggaaa acggtgtaac 1800
aagggtgaac actatcccat atcaccagct caccgtcttt cattgccata cggaattccg 1860
gatgagcatt catcaggcgg gcaagaatgt gaataaaggc cggataaaac ttgtgcttat 1920
ttttctttac ggtctttaaa aaggccgtaa tatccagctg aacggtctgg ttataggtac 1980
attgagcaac tgactgaaat gcctcaaaat gttctttacg atgccattgg gatatatcaa 2040
cggtggtata tccagtgatt tttttctcca ttttagcttc cttagctcct gaaaatctcg 2100
ataactcaaa aaatacgccc ggtagtgatc ttatttcatt atggtgaaag ttggaacctc 2160
ttacgtgccg atcaacgtct cattttcgcc aaaagttggc ccagggcttc ccggtatcaa 2220
cagggacacc aggatttatt tattctgcga agtgatcttc cgtcacaggt atttattcgg 2280
cgcaaagtgc gtcgggtgat gctgccaact tagtcgacta caggtcacta ataccatcta 2340
agtagttgat tcatagtgac tggatatgtt gtgttttaca gtattatgta gtctgttttt 2400
tatgcaaaat ctaatttaat atattgatat ttatatcatt ttacgtttct cgttcagctt 2460
tcttgtacaa agttggcatt ataagaaagc attgcttatc aatttgttgc aacgaacagg 2520
tcactatcag tcaaaataaa atcattattt gccatccagc tgatatcccc tataggtcat 2580
agctgtttcc tggcagctct ggcccgtgtc tcaaaatctc tgatgttaca ttgtacaaga 2640
taaaataata tcatcatgaa caataaaact gtctgcttac ataaacagta atacaagggg 2700
tgttatgagc catattcaac gggaaacgtc gaggccgcga ttaaattcca acatggatgc 2760
tgatttatat gggtataaat gggctcgcga taatgtcggg caatcaggtg cgacaatcta 2820
tcgcttgtat gggaagcccg atgcgccaga gttgtttctg aaacatggca aaggtagcgt 2880
tgccaatgat gttacagatg agatggtcag actaaactgg ctgacggaat ttatgccact 2940
tccgaccatc aagcatttta tccgtactcc tgatgatgca tggttactca ccactgcgat 3000
ccccggaaaa acagcgttcc aggtattaga agaatatcct gattcaggtg aaaatattgt 3060
tgatgcgctg gcagtgttcc tgcgccggtt gcactcgatt cctgtttgta attgtccttt 3120
taacagcgat cgcgtatttc gcctcgctca ggcgcaatca cgaatgaata acggtttggt 3180
tgatgcgagt gattttgatg acgagcgtaa tggctggcct gttgaacaag tctggaaaga 3240
aatgcataaa cttttgccat tctcaccgga ttcagtcgtc actcatggtg atttctcact 3300
tgataacctt atttttgacg aggggaaatt aataggttgt attgatgttg gacgagtcgg 3360
aatcgcagac cgataccagg atcttgccat cctatggaac tgcctcggtg agttttctcc 3420
ttcattacag aaacggcttt ttcaaaaata tggtattgat aatcctgata tgaataaatt 3480
gcagtttcat ttgatgctcg atgagttttt ctaatcagaa ttggttaatt ggttgtaaca 3540
ctggcagagc attacgctga cttgacggga cggcgcaagc tcatgaccaa aatcccttaa 3600
cgtgagttac gcgcgcgtcg ttccactgag cgtcagaccc cgtagaaaag atcaaaggat 3660
cttcttgaga tccttttttt ctgcgcgtaa tctgctgctt gcaaacaaaa aaaccaccgc 3720
taccagcggt ggtttgtttg ccggatcaag agctaccaac tctttttccg aaggtaactg 3780
gcttcagcag agcgcagata ccaaatactg ttcttctagt gtagccgtag ttagcccacc 3840
acttcaagaa ctctgtagca ccgcctacat acctcgctct gctaatcctg ttaccagtgg 3900
ctgctgccag tggcgataag tcgtgtctta ccgggttgga ctcaagacga tagttaccgg 3960
ataaggcgca gcggtcgggc tgaacggggg gttcgtgcac acagcccagc ttggagcgaa 4020
cgacctacac cgaactgaga tacctacagc gtgagctatg agaaagcgcc acgcttcccg 4080
aagggagaaa ggcggacagg tatccggtaa gcggcagggt cggaacagga gagcgcacga 4140
gggagcttcc agggggaaac gcctggtatc tttatagtcc tgtcgggttt cgccacctcg 4200
acttgagcgt cgatttttgt gatgctcgtc aggggggcgg agcctatgga aaaacgccag 4260
caacgcggcc tttttacggt tcctggcctt ttgctggcct tttgctcaca tgttctttcc 4320
tgcgttatcc cctgattctg tggataaccg tattaccgcc tttgagtgag ctgataccgc 4380
tcgccgcagc cgaacgaccg agcgcagcga gtcagtgagc                       4420
```

<210> 41
<211> 29
<212> DNA
<213> Artificial Sequence

<220>
<223> Syn thetic Nucleotide

<400> 41
ggggacaagt ttgtacaaaa aagcaggct 29

<210> 42
<211> 29
<212> DNA
<213> Artificial Sequence

<220>
<223> Synthetic Nucleotide

<400> 42

acccagcttt cttgtacaaa gtggtcccc 29

<210> 43
<211> 2899
<212> DNA
<213> Artificial Sequence

<220>
<223> vector

<400> 43

```
gaggaagcgg aaggcgagag tagggaactg ccaggcatca aactaagcag aaggcccctg 60
acggatggcc tttttgcgtt tctacaaact ctttctgtgt tgtaaaacga cggccagtct 120
taagctcggg cctcaaataa tgattttaga ttaacggtct ccttttctcg agcggataaa 180
tgtgagcgga taacattgac attgtgagcg gataacaaga tactgagcac atcagcagga 240
cgcactgacc gcgggatccc ggtgcagaaa ataaggagga aaaaaaaatg agcaaaggtg 300
aagaactgtt caccggcgtt gtgccaattc tggttgagct ggatggtgac gtgaatggcc 360
acaaattttc cgtgtctggt gaaggcgagg gtgatgctac ttatggcaaa ctgactctga 420
aactgatctg taccaccggc aaactgcctg ttccgtggcc aactctggtc actactctgg 480
gttacggcct gatgtgtttt gcgcgttacc cggatcacat gaaacagcat gacttcttca 540
aatctgccat gccggaaggc tatgtccaag aacgtacgat ctttttcaag gacgacggca 600
actataaaac ccgtgccgaa gttaaattcg agggtgacac cctggttaac cgcatcgaac 660
tgaaaggcat tgacttcaaa gaggacggca acattctggg tcacaagctg gaatacaact 720
acaactccca caacgtttac attactgctg acaagcagaa aaacggcatc aaagcaaact 780
tcaagatccg tcacaacatt gaagatggtg gcgtacagct ggcagatcac taccagcaga 840
acactccaat cggtgatggc ccagtactgc tgccagataa ccattacctg tcctaccaga 900
gcaaactgtc taaagacccg aacgaaaaac gtgaccacat ggtactgctg gaatttgtta 960
ccgcggcagg cattacccac ggtatggacg aactgtataa ataaccccag agaccgttaa 1020
tcgccatcca gctgatattc cctatagtgc atggtcatag ctgtttcctg gcagctctgg 1080
cccgtgtctc aaaatctctg atgttacatt gtacaagata aaataatatc atcatgaaca 1140
ataaaactgt ctgcttacat aaacagtaat acaaggggtg ttatgagcca tattcaacgg 1200
gaaacgtcga ggccgcgatt aaattccaac atggatgctg atttatatgg gtataaatgg 1260
gctcgcgata atgtcgggca atcaggtgcg acaatctatc gcttgtatgg gaagcccgat 1320
gcgccagagt tgtttctgaa acatggcaaa ggtagcgttg ccaatgatgt tacagatgag 1380
atggtcagac taaactggct gacggaattt atgccacttc cgaccatcaa gcattttatc 1440
cgtactcctg atgatgcatg gttactcacc actgcgatcc ccggaaaaac agcgttccag 1500
gtattagaag aatatcctga ttcaggtgaa aatattgttg atgcgctggc agtgttcctg 1560
cgccggttgc actcgattcc tgtttgtaat tgtcctttta acagcgatcg cgtatttcgc 1620
ctcgctcagg cgcaatcacg aatgaataac ggtttggttg atgcgagtga ttttgatgac 1680
gagcgtaatg gctggcctgt tgaacaagtc tggaaagaaa tgcataaact tttgccattc 1740
tcaccggatt cagtcgtcac tcatggtgat ttctcacttg ataaccttat ttttgacgag 1800
gggaaattaa taggttgtat tgatgttgga cgagtcggaa tcgcagaccg ataccaggat 1860
cttgccatcc tatggaactg cctcggtgag ttttctcctt cattacagaa acggcttttt 1920
caaaaatatg gtattgataa tcctgatatg aataaattgc agtttcattt gatgctcgat 1980
gagtttttct aatcagaatt ggttaattgg ttgtaacact ggcagagcat tacgctgact 2040
tgacgggacg gcgcaagctc atgaccaaaa tcccttaacg tgagttacgc gcgcgtcgtt 2100
ccactgagcg tcagaccccg tagaaaagat caaaggatct tcttgagatc ctttttttct 2160
gcgcgtaatc tgctgcttgc aaacaaaaaa accaccgcta ccagcggtgg tttgtttgcc 2220
ggatcaagag ctaccaactc tttttccgaa ggtaactggc ttcagcagag cgcagatacc 2280
aaatactgtt cttctagtgt agccgtagtt agcccaccac ttcaagaact ctgtagcacc 2340
gcctacatac ctcgctctgc taatcctgtt accagtggct gctgccagtg gcgataagtc 2400
gtgtcttacc gggttggact caagacgata gttaccggat aaggcgcagc ggtcgggctg 2460
aacggggggt tcgtgcacac agcccagctt ggagcgaacg acctacaccg aactgagata 2520
cctacagcgt gagctatgag aaagcgccac gcttcccgaa gggagaaagg cggacaggta 2580
tccggtaagc ggcagggtcg gaacaggaga gcgcacgagg gagcttccag ggggaaacgc 2640
ctggtatctt tatagtcctg tcgggtttcg ccacctctga cttgagcgtc gatttttgtg 2700
atgctcgtca ggggggcgga gcctatggaa aaacgccagc aacgcggcct ttttacggtt 2760
cctggccttt tgctggcctt ttgctcacat gttctttcct gcgttatccc ctgattctgt 2820
ggataaccgt attaccgcct ttgagtgagc tgataccgct cgccgcagcc gaacgaccga 2880

gcgcagcgag tcagtgagc 2899
```

<210> 44
<211> 39
<212> DNA

<213> Artificial Sequence

<220>
<223> Synthetic Nucleotide

<220>
<221> misc_feature
<222> 15, 16, 17, 18, 19, 20, 21, 22, 23, 24, 25
<223> n = A,T,C or G

<400> 44
aacggtctcc ttttnnnnnn nnnnncccca gagaccgtt 39

<210> 45
<211> 11
<212> DNA
<213> Artificial Sequence

<220>
<223> oligonucleotide

<400> 45
ggtctccttt t 11

<210> 46
<211> 11
<212> DNA
<213> Artificial Sequence

<220>
<223> Oligonucleotide

<400> 46
ccccagagac c 11

<210> 47
<211> 1556
<212> DNA
<213> Artificial Sequence

<220>
<223> vector

<400> 47

```
atgatcgagc agctgctgga atactggtac gtggttgtgc ctgttctgta tattatcaaa 60
cagctgctgg cgtacactaa aacgcgtgtc ctgatgaaga aactgggcgc agcgccggtg 120
actaacaaac tgtacgataa cgctttcggc atcgtaaatg gttggaaagc cctgcagttt 180
aagaaagagg gtcgtgcgca agaatataac gactataaat tcgatcattc taagaacccg 240
agcgtgggta cttatgtgtc tatcctgttc ggtactcgca tcgtggtaac taaagaccca 300
gaaaacatca aagcaatcct ggcgacgcaa ttcggcgact tctctctggg taaacgtcac 360
acgctgttca aacctctgct gggcgatggc attttcaccc tggatggtga aggttggaaa 420
cattcccgtg cgatgctgcg tccgcagttt gcgcgtgaac aggttgcgca cgttacgtct 480
ctggagccgc acttccagct gctgaagaaa catatcctga aacacaaagg cgagtatttc 540
gatatccagg agctgttctt ccgtttcacc gtagattccg ctaccgaatt tctgttcggt 600
gaatctgttc atagcctgaa agatgaaagc atcggcatca accaggatga catcgacttc 660
gctggtcgca aggatttcgc agaatccttc aataaagctc aggaatatct ggcgatccgt 720
actctggtgc aaactttcta ttggctggtt aacaataaag agtttcgcga ctgtaccaaa 780
tccgttcata aattcactaa ctactacgtt cagaaagctc tggatgcatc cccggaagaa 840
```

```
ctggaaaagc agtccggtta cgttttcctg tacgaactgg tgaaacagac tcgtgacccg 900
aacgtcctgc gtgaccagtc tctgaacatc ctgctggccg gccgtgacac taccgctggc 960
ctgctgtcct tcgcggtctt cgagctggcc cgtcatccgg aaatctgggc caaactgcgt 1020
gaagaaatcg aacagcaatt cggcctgggt gaggactccc gtgttgaaga aatcactttc 1080
gaatctctga aacgttgcga atatctgaaa gcattcctga acgaaacgct gcgtatctac 1140
ccgtccgttc cgcgcaactt ccgcattgct accaagaaca cgaccctgcc gcgtggcggt 1200
ggcagcgacg gcacctctcc gatcctgatt caaaagggtg aagcagtatc ctacggtatt 1260
aactccaccc acctggaccg gtatactacg gtccggacgc ggcagaattt cgtccagagc 1320
gctggtttga accgtctacc aagaagctgg gttgggctta tctgccgttc aacggcggcc 1380
ctcgtatctg tctgggtcag cagtttgccc tgaccgaggc aggctacgtt ctggttcgcc 1440
tggtccaaga attttctcac gtacgtagcg acccggacga agtttacccg ccgaagcgcc 1500
tgaccaacct gactatgtgc ctgcaagatg gcgctatcgt caaatttgat taataa     1556
```

<210> 48

<211> 1557

<212> DNA

<213> Artificial Sequence

<220>

<223> vector

<400> 48

```
atgatcgaac aactgctgga atactggtac gtggttgtgc ctgttctgta tattatcaaa 60
cagctgctgg cgtacactaa aacgcgtgtc ctgatgaaga aactgggcgc agcgccggtg 120
actaacaaac tgtacgataa cgctttcggc atcgtaaatg gttggaaagc cctgcagttt 180
aagaaagagg gtcgtgcgca agaatataac gactataaat tcgatcattc taagaacccg 240
agcgtgggta cttatgtgtc tatcctgttc ggtactcgca tcgtggtaac taaagaccca 300
gaaaacatca aagcaatcct ggcgacgcaa ttcggcgact tctctctggg taaacgtcac 360
acgctgttca aacctctgct gggcgatggc attttcaccc tggatggtga aggttggaaa 420
cattcccgtg cgatgctgcg tccgcagttt gcgcgtgaac aggttgcgca cgttacgtct 480
ctggagccgc acttccagct gctgaagaaa catatcctga aacacaaagg cgagtatttc 540
gatatccagg agctgttctt ccgtttcacc gtagattccg ctaccgaatt tctgttcggt 600
gaatctgttc atagcctgaa agatgaaagc atcggcatca accaggatga catcgacttc 660
gctggtcgca aggatttcgc agaatccttc aataaagctc aggaatatct ggcgatccgt 720
actctggtgc aaactttcta ttggctggtt aacaataaag agtttcgcga ctgtaccaaa 780
tccgttcata aattcactaa ctactacgtt cagaaagctc tggatgcatc cccggaagaa 840
ctggaaaagc agtccggtta cgttttcctg tacgaactgg tgaaacagac tcgtgacccg 900
aacgtcctgc gtgaccagtc tctgaacatc ctgctggccg gccgtgacac taccgctggc 960
ctgctgtcct tcgcggtctt cgagctggcc cgtcatccgg aaatctgggc caaactgcgt 1020
gaagaaatcg aacagcaatt cggcctgggt gaggactccc gtgttgaaga aatcactttc 1080
gaatctctga aacgttgcga atatctgaaa gcattcctga acgaaacgct gcgtatctac 1140
ccgtccgttc cgcgcaactt ccgcattgct accaagaaca cgaccctgcc gcgtggcggt 1200
ggcagcgacg gcacctctcc gatcctgatt caaaagggtg aagcagtatc ctacggtatt 1260
aactccaccc acctggaccc ggtatactac ggtccggacg cggcagaatt tcgtccagag 1320
cgctggtttg aaccgtctac caagaagctg ggttgggctt atctgccgtt caacggcggc 1380
cctcgtatct gtctgggtca gcagtttgcc ctgaccgagg caggctacgt tctggttcgc 1440
ctggtccaag aattttctca cgtacgtagc gacccggacg aagtttaccc gccgaagcgc 1500
ctgaccaacc tgactatgtg cctgcaagat ggcgctatcg tcaaatttga ttaataa    1557
```

<210> 49

<211> 1574

<212> DNA

<213> Artificial Sequence

<220>

<223> Polynucleotide

<400> 49

```
tgctggggaa aagtaaacac acacaggcgc actcgagaac agatgagttc tttggacgag 60
gatgaagagg acttcgaaat gctggacacg gagaacctcc agtttatggg gaagaagatg 120
tttggcaaac aggccggcga agacgagagt gatgattttg ctatagggg tagcaccccg 180
accaataaac tgaaatttta tccatatgcg aacaacaaat tgacaagagc tacggggacc 240
ttgaacctgt cattaagtaa tgcagctttg tcagaggcta actccaaatt tcttgggaaa 300
```

```
attgaagaag aggaagaaga ggaggaagaa ggcaaggatg aggaaagcgt ggatgctcgt 360
attaaaaggt ggtctccgtt ccatgaaaat gaaagtgtta ctactcctat tgcaaaaaga 420
gctgcggaaa aaacgaacag tcctattgct ctcaaacaat ggaaccagcg atggtttccg 480
aaaaatgatg ctcgcactga aaatacatcc tcatcctctt catatagcgt cgctaaacct 540
aaccaatcag cctttacgtc ttcgggcctc gtatctaaaa tgtctatgga cacttcgtta 600
taccctgcga aattgaggat accagaaaca ccagtgaaaa aatcacccctt agtggaggga 660
agagaccata agcatgtcca cctttcgagt tcgaaaaatg catcgtcttc tctaagtgtt 720
tccccttttaa attttgttga agacaataat ttacaagaag accttttatt ttcagattct 780
ccgtcttcga aagctttacc ttccatccat gtaccaacca tagacgcatc cccactgagc 840
gaggcaaaat atcatgcaca tgatcgtcac aataaccaga caaacatcct gtctcccact 900
aatagcttgg ttaccaacag ctctccacaa acattgcatt ctaacaagtt caaaaaaatc 960
aaaagagcaa ggaattcggt tattttgaaa aatagagagc taacaaacag tttacaacaa 1020
ttcaaagatg atttatacgg cacggacgag aatttcccac ctccaatcat aatatcaagt 1080
catcattcaa ctagaaagaa ccctcaacct tatcaatttc gtggacgcta tgacaatgac 1140
gctgacgaag agatctccac tccaacaaga cgaaaatcta ttattggggc agcatctcaa 1200
acacatagag aaagcagacc attgtcactc tcctctgcca tcgtgacaaa cacaacaagt 1260
gcagagacgc attccatatc ttccaccgat tcttcgccgt taaattccaa aaggcgtcta 1320
atctcttcaa ataagttatc agcaaatcca gattcccatc ttttcgaaaa atttacgaat 1380
gtgcattcca ttggtaaagg ccagttttcc acggtctacc aggttacgtt tgcccaaaca 1440
aacaaaaagt atgcaatcaa agccattaaa ccaaacaaat ataattcctt gaaacgcata 1500
ttactggaaa ttaaaatact aaacgaggta acaaaccaaa ttaccatgga tcaagaaggg 1560
aaggaataca tcat                                                   1574
```

<210> 50
<211> 17
<212> DNA
<213> Artificial Sequence

<220>
<223> Oligonucleotide

<400> 50
gaagaagagg aagaaga 17

<210> 51
<211> 17
<212> DNA
<213> Artificial Sequence

<220>
<223> Oligonucleotide

<400> 51
gaagaagagg aggaaga 17

<210> 52
<211> 16
<212> DNA
<213> Artificial Sequence

<220>
<223> Oligonucleotide

<400> 52
gaagaggagg aagaag 16

<210> 53
<211> 369
<212> PRT
<213> Artificial Sequence

<220>

<223> Proteinase K

<400> 53

```
Ala Pro Ala Val Glu Gln Arg Ser Glu Ala Ala Pro Leu Ile Glu Ala
1               5                   10                  15
Arg Gly Glu Met Val Ala Asn Lys Tyr Ile Val Lys Phe Lys Glu Gly
            20                  25                  30
Ser Ala Leu Ser Ala Leu Asp Ala Ala Met Glu Lys Ile Ser Gly Lys
        35                  40                  45
Pro Asp His Val Tyr Lys Asn Val Phe Ser Gly Phe Ala Ala Thr Leu
    50                  55                  60
Asp Glu Asn Met Val Arg Val Leu Arg Ala His Pro Asp Val Glu Tyr
65                  70                  75                  80
Ile Glu Gln Asp Ala Val Val Thr Ile Asn Ala Ala Gln Thr Asn Ala
                85                  90                  95
Pro Trp Gly Leu Ala Arg Ile Ser Ser Thr Ser Pro Gly Thr Ser Thr
            100                 105                 110
Tyr Tyr Tyr Asp Glu Ser Ala Gly Gln Gly Ser Cys Val Tyr Val Ile
        115                 120                 125
Asp Thr Gly Ile Glu Ala Ser His Pro Glu Phe Glu Gly Arg Ala Gln
    130                 135                 140
Met Val Lys Thr Tyr Tyr Tyr Ser Ser Arg Asp Gly Asn Gly His Gly
145                 150                 155                 160
Thr His Cys Ala Gly Thr Val Gly Ser Arg Thr Tyr Gly Val Ala Lys
                165                 170                 175
Lys Thr Gln Leu Phe Gly Val Lys Val Leu Asp Asp Asn Gly Ser Gly
            180                 185                 190
Gln Tyr Ser Thr Ile Ile Ala Gly Met Asp Phe Val Ala Ser Asp Lys
        195                 200                 205
Asn Asn Arg Asn Cys Pro Lys Gly Val Val Ala Ser Leu Ser Leu Gly
    210                 215                 220
Gly Gly Tyr Ser Ser Ser Val Asn Ser Ala Ala Ala Arg Leu Gln Ser
225                 230                 235                 240
Ser Gly Val Met Val Ala Val Ala Ala Gly Asn Asn Asn Ala Asp Ala
                245                 250                 255
Arg Asn Tyr Ser Pro Ala Ser Glu Pro Ser Val Cys Thr Val Gly Ala
            260                 265                 270
Ser Asp Arg Tyr Asp Arg Arg Ser Ser Phe Ser Asn Tyr Gly Ser Val
        275                 280                 285
Leu Asp Ile Phe Gly Pro Gly Thr Ser Ile Leu Ser Thr Trp Ile Gly
    290                 295                 300
Gly Ser Thr Arg Ser Ile Ser Gly Thr Ser Met Ala Thr Pro His Val
305                 310                 315                 320
Ala Gly Leu Ala Ala Tyr Leu Met Thr Leu Gly Lys Thr Thr Ala Ala
                325                 330                 335
Ser Ala Cys Arg Tyr Ile Ala Asp Thr Ala Asn Lys Gly Asp Leu Ser
            340                 345                 350
Asn Ile Pro Phe Gly Thr Val Asn Leu Leu Ala Tyr Asn Asn Tyr Gln
        355                 360                 365
Ala
```

<210> 54
<211> 1107
<212> DNA
<213> Artificial Sequence

<220>
<223> Coding for Proteinase K

<400> 54

```
gctccggcag ttgaacagcg ttctgaagcg gcgccgctga tcgaggcgcg tggtgagatg 60
gttgctaaca aatacattgt gaaattcaag gagggctctg ctctgtctgc actggacgcc 120
```

```
gcaatggaaa agatcagcgg caagccggac cacgtgtaca aaaacgtgtt ttccggtttc 180
gccgctactc tggatgaaaa tatggttcgt gttctgcgtg cgcacccgga tgtagaatat 240
atcgaacagg atgcagtcgt aaccatcaat gctgctcaga ccaatgcgcc gtggggtctg 300
gcacgtattt cttctacctc cccgggtacc agcacctatt attacgacga aagcgccggc 360
cagggctctt gcgtttacgt tattgacacc ggcatcgaag cttctcatcc agaattcgag 420
ggtcgtgcgc agatggtgaa aacctactac tactcctctc gcgatggcaa cggtcatggc 480
acgcattgcg caggcacggt aggctcccgt acgtacggtg ttgcaaaaaa aacccagctg 540
ttcggcgtta aagtgctgga cgataacggt tctggtcagt actccaccat catcgcaggt 600
atggacttcg tagcgtccga caaaaacaac cgtaactgtc cgaaaggcgt cgttgcgagc 660
ctgagcctgg gtggtggcta ttcttcctcc gtgaactctg cggcggcccg cctgcagagc 720
tctggtgtaa tggttgcagt agccgcaggc aacaacaacg ctgatgcacg taactactct 780
ccggcttccg aaccatctgt gtgtaccgtg ggtgcatccg atcgttacga ccgccgtagc 840
tctttttcta actacggctc cgtgctggac attttcggcc cgggtacttc tattctgtct 900
acttggatcg gcggttctac ccgcagcatc agcggtactt ctatggcgac cccgcacgtg 960
gcaggcctgg cggcttatct gatgactctg ggtaaaacca ccgcggcgag cgcgtgtcgt 1020
tacatcgcgg atactgctaa caaaggtgac ctgtctaaca tccctttcgg taccgtcaac 1080
ctgctggcat acaacaacta ccaagcg                                     1107
```

<210> 55
<211> 12
<212> DNA
<213> Artificial Sequence

<220>
<223> Nucleotide

<400> 55
agcggcgccg ct 12

<210> 56
<211> 12
<212> DNA
<213> Artificial Sequence

<220>
<223> Nucleotide

<400> 56
ccgtacgtac gg 12

<210> 57
<211> 1107
<212> DNA
<213> Artificial Sequence

<220>
<223> Coding for Proteinase K

<400> 57

```
gctccagcgg ttgaacagcg cagcgaggcc gcaccgctga tcgaagcccg tggtgaaatg 60
gtggcaaaca aatacattgt caagttcaaa gaaggttccg cgctgagcgc tctggatgct 120
gcaatggaaa aaatctccgg taaaccggac cacgtatata aaaatgtctt ttctggcttc 180
gcggctactc tggatgagaa catggttcgt gtgctgcgtg cgcatccgga tgttgaatac 240
attgaacagg acgcagttgt aacgattaac gctgcccaaa ctaacgcgcc atggggcctg 300
gcccgcatta gctccacctc cccaggtact tccacttatt actacgacga atccgcaggt 360
cagggttcct gcgtatatgt tatcgacacc ggtatcgaag cgtcccaccc ggaatttgag 420
ggtcgtgcgc aaatggtgaa gacctactac tactcttccc gtgacggtaa cggtcacggt 480
acccactgtg cgggtactgt aggtagccgt acctatggtg ttgccaaaaa aacccagctg 540
tttggcgtta aagtgctgga tgataatggc tccggtcagt actccaccat catcgctggc 600
atggactttg tcgcaagcga caaaaacaac cgcaactgcc cgaaaggtgt tgtggcttct 660
ctgtccctgg gtggtggcta tagctcctct gtgaactctg cggcagcgcg tctgcaatcc 720
tccggcgtga tggtcgcggt tgccgcaggt aacaacaacg cggatgcgcg caactactct 780
cctgcatccg aaccgtccgt ttgtactgtt ggtgcgtctg accgttacga ccgtcgttct 840

tctttctcca actacggttc tgtactggac atcttcggtc ctggcacctc catcctgtct 900
acgtggattg gcggtagcac ccgtagcatc tctggtacta gcatggctac cccgcacgta 960
gcaggcctgg cggcatatct gatgacgctg ggcaagacta ccgcggctag cgcttgccgt 1020
tacatcgcgg ataccgcgaa caaaggcgac ctgtctaaca tcccgttcgg caccgtgaac 1080
ctgctggcat acaacaacta tcaggcg                                    1107
```

<210> 58
<211> 18
<212> DNA
<213> Artificial Sequence

<220>
<223> RNA Stem Loop Structure

<400> 58
tttgtcgcaa gcgacaaa 18

<210> 59
<211> 1107
<212> DNA
<213> Artificial Sequence

<220>
<223> Polynucleotide

<400> 59

```
gcgccggcag tagaacagcg ttctgaagca gcaccgctga tcgaagctcg cggcgaaatg 60
gtagcgaaca aatatattgt aaaattcaaa gaaggctctg cactgtctgc gctggatgct 120
gcgatggaga aaatctctgg taaaccggat cacgtataca agaacgtttt ttctggcttc 180
gctgcaacgc tggatgaaaa catggtgcgt gtactgcgtg cgcacccgga tgtggagtac 240
atcgaacagg acgcagttgt gaccatcaac gcggcgcaga ctaacgctcc gtggggcctg 300
gctcgcatct cttccacctc cccgggcact tccacctact actatgatga gtctgctggt 360
cagggtagct gtgtttacgt tatcgatacg ggcatcgaag cttcccaccc ggaattcgaa 420
ggccgtgcgc agatggtgaa aacctattac tattcttctc gtgatggcaa tggccacggc 480
acccactgcg ccggcaccgt tggttctcgc acctacggtg tggcaaagaa aacccagctg 540
ttcggtgtga aggttctgga cgataacggt tccggccagt actccactat catcgccggc 600
atggacttcg ttgcctccga caaaaataac cgtaattgcc cgaaaggtgt tgttgcttcc 660
ctgagcctgg gtggcggtta ttccagctct gtgaactctg cagccgctcg cctgcagtcc 720
tctggcgtta tggtagccgt cgcggctggt aacaacaacg cggatgcacg caattactcc 780
ccggcctccg aaccttctgt ctgtaccgtt ggcgctagcg accgttatga tcgtcgctct 840
agcttctcta actatggttc cgtactggat atcttcggcc cgggtacctc tattctgtcc 900
acttggattg gcggctctac ccgctctatc tccggtacct ctatggccac gccgcatgtc 960
gcaggcctgg cagcttacct gatgactctg ggtaaaacta ccgcggcctc cgcttgccgc 1020
tacattgccg acactgctaa caaaggcgac ctgagcaaca ttccattcgg caccgttaac 1080
ctgctggcct acaacaatta ccaggca                                    1107
```

<210> 60

<211> 419
<212> PRT
<213> Artificial Sequence

<220>
<223> Polypeptide

<400> 60

```
Met Ala Gln His Asp Glu Ala Gln Gln Asn Ala Phe Tyr Gln Val Leu
 1               5                  10                  15
Asn Met Pro Asn Leu Asn Ala Asp Gln Arg Asn Gly Phe Ile Gln Ser
            20                  25                  30
Leu Lys Asp Asp Pro Ser Gln Ser Ala Asn Val Leu Gly Glu Ala Gln
        35                  40                  45
Lys Leu Asn Asp Ser Gln Ala Pro Lys Ala Asp Ala Gln Asn Asn Phe
    50                  55                  60

Asn Lys Asp Gln Gln Ser Ala Phe Tyr Glu Ile Leu Asn Met Pro Asn
65                  70                  75                  80
Leu Asn Glu Ala Gln Arg Asn Gly Phe Ile Gln Ser Leu Lys Asp Asp
                85                  90                  95
Pro Ser Gln Ser Thr Asn Val Leu Gly Glu Ala Lys Lys Leu Asn Glu
            100                 105                 110
Ser Gln Ala Pro Lys Asp Asn Asn Phe Asn Lys Glu Gln Gln Asn Ala
        115                 120                 125
Phe Tyr Glu Ile Leu Asn Met Pro Asn Leu Asn Glu Glu Gln Arg Asn
    130                 135                 140
Gly Phe Ile Gln Ser Leu Lys Asp Asp Pro Ser Gln Ser Ala Asn Leu
145                 150                 155                 160
Leu Ser Glu Ala Lys Lys Leu Asn Glu Ser Gln Ala Pro Lys Asp Asn
                165                 170                 175
Lys Phe Asn Lys Glu Gln Gln Asn Ala Phe Tyr Glu Ile Leu His Leu
            180                 185                 190
Pro Asn Leu Asn Glu Glu Gln Arg Asn Gly Phe Ile Gln Ser Leu Lys
        195                 200                 205
Asp Asp Pro Ser Gln Ser Ala Asn Leu Leu Ala Glu Ala Lys Lys Leu
    210                 215                 220
Asn Asp Ala Gln Ala Pro Lys Asp Asn Lys Phe Asn Lys Glu Gln Gln
225                 230                 235                 240
Asn Ala Phe Tyr Glu Ile Leu His Leu Pro Asn Leu Thr Glu Glu Gln
                245                 250                 255
Arg Asn Gly Phe Ile Gln Ser Leu Lys Asp Asp Pro Ser Val Ser Lys
            260                 265                 270
Glu Ile Leu Ala Glu Ala Lys Lys Leu Asn Asp Ala Gln Ala Pro Lys
        275                 280                 285
Glu Glu Asp Asn Asn Lys Pro Gly Lys Glu Asp Asn Asn Lys Pro Gly
    290                 295                 300
Lys Glu Asp Asn Asn Lys Pro Gly Lys Glu Asp Asn Asn Lys Pro Gly
305                 310                 315                 320
Lys Glu Asp Asn Asn Lys Pro Gly Lys Glu Asp Asn Asn Lys Pro Gly
                325                 330                 335
Lys Glu Asp Gly Asn Lys Pro Gly Lys Glu Asp Asn Lys Lys Pro Gly
            340                 345                 350
Lys Glu Asp Gly Asn Lys Pro Gly Lys Glu Asp Asn Lys Lys Pro Gly
        355                 360                 365
Lys Glu Asp Gly Asn Lys Pro Gly Lys Glu Asp Gly Asn Lys Pro Gly
    370                 375                 380
Lys Glu Asp Gly Asn Gly Val His Val Val Lys Pro Gly Asp Thr Val
385                 390                 395                 400
Asn Asp Ile Ala Lys Ala Asn Gly Thr Thr Ala Asp Lys Ile Ala Ala
                405                 410                 415
Asp Asn Lys
```

<210> 61
<211> 175
<212> PRT
<213> Artificial Sequence

<220>
<223> Polypeptide

<400> 61

```
Met Ala Gln His Asp Glu Ala Gln Gln Asn Ala Phe Tyr Gln Val Leu
 1              5                   10                  15
Asn Met Pro Asn Leu Asn Ala Asp Gln Arg Asn Gly Phe Ile Gln Ser
            20                  25                  30
Leu Lys Asp Asp Pro Ser Gln Ser Ala Asn Val Leu Gly Glu Ala Gln
        35                  40                  45

Lys Leu Asn Asp Ser Gln Ala Pro Lys Ala Asp Ala Gln Asn Asn Phe
    50                  55                  60
Asn Lys Asp Gln Gln Ser Ala Phe Tyr Glu Ile Leu Asn Met Pro Asn
65                  70                  75                  80
Leu Asn Glu Ala Gln Arg Asn Gly Phe Ile Gln Ser Leu Lys Asp Asp
                85                  90                  95
Pro Ser Gln Ser Thr Asn Val Leu Gly Glu Ala Lys Lys Leu Asn Glu
            100                 105                 110
Ser Gln Ala Pro Lys Ala Asp Asn Asn Phe Asn Lys Glu Gln Gln Asn
        115                 120                 125
Ala Phe Tyr Glu Ile Leu Asn Met Pro Asn Leu Asn Glu Glu Gln Arg
    130                 135                 140
Asn Gly Phe Ile Gln Ser Leu Lys Asp Asp Pro Ser Gln Ser Ala Asn
145                 150                 155                 160
Leu Leu Ser Glu Ala Lys Lys Leu Asn Glu Ser Gln Ala Pro Lys
                165                 170                 175
```

<210> 62
<211> 136
<212> PRT
<213> Artificial Sequence

<220>
<223> Polypeptide

<400> 62

```
Ala Asp Asn Lys Phe Asn Lys Glu Gln Gln Asn Ala Phe Tyr Glu Ile
 1              5                   10                  15
Leu His Leu Pro Asn Leu Asn Glu Glu Gln Arg Asn Gly Phe Ile Gln
            20                  25                  30
Ser Leu Lys Asp Asp Pro Ser Gln Ser Ala Asn Leu Leu Ala Glu Ala
        35                  40                  45
Lys Lys Leu Asn Asp Ala Gln Ala Pro Lys Asp Asn Lys Phe Asn Lys
    50                  55                  60
Glu Gln Gln Asn Ala Phe Tyr Glu Ile Leu His Leu Pro Asn Leu Thr
65                  70                  75                  80
Glu Glu Gln Arg Asn Gly Phe Ile Gln Ser Leu Lys Asp Asp Pro Ser
                85                  90                  95
Val Ser Lys Glu Ile Leu Ala Glu Ala Lys Lys Leu Asn Asp Ala Gln
            100                 105                 110
Ala Pro Lys Glu Glu Asp Asn Asn Lys Pro Gly Lys Glu Asp Asn Asn
        115                 120                 125
Lys Pro Gly Lys Glu Asp Asn Asn
    130                 135
```

<210> 63
<211> 110
<212> PRT
<213> Artificial Sequence

<220>
<223> Oligonucleotide

<400> 63

```
Lys Pro Gly Lys Glu Asp Asn Asn Lys Pro Gly Lys Glu Asp Asn Asn
 1               5                  10                  15
Lys Pro Gly Lys Glu Asp Asn Asn Lys Pro Gly Lys Glu Asp Gly Asn
            20                  25                  30
Lys Pro Gly Lys Glu Asp Asn Lys Lys Pro Gly Lys Glu Asp Gly Asn
        35                  40                  45
Lys Pro Gly Lys Glu Asp Asn Lys Lys Pro Gly Lys Glu Asp Gly Asn


    50                  55                  60
Lys Pro Gly Lys Glu Asp Gly Asn Lys Pro Gly Lys Glu Asp Gly Asn
65                  70                  75                  80
Gly Val His Val Val Lys Pro Gly Asp Thr Val Asn Asp Ile Ala Lys
                85                  90                  95
Ala Asn Gly Thr Thr Ala Asp Lys Ile Ala Ala Asp Asn Lys
            100                 105                 110
```

<210> 64
<211> 548
<212> DNA
<213> Artificial Sequence

<220>
<223> Vector

<400> 64

```
ggtaccccgg taacgcgtat ggcgcaacat gacgaagctc agcagaacgc tttttaccag 60
gtactgaaca tgccgaacct gaacgcggat cagcgcaacg gtttcatcca gagcctgaaa 120
gacgaccctt ctcagtccgc aaacgttctg ggcgaggctc agaaactgaa cgacagccag 180
gccccaaaag cagatgctca gcaaaataac ttcaacaagg accagcagag cgcattctac 240
gaaatcctga acatgccaaa tctgaacgaa gctcaacgca acggcttcat tcagtctctg 300
aaagacgatc cgtcccagtc cactaacgtt ctgggtgaag ctaagaagct gaacgaatcc 360
caggcaccaa aagcagacaa caacttcaac aaagagcagc agaacgcttt ctatgaaatc 420
ttgaacatgc ctaacctgaa tgaagaacag cgtaacggct tcatccagtc tctgaaggac 480
gaccctagcc agtctgctaa cctgctgtcc gaagcaaaaa aactgaacga gtcccaggct 540
ccaaaagc                                                          548
```

<210> 65
<211> 407
<212> DNA
<213> Artificial Sequence

<220>
<223> Synthetic Nucleotide

<400> 65

```
ggataacaaa ttcaacaagg agcagcagaa cgcattctac gaaatcctgc acctgccgaa 60
cctgaacgaa gaacagcgta acggtttcat ccaatccctg aaagacgatc cttcccagtc 120
cgcaaatctg ctggcagaag caaagaaact gaacgacgca caggcaccga aggctgacaa 180
caagttcaac aaagagcagc agaatgcctt ctacgagatt ctgcatctgc caaacctgac 240
tgaggagcag cgcaacggtt tcattcagtc cctgaaggac gacccaagcg tcagcaagga 300
aatcctggct gaggcgaaaa aactgaacga tgcacaggct ccgaaggaag aagacaacaa 360
taaacctggt aaagaagata ataataagcc tggcaaggaa gataaca               407
```

<210> 66

<211> 363
<212> DNA
<213> Artificial Sequence

<220>
<223> Synthetic Nucleotide

<400> 66

```
acaagccggg caaggaggac aacaataaac cgggcaaaga ggataataac aagcctggta 60
aggaagacaa caacaaacca ggcaaagaag atggcaacaa gccgggtaag gaggataata 120
aaaaaccagg caaggaagac ggcaacaaac ctggcaagga ggataacaaa aagccaggca 180
aggaggatgg taataaaccg ggcaaagaag acggcaacaa gcctggtaaa gaagacggta 240
acggtgtaca cgtcgttaaa cctggtgaca ccgtgaacga catcgctaag gctaatggca 300
ccacggcaga caagattgca gcggacaata aattagctga taaataagga tccgcggaag 360
ctt                                                               363
```

<210> 67
<211> 29
<212> DNA
<213> Artificial Sequence

<220>
<223> Oligonucleotide

<400> 67
ggggacaagt ttgtacaaaa aagcaggct 29

<210> 68
<211> 29
<212> DNA
<213> Artificial Sequence

<220>
<223> Oligonucleotide

<400> 68
acccagcttt cttgtacaaa gtggtcccc 29

<210> 69
<211> 615
<212> DNA
<213> Artificial Sequence

<220>
<223> Synthetic Polynucleotide

<400> 69

```
ggggacaagt ttgtacaaaa aagcaggctg gtaccccggt aacgcgtatg gcgcaacatg 60
acgaagctca gcagaacgct ttttaccagg tactgaacat gccgaacctg aacgcggatc 120
agcgcaacgg tttcatccag agcctgaaag acgacccttc tcagtccgca aacgttctgg 180
gcgaggctca gaaactgaac gacagccagg ccccaaaagc agatgctcag caaaataact 240
tcaacaagga ccagcagagc gcattctacg aaatcctgaa catgccaaat ctgaacgaag 300
ctcaacgcaa cggcttcatt cagtctctga aagacgatcc gtcccagtcc actaacgttc 360
tgggtgaagc taagaagctg aacgaatccc aggcaccaaa agcagacaac aacttcaaca 420
aagagcagca gaacgctttc tatgaaatct tgaacatgcc taacctgaat gaagaacagc 480
gtaacggctt catccagtct ctgaaggacg accctagcca gtctgctaac ctgctgtccg 540
aagcaaaaaa actgaacgag tcccaggctc caaaagcgga gagaccaccc agctttcttg 600
tacaaagtgg tcccc                                                  615
```

<210> 70
<211> 483
<212> DNA
<213> Artificial Sequence

<220>
<223> Synthetic Polynucleotide

<400> 70

ggggacaagt ttgtacaaaa aagcaggctg gtctcagcgg ataacaaatt caacaaggag 60
cagcagaacg cattctacga aatcctgcac ctgccgaacc tgaacgaaga acagcgtaac 120
ggtttcatcc aatccctgaa agacgatcct tcccagtccg caaatctgct ggcagaagca 180
aagaaactga acgacgcaca ggcaccgaag gctgacaaca agttcaacaa agagcagcag 240
aatgccttct acgagattct gcatctgcca aacctgactg aggagcagcg caacggtttc 300
attcagtccc tgaaggacga cccaagcgtc agcaaggaaa tcctggctga ggcgaaaaaa 360
ctgaacgatg cacaggctcc gaaggaagaa gacaacaata aacctggtaa agaagataat 420
aataagcctg gcaaggaaga taacaacaga gaccacccag ctttcttgta caaagtggtc 480
ccc 483

<210> 71
<211> 430
<212> DNA
<213> Artificial Sequence

<220>
<223> Synthetic Polynucleotide

<400> 71

ggggacaagt ttgtacaaaa aagcaggctg gtctcacaac aagccgggca aggaggacaa 60
caataaaccg ggcaaagagg ataataacaa gcctggtaag gaagacaaca acaaaccagg 120
caaagaagat ggcaacaagc cgggtaagga ggataataaa aaaccaggca aggaagacgg 180
caacaaacct ggcaaggagg ataacaaaaa gccaggcaag gaggatggta ataaaccggg 240
caaagaagac ggcaacaagc ctggtaaaga agacggtaac ggtgtacacg tcgttaaacc 300
tggtgacacc gtgaacgaca tcgctaaggc taatggcacc acggcagaca agattgcagc 360
ggacaataaa ttagctgata aataaggatc cgcggaagct tacccagctt tcttgtacaa 420
agtggtcccc 430

<210> 72
<211> 43
<212> DNA
<213> Artificial Sequence

<220>
<223> Oligonucleotide

<400> 72
ggggacaagt ttgtacaaaa aagcaggctg gtctcacaac aag 43

<210> 73
<211> 49
<212> DNA
<213> Artificial Sequence

<220>
<223> Oligonucleotide

<400> 73
gacaacaata aaccgggcaa agaggataat aacaagcctg gtaaggaag 49

<210> 74
<211> 58
<212> DNA
<213> Artificial Sequence

<220>
<223> Oligonucleotide

<400> 74
acaacaacaa accaggcaaa gaagatggca acaagccggg taaggaggat aataaaaa 58

<210> 75
<211> 50
<212> DNA
<213> Artificial Sequence

<220>
<223> Oligonucleotide

<400> 75
accaggcaag gaagacggca acaaacctgg caaggaggat aacaaaaagc 50

<210> 76
<211> 54
<212> DNA
<213> Artificial Sequence

<220>
<223> Oligonucleotide

<400> 76
caggcaagga ggatggtaat aaaccgggca aagaagacgg caacaagcct ggta 54

<210> 77
<211> 49
<212> DNA
<213> Artificial Sequence

<220>
<223> oligonucleotide

<400> 77
aagaagacgg taacggtgta cacgtcgtta aacctggtga caccgtgaa 49

<210> 78
<211> 60
<212> DNA
<213> Artificial Sequence

<220>
<223> Oligonucleotide

<400> 78
cgacatcgct aaggctaatg gcaccacggc agacaagatt gcagcggaca ataaattagc 60

<210> 79
<211> 53
<212> DNA

<213> Artificial Sequence

<220>
<223> Oligonucleotide

<400> 79
ataaataagg atccgcggaa gcttacccag ctttcttgta caaagtggtc ccc 53

<210> 80
<211> 60
<212> DNA
<213> Artificial Sequence

<220>
<223> Oligonucleotide

<400> 80
tttgcccggt ttattgttgt cctccttgcc cggcttgttg tgagaccagc ctgctttttt 60

<210>81
<211> 52
<212> DNA
<213> Artificial Sequence

<220>
<223> Oligonucleotide

<400> 81
cttctttgcc tggtttgttg ttgtcttcct taccaggctt gttattatcc tc 52

<210> 82
<211> 61
<212> DNA
<213> Artificial Sequence

<220>
<223> Oligonucleotide

<400> 82

```
ggtttgttgc cgtcttcctt gcctggtttt ttattatcct ccttacccgg cttgttgcca 60
t                                                                 61
```

<210> 83
<211> 41
<212> DNA
<213> Artificial Sequence

<220>
<223> Oligonucleotide

<400> 83
taccatcctc cttgcctggc tttttgttat cctccttgcc a 41

<210> 84
<211> 57
<212> DNA
<213> Artificial Sequence

<220>
<223> Oligonucleotide

<400> 84
gtacaccgtt accgtcttct ttaccaggct tgttgccgtc ttctttgccc ggtttat 57

<210> 85
<211> 55
<212> DNA
<213> Artificial Sequence

<220>
<223> Oligonucleotide

<400> 85
gtggtgccat tagccttagc gatgtcgttc acggtgtcac caggtttaac gacgt 55

<210> 86
<211> 60
<212> DNA
<213> Artificial Sequence

<220>
<223> Oligonucleotide

<400> 86
taagcttccg cggatcctta tttatcagct aatttattgt ccgctgcaat cttgtctgcc 60

<210> 87
<211> 28
<212> DNA
<213> Artificial Sequence

<220>
<223> Oligonucleotide

<400> 87
ggggaccact ttgtacaaga aagctggg 28

<210> 88
<211> 1306
<212> DNA
<213> Artificial Sequence

<220>
<223> Synthetic Polynucleotide

<400> 88

```
ccggtaacgc gtatggcgca acatgacgaa gctcagcaga acgcttttta ccaggtactg 60
aacatgccga acctgaacgc ggatcagcgc aacggtttca tccagagcct gaaagacgac 120
ccttctcagt ccgcaaacgt tctgggcgag gctcagaaac tgaacgacag ccaggcccca 180
aaagcagatg ctcagcaaaa taacttcaac aaggaccagc agagcgcatt ctacgaaatc 240
ctgaacatgc caaatctgaa cgaagctcaa cgcaacggct tcattcagtc tctgaaagac 300
gatccgtccc agtccactaa cgttctgggt gaagctaaga agctgaacga atcccaggca 360
ccaaaagcag acaacaactt caacaaagag cagcagaacg ctttctatga aatcttgaac 420
atgcctaacc tgaatgaaga acagcgtaac ggcttcatcc agtctctgaa ggacgaccct 480
agccagtctg ctaacctgct gtccgaagca aaaaaactga acgagtccca ggctccaaaa 540
gcggataaca aattcaacaa ggagcagcag aacgcattct acgaaatcct gcacctgccg 600
aacctgaacg aagaacagcg taacggtttc atccaatccc tgaaagacga tccttcccag 660
tccgcaaatc tgctggcaga agcaaagaaa ctgaacgacg cacaggcacc gaaggctgac 720
aacaagttca acaaagagca gcagaatgcc ttctacgaga ttctgcatct gccaaacctg 780
actgaggagc agcgcaacgg tttcattcag tccctgaagg acgacccaag cgtcagcaag 840
gaaatcctgg ctgaggcgaa aaaactgaac gatgcacagg ctccgaagga agaagacaac 900
aataaacctg gtaaagaaga taataataag cctggcaagg aagataacaa caagccgggc 960
aaggaggaca acaataaacc gggcaaagag gataataaca agcctggtaa ggaagacaac 1020
aacaaaccag gcaaagaaga tggcaacaag ccgggtaagg aggataataa aaaaccaggc 1080
aaggaagacg gcaacaaacc tggcaaggag gataacaaaa agccaggcaa ggaggatggt 1140
aataaaccgg gcaaagaaga cggcaacaag cctggtaaag aagacggtaa cggtgtacac 1200
gtcgttaaac ctggtgacac cgtgaacgac atcgctaagg ctaatggcac cacggcagac 1260
aagattgcag cggacaataa attagctgat aaataaggat ccgcgg                1306
```

<210> 89

<211> 369

<212> PRT

<213> Artificial Sequence

<220>

<223> T7 Endonuclease

<400> 89

```
Ala Pro Ala Val Glu Gln Arg Ser Glu Ala Ala Pro Leu Ile Glu Ala
1               5                   10                  15
Arg Gly Glu Met Val Ala Asn Lys Tyr Ile Val Lys Phe Lys Glu Gly
            20                  25                  30
Ser Ala Leu Ser Ala Leu Asp Ala Ala Met Glu Lys Ile Ser Gly Lys
        35                  40                  45
Pro Asp His Val Tyr Lys Asn Val Phe Ser Gly Phe Ala Ala Thr Leu
    50                  55                  60
Asp Glu Asn Met Val Arg Val Leu Arg Ala His Pro Asp Val Glu Tyr
65                  70                  75                  80
Ile Glu Gln Asp Ala Val Val Thr Ile Asn Ala Ala Gln Thr Asn Ala
                85                  90                  95
Pro Trp Gly Leu Ala Arg Ile Ser Ser Thr Ser Pro Gly Thr Ser Thr
            100                 105                 110
```

```
Tyr Tyr Tyr Asp Glu Ser Ala Gly Gln Gly Ser Cys Val Tyr Val Ile
        115                 120                 125
Asp Thr Gly Ile Glu Ala Ser His Pro Glu Phe Glu Gly Arg Ala Gln
    130                 135                 140
Met Val Lys Thr Tyr Tyr Tyr Ser Ser Arg Asp Gly Asn Gly His Gly
145                 150                 155                 160
Thr His Cys Ala Gly Thr Val Gly Ser Arg Thr Tyr Gly Val Ala Lys
                165                 170                 175
Lys Thr Gln Leu Phe Gly Val Lys Val Leu Asp Asp Asn Gly Ser Gly
            180                 185                 190
Gln Tyr Ser Thr Ile Ile Ala Gly Met Asp Phe Val Ala Ser Asp Lys
        195                 200                 205
Asn Asn Arg Asn Cys Pro Lys Gly Val Val Ala Ser Leu Ser Leu Gly
    210                 215                 220
Gly Gly Tyr Ser Ser Ser Val Asn Ser Ala Ala Ala Arg Leu Gln Ser
225                 230                 235                 240
Ser Gly Val Met Val Ala Val Ala Ala Gly Asn Asn Asn Ala Asp Ala
                245                 250                 255
Arg Asn Tyr Ser Pro Ala Ser Glu Pro Ser Val Cys Thr Val Gly Ala
            260                 265                 270
Ser Asp Arg Tyr Asp Arg Arg Ser Ser Phe Ser Asn Tyr Gly Ser Val
        275                 280                 285
Leu Asp Ile Phe Ala Pro Gly Thr Ser Ile Leu Ser Thr Trp Ile Gly
    290                 295                 300
Gly Ser Thr Arg Ser Ile Ser Gly Thr Ser Met Ala Thr Pro His Val
305                 310                 315                 320
Ala Gly Leu Ala Ala Tyr Leu Met Thr Leu Gly Lys Thr Thr Ala Ala
                325                 330                 335
Ser Ala Cys Arg Tyr Ile Ala Asp Thr Ala Asn Lys Gly Asp Leu Ser
            340                 345                 350
Asn Ile Pro Phe Gly Thr Val Asn Leu Leu Ala Tyr Asn Asn Tyr Gln
        355                 360                 365
Ala
```

<210> 90
<211> 1107
<212> DNA
<213> Artificial Sequence

<220>
<223> Synthetic Polynucleotde

<400> 90

```
gcaccggccg ttgaacagcg ttctgaagca gctcctctga ttgaggcacg tggtgaaatg 60
gtagcaaaca agtacatcgt gaagttcaag gagggttctg ctctgtctgc tctggatgct 120
gctatggaaa agatctctgg caagcctgat cacgtctata agaacgtgtt cagcggtttc 180
gcagcaactc tggacgagaa catggtccgt gtactgcgtg ctcatccaga cgttgaatac 240
atcgaacagg acgctgtggt tactatcaac gcggcacaga ctaacgcacc ttggggtctg 300
gcacgtattt cttctacttc cccgggtacg tctacttact actacgacga gtctgccggt 360
caaggttctt gcgtttacgt gatcgatacg ggcatcgagg cttctcatcc tgagtttgaa 420
ggccgtgcac aaatggtgaa gacctactac tactcttccc gtgacggtaa tggtcacggt 480
actcattgcg caggtactgt tggtagccgt acctacggtg ttgctaagaa aacgcaactg 540
ttcggcgtta aagtgctgga cgacaacggt tctggtcagt actccaccat tatcgcgggt 600
atggatttcg tagcgagcga taaaaacaac cgcaactgcc cgaaaggtgt tgtggcttct 660
ctgtctctgg gtggtggtta ctcctcttct gttaacagcg cagctgcacg tctgcaatct 720
tccggtgtca tggtcgcagt agcagctggt aacaataacg ctgatgcacg caactactct 780
cctgctagcg agccttctgt ttgcaccgtg ggtgcatctg atcgttatga tcgtcgtagc 840
tccttcagca actatggttc cgtcctggat atcttcgcgc ctggtacttc tatcctgtct 900
acctggattg gcggtagcac tcgttccatt tccggtacga gcatggctac tccacatgtt 960
gctggtctgg cagcatacct gatgaccctg ggtaagacca ctgctgcatc cgcttgtcgt 1020
tacatcgcgg atactgcgaa caaaggcgat ctgtctaaca tcccgttcgg caccgttaat 1080

ctgctggcat acaacaacta tcaggct                                     1107
```

<210> 91

<211> 30
<212> DNA
<213> Artificial Sequence

<220>
<223> Oligonucleotide

<400> 91
taacaggagg aattaaccat gaaaaaactg 30

<210> 92
<211> 29
<212> DNA
<213> Artificial Sequence

<220>
<223> Oligonucleotide

<400> 92
taatctgtat caggctgaaa atcttctct 29

<210> 93
<211> 36
<212> DNA
<213> Artificial Sequence

<220>
<223> Oligonucleotide

<400> 93
taacaggagg aattaaccat gaaaaaactg ctgttc 36

<210> 94
<211> 41
<212> DNA
<213> Artificial Sequence

<220>
<223> Oligonucleotide

<400> 94
aagtacatcg tgaagttcaa ggagggttct gctctgtctg c 41

<210> 95
<211> 43
<212> DNA
<213> Artificial Sequence

<220>
<223> Oligonucleotide

<400> 95
cgttgaatac atcgaacagg acgctgtggt tactatcaac gcg 43

<210> 96
<211> 38
<212> DNA
<213> Artificial Sequence

<220>
<223> Oligonucleotide

<400> 96
ggcatcgagg cttctcatcc tgagtttgaa ggccgtgc 38

<210> 97
<211> 41
<212> DNA
<213> Artificial Sequence

<220>
<223> Oligonucleotide

<400> 97
ttaaagtgct ggacgacaac ggttctggtc agtactccac c 41

<210> 98
<211> 37
<212> DNA
<213> Artificial Sequence

<220>
<223> Oligonucleotide

<400> 98
cgtctgcaat cttccggtgt catggtcgca gtagcag 37

<210> 99
<211> 43
<212> DNA
<213> Artificial Sequence

<220>
<223> Oligonucleotide

<400> 99
cgttacatcg cggatactgc gaacaaaggc gatctgtcta aca 43

<210> 100
<211> 42
<212> DNA
<213> Artificial Sequence

<220>
<223> Oligonucleotide

<400> 100
ccaccagcgg aatcgcgaac agcagttttt tcatggttaa tt 42

<210> 101
<211> 39
<212> DNA
<213> Artificial Sequence

<220>
<223> Oligonucleotide

<400> 101
ttttccatag cagcatccag agcagacaga gcagaaccc 39

<210> 102
<211> 39
<212> DNA
<213> Artificial Sequence

<220>
<223> Oligonucleotide

<400> 102
aggtgcgtta gtctgtgccg cgttgatagt aaccacagc 39

<210> 103
<211> 44
<212> DNA
<213> Artificial Sequence

<220>
<223> Oligonucleotide

<400> 103
gtagtagtag gtcttcacca tttgtgcacg gccttcaaac tcag 44

<210> 104
<211> 42
<212> DNA
<213> Artificial Sequence

<220>
<223> Oligonucleotide

<400> 104
cgaaatccat acccgcgata atggtggagt actgaccaga ac 42

<210> 105
<211> 41
<212> DNA
<213> Artificial Sequence

<220>
<223> Oligonucleotide

<400> 105
gcatcagcgt tattgttacc agctgctact gcgaccatga c 41

<210> 106
<211> 42
<212> DNA
<213> Artificial Sequence

<220>
<223> Oligonucleotide

<400> 106
acgagtgcta ccgccaatcc aggtagacag gatagaagta cc 42

<210> 107
<211> 38
<212> DNA
<213> Artificial Sequence

<220>
<223> Oligonucleotide

<400> 107
cggtgccgaa cgggatgtta gacagatcgc ctttgttc 38

<210> 108
<211> 36
<212> DNA
<213> Artificial Sequence

<220>
<223> Oligonucleotide

<400> 108
gcgattccgc tggtggtgcc gttctatagc catagc 36

<210> 109
<211> 41
<212> DNA
<213> Artificial Sequence

<220>
<223> Oligonucleotide

<400> 109
tctggatgct gctatggaaa agatctctgg caagcctgat c 41

<210> 110
<211> 35
<212> DNA
<213> Artificial Sequence

<220>
<223> Oligonucleotide

<400> 110
gcacagacta acgcaccttg gggtctggca cgtat 35

<210> 111
<211> 45
<212> DNA
<213> Artificial Sequence

<220>
<223> Oligonucleotide

<400> 111
acaaatggtg aagacctact actactcttc ccgtgacggt aatgg 45

<210> 112
<211> 44
<212> DNA

<213> Artificial Sequence

<220>
<223> Oligonucleotide

<400> 112
attatcgcgg gtatggattt cgtagcgagc gataaaaaca accg 44

<210> 113
<211> 41
<212> DNA
<213> Artificial Sequence

<220>
<223> Oligonucleotide

<400> 113
ctggtaacaa taacgctgat gcacgcaact actctcctgc t 41

<210> 114
<211> 37
<212> DNA
<213> Artificial Sequence

<220>
<223> Oligonucleotide

<400> 114
attggcggta gcactcgttc catttccggt acgagca 37

<210> 115
<211> 38
<212> DNA
<213> Artificial Sequence

<220>
<223> Oligonucleotide

<400> 115
tcccgttcgg caccgttaat ctgctggcat acaacaac 38

<210> 116
<211> 35
<212> DNA
<213> Artificial Sequence

<220>
<223> Oligonucleotide

<400> 116
ggccggtgcc atggtgctat ggctatagaa cggca 35

<210> 117
<211> 43
<212> DNA
<213> Artificial Sequence

<220>

<223> oligonucleotide

<400> 117
gctgaacacg ttcttataga cgtgatcagg cttgccagag atc 43

<210> 118
<211> 36
<212> DNA
<213> Artificial Sequence

<220>
<223> Oligonucleotide

<400> 118
acccggggaa gtagaagaaa tacgtgccag acccca 36

<210> 119
<211> 38
<212> DNA
<213> Artificial Sequence

<220>
<223> Oligonucleotide

<400> 119
gcgcaatgag taccgtgacc attaccgtca cgggaaga 38

<210> 120
<211> 40
<212> DNA
<213> Artificial Sequence

<220>
<223> oligonucleotide

<400> 120
acacctttcg ggcagttgcg gttgttttta tcgctcgcta 40

<210> 121
<211> 37
<212> DNA
<213> Artificial Sequence

<220>
<223> Oligonucleotide

<400> 121
gcaaacagaa ggctcgctag caggagagta gttgcgt 37

<210> 122
<211> 38
<212> DNA
<213> Artificial Sequence

<220>
<223> Oligonucleotide

<400> 122

gcaacatgtg gagtagccat gctcgtaccg gaaatgga 38

<210> 123
<211> 43
<212> DNA
<213> Artificial Sequence

<220>
<223> Oligonucleotide

<400> 123
tgatggtcga cagcctgata gttgttgtat gccagcagat taa 43

<210> 124
<211> 35
<212> DNA
<213> Artificial Sequence

<220>
<223> Oligonucleotide

<400> 124
accatggcac cggccgttga acagcgttct gaagc 35

<210> 125
<211> 42
<212> DNA
<213> Artificial Sequence

<220>
<223> Oligonucleotide

<400> 125
acgtctataa gaacgtgttc agcggtttcg cagcaactct gg 42

<210> 126
<211> 42
<212> DNA
<213> Artificial Sequence

<220>
<223> Oligonucleotide

<400> 126
ttcttctact tccccgggta cgtctactta ctactacgac ga 42

<210> 127
<211> 37
<212> DNA
<213> Artificial Sequence

<220>
<223> Oligonucleotide

<400> 127
tcacggtact cattgcgcag gtactgttgg tagccgt 37

<210> 128

<211> 38
<212> DNA
<213> Artificial Sequence

<220>
<223> Oligonucleotide

<400> 128
caactgcccg aaaggtgttg tggcttctct gtctctgg 38

<210> 129
<211> 35
<212> DNA
<213> Artificial Sequence

<220>
<223> Oligonucleotide

<400> 129
agcgagcctt ctgtttgcac cgtgggtgca tctga 35

<210> 130
<211> 37
<212> DNA
<213> Artificial Sequence

<220>
<223> Oligonucleotide.

<400> 130
tggctactcc acatgttgct ggtctggcag catacct 37

<210> 131
<211> 46
<212> DNA
<213> Artificial Sequence

<220>
<223> Oligonucleotide

<400> 131
tatcaggctg tcgaccatca tcatcatcat cattgagttt aaacgg 46

<210> 132
<211> 39
<212> DNA
<213> Artificial Sequence

<220>
<223> Oligonucleotide

<400> 132
tgcctcaatc agaggagctg cttcagaacg ctgttcaac 39

<210> 133
<211> 38
<212> DNA
<213> Artificial Sequence

<220>
<223> Ologonucleotide

<400> 133
acacggacca tgttctcgtc cagagttgct gcgaaacc 38

<210> 134
<211> 41
<212> DNA
<213> Artificial Sequence

<220>
<223> Oligonucleotide

<400> 134
gaaccttgac cggcagactc gtcgtagtag taagtagacg t 41

<210> 135
<211> 40
<212> DNA
<213> Artificial Sequence

<220>
<223> Oligonucleotide

<400> 135
tttcttagca acaccgtagg tacggctacc aacagtacct 40

<210> 136
<211> 41
<212> DNA
<213> Artificial Sequence

<220>
<223> Oligonucleotide

<400> 136
cagaagagga gtaaccacca cccagagaca gagaagccac a 41

<210> 137
<211> 38
<212> DNA
<213> Artificial Sequence

<220>
<223> Oligonucleotide

<400> 137
gagctacgac gatcataacg atcagatgca cccacggt 38

<210> 138
<211> 38
<212> DNA
<213> Artificial Sequence

<220>
<223> Oligonucleotide

<400> 138
tggtcttacc cagggtcatc aggtatgctg ccagacca 38

<210> 139
<211> 45
<212> DNA
<213> Artificial Sequence

<220>
<223> Oligonucleotide

<400> 139
aaaacagcca agctggagac cgtttaaact caatgatgat gatga 45

<210> 140
<211> 40
<212> DNA
<213> Artificial Sequence

<220>
<223> Oligonucleotide

<400> 140
agctcctctg attgaggcac gtggtgaaat ggtagcaaac 40

<210> 141
<211> 37
<212> DNA
<213> Artificial Sequence

<220>
<223> Oligonucleotide

<400> 141
acgagaacat ggtccgtgta ctgcgtgctc atccaga 37

<210> 142
<211> 40
<212> DNA
<213> Artificial Sequence

<220>
<223> Oligonucleotide

<400> 142
gtctgccggt caaggttctt gcgtttacgt gatcgatacg 40

<210> 143
<211> 37
<212> DNA
<213> Artificial Sequence

<220>
<223> Oligonucleotide

<400> 143
acctacggtg ttgctaagaa aacgcaactg ttcggcg 37

<210> 144
<211> 38
<212> DNA
<213> Artificial Sequence

<220>
<223> Oligonucleotide

<400> 144
gtggtggtta ctcctcttct gttaacagcg cagctgca 38

<210> 145
<211> 42
<212> DNA
<213> Artificial Sequence

<220>
<223> Oligonucleotide

<400> 145
tcgttatgat cgtcgtagct ccttcagcaa ctatggttcc gt 42

<210> 146
<211> 37
<212> DNA
<213> Artificial Sequence

<220>
<223> Oligonucleotide

<400> 146
gatgaccctg ggtaagacca ctgctgcatc cgcttgt 37

<210> 147
<211> 41
<212> DNA
<213> Artificial Sequence

<220>
<223> Oligonucleotide

<400> 147
tctccagctt ggctgttttg gcggatgaga gaagattttc a 41

<210> 148
<211> 43
<212> DNA
<213> Artificial Sequence

<220>
<223> oligonucleotide

<400> 148
cctggatatc ttcgcgcctg gtacttctat cctgtctacc tgg 43

<210> 149
<211> 44
<212> DNA

<213> Artificial Sequence

<220>
<223> Oligonucleotide

<400> 149
tccttgaact tcacgatgta cttgtttgct accatttcac cacg 44

<210> 150
<211> 40
<212> DNA
<213> Artificial Sequence

<220>
<223> Oligonucleotide

<400> 150
gtcctgttcg atgtattcaa cgtctggatg agcacgcagt 40

<210> 151
<211> 41
<212> DNA
<213> Artificial Sequence

<220>
<223> Oligonucleotide

<400> 151
gatgagaagc ctcgatgccc gtatcgatca cgtaaacgca a 41

<210> 152
<211> 37
<212> DNA
<213> Artificial Sequence

<220>
<223> Oligonucleotide

<400> 152
cgttgtcgtc cagcacttta acgccgaaca gttgcgt 37

<210> 153
<211> 35
<212> DNA
<213> Artificial Sequence

<220>
<223> Oligonucleotide

<400> 153
accggaagat tgcagacgtg cagctgcgct gttaa 35

<210> 154
<211> 37
<212> DNA
<213> Artificial Sequence

<220>

<223> Oligonucleotide

<400> 154
gcagtatccg cgatgtaacg acaagcggat gcagcag 37

<210> 155
<211> 37
<212> DNA
<213> Artificial Sequence

<220>
<223> oligonucleotide

<400> 155
taatctgtat caggctgaaa atcttctctc atccgcc 37

<210> 156
<211> 40
<212> DNA
<213> Artificial Sequence

<220>
<223> Oligonucleotide

<400> 156
aggcgcgaag atatccagga cggaaccata gttgctgaag 40

<210> 157
<211> 1382
<212> DNA
<213> Artificial Sequence

<220>
<223> Synthetic Polynucleotide

<400> 157

```
cggggacaag tttgtacaaa aaagcaggct gctcttcgcc tgctggctgg taatcgccag 60
caggcctttt tatttggggg agagggaagt catgaaaaaa ctaacctttg aaattcgatc 120
tccaccacat cagctctgaa gcaacgtaaa aaaacccgcc ccggcgggtt tttttatacc 180
cgtagtatcc ccacttatct acaatagctg tccttaatta aggttgaata aataaaaaca 240
gccgttgcca gaaagaggca cggctgtttt tattttctag tgagaccggg accagtttat 300
taagcgccag tgctatgacg accttctgcg cgctcgtact gttcgacaat ggtgtaatct 360
tcgttgtgag aagtgatgtc cagcttgatg tcagttttgt aagcgcccgg cagttgcaca 420
ggtttttttg ccatgtacgt agtttttacc tctgcgtcgt agtgaccacc gtccttcagc 480
ttcaggcgca ttttaatttc gcccttcagg gcaccatctt ccgggtacat acgctcagtg 540
gacgcttccc aacccatcgt ctttttctgc attacaggac cgtcagacgg gaagttagta 600
ccgcgcagct tcactttgta gatgaactcg ccgtcttgca ggctagagtc ttgggtcaca 660
gtcaccacac caccgtcctc gaagttcata acacgttccc atttgaaacc ttccgggaaa 720
gacagtttca ggtaatccgg aatatccgcc gggtgtttaa cgtacgcctt agagccatac 780
tggaactgag ggctcagaat atcccatgca aaaggcagtg ggccaccttt ggtcactttc 840
agtttcgcgg tctgagtacc ctcgtaagga cggccttcac cttcaccctc gatttcaaat 900
tcgtggccat ttacagagcc ctccatacgc actttgaagc gcatgaactc cttgattaca 960
tcttcagagg aggccatttt tttttcctcc ttattttctc aagcctaggt ctgtgtgaaa 1020
ttgttatccg ctcacaattg aatctatcat aattgtgagc gctcacaatt gtaaaggtta 1080
gatccgctaa tcttatggat aaaaatgcta tgttcccccc ggggggatat caacaggagt 1140
```

```
ccaagcgacc ggtggttgca tgtctagcta gctagaacag gactagtcct gagtaatagt 1200
caaaagcctc cggtcggagg cttttgactt tctgaaatgt aatcacactg gctcaccttc 1260
gggtgggcct ttctgcgttt ataagaagga aaaaagcggc cgcaaaagga aaaaattatt 1320
cgtatagcat acattatacg aagttataag cttacccagc tttcttgtac aaagtggtcc 1380
cc                                                                1382
```

<210> 158
<211> 21
<212> DNA
<213> Artificial Sequence

<220>
<223> RNA Stem Loop Structure

<400> 158
ccgtgacggt aacggtcacg g 21

## Description

### 1. FIELD OF THE INVENTION

This invention relates to methods for designing and synthesizing nucleic acids, and to novel polynucleotides.

### 2. BACKGROUND OF THE INVENTION

Several methods have been described for the synthesis of oligonucleotides using phosphoramidite chemistry, which are now capable of achieving nucleotide coupling efficiencies of 99%. The primary markets for commercial oligonucleotide synthesis are synthesis of oligonucleotide arrays for genomic and expression applications, and for use as PCR primers, for which such efficiencies are adequate. Since 1990 there has been little work done improving oligonucleotide chemistries to increase coupling efficiencies, the focus has instead been on increasing throughput with existing chemistries. Increased coupling efficiencies would provide a significant benefit to growing applications such as synthesis of long polynucleotides by assembly of oligonucleotides, accurate detection of single nucleotide polymorphisms in individuals and populations, the manufacture of high quality microarray chips for use in clinical diagnostics, haplotyping, real-time polymerase chain reaction, small inhibitory RNAs (siRNAs) used for validation of drug targets, expression array production, and chip-based sequencing. There is therefore a need in the art for synthetic processes that reduce synthesis errors and increase oligonucleotide coupling efficiencies.

Several methods have been described for the synthesis of larger polynucleotides by the assembly of oligonucleotides, using combinations of ligation, polymerase chain reaction and ligase chain reaction. See, for example, Hayden et al., 1988, DNA 7, 571-7.; Ciccarelli et al., 1991, Nucleic Acids Res 19, 6007-13; Jayaraman et al., 1991, Proc Natl Acad Sci U S A 88, 4084-8.; Jayaraman et al., 1992, Biotechniques 12: 392-8.; Graham et al., 1993, Nucleic Acids Res 21: 4923-8.; Kobayashi et al., 1997, Biotechniques 23: 500-3.; Au et al., 1998, Biochem Biophys Res Commun. 248: 200-203; Hoover et al., 2002, Nucleic Acids Res 30: e43, each of which is hereby incorporated by reference in its entirety. The assembly of polynucleotides from oligonucleotides is an error-prone process. Errors arise from the chemical synthesis of oligonucleotides, and the enzymatic processes used to assemble these oligonucleotides into longer polynucleotides. These errors increase the cost and time taken to synthesize polynucleotides. There is therefore a need in the art for synthetic processes that reduce synthesis errors and synthesis time.

The article by Shuttleworth et al., Gene, 58 (1987), 283-295, discloses a nucleotide sequence analysis of the gene for protein A from *staph. aureus* Cowan 1.

The article by Jine et al., Journal of Biotechnology, 22 (1992), 271-282, discloses a study relating to high level expression of staphylococcal nuclease R gene in *E. coli.*

### 3. SUMMARY OF THE INVENTION

The present invention relates to polynucleotides, vectors, plasmids and expression systems as defined in the claims.

### 4. BRIEF DESCRIPTION OF THE FIGURES

Figure 1 illustrates a flowchart showing the standard coupling process for oligonucleotide synthesis in accordance with the prior art. See also, Gait, 1984, Practical approach series, xiii, 217). Minor modifications have also been described in Matteucci & Caruthers, 1981, J Am Chem Soc. 103, 3185-3191; Pon et al., 1985, Tetrahedron Lett. 26, 2525-2528; Adams et al., 1983, J Am Chem Soc 105, 661-663; McBride et al., 1986; J Am Chem Soc 108, 2040-2048; Letsinger et al., 1984, Tetrahedron 40,137-143; Hayakawa et al., 1990, J Am Chem Soc 112, 1691-1696; and Hayakawa & Kataoka., 1998, J Am Chem Soc 120, 12395-12401.

Figures 2A - 2C illustrate the effect of a capping procedure on the distribution of truncated oligomers. (A) Expected distribution of oligonucleotide products with and without capping. (B) HPLC trace showing the observed distribution of oligonucleotide products without capping. (C) Proposed explanation for failures in elongation: oligonucleotide packing produces populations that grow as desired (202A and 202E), are trapped by neighboring chains (202B) or protected by neighboring trityl groups (202D) resulting in n-1, n-2, n-3 etc. byproducts, or nonoxidized (202C) that will generate n-1 byproducts.

Figures 3A - 3D illustrate the stability of the trityl protection group. Samples of 5NO-dimethoxytrityl-bisthymydyllthymidine were incubated at 25°C for 60 hours in 0.5M phosphate buffer at the pH indicated, then analyzed by HPLC. Protected oligonucleotides are indicated as the DMTr-T3 peak to the right of each trace, loss of protection is seen as an increase in height of the T3 peak towards the left of each trace. (A) start, (B) pH 7.0, (C) pH 6.0, (D) pH 5.0.

Figures 4A-4F illustrate optimization of phosphodiesterase cleavage of non-tritylated oligonucleotides. A total of 1nmol of $dT_{20}$ (QIAgen) in 10 μl of 0.5M phosphate buffer was treated for sixteen hours with calf spleen phosphodiesterase II (Sigma cat # P9041) and analyzed by HPLC. (A) undigested, (B) 0.01U enzyme 25°C, pH 7.0, (C) 0.01U enzyme 37°C, pH 7.0, (D) 0.01U enzyme 25°C, pH 6.0, (E) 0.01U enzyme 25°C, pH 5.0, and (F) 0.1U enzyme 25°C, pH 7.0. Undigested 20mer is the large peak to the right of trace A. Completely digested monomer is the large peak to the left of traces B-F.

Figures 5A-5C illustrate phosphodiesterase-II-assisted oligonucleotide purification. An oligomer of $dT_{15}$ was synthesized on CPG 2000A without capping, treated with phosphodiesterase and analyzed by HPLC. (A) ~80nmol of fully deprotected $dT_{15}$ on 2 mg CPG treated with 1U enzyme for 30 hours at 37°C prior to cleavage, (B) - 40nmol of $dT_{15}$ cleaved from 1 mg CPG untreated with phosphodiesterase, (C) ~10nmol of trityl protected $dT_{15}$ from the same synthesis as trace B, cleaved from 1 mg CPG and treated with 0.1U enzyme for 16 hours at 25°C. Following cleavage, the enzyme was denaturated by heating to 65°C for 30 minutes, the oligomer detritylated by acetic acid for 2 hours and neutralized with 10 M ammonia. Undigested 15mer is the large peak to the right of each trace. Truncated oligomers are labeled.

Figures 6A-6C illustrate HPLC purification of tritylated oligonucleotides. 9mer oligodeoxythymidine was synthesized under standard conditions without capping (6A), (6B) with $Ac_2O$-DMAP capping before oxidation (0.1M THF:L:W = 4:1:1) and (6C) with $Ac_2O$-DMA capping after oxidation (0.1M THF:L:W = 4:1:1). Oligonucleotides were cleaved without detritylation and HPLC purified on a XTerra MS-C18. Untritylated oligonucleotides (traces A in 6A, 6B and 6C) were separated from full-length tritlated oligonucleotides (traces B in 6A, 6B and 6C) which were eluted after 8min of washing with 0.1 % TFA. Oligonucleotides were then detritylated and analyzed by HPLC. The full-length 9mer is the large peak to the right of traces B.

Figures 7A-7H illustrate two classes of chain elongation failures. Tetramers of homo-thymidine (A, B), homo-cytidine (C, D), homo-adenine (E, F) and homo-guanine (G, H) were synthesized without capping on a CPG support and cleaved without detritylation. HPLC was then used to separate the tritylated (the large peak to the right of traces A, C, E and G) from the non-tritylated oligomers (the small peak to the left of traces A, C, E and G), or to separate tritylated trimer (the small peak to the right of traces B, D, F and H) from tritylated tetramer (the large peak to the left of traces B, D, F and H).

Figures 8A-8C illustrate comparison of capping reagents. A single CPG-linked thymidine was capped with (A) acetic anhydride/NMI (B) $Pac_2O$/NMI or (C) DMPA for the times indicated. Incomplete capping was measured by coupling a second thymidine. Capped (T1, the large peak to the left of traces in A and B) and dimer (T2, produced from uncapped chains) peaks were separated by HPLC.

Figures 9A-F illustrate efficiency of capping after fifteen seconds and after one minute. A first CPG-supported thymidine was capped for fifteen seconds with (A) N-MI:Lut:THF = 1:1.5:7, (B) N-MI:Lut:THF = 1.5:1.5:7 (C) N-MI:Lut:DIOX =1.5:1.5:7, (D) N-I:Lut:DMA = 1.5:1.5:7, (E) N-MI:Lut:TOL =1.5:1.5:7, (F) DMAP:Lut:DMA = 1.5:1.5:7 (N-MI = N-methylimidazole; Lut = 2,6-lutidine; THF = tetrahydrafuran; DIOX = dioxane; DMA = dimethylacetamide; and DMAP = N,N-dimethylaminopyridine). The base was then coupled to a second thymidine which reacted at the unprotected positions. Oligonucleotides were detritylated, cleaved from the support and analyzed by HPLC. Relative Capping Efficiency (RCE) was calculated as the ratio of T1 to T2. RCE after fifteen seconds was (A) 77.2%, (B) 77.8%, (C) 65.4%, (D) 50.4%, (E) 95.6%, and (F) 100%. RCE after one minute was (A) 99.1%, B) 99.2%, (C) 97.1%, (D) 93.8%, (E) 100%, and (F) 100%.

Figures 10A-F illustrate comparison of oxidation conditions. A single CPG-linked thymidine was coupled to a second thymidine and oxidized with 0.1M iodine in THF:2,6-lutidine:Water 40:10:1 in accordance with Gait, 1984, Practical Approach Series, xiii, 217, for (A) five seconds, (B) twenty seconds, (C) one minute, (D) ten minutes or (E) with 0.1M iodine in THF:2,6-lutidine:water 40:10:1 for 15 seconds or (F) 0.08M iodine in THF: 2,6-lutidine:Water 4:1:1 for 15 seconds. The dimer was then detritylated, cleaved from CPG and analyzed by HPLC. The T2 peak (to the right of each trace) corresponds to completely oxidized chains, the T1 peak (to the left of each trace) corresponds to incomplete oxidation followed by bond cleavage upon detritylation.

Figure 11 illustrate products resulting from incomplete chain oxidation in accordance with the prior art.

Figures 12A-G compare oxidation reagents. A single CPG-linked thymidine was coupled to a second thymidine and oxidized for fifteen seconds with (A) no oxidizer; (B) 0.08M iodine in THF:2,6-lutidine:water = 4:1:1 (stored for three months at 25°C, no precipitation as described by Pon, 1987, Nucleic Acids Res 15, 7203; (C) freshly prepared 1.25M iodine in THF:2,6-lutidine:water 4:1:1; (D) 1M t-butyl hydroperoxide (TBHP)/toluene (stored at 25°C for three months in a dark glass bottle as described in Hayakawa et al., 1986, Tetrahedron Lett 27, 4191-4194; (E) $CCl_4$ oxidation as described in Padiya and Salunkhe, 1998, J Chem Research, 804; two month old solutions A and B were mixed immediately before use; (F) 3.3M TBHP/toluene stored at 25'C for three months in a dark glass bottle; and (G) ten minute oxidation with iodine aqueous solution (same as B). The dimer was then detritylated, cleaved from CPG and analyzed by HPLC. The T2 peak (to the right of each trace) corresponds to completely oxidized chains, the T1 peak (to the left of each trace) corresponds to incomplete oxidation followed by bond cleavage upon detritylation.

Figure 13 illustrates a modified oligonucleotide synthesis procedure. See Eadie & Davidson, 1987, Nucleic Acids Res 15, 8333-49; Boal et al., 1996; Nucleic Acids Res 24, 3115; and Kwiatkowski et al., 1996, Nucleic Acids Res 24, 4632-4638.

Figures 14A-14F illustrate a comparison of efficiency of standard and modified coupling protocols. HPLC chromatograms of (14A) high quality dT20, (14B) low quality dT20, (14C) gel-purified dT20 (from ABRF NARG 2000-2001 DNA synthesis studies that covered 20 DNA synthesis core facilities and 30 DNA synthesizers. See, Gunthorpe *et al.,* 2001, http://www.abrf.org/ResearchGroups/NucleicAcids/EPosters/NARG_00_01_poster.pdf, (14D) dT10 purchased from QIAgen, (14E) dT9, and (14F) dT16 synthesized using a modified protocol.

Figures 15A-15O illustrate quartz surface reorganization in which 7mm quartz rods were broken and kept under vacuum (15A) - (15F) or in air (15G) - (15L) before measuring the surface wetability with a 2 µl water drop. Broken glass vacuum: (15A) 0h (0N), (15B) 0.5h(48N), (15C) 2h (58N), (15D), 5h (61N), (15E) 17h (64N), and (15F) 48h (69N). Broken glass atmosphere: (15G) 0h, (15H) 2h, (15I) 24h, (15J) 32h, (15K) 75h, (15L) old surface (87'). Freshly polished glass rod: (15M) 220mesh, (15N) 600mesh. (150). Quantification of (15A) through (15F) was by measuring the contact angle between the water and the surface.

Figures 16A-16O illustrates activation of glass surfaces. All silanoyl groups were removed by heating a freshly broken quartz rod in a vacuum at 125°C for 1hour. The rod was then treated with (16A) - (16B) 10M Ammonium hydroxide (16A) start, (16B) after 24 hours; (16C) - (16D) 10M HCl (16C) start, (16D) after 24 hours; (16E) - (16F) trifluoroacetic acid (E) start, (F) after 24 hours; (G) - (1) 65% nitric acid, (G) start, (H) after 1 hour, (161) after 24 hours; (16J) - (16K) 50% w/v sodium hydroxide; (16J) start, (16K) after 24 hours; (16L) - (16M) sodium fluoride, (16L) start, (16M) after 24 hours. Cleavage of Si-O-Si bonds was assessed by measuring changes in the contact angle of a two µl water drop.

Figures 17A-17J illustrate derivatization of rod surfaces. (17A) The sides of the rods are protected with trimethylsilane (TMS) while the ends are derivatized with aminopropylsilane (APS). The polished surfaces of quartz rods were activated using 50% w/v sodium hydroxide for 11 minutes at 25°C followed by 5 minutes with concentrated nitric acid before treatment with (17B) - (17E) trimethylsilane (17B) start, (17C) 6 seconds, (17D) 12 seconds, (17E) 60 seconds; (17F) - (17I) aminopropylsilane from a freshly opened bottle (17F) 1 minute, (17G) 5 minutes, (17H) 10 minutes, (17I) 20 minutes, or (17J) aminopropylsilane from an old bottle. Hydrophobicity was assessed by measuring changes in the contact angle of a two µl water drop.

Figures 18A-18B illustrate the loading of APS and first nucleotide. The loading of dimethoxytritylthymidine onto derivatized glass surfaces was measured by comparison to the curve "peak area - concentration". (A) Loading was measured on surfaces derivatized by exposure to 1% aminopropylsilane (APS) in EtOH for different times. (B) Loading was measured on surfaces derivatized with aminopropylsilane for eight minutes then loaded.

Figures 19A-19F illustrate single and twelve channel devices for oligonucleotide synthesis: (19A) a single channel CPG reaction vessel, (19B) twelve-pin activated glass rods, (19C) rods in prototype reactor, (19D) removing a microtiter plate from reactor (19E) illustrates the use of a humidity sensor to ensure water-free conditions. Cleavage from glass rods was carried out in gaseous ammonia at 55°C inside an autoclave (19F).

Figures 20A-20C illustrate oligonucleotide synthesis on different supports. A polythymidine 9mer was synthesized, cleaved, detritylated and analyzed by HPLC. (20A) Synthesis on derivatized quartz rod with capping prior to oxidation.

(20B) synthesis on derivatized quartz rod following the modified protocol shown in Figure 13. (20C) Synthesis on CPG in parallel with the synthesis in (20B).

Figure 21. A schematic representation of the assembly of oligonucleotides into a polynucleotide. Oligonucleotides are represented by arrows pointing from 5' to 3'. In this example the polynucleotide is assembled from sixteen oligonucleotides, eight for each strand. Each oligonucleotide is labeled: those that comprise the top strand of the polynucleotide with one capital letter, those that comprise the bottom strand with two lower case letters. These letters indicate the two top strand oligonucleotides to which the bottom strand is complementary. In this representation the oligonucleotides are shown precisely abutting one another, that is the 3'-most base of each oligonucleotide is the base following the 5'-most base of the preceding oligonucleotide, so that the consecutive sequences of the top strand oligonucleotides are identical to the top strand of the polynucleotide sequence. Similarly the consecutive sequences of the bottom strand oligonucleotides are identical to the bottom strand of the polynucleotide sequence. Other oligonucleotide arrangements are also possible: the oligonucleotides may not precisely abut one another. In one case there could be a gap between two adjacent oligonucleotides which is "covered" by the sequence in the complementary oligonucleotide. In another case there could be overlap between two adjacent oligonucleotides. In this scheme and in the text of this application the term "correct annealing partner" refers to oligonucleotides whose annealing will result in the subsequent synthesis of the desired polynucleotide. In this figure for example, the correct annealing partners for oligonucleotide B are oligonucleotide ab and oligonucleotide bc. The term "incorrect annealing partner" refers to oligonucleotides whose annealing will not result in the subsequent synthesis of the desired polynucleotide. In this figure, for example, the incorrect annealing partners for oligonucleotide B are all oligonucleotides other than oligonucleotide ab and oligonucleotide bc.

Figure 22 illustrates the frequency of codon usage in *Escherichia coli* class II (highly expressed) genes. The table shows the three letter amino acid code, a three nucleotide codon that encodes that amino acid, and the frequency with which that codon appears in highly expressed *Escherichia coli* genes.

Figure 23 illustrates a table reflecting the bias of codon usage in human *(Homo sapiens)* genes. The table shows the three letter amino acid code, a three nucleotide codon that encodes that amino acid, and the frequency with which that codon appears in human genes.

Figure 24 illustrates a table reflecting a combination of the biases of codon usage in human (*Homo sapiens*) genes and *Escherichia coli* class II (highly expressed) genes. The table was constructed from those shown in Figures 23 and 24 as follows. Any codon that occurred with a frequency of less than 0.05 in either human or highly expressed *Escherichia coli* genes was eliminated by setting its frequency in the new table to zero. For example the codon TTA encodes Leu with a frequency of 0.07 in human genes, but only 0.03 in highly expressed *E. coli* genes, so its frequency in the hybrid table is set to 0. The remaining non-zero codon frequencies were calculated by averaging the values in the two organisms, for example the codon TTT encodes Phe with a frequency of 0.29 in highly expressed *E. coli* genes and a frequency of 0.45 in human genes so its value is set to the average of these values, 0.37, in the hybrid table. This calculation will yield frequencies that do not sum to 1 for amino acids for which one or more codon has been eliminated because it fell below the threshold (in this case Thr, Arg, Ser, Ile, Pro, Leu and Gly). For these amino acids, the frequencies have been normalized by dividing the frequency for each codon by the sum of the codon frequencies for that amino acid.

Figure 25 illustrates a table reflecting the bias of codon usage in mouse *(Mus musculus)* genes. The table shows the three letter amino acid code, a three nucleotide codon that encodes that amino acid, and the frequency with which that codon appears in mouse genes.

Figure 26 illustrates an automated process for designing a polynucleotide to encode a provided polypeptide sequence, incorporating functional and synthetic constraints. The steps in the process are: (01) input a polypeptide sequence for which an encoding polynucleotide is desired; (02) select a codon bias table that reflects the distribution of codons found in genes, or a class of genes (e.g. highly expressed genes) in one or more expression organisms; (03) select a threshold frequency (codons that are used with a frequency below this threshold will be rejected from the design); (04) select the next amino acid in the polypeptide; (05) select a codon that encodes the amino acid, by using the codon bias table to provide the probability of selection; (06) ensure that the selected codon is above the threshold (if it is not return to 05, otherwise proceed to 07); (07) check that the last N nucleotides have a GC content within defined limits, the number of nucleotides (N) and the GC content are both parameters that can be varied in the method. If this criterion is not satisfied proceed to 11, otherwise proceed to 08; (08) check that the last M nucleotides of sequence do not contain a forbidden restriction site, the number of nucleotides (M) and the list of sites to be avoided are both parameters that can be varied in the method. If the sequence does contain a forbidden site proceed to 11. Otherwise proceed to 09; (09) check whether the entire polynucleotide sequence contains a disallowed repeat. The parameters for repeats may be varied in the method. If the sequence does contain a disallowed repeat proceed to 11. Otherwise proceed to 10; (10) accept the codon and proceed to 04; (11-14) if any of the criteria from steps 07, 08 or 09 are not met, the method requires that the process move back some length of sequence (Z amino acids, where Z is preferably between 2 and 20 amino acids, more preferably between 5 and 10 amino acids) in the polypeptide, delete the codons that were selected for those amino acids and reselect those codons (Steps 11 and 12). Because the codons are selected probabilistically, different iterations of the process will produce different sequences that still fulfill the functional codon bias criteria. This process is repeated X number of times, where X is preferably less than 10,000, and more preferably less than 1,000. If X iterations are repeated without meeting all of the desired criteria, a report is generated describing the failure, the codon is accepted, and the process proceeds to the next amino acid. This is to prevent the method from becoming trapped in an endless loop if no solutions are available. The report will then allow manual adjustment of the constraints to obtain an acceptable solution (such as reducing the threshold for a single position or relaxing the repeat or GC content requirement).

Figure 27 illustrates an automated process for designing a polynucleotide to encode a provided polypeptide sequence, incorporating functional and synthetic constraints. The steps in the process are (01) input a polypeptide sequence for which an encoding polynucleotide is desired; (02) select a codon bias table that reflects the distribution of codons found in genes, or a class of genes (e.g. highly expressed genes) in one or more expression organisms; (03) select a threshold frequency. Codons that are used with a frequency below this threshold will be rejected from the design. (04) Select the next amino acid in the polypeptide. (05) Select a codon that encodes the amino acid, by using the codon bias table to provide the probability of selection. (06) Ensure that the selected codon is above the threshold. If it is not return to 05. Otherwise proceed to 07. (07) Check that the last N nucleotides have a GC content within defined limits. The number of nucleotides (N) and the GC content are both parameters that can be varied in the method. If this criterion is not satisfied proceed to 11. Otherwise proceed to 08. (08) Check that the last M nucleotides of sequence do not contain a forbidden restriction site. The number of nucleotides (M) and the list of sites to be avoided are both parameters that can be varied in the method. If the sequence does contain a forbidden site proceed to 11. Otherwise proceed to 09. (09) Check whether the last P nucleotides contain a subsequence that will anneal to any subsequence in the polynucleotide (or its reverse complement) with a calculated Tm of > Y°C. The number of nucleotides (P) and the annealing temperature are both parameters that can be varied in the method. If the sequence does contain a forbidden subsequence proceed to 11. Otherwise proceed to 10. (10) Accept the codon and proceed to 04. (11-14) If any of the criteria from steps 07, 08 or 09 are not met, the move back some length of sequence (Z amino acids, where Z is preferably between 2 and 20 amino acids, more preferably between 5 and 10 amino acids) in the polypeptide, delete the codons that were selected for those amino acids and reselect those codons (Steps 11 and 12). Because the codons are selected probabilistically, different iterations of the process will produce different sequences that still fulfill the functional codon bias criteria. This process is repeated X number of times, where X is preferably less than 10,000, more preferably less than 1,000. If X iterations are repeated without meeting all of the desired criteria, a report is generated describing the failure, the codon is accepted, and the process proceeds to the next amino acid. This is to prevent the method from becoming trapped in an endless loop if no solutions are available. The report will then allow manual adjustment of the constraints to obtain an acceptable solution (such as reducing the threshold for a single position or relaxing the repeat or GC content requirement).

Figure 28 illustrates an automatable process for modifying a designed polynucleotide to alter some properties (such as restriction sites, GC content and repeated subsequences) while retaining others (such as overall codon bias). (01) input a polypeptide sequence for which an encoding polynucleotide is desired; (02) select a codon bias table that reflects the distribution of codons found in genes, or a class of genes (e.g. highly expressed genes) in one or more expression organisms; (03) select a threshold frequency. Codons that are used with a frequency below this threshold will be rejected from the design. (04) Select an initial sequence design. This may be accomplished by using a method disclosed herein, or by selecting codons using a codon bias table but without applying any additional constraints. (05) identify whether any subsequence of N nucleotides has a GC content outside defined limits. The number of nucleotides (N) and the GC content are both parameters that can be varied in the method. If there are any such subsequences, proceed to 10. Otherwise proceed to 06. (06) Identify whether the polynucleotide contains any forbidden restriction sites. The list of sites to be avoided is a parameter that can be varied in the method. If the sequence does contain a forbidden site proceed to 10. Otherwise proceed to 07. (07) Check whether the polynucleotide contains any subsequences that will anneal to any subsequence in the polynucleotide (or its reverse complement) with a calculated Tm of > Y°C. The length of such subsequences is preferably between 6 and 40 nucleotides, more preferably between 8 and 30 nucleotides and even more preferably between 10 and 25 nucleotides. The size of the subsequence and the annealing temperature are both parameters that can be varied in the method. If the sequence does contain a forbidden subsequence proceed to 10. Otherwise proceed to 08. (08) Accept the sequence. (09-14) If the design fails any of the criteria from steps 05, 06 or 07, the method selects one codon in one of the regions that does not conform to the design specifications, and replaces it using another codon selected probabilistically from a codon bias table. The new polynucleotide sequence is then assessed to see whether it more closely conforms to the design specifications than the sequence before the replacement. If it does, the replacement is accepted, if not it is rejected.

Figure 29 illustrates an automatable process for designing a set of half-oligonucleotides as a basis for an oligonucleotide set for assembly into a polynucleotide. The half-oligonuclotides are designed to have a very close range of calculated annealing temperatures. (01) Input a polynucleotide sequence. (02) Select an annealing temperature Z°C, where Z is preferably between 40°C and 80°C, more preferably between 50°C and 76°C, even more preferably between 60°C and 74°C. (04, 05 and 07) Starting at the first position in the polynucleotide, begin adding nucleotides until a subsequence is obtained with an annealing temperature greater than the set annealing temperature. (06) Define the subsequence as one "half oligonucleotide". Repeat the process by resetting the start of a new half oligonucleotide (OA, with A set to A+1) to the first nucleotide following the just completed half oligonucleotide (set NB+1 to N1). The process continues until the entire polynucleotide has been divided into half-olignucleotides.

Figure 30 illustrates an automatable process for combining pairs of half-oligonucleotides to design an oligonucleotide set for assembly into a polynucleotide. This process can be encoded into a computer program. This process produces a set of oligonucleotide designs, each with a tight range of annealing temperatures. (01) input a polynucleotide sequence. (02) Calculate a set of half olignucleotides. For example, by using the process shown schematically in Figure 29. (03) Create a set of forward oligonucleotides by combining the first with second, the third with the fourth, the fifth with the sixth half oligonucleotides and so on. (04) Create a set of reverse oligonucleotides by combining the second with the third, the fourth with the fifth, the sixth with the seventh half oligonucleotides and so on. Each of these sequences should then be reverse complemented to provide the set of reverse oligonucleotides. (05) The forward and reverse set of oligonucleotides are then saved. (06) A new set of forward and reverse oligonucleotides are then created, with the starting point for the first half-oligonucleotide advanced by 1 nucleotide from the previous set. This process is repeated until the starting position is the first nucleotide of OF2 from the first set. A set of oligonucleotides starting from this position would be identical to the first set, except that OF1 would be missing.

Figure 31 illustrates an automatable process for selecting an oligonucleotide set suitable for assembly into a polynucleotide. (01) Input a polynucleotide sequence. (02) Identify and flag any subsequences that are repetitive defmed either by annealing properties with other parts of the polynucleotide, or by sequence matches. The annealing temperature and the length of sequence match are both parameters that can be varied in the method. (03) Input candidate oligonucleotide sets. Such sets can be produced by many methods, including for example by the methods shown in Figures 29 and 30. (04) Select one of the candidate sets. (05) Calculate the annealing temperatures for all of the correct annealing partners in the oligonucleotide set. Calculate the highest and lowest annealing temperatures within the set. (06) Determine whether the range of annealing temperatures for the correct annealing partners within the set is smaller than some specified value (A). If yes, proceed to 07. If no, proceed to 11. The annealing temperature range is a parameter that can be varied in the method. (07) Determine whether the range of oligonucleotide lengths within the set is between two specified values (C and D). If yes, proceed to 08. If no, proceed to 11. The lower and upper limits are parameters that can be varied in the method. (08) Determine whether there are an even number of oligonucleotides in the set. If yes, proceed to 09. If no, proceed to 11. (09) Determine whether there are repeat sequences (flagged in 02) at the end of any oligonucleotide. If no, proceed to 10. If yes, proceed to 11. (10) Determine whether any pair of incorrect annealing partners have an annealing temperature closer than a defined value (B) to the lowest annealing temperature between correct annealing partners. The value (B) is a parameter that can be varied in the method. If yes, proceed to 11. If no, proceed to 12. (11) If the set fails based on any of the criteria described, a new set of oligonucleotides may be selected and tested. If all sets fail, the adjustable parameters may be altered until an oligonucleotide set is identified that fulfills the relaxed selection criteria. (12) If a set passes all selection criteria, it is accepted.

Figure 32 illustrates a PCR protocol for assembly of a gene of length < 500 bp. The exact annealing temperature depends upon the calculated annealing temperatures of the correct annealing partners in the oligonucleotide set. For example, if the calculated annealing temperatures are in the range from 62°C to 65°C, the PCR annealing temperature should be between 58°C and 65°C.

Figure 33 illustrates a PCR protocol for assembly of a gene of length 500-750 bp. The exact annealing temperature depends upon the calculated annealing temperatures of the correct annealing partners in the oligonucleotide set.

For example, if the calculated annealing temperatures are in the range form 62°C to 65°C, the PCR annealing temperature should be between 58°C and 65°C.

Figure 34 illustrates a PCR protocol for assembly of a gene of length 750-1,000 bp. The exact annealing temperature depends upon the calculated annealing temperatures of the correct annealing partners in the oligonucleotide set. For example, if the calculated annealing temperatures are in the range form 62°C to 65°C, the PCR annealing temperature should be between 58°C and 65°C.

Figure 35 illustrates a PCR protocol for assembly of a gene of length 1,000-1,500 bp. The exact annealing temperature depends upon the calculated annealing temperatures of the correct annealing partners in the oligonucleotide set. For example, if the calculated annealing temperatures are in the range form 62°C to 65°C, the PCR annealing temperature should be between 58°C and 65°C.

Figure 36 illustrates a PCR protocol for assembly of a gene of length 1,500-2,000 bp. The exact annealing temperature depends upon the calculated annealing temperatures of the correct annealing partners in the oligonucleotide set. For example, if the calculated annealing temperatures are in the range form 62°C to 65°C, the PCR annealing temperature should be between 58°C and 65°C.

Figure 37 illustrates a dot-plot representation of repetitive sequence elements within a polypeptide. The same sequence is represented on the vertical and horizontal axes. The entire sequence was scanned using all consecutive overlapping 3 amino acid sequence elements. Dots and lines off the diagonal indicate repeated sequence elements within the polynucleotide.

Figure 38 illustrates a dot-plot representation of repetitive sequence elements within Part 1 of the polynucleotide shown in Figure 37. The same sequence is represented on the vertical and horizontal axes. The entire sequence was scanned using all consecutive overlapping 12 base pair sequence elements. Dots and lines off the diagonal indicate repeated sequence elements within the polynucleotide.

Figure 39 illustrates a dot-plot representation of repetitive sequence elements within Part 2 of the polynucleotide shown in Figure 37. The same sequence is represented on the vertical and horizontal axes. The entire sequence was scanned using all consecutive overlapping 12 base pair sequence elements. Dots and lines off the diagonal indicate repeated sequence elements within the polynucleotide.

Figure 40 illustrates a dot-plot representation of repetitive sequence elements within Part 3 of the polynucleotide shown in Figure 37. The same sequence is represented on the vertical and horizontal axes. The entire sequence was scanned using all consecutive overlapping 12 base pair sequence elements. Dots and lines off the diagonal indicate repeated sequence elements within the polynucleotide.

Figure 41 illustrates type IIS restriction sites useful for joining sections of a polynucleotide. The figure shows different type IIs restriction enzymes that may be used to generate compatible sticky ends useful for subsequent ligation of two or more DNA fragments. The targeted overhangs resulting from digestion are indicated in bold letters with alphabetic subscripts (*e.g.* $N_AN_B$ *etc).* Other nucleotides within the polynucleotide sequence are indicated with numerical subscripts, negative numbers indicating that the bases are before (*i.e.* 5' of) the targeted ligation overhang, positive numbers indicating that the bases are after (*i.e.* 3' of) the targeted ligation overhang. The figure shows a general scheme by which compatible ends may be generated in synthetic DNA segments, by adding the indicated sequences to the 3' end of the intended 5' segment, and to the 5' end of the intended 3' segment. Providing the same kind of overhang is produced (*i.e.* the same number of bases and either 3' or 5'), different restriction enzymes may be used to digest the different fragments.

Figure 42 illustrates an automatable process for selecting an oligonucleotide set suitable for assembly into a polynucleotide using ligation- or ligation chain reaction-based methods. This process can be encoded into a computer program. (01) Input a polynucleotide sequence. (02) Input a candidate set of oligonucleotides. Such sets can be produced by many methods, including for example by the methods shown in Figures 29 and 30. (03) For ligation-based assembly methods, the most important sequence recognition occurs at the ends of the sequence. Sequence designs that minimize incorrect ligation are thus those that minimize sequence similarities at the end of the oligonucleotides. This step defines the sequences at the ends. The length of this sequence is a parameter that can be varied within the method. (04) Determine whether the 5' ends of all the oligos are unique. If yes, proceed to 05. If no, proceed to 09. (05) Determine whether the 3' ends of all the oligos are unique. If yes, proceed to 06. If no, proceed to 09. (06) Determine whether the minimum annealing temperatures for the correct annealing partners within the set is greater than some specified temperature (A). If yes, proceed to 07. If no, proceed to 09. The annealing temperature range is a parameter that can be varied in the method. (07) Determine whether the range of oligonucleotide lengths within the set is between two specified values (C and D). If yes, proceed to 08. If no, proceed to 11. The lower and upper oligonucleotide lengths are parameters that can be varied in the method. (08) Accept the design. (09) Count the number of attempts to modify the oligonucleotide set (X). This number is a parameter that can be varied in the method. If the number of attempts exceeds the set number, choose a new set of oligonucleotides and proceed to 02. If the number of attempts does not exceed X, proceed to 10. (10-14) If the design fails any of the criteria from steps 04, 05, 06 or 07, the method selects one oligonucleotide that does not conform to the design specifications, and moves the boundary between it and an adjacent oligonucleotide. The new oligonucleotide set is then assessed to see whether it more closely conforms to the design specifications than the set before the replacement. If it does, the replacement is accepted, if not it is rejected.

Figure 43 illustrates the thermocycling protocol for assembly of a gene by ligation using a thermostable DNA ligase. The exact annealing temperature depends upon the calculated annealing temperatures of the correct annealing partners in the oligonucleotide set. For example, if the calculated annealing temperatures are in the range from 62°C to 65°C, the PCR annealing temperature should be between 58°C and 65°C.

Figure 44 illustrates an automatable process for designing a polynucleotide in parts. This process can be encoded into a computer program. (01) Input a polypeptide sequence. (02) Calculate a polynucleotide sequence that encodes the polypeptide. Processes such as those shown in Figures 26, 27 or 28 are possible ways of calculating the polynucleotide. Varying the parameters within these methods will result in different polynucleotides. (03) Calculate an oligonucleotide set that will assemble into the calculated polynucleotide. Processes such as those shown in Figures 29, 30 and 31 are possible ways of calculating the oligonucleotide sets. Varying the parameters within these methods will result in different oligonucleotide sets. (04) Determine whether any pair of incorrect annealing partners have an annealing temperature closer than a defined value (B) to the lowest annealing temperature between correct annealing partners. The aim of this step is to determine whether there are oligonucleotides that are likely to present a problem by annealing to incorrect partners during the assembly process. The value B is a parameter that can be varied in the method. If no, proceed to 09. If yes, proceed to 05. (05) Determine whether the length of the polynucleotide is less than N base pairs long. The value N is a parameter that can be varied in the method. If yes, then further division is undesirable, and the design criteria should be changed to allow ligase-based assembly instead of polymerase-based assembly, so proceed to 06. If no, proceed to 07. (06) Calculate an oligonucleotide set to assemble into the polynucleotide using a ligase-based method. One example of such a method is the process shown in Figure 26. (07) Divide the polypeptide into two sub-sequences. There are many different ways to divide the polypeptide. For example it can be divided between two residues such that the division separates two incorrect annealing partners with high annealing temperatures within the oligonucleotide set. The polypeptide can also be divided randomly. (08) For each part of the polypeptide design a polynucleotide segment to encode it. Many methods are available for design of polynucleotide encoding a specific polypeptide sequence, including those shown in Figures 26, 27 and 28. Each polynucleotide may also include restriction sites useful in joining the polynucleotide segments together; for example the type IIs restriction sites shown in Figure 40 may be added to the ends of the sequence in order to produce a complementary overlap between polynucleotide segments. In addition a recombinase-recognition sequence may be added to the end of each polynucleotide segment to facilitate independent cloning of each polynucleotide segment by a recombinase-based method. Since steps 03 to 08 are iterative, the original polypeptide may be divided into more than 2 sub-sequences. It is important to ensure that the resultant polynucleotide segments can be joined, for example by overlap extension or restriction digestion and ligation, to form a single polynucleotide. Return to 03. (09) Count the number of polynucleotides. If the number is $< P$ accept the design. If the number is $> P$ reject the design and return to 01. Because the design methods are probabilistic, a repeat of the process will yield a different solution that may conform to the design criteria. The value P is a parameter that can vary within the method.

Figure 45 illustrates an automatable process for designing a polynucleotide in parts. This process can be encoded into a computer program. (01) Input a polynucleotide sequence. (02) Calculate an oligonucleotide set that will assemble into the calculated polynucleotide. Processes such as those shown in Figures 29, 30 and 31 are possible ways of calculating the oligonucleotide sets. Varying the parameters within these methods will result in different oligonucleotide sets. (03) Determine whether any pair of incorrect annealing partners have an annealing temperature closer than a defined value (B) to the lowest annealing temperature between correct annealing partners. The aim of this step is to determine whether there are oligonucleotides that are likely to present a problem by annealing to incorrect partners during the assembly process. The value B is a parameter that can be varied in the method. If no, proceed to 08. If yes, proceed to 04. (04) Determine whether the length of the polynucleotide is less than N base pairs long. The value N is a parameter that can be varied in the method. If yes, then further division is undesirable, and the design criteria should be changed to allow ligase-based assembly instead of polymerase-based assembly, so proceed to 06. If no, proceed to 07. (05) Calculate an oligonucleotide set to assemble into the polynucleotide using a ligase-based method. One example of such a method is the process shown in Figure 26. (06) Divide the polynucleotide into two sub-sequences. There are many different ways to divide the polynucleotide. For example it can be divided between two residues such that the division separates two incorrect annealing partners with high annealing temperatures within the oligonucleotide set. The polynucleotide can also be divided randomly. (08) For each part of the polynucleotide, add overlap sequences or restriction sites that will be useful in joining the polynucleotide segments together; for example the type IIs restriction sites shown in Figure 25 may be added to the ends of the sequence in order to produce a complementary overlap between polynucleotide segments. In addition a recombinase-recognition sequence may be added to the end of each polynucleotide segment to facilitate independent cloning of each polynucleotide segment by a recombinase-based method. Since steps 03 to 08 are iterative, the original polynucleotide may be divided into more than 2 sub-sequences. It is important to ensure that the resultant polynucleotide segments can be joined, for example by overlap extension or restriction digestion and ligation, to form a single polynucleotide. Return to 02. (09) Count the number of polynucleotides. If the number is < P accept the design. If the number is > P reject the design and return to 01. Because the parameters for oligonucleotide design may be tuned differently, a repeat of the process may yield a different solution that may conform to the design criteria. Variation of the point of polynucleotide division can also give different results. The value P is a parameter that can vary within the method.

Figure 46 illustrates a sequence of a vector (SEQ ID NO: 39) lacking most common restriction sites, carrying a kanamycin resistance gene and a pUC origin of replication. Inserts may be cloned into the EcoRV site.

Figure 47 illustrates a sequence of a vector (SEQ ID NO: 40) lacking most common restriction sites, carrying a kanamycin resistance gene and a pUC origin of replication. Inserts carrying the appropriate ends, for example 5'-GGGGACAAGTTTGTACAAAAAAGCAGGCT-3' (SEQ ID NO: 41) at the 5' end and 5'-ACCCAGCTTTCTTGTACAAAGTGGTCCCC-3' (SEQ ID NO: 42) may be cloned into recombination sites in this vector using a commercially available lambda recombinase.

Figure 48 illustrates a sequence of a vector (SEQ ID NO: 43) lacking most common restriction sites, carrying a kanamycin resistance gene and a pUC origin of replication. This vector is useful for construction of genes in parts. Digestion of the vector shown in Figure 48 with the restriction enzyme BsaI excises a stuffer cassette sequence and leaves the vector with a TTTT overhang at one end and a CCCC overhang at the other end: aacggtctcCTTTTNNNNNN......NNNNNNccccagagaccgtt (SEQ ID NO: 44). Addition of the sequence 5'-GGTCTCCTTTT-3' (SEQ ID NO: 45) to the 5' end of the 5' part of a synthetic polynucleotide synthesized in parts and addition of the sequence 5'-CCCCAGAGACC-3' (SEQ ID NO: 46) to the 3' end of the 3' part of a synthetic polynucleotide synthesized in parts, followed by digestion of the parts with BsaI, will create overhangs complementary to those of the vector.

Figure 49 illustrates components of an integrated device for synthesizing polynucleotides in accordance with the present invention. One or more of these modules may be designed to perform some or all of the processes required to synthesize polynucleotides, thereby resulting in a partially or fully integrated device. The design module is primarily bioinformatic module that performs the following tasks: (1) polynucleotide design, for example design of a polynucleotide to encode a specific polypeptide, reduction or elimination of repeat elements, design of two or more polynucleotides for synthesis and joining to form a single polynucleotide, (2) oligonucleotide design, for example reduction or elimination of annealing regions in incorrect annealing partners, design of a "constant Tm" set, (3) select the assembly conditions appropriate for the designed oligonucleotide set, for example the annealing temperature, the number of cycles and time for each cycle, the use of polymerase or ligase-based assembly conditions. The oligonucleotide synthesis module performs the physical process of oligonucleotide synthesis. The input to this module is a set of oligonucleotide sequences that is provided by the design module. The oligonucleotide synthesis module could be an outside oligonucleotide vendor that receives the sequence information electronically either directly from the design module, or via an intermediary such as an ordering system. The oligonucleotide synthesis module could also be an oligonucleotide synthesis machine that is physically or electronically linked to and instructed by the design module. The oligonucleotide synthesis module could synthesize oligonucleotides using standard phosphoramidite chemistry, or using the modifications described here. The synthesis module performs the physical process of assembling oligonucleotides into a polynucleotide. The synthesis module receives informational input from the design module, to set the parameters and conditions required for successful assembly of the oligonucleotides. It also receives physical input of oligonucleotides from the oligonucleotide synthesis module. The synthesis module is capable of performing variable temperature incubations required by polymerase chain reactions or ligase chain reactions in order to assemble the mixture of oligonucleotides into a polynucleotide. For example the synthesis module can include a thermocycler based on Peltier heating and cooling, or based on microfluidic flow past heating and cooling regions. The synthesis module also performs the tasks of amplifying the polynucleotide, if necessary, from the oligonucleotide assembly reaction. The synthesis module also performs the task of ligating or recombining the polynucleotide into an appropriate cloning vector. The transformation module performs the following tasks: (1) transformation of the appropriate host with the polynucleotide ligated into a vector, (2) separation and growth of individual transformants (e.g. flow-based separations, plating-based separations), (3) selection and preparation of individual transformants for analysis. The analysis module performs the following tasks (1) determination of the sequence of each independent transformant, (2) comparison of the determined sequence with the sequence that was designed, and (3) identification of transformants whose sequence matches the designed sequence.

Figure 50 illustrates the design for an oligonucleotide reaction vessel using argon flow in accordance with the present invention. Vacuum filtration is replaced by an argon purging procedure with pressure regulated using a manometer. An optional stopcock regulates the argon input. Another optional stopcock for closing waste permits steps that require keeping liquid inside the funnel longer then one minute.

Figure 51 illustrates the design for a temperature-controlled reaction vessel in accordance with the present invention. A Peltier temperature control block is used to regulate the temperature of the reaction chambers to enhance differentiation in the rates of wanted reactions and unwanted side-reactions.

Figure 52 illustrates two designed P450 sequences. The first (A) (SEQ ID NO: 47) has an inverted repeat at the beginning. The second (B) (SEQ ID NO: 48) has a removal of that repeat by substitution of two nucleotides (i.e. choice of two alternative codons) increased expression between 5- and 10-fold.

## 5. DETAILED DESCRIPTION OF THE INVENTION

Before the present invention is described in detail, it is to be understood that this invention is not limited to the particular methodology, devices, solutions or apparatuses described, as such methods, devices, solutions or apparatuses can, of course, vary. It is also to be understood that the terminology used herein is for the purpose of describing particular embodiments only, and is not intended to limit the scope of the present invention.

### 5.1 DEFINITIONS

Use of the singular forms "a," "an," and "the" include plural references unless the context clearly dictates otherwise. Thus, for example, reference to "a polynucleotide" includes a plurality of polynucleotides, reference to "a substrate" includes a plurality of such substrates, reference to "a variant" includes a plurality of variants, and the like. Terms such as "connected," "attached," "linked," and "conjugated" are used interchangeably herein and encompass direct as well as indirect connection, attachment, linkage or conjugation unless the context clearly dictates otherwise. Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the invention. Where a value being discussed has inherent limits, for example where a component can be present at a concentration of from 0 to 100%, or where the pH of an aqueous solution can range from 1 to 14, those inherent limits are specifically disclosed. Where a value is explicitly recited, it is to be understood that values which are about the same quantity or amount as the recited value are also within the scope of the invention. Where a combination is disclosed, each subcombination of the elements of that combination is also specifically disclosed and is within the scope of the invention. Conversely, where different elements or groups of elements are individually disclosed, combinations thereof are also disclosed. Where any element of an invention is disclosed as having a plurality of alternatives, examples of that invention in which each alternative is excluded singly or in any combination with the other alternatives are also hereby disclosed; more than one element of an invention can have such exclusions, and all combinations of elements having such exclusions are hereby disclosed.

Unless defined otherwise herein, all technical and scientific terms used herein have the same meaning as commonly understood by one of ordinary skill in the art to which this invention belongs. Singleton, et al., Dictionary of Microbiology and Molecular Biology, 2nd Ed., John Wiley and Sons, New York (1994), and Hale & Marham, The Harper Collins Dictionary of Biology, Harper Perennial, NY, 1991, provide one of skill with a general dictionary of many of the terms used in this invention. Although any methods and materials similar or equivalent to those described herein can be used in the practice or testing of the present invention, the preferred methods and materials are described. Unless otherwise indicated, nucleic acids are written left to right in 5' to 3' orientation; amino acid sequences are written left to right in amino to carboxy orientation, respectively. The terms defined immediately below are more fully defined by reference to the specification as a whole.

The terms "polynucleotide," "oligonucleotide," "nucleic acid" and "nucleic acid molecule" and "gene" are used interchangeably herein to refer to a polymeric form of nucleotides of any length, and may comprise nbonucleotides, deoxyribonucleotides, analogs thereof, or mixtures thereof. This term refers only to the primary structure of the molecule. Thus, the term includes triple-, double- and single-stranded deoxyribonucleic acid ("DNA"), as well as triple-, double- and single-stranded ribonucleic acid ("RNA"). It also includes modified, for example by alkylation, and/or by capping, and unmodified forms of the polynucleotide. More particularly, the terms "polynucleotide," "oligonucleotide," "nucleic acid" and "nucleic acid molecule" include polydeoxyribonucleotides (containing 2-deoxy-D-ribose), polyribonucleotides (containing D-ribose), including tRNA, rRNA, hRNA, siRNA and mRNA, whether spliced or unspliced, any other type of polynucleotide which is an N- or C-glycoside of a purine or pyrimidine base, and other polymers containing nonnucleotidic backbones, for example, polyamide (*e.g.*, peptide nucleic acids ("PNAs")) and polymorpholino (commercially available from the Anti-Virals, Inc., Corvallis, Oreg., as Neugene) polymers, and other synthetic sequence-specific nucleic acid polymers providing that the polymers contain nucleobases in a configuration which allows for base pairing and base stacking, such as is found in DNA and RNA. There is no intended distinction in length between the terms "polynucleotide," "oligonucleotide," "nucleic acid" and "nucleic acid molecule," and these terms are used interchangeably herein. These terms refer only to the primary structure of the molecule. Thus, these terms include, for example, 3'-deoxy-2', 5'-DNA, oligodeoxyribonucleotide N3' P5' phosphoramidates, 2'-O-alkyl-substituted RNA, double- and single-stranded DNA, as well as double- and single-stranded RNA, and hybrids thereof including for example hybrids between DNA and RNA or between PNAs and DNA or RNA, and also include known types of modifications, for example, labels, alkylation, "caps," substitution of one or more of the nucleotides with an analog, internucleotide modifications such as, for example, those with uncharged linkages (*e.g.*, methyl phosphonates, phosphotriesters, phosphoramidates, carbamates, *etc.*), with negatively charged linkages (*e.g.*, phosphorothioates, phosphorodithioates, *etc.),* and with positively charged linkages (*e.g.*, aminoalkylphosphoramidates, aminoalkylphosphotriesters), those containing pendant moieties, such as, for example, proteins (including enzymes (*e.g.* nucleases), toxins, antibodies, signal peptides, poly-L-lysine, *etc.*), those with intercalators (*e.g.*, acridine, psoralen, *etc.*), those containing chelates (of, *e.g.*, metals, radioactive metals, boron, oxidative metals, *etc.*), those containing allcylators, those with modified linkages (*e.g.*, alpha anomeric nucleic acids, *etc.*), as well as unmodified forms of the polynucleotide or oligonucleotide.

Where the polynucleotides are to be used to express encoded proteins, nucleotides that can perform that function or which can be modified (*e.g.*, reverse transcribed) to perform that function are used. Where the polynucleotides are to be used in a scheme that requires that a complementary strand be formed to a given polynucleotide, nucleotides are used which permit such formation.

It will be appreciated that, as used herein, the terms "nucleoside" and "nucleotide" will include those moieties which contain not only the known purine and pyrimidine bases, but also other heterocyclic bases which have been modified. Such modifications include methylated purines or pyrimidines, acylated purines or pyrimidines, or other heterocycles. Modified nucleosides or nucleotides can also include modifications on the sugar moiety, *e.g.*, where one or more of the hydroxyl groups are replaced with halogen, aliphatic groups, or is functionalized as ethers, amines, or the like.

Standard A-T and G-C base pairs form under conditions which allow the formation of hydrogen bonds between the N3--H and C4-oxy of thymidine and the NI and C6--NH2, respectively, of adenosine and between the C2-oxy, N3 and C4--NH2, of cytidine and the C2--$NH_2$, N'--H and C6-oxy, respectively, of guanosine. Thus, for example, guanosine (2-amino-6-oxy-9-.beta.-D-ribofuranosyl-purine) may be modified to form isoguanosine (2-oxy-6-amino-9-.beta.-D-ribofuranosyl-purine). Such modification results in a nucleoside base which will no longer effectively form a standard base pair with cytosine. However, modification of cytosine (1-.beta.-D-ribofuranosyl-2-oxy-4-amino-pyrimidine) to form isocytosine (1-.beta.-D-ribofuranosyl-2-amino-4-oxy-pyrimidine-) results in a modified nucleotide which will not effectively base pair with guanosine but will form a base pair with isoguanosine (U.S. Pat. No. 5,681,702 to Collins et al., hereby incorporated by reference in its entirety). Isocytosine is available from Sigma Chemical Co. (St. Louis, Mo.); isocytidine may be prepared by the method described by Switzer et al. (1993) Biochemistry 32:10489-10496 and references cited therein; 2'-deoxy-5-methyl-isocytidine may be prepared by the method of Tor et al., 1993, J. Am. Chem. Soc. 115:4461-4467 and references cited therein; and isoguanine nucleotides may be prepared using the method described by Switzer *et al.*, 1993, supra, and Mantsch et al., 1993, Biochem. 14:5593-5601, or by the method described in U.S. Pat. No. 5,780,610 to Collins et al., each of which is hereby incorporated by reference in its entirety. Other nonnatural base pairs may be synthesized by the method described in Piccirilli et al., 1990, Nature 343:33-37, hereby incorporated by reference in it entirety, for the synthesis of 2,6-diaminopyrimidine and its complement (1-methylpyrazolo-[4,3]pyrimidine-5,7-(4H,6H)-dione. Other such modified nucleotidic units which form unique base pairs are known, such as those described in Leach et al. (1992) J. Am. Chem. Soc. 114:3675-3683 and Switzer et al., supra.

The phrase "DNA sequence" refers to a contiguous nucleic acid sequence. The sequence can be either single stranded or double stranded, DNA or RNA, but double stranded DNA sequences are preferable. The sequence can be an oligonucleotide of 6 to 20 nucleotides in length to a full length genomic sequence of thousands or hundreds of thousands of base pairs.

The term "protein" refers to contiguous "amino acids" or amino acid "residues." Typically, proteins have a function. However, for purposes of this invention, proteins also encompass polypeptides and smaller contiguous amino acid sequences that do not have a functional activity. The functional proteins of this invention include, but are not limited to, esterases, dehydrogenases, hydrolases, oxidoreductases, transferases, lyases, ligases, receptors, receptor ligands, cytokines, antibodies, immunomodulatory molecules, signalling molecules, fluorescent proteins and proteins with insecticidal or biocidal activities. Useful general classes of enzymes include, but are not limited to, proteases, cellulases, lipases, hemicellulases, laccases, amylases, glucoamylases, esterases, lactases, polygalacturonases, galactosidases, ligninases, oxidases, peroxidases, glucose isomerases, nitrilases, hydroxylases, polymerases and depolymerases. In addition to enzymes, the encoded proteins which can be used in this invention include, but are not limited to, transcription factors, antibodies, receptors, growth factors (any of the PDGFs, EGFs, FGFs, SCF, HGF, TGFs, TNFs, insulin, IGFs, LIFs, oncostatins, and CSFs), immunomodulators, peptide hormones, cytokines, integrins, interleukins, adhesion molecules, thrombomodulatory molecules, protease inhibitors, angiostatins, defensins, cluster of differentiation antigens, interferons, chemokines, antigens including those from infectious viruses and organisms, oncogene products, thrombopoietin, erythropoietin, tissue plasminogen activator, and any other biologically active protein which is desired for use in a clinical, diagnostic or veterinary setting. All of these proteins are well defined in the literature and are so defined herein. Also included are deletion mutants of such proteins, individual domains of such proteins, fusion proteins made from such proteins, and mixtures of such proteins; particularly useful are those which have increased half-lives and/or increased activity.

"Polypeptide" and "protein" are used interchangeably herein and include a molecular chain of amino acids linked through peptide bonds. The terms do not refer to a specific length of the product. Thus, "peptides," "oligopeptides," and "proteins" are included within the definition of polypeptide. The terms include polypeptides containing in co- and/or post-translational modifications of the polypeptide made in vivo or in vitro, for example, glycosylations, acetylations, phosphorylations, PEGylations and sulphations. In addition, protein fragments, analogs (including amino acids not encoded by the genetic code, e.g. homocysteine, ornithine, p-acetylphenylalanine, D-amino acids, and creatine), natural or artificial mutants or variants or combinations thereof, fusion proteins, derivatized residues (*e.g*. alkylation of amine groups, acetylations or esterifications of carboxyl groups) and the like are included within the meaning of polypeptide.

"Amino acids" or "amino acid residues" may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

The terms "codon usage table" or "codon bias table" are used interchangeably to describe a table which correlates each codon that may be used to encode a particular amino acid, with the frequencies with which each codon is used to encode that amino acid in a specific organism, or within a specified class of genes within that organism. Many examples of such tables can be found at http://www.kazusa.or.jp/codon/http://www.kazusa.or.jp/codon/, which is hereby incorporated by reference. A "hybrid codon usage table" or "hybrid codon bias table" can also be constructed by combining two or more codon usage tables according to a variety of possible rules, some of which will be enumerated in more detail elsewhere in this document.

The terms "threshold" or "cutoff" are used interchangeably to refer to the minimum allowable frequency in using a codon bias table. For example if a threshold or cutoff of 10% is set for a codon bias table, then no codons that are used less frequently than 10% of the time are accepted for subsequent polynucleotide design and synthesis. Thresholds may be expressed as percentages (*e.g.*, the percentage of time that an organism or class of genes within an organism uses a specified codon to encode an amino acid) or as frequencies (0.1 would be the frequency of codon usage that could also be expressed as 10%).

The term "splice variant" or "splicing variant" refers to the different possible RNA products that may be produced by a cell that transcribes a segment of DNA to produce an RNA molecule. These different products result from the action of the RNA splicing and transportation machinery, whose specificity of function differs from cell to cell, causing different signals within an RNA sequence to be recognized as intron donor and acceptor sites, and leading to different RNA products.

The term "expression system" refers to any in vivo or in vitro biological system that is used to produce one or more protein encoded by a polynucleotide.

The term "annealing temperature" or "melting temperature" or "transition temperature" refers to the temperature at which a pair of nucleic acids is in a state intermediate between being fully annealed and fully melted. The term refers to the behavior of a population of nucleic acids: the "annealing temperature" or "melting temperature" or "transition temperature" is the temperature at which 50% of the molecules are annealed and 50% are separate. Annealing temperatures can be determined experimentally. There are also methods well know in the art for calculating these temperatures.

The term "constant Tm set" refers to a set of nucleic acid sub-sequences, designed such that the annealing temperature of each member of the set to its reverse complement sequence are within a very narrow range. Typically such a set is created by sequentially adding nucleotides to a sequence until a defined annealing temperature has been reached.

## 5.2 SYNTHESIS OF OLIGONUCLEOTIDES

### 5.2.1 REMOVAL OF NON-TRITYLATED TRUNCATED CHAINS

Oligonucleotides that are useful for assembly of polynucleotides and other demanding applications must meet different performance criteria from oligonucleotides for standard applications. Frequently for high-quality applications only relatively small amounts of oligonucleotides are required: preferably less than 100 pmol of oligonucleotide, more preferably less than 50 pmol of oligonucleotide, more preferably less than 10 pmol of oligonucleotide and more preferably less than 5 pmol of oligonucleotide. The purity is important, with oligonucleotides containing internal deletions or apurinic residues being particularly harmful to many applications. The following are a list of modifications to the current generally used phosphoramidite-based chemistry for oligonucleotide synthesis. These modifications improve the quality of oligonucleotides for subsequent applications including but not limited to assembly into polynucleotides.

The standard oligonucleotide coupling process described in Gait's practical handbook, Gait, 1984, Practical approach series, xiii, 217, hereby incorporated by reference is shown in Figure 1. Despite the high efficiency of phosphoramidite chemistry, chain elongation is not quantitative. The standard capping process, stepwise acetylation of the unphosphitylated chains, Beaucage and Radhakrishnan, 1992, Tetrahedron, 48, 2223-2311, hereby incorporated by reference in its entirety, using acetic anhydride, 2,6-lutidine and N-methylimidazole in tetrahydrofuran is implemented to prevent further extension of oligonucleotide chains that do not incorporate the last base. Oligonucleotides synthesized with this capping step should thus contain a ladder of products corresponding to the extension failures at each cycle that are then capped. If this capping step is omitted, extension failures in one cycle are expected to extend in the subsequent cycle resulting in a large n-1 peak and much reduced peaks for n-2, n-3 *etc.* as illustrated in Figure 2A. In contrast with this expectation, it has been determined that when a 15-mer of polydezoxythymidine is synthesized on CPG-(2000A) without capping, there is a ladder of products that more closely resembled the products expected in the presence of capping (Figure 2B). This shows that the failure of growing oligonucleotide chains to extend quantitatively results in part from a sub-population of chains that become non-reactive. Oligonucleotide packing produces populations that (1) grow as desired, (2) are permanently trapped by neighboring chains or (3) permanently protected by neighboring trityl groups resulting in n-1, n-2, n-3 *etc.* byproducts, or (4) are nonoxidized and generate n-1 byproducts.

Oligonucleotides that are not extended for one or more cycles, and that then reenter the active pool are even more deleterious to ultimate function than oligonucleotides that are truncated but otherwise correct in sequence. The former class of oligonucleotides contains internal deletions of one or more base; incorporation of such deletions is a very serious limitation, for example in the assembly of polynucleotides from oligonucleotides. It is therefore important to ensure that an unextended oligonucleotide chain does not reenter the reactive pool. This is the intention of the capping step, but the experiments summarized in Figure 2 show that if oligonucleotide chains become unavailable for extension for multiple cycles they may also be unavailable for the capping reaction.

Oligonucleotide packing produces truncated n-1, n-2, n-3 *etc.* byproducts as a result of trapping by neighboring chains or protected by neighboring trityl groups. These byproducts are not themselves tritylated because the chain extension failure is a failure to extend that follows the detritylation step at the beginning of the cycle. Such permanently terminated chains will be truncated but otherwise correct in sequence. Short truncated oligonucleotides can be problematic. For example, they are problematic when using them to synthesize genes containing repetitive sequences. Short truncated oligonucleotides can, in principle, be removed using the enzyme phosphodiesterase, though it has previously been reported that the DMT-protection is unstable under phosphodiesterase digestion conditions. See, Urdea and Horn, 1986, Tetrahedron Lett 27, 2933-2936, which is hereby incorporated by reference. As illustrated in Figure 3, it has been determined that the instability of the trityl protection group is primarily a function of pH. The protection is stable for 60 hours at pH 7 (Figure 3B). Although the oligonucleotide hydrolysis activity of phosphodiesterase decreases at higher pHs (Figure 4), 1 nmol of 20mer can be completely removed by 0.1 U of enzyme at 25°C at pH 7.0 (Figure 4F).

Accordingly, there is provided a method of treating a synthetic oligonucleotide product. In the method, synthetic oligonucleotide product is cleaved from a solid support in the absence of a final detritylation step. The cleaved oligonucleotide product is then treated with a phosphodiesterase or a pyrophosphatase at a pH greater than 5.5. In some embodiments the cleaved oligonucleotide product is alternatively treated with a phosphodiesterates or a pyrophosphatase at a pH greater than 5.6, or a pH greater than 5.7, or a pH greater than 5.8, or a pH greater than 5.9, or a pH greater than 6.0, or a pH greater than 6.1, or a pH greater than 6.2, or a pH greater than 6.3, or a pH greater than 6.4, or a pH greater than 6.5. In some embodiments the treating step is performed for between 20 minutes and 24 hours, between 25 minutes and 2 hours, less than 5 hours or between 18 minutes and 24 minutes.

Any pyrophosphatase or phosphodiesterase can be used to accomplish such enzymatic cleavage. For example, any pyrophosphatase or phosphodiesterase described by Bollen et al., 2000, Critical Reviews in Biochemistry and Molecular Biology 35, 393-432, which is hereby incorporated by reference in its entirety, can be used. Nucleotide pyrophosphatases/phosphodiesterases (NPPs) release nucleoside 5'-monophosphates from nucleotides and their derivatives. They exist both as membrane proteins, with an extracellular active site, and as soluble proteins in body fluids. NPPs include, but are not limited to the mammalian ecto-enzymes NPP1 (PC-1), NPP2 (autotaxin) and NPP3 (B10; gp130RB13-6). These are modular proteins consisting of a short N-terminal intracellular domain, a single transmembrane domain, two somatomedin-B-like domains, a catalytic domain, and a C-terminal nuclease-like domain. The catalytic domain of NPPs is conserved from prokaryotes to mammals and shows structural and catalytic similarities with the catalytic domain of other phospho-/sulfo-coordinating enzymes such as alkaline phosphatases. Hydrolysis of pyrophosphate/phosphodiester bonds by NPPs occurs via a nucleotidylated threonine. NPPs are also known to auto(de)phosphorylate this active-site threonine, a process accounted for by an intrinsic phosphatase activity, with the phosphorylated enzyme representing the catalytic intermediate of the phosphatase reaction.

In some cases, the method further comprises detritylating a tritylated oligonucleotide in the oligonucleotide product after the treating step. In some embodiments, the method further comprises physically separating a tritylated oligonucleotide from a non-tritylated oligonucleotide in the cleaved oligonucleotide product, where the tritylated oligonucleotide is a full length oligonucleotide; and detritylating the tritylated oligonucleotide.

Phosphodiesterase can selectively remove oligonucleotides lacking a 5'-trityl group. Figure 5 shows that phosphodiesterase does not cleave fully unprotected oligonucleotides still bound to the CPG support (Figure 5A). This is not surprising, since the target population of untritylated oligomers is inaccessible even to small chemical reagents. In contrast, when the trityl protected oligomers are cleaved from CPG, phosphodiesterase treatment removes most of truncated byproducts (< n-2) (compare Figure 5B and C).

Capped and uncapped oligonucleotides can be separated from the full-length tritylated product by HPLC (compare traces A and B in Figure 6). Treating tritylated oligonucleotides with phosphodiesterase and then performing a subsequent reverse phase separation to separate the tritylated (full-length) from the non-tritylated (truncated) oligonucleotides allows simultaneous purification of a pool of oligonucleotides. This approach removes the major limitation of subsequent hydrophobic purification by increasing the difference in retention time between fractions of truncated and desired products. This procedure provides a format that is readily amenable to high throughput implementation.

### 5.2.2 ELIMINATING SOURCES OF INTERNALLY DELETED CHAINS BY IMPROVED CAPPING

The second class of truncation products shown in Figure 2 are oligonucleotides that failed to add a base in one or more cycles of elongation, but were then able to reenter a subsequent cycle and continue extending. These truncated but growing oligonucleotides are tritylated like the full-length oligonucleotides and correspond to the small population of oligomers that are resistant to phosphodiesterase treatment in Figure 4C. In contrast to the physically trapped truncated oligonucleotides, these chains are active participants in the ongoing synthesis, they will have internal deletions corresponding to the cycle(s) in which they did not participate, and they will also have a 5' trityl group.

The two different classes of extension failures are shown in Figure 7. Homo-tetramers of each base are synthesized and cleaved from the CPG support without detritylation. Tritylated and non-tritylated oligomers are then separated by HPLC. Consistent with a physical trapping explanation, a larger sub-population of untritylated truncated chains and truncated byproducts is observed for more sterically hindered nucleotides (dC, dA, and dG). Tritylated truncated chains corresponding to oligonucleotides lacking one or more addition but still active participants in the extension cycle are also observed.

While phosphodiesterase is a suitable treatment for removing the physically inaccessible (trapped) chain extension failures, an alternative stratagem can be used to eliminate this second class of chemically available failures. There are two ways to address this population: permanent capping to prevent further extension of unreacted chains (this capping is a standard part of conventional oligonucleotide synthesis), and optimizing the reaction steps to maximize the efficiency of nucleotide addition.

Any unextended chains that are physically accessible should be prevented from undergoing further extension to ensure optimal quality for gene synthesis. Different capping methods have been used to prevent further cycles of oligonucleotide polymerization on unextended chains. See, for example, Matteucci and Caruthers, 1981, J Am Chem Soc, 103, 3185-3191; Eadie and Davidson, 1987, Nucleic Acids Res 15, 8333-49; Chaix et al., 1989, Tetrahedron Lett 30, 71-74; and Yu et al., 1994, Tetrahedron Lett 34, 8565-8568, each of which is hereby incorporated by reference in its entirety. Early protocols used dimethylaminopyridine (DMAP) catalyzed capping after oxidation. These methods were subsequently replaced with capping by acetic anhydride and N-methylimidasole (NMI) in tetrahydrofuran (THF) before the oxidation step. This change was introduced to reduce the oxidation and acetylation of guanidine residues.

The efficiency of capping using acetic anhydride in presence of N-methylimidazole (NMI), phenoxyacetic anhydride ($Pac_2O$) and dimethoxy N,N-diisopropyl phosphoramidite (DMPA) was compared. It was determined that a capping time of between 1 and 5 minutes is required for quantitative capping using acetic anhydride (Figure 8A), while

capping with $Pac_2O$ was essentially complete after 30 seconds (Figure 8B) and capping with DMPA took only 15 seconds for completion (Figure 8C). This result was confirmed by using different capping mixtures for 15 seconds (Figure 9). Quantitative capping was found only with N,N-dimethylaminopyridine (DMAP) (Figure 9F) although a 1.5:1.5:7 mixture of N-methylimidazole : 2,6-lutidine : toluene gave quantitative capping after a minute (Figure 9E).

Complete elimination of oligonucleotides that have failed to react in any other cycle from further cycles is important to quality for gene synthesis. Therefore DMAP capping at least for nucleotides A, C and T is proposed. The $O^6$-position of guanidine modification problem caused by this capping reagent can be efficiently avoided by engineering the nucleic acid synthesis instrument with the capability to perform oxidation before or after capping, by applying the full protection strategy, or by combining both approaches $Ac_2O$-NMI capping before oxidation and $Ac_2O$-DMAP capping after oxidation.

From Figures 8 and 9 it is clear that DMAP capping after oxidation provides superior capping protection to N-methylimidazole capping before oxidation. Figure 6 also shows that the standard capping step before oxidation reduces the number of truncated oligonucleotides relative to an uncapped protocol (Figure 61A, 2A). Moving the capping step to follow oxidation reduces the levels of truncated oligonucleotides further (Figure 6 2A, 3A), particularly noticeable with the reduced levels of tritylated n-1 product (i.e., T8). Complete elimination from further cycles of oligonucleotides that have failed to react in any other cycle is absolutely critical to oligonucleotide quality. Using DMAP capping at least for nucleotides A, C and T will therefore improve overall oligonucleotide quality. A problem has been reported with modification of the $O^6$-position of guanidine caused by this capping reagent. See Eadie and Davidson, 1987, Nucleic Acids Res 15, 8333-49; Pon et al., 1985, Tetrahedron Lett. 26, 2525-2528; Pon et al., 1985, Nucleic Acids Res 13, 6447-65; and Pon et al., 1986, Nucleic Acids Res 14, 6453-70, each of which is hereby incorporated by reference in its entirety. This can be efficiently avoided by either performing capping before oxidation for addition of dG but after oxidation for A, C and T. Alternatively growing chains may be $Ac_2O$-NMI capped before oxidation and $Ac_2O$-DMAP capped after oxidation.

Accordingly, there is provided a method of synthesizing an oligonucleotide comprising an $n^{th}$ nucleotide and an $n+1^{th}$ nucleotide, where the $n^{th}$ nucleotide and the $n+1^{th}$ nucleotide are coupled to each other in the oligonucleotide. In the method, the $n^{th}$ nucleotide is detritylated when the $n^{th}$ nucleotide is a terminal nucleotide of a nucleic acid attached to a solid support. The $n+1^{th}$ nucleotide is coupled to the $n^{th}$ nucleotide. The nucleic acid attached to the solid support is then exposed with a first capping reagent, prior to an oxidation step, when the $n+1^{th}$ nucleotide is deoxyguanosine. The oxidation step is then performed. The nucleic acid is attached to the solid support with a second capping reagent, after the oxidation step, when the $n+1^{th}$ nucleotide is deoxycytosine, deoxythymidine or deoxyadenosine. In some embodiments, the oligonucleotide comprises a plurality of nucleotides and the aforementioned steps are repeated for all or a portion of the nucleotides in the plurality of nucleotides, thereby synthesizing the oligonucleotide. In some embodiments, the method further comprises separating the nucleic acid from the solid support thereby deriving the oligonucleotide and then separating the oligonucleotide from one or more truncated by-products. In some embodiments, the first capping reagent is N-methylimidazole or the like and the second capping reagent is N,N-dimethylaminopyridine or the like. In some embodiments the oligonucleotide comprises between 10 nucleotides and 100 nucleotides, between 5 nucleotides and 50 nucleotides, or between 3 nucleotides and 40 nucleotides. In some embodiments, the nucleic acid attached to the solid has a length of one nucleotide or greater.

Another aspect provides a method of synthesizing an oligonucleotide comprising an $n^{th}$ nucleotide and an $n+1^{th}$ nucleotide, where the $n^{th}$ nucleotide and the $n+1^{th}$ nucleotide are adjacent to each other in the oligonucleotide. IN the method, the $n^{th}$ nucleotide is detritylated when the $n^{th}$ nucleotide is a terminal nucleotide of a nucleic acid attached to a solid support. The $n+1^{th}$ nucleotide is then coupled with the $n^{th}$ nucleotide. The nucleic acid attached to the solid support is then exposed with a first capping reagent, prior to an oxidation step. Then an oxidation step is performed. After the oxidation step, the nucleic acid attached to the solid support is exposed with a second capping reagent. In some embodiments the oligonucleotide comprises a plurality of nucleotides and the aforementioned steps are repeated for all or a portion of the nucleotides in the plurality of nucleotides. In some embodiments, the method further comprises separating the nucleic acid from the solid support, thereby deriving the oligonucleotide. In some embodiments, the oligonucleotide is separated from one or more truncated by-products. In some embodiments, the first capping reagent is N-methylimidazole and the second capping reagent is N,N-dimethylaminopyridine. In some embodiments, oligonucleotide comprises between 10 and 100 nucleotides, between 5 nucleotides and 50 nucleotides, or between 3 nucleotides and 40 nucleotides. In some embodiments, the nucleic acid attached to the solid has a length of one nucleotide or greater.

### 5.2.3 ELIMINATING SOURCES OF INTERNALLY DELETED CHAINS BY IMPROVED OXIDATION

Two strategies are available to prevent extension of oligonucleotide chains that have failed to add a base in one or more cycle. One is to efficiently block further extension of unextended chains. This is why it has been proposed here to switch to the superior capping agent DMAP. The second stratagem is to maximize the coupling of bases.

The data in Figures 6, 7, 8 and 9 present a dilemma. Even if coupling and capping were each only 99% efficient, statistically only 1% of 1% of chains (*i.e.* 1 in 10,000) should fail in both reactions. The resulting internal deletion within an oligonucleotide should therefore be extremely rare. In practice, however, these deletions are seen at a rate about 30-fold higher: synthetic genes made from commercial oligonucleotides frequently contain between 2 and 5 internal deletions per 1,000 bases. Systematic exploration of reaction conditions to optimize coupling efficiency, revealed that the assay for incomplete oxidation was also measuring exactly the kind of error for which avoidance was sought.

Letsinger's method of nucleotidic phosphite triester oxidation has been the standard chemistry for almost thirty years. See, Letsinger et al., 1975, J Am Chem Soc, 3278-3279, which is hereby incorporated by reference. However, there is no clear consensus in the literature for the iodine and/or water ratios, type of base for iodic acid neutralization or duration of reaction. Several different oxidation conditions were tested by synthesizing a dimmer, then detritylating, cleaving and analyzing by HPLC. Incompletely oxidized phosphate bonds were cleaved by the detritylating conditions, resulting in monomer. Dimer stability was used as a measure of the completeness of oxidation (Figure 10).

Using 0.1M iodine in THF:2,6-lutidine:Water 40:10:1, oxidation was only 82% complete after 1 minute and 98% after 10 minutes (Figure 10A-D). In comparison a 10-fold increase in water concentration resulted in 93% oxidation in just 15 seconds (compare Figure 10E and F).

Loss of an incompletely oxidized base at the detritylation step would result in exactly the kind of internal deletions that we wish to avoid in oligonucleotides to be used as building blocks for synthetic genes (see Figure 11). It is thus important that oxidation be as complete as possible. Several different reagents were evaluated in a 15 second oxidation test (Figure 12). From these test it was found that the most efficient oxidation reagent was freshly prepared iodine/water oxidizer (Figure 12C).

An aspect provides a method of synthesizing an oligonucleotide comprising an $n^{th}$ nucleotide and an $n+1^{th}$ nucleotide, where the $n^{th}$ nucleotide and the $n+1^{th}$ nucleotide are coupled to each other in the oligonucleotide. The method comprises detritylating the $n^{th}$ nucleotide when the $n^{th}$ nucleotide is a terminal nucleotide of a nucleic acid attached to a solid support. Then the $n+1^{th}$ nucleotide is coupled with the $n^{th}$ nucleotide. The nucleic acid is then exposed to a capping reagent prior to an exposing step. The nucleic acid is then exposed to an oxidizing solution comprising a plurality of components, where a first component and a second component in the plurality of components are mixed together less than twelve hours prior to exposing the nucleic acid to the oxidizing solution. In some embodiments, the oligonucleotide comprises a plurality of nucleotides and the aforementioned steps are repeated for all or a portion of the nucleotides in the plurality of nucleotides, thereby synthesizing said oligonucleotide. In some embodiments, the method further comprises separating the nucleic acid from the solid support, thereby deriving the oligonucleotide and then separating the oligonucleotide from one or more truncated by-products. In some embodiments, the first component is iodine. In some embodiments, the iodine concentration in the oxidizing solution is between 0.05M and 0.5M. In some embodiments, the second component is THF:2,6-lutidine:water 4:1:1. In some embodiments, the method further comprise exposing the nucleic acid to a capping reagent after the exposing step.

### 5.2.4 A COMBINED PROTOCOL FOR IMPROVED OLIGONCULEOTIDE QUALITY

By combining the modifications to the standard procedure, new oligonucleotide synthesis procedures have been designed as illustrated in Figure 13. The main features of this protocol are (1) oxidation is performed with freshly prepared iodine in THF:2,6-lutidine:water (4:4:1); (2) a second capping step is performed after oxidation using acetic anhydride and DMAP; (3) oligonucleotides are cleaved and deprotected in gaseous ammonia with the final trityl group in place; (4) truncated and cleaved depurinated olignucleotides are optionally digested with phosphodiesterase and (5) there is an optional trityl-based HPLC purification prior to detritylation.

### 5.3 SYNTHESIS OF OLIGONUCLEOTIDES ON NON-POROUS SOLID SUPPORTS

The major applications for commercially synthesized oligonucleotides are as PCR primers or DNA micro-array probes, neither of which demand the same level of quality as building blocks for synthetic genes. Current commercial synthesizers use controlled-pore glass as a support for oligonucleotide synthesis, the design of such reaction vessels has already reached the minimal reaction volume (-45 μl) at which a two component reaction and resin can still form a homogeneous suspension without sticking to the walls and leaking out from the supported filter. Porous support materials have the disadvantage that they may trap reagents, chemicals may leak during the reaction and there may be unpredictable plugging and unplugging of pores by gases and micro particles. A non-porous glass support will reduce or eliminate these problems, and allow smaller reaction volumes for oligonucleotide synthesis (~5ul) together with the high quality needed for subsequent polynucleotide assembly.

Non-porous surfaces suitable as substrates on which to perform oligonucleotide synthesis include polished Quartz (100% $SiO_2$) or Pyrex (81% $SiO_2$) discs or plates from Chemglass with an exposed surface area of less than 1000 $mm^2$, preferably less than 300 $mm^2$, more preferably less than 100 $mm^2$.

### 5.3.1 SURFACE PREPARATION

A freshly exposed glass surface is known to rapidly increase in surface hydrophobicity, a tendency that has been ascribed to adsorption of impurities from the air. See Petri et al., 1999, Langmuir 15, 4520-4523, which is hereby incorporated by reference in its entirety. By measuring the contact angle of a freshly broken glass surface with a water drop it was determined that when placed in a vacuum the surface becomes more hydrophobic even more rapidly than in air (Figure 15A and B). The most dramatic thermodynamically driven stabilization, by formation of new Si-O-Si bonds, occurs within first hour. Broken bond stabilization by air keeps the surface hydrophilic much longer. Using freshly polished and activated glass surfaces for derivatization will thus minimize reproducibility problems (Figure 15C).

### 5.3.2 SURFACE ACTIVATION

Glass surfaces are activated by hydrolysis of Si-O-Si bonds, typically by boiling the glass in inorganic acid. See, Allenmark, 1988, Ellis Horwood series in analytical chemistry 224. Such a method is not easy to apply to manufacturing. However, it has been determined herein that treatment of glass with 50% sodium hydroxide works as a suitable alternative (Figure 16E).

### 5.3.3 SURFACE DERIVATIZATION AND LOADING

To localize oligonucleotide synthesis to the flat end of the rod the rod sides can be chemically protected, for example with trimethylsilane (Figure 17A). Silanization can be monitored on a freshly activated glass surface by measuring changes in the contact angle of a 2 μl water drop (Figure 17B). Once the sides of the rod are silanized, the end can be derivatized, for example with aminopropylsilane. Longer exposure of the surface to aminopropylsilane, or use of aged aminopropylsilane produces a more hydrophobic surface (Figure 17C and D) which is less useful. A short derivatization step was selected because incomplete or irreproducible rod derivatization can cause low coupling efficiencies (Figure 18A).

The derivatized surface can be loaded with functional groups for oligonucleotide synthesis such as dimethoxytritylthymidine succinate to load the first nucleotide onto the surface.

Attachment of the first nucleotide can be performed by 2-(1-H-benzotriazole-1-yl)-1,1,2,2-tetramethyluronium hexafluorophosphate (HBTU), 2000 Novabiochem catalog, for example, by injecting 5-10 μl drops of reagents on top of vertically installed rods (4 mm diameter). Preferably, the rod walls are freshly treated with trimethylchlorosilane to prevent drops from slipping down. Alternatively, the reaction area can be oriented downwards.

Synthesized oligonucleotides can be released from the end of glass reaction pins by gaseous ammonia, which effects a rapid, mild deprotection and cleavage of oligodesoxyribonucleotides from the support. Under these conditions the rate of isobutyril-dG deprotection is comparable with the removal of 4-(tert-butyl)-phenoxyacetyl group by aqueous ammonia at room temperature. See, for example, Boal et al., 1996, Nucleic Acids Res 24, 3115-7, which is hereby incorporated by reference in its entirety.

To reduce or eliminate the fraction of oligonucleotides with low reactivity towards polymerization on non-porous supports, oligonucleotide chains may be supported by glass rods derivatized with polyethylene glycol or polypropylene rods functionalized by ammonium plasma. See, for example, Chu et al., 1992, Electrophoresis 13, 105-14, which is hereby incorporated by reference in its entirety.

## 5.4 DEVICES FOR OLIGONUCLEOTIDE SYNTHESIS

### 5.4.1 REACTOR DESIGN

Oligonucleotides that are useful for assembly of polynucleotides must meet higher performance criteria than oligonucleotides for many other applications. Only relatively small amounts of oligonucleotides are required: preferably less than 10 pmol of oligonucleotide and more preferably less than 5 pmol of oligonucleotide. Purity is important, and oligonucleotides containing internal deletions or apurinic residues are particularly deleterious.

The major applications for commercially synthesized oligonucleotides are as PCR primers or DNA micro-array probes, neither of which demands the same level of quality as building blocks for synthetic genes. Current commercial synthesizers use controlled-pore glass as a support for oligonucleotide synthesis, the design of such reaction vessels has already reached the minimal reaction volume (-45 μl) at which a two component reaction and resin can still form a homogeneous suspension without sticking to the walls and leaking out from the supported filter. Porous support materials have the disadvantage that they may trap reagents, chemicals may leak during the reaction and there may be unpredictable plugging and unplugging of pores by gases and microparticles. A non-porous glass support will reduce or eliminate these problems, and allow smaller reaction volumes for oligonucleotide synthesis (~5ul) together with the high quality needed for subsequent polynucleotide assembly.

Non-porous surfaces suitable as substrates on which to perform oligonucleotide synthesis include polished quartz (100% $SiO_2$) or Pyrex (81% $SiO_2$) discs or plates from Chemglass with an exposed surface area of less than 1000 $mm^2$, preferably less than 300 $mm^2$, and more preferably less than 100 $mm^2$.

Modifications to the standard reaction vessel for CPG-supported olignoucleotide synthesis (Gait, 1984, Practical approach series, xiii, 217; Ito et al., 1982, Nucleic Acids Res 10, 1755, each of which is hereby incorporated by reference) improve oligonucleotide quality. The punching that frequently causes vortex formation during argon purging and contamination by chemicals stuck to the septa can be reduced or eliminated by using a technique based on positive pressure inert gas flow. Instead of punching through a septum, chemicals are added through an open channel with an argon flow to prevent air entering the reactor. The risk of air bypassing can be removed by using an argon purging procedure instead of vacuum filtration. Only one flow regulator (such as a stopcock) for regulating the argon input is required. All air sensitive solutions can be pressurized with an inert gas such as argon. An example of such a device is shown in Figure 50.

Accordingly, there is also provided a device for synthesizing oligonucleotides. The apparatus comprises (i) a reaction vessel for containing substrate supported seed nucleotides, (ii) an open channel in fluid communication with the reaction vessel, (iii) and a positive-pressure inert gas flow regulated by a stopcock, where the positive-pressure inert gas flow is configured to add chemicals through said open channel. In some embodiments, the positive-pressure inert gas flow is an argon gas flow.

### 5.4.2 COMBINED SYNTHESIZER AND CHEMISTRY IMPROVEMENTS

By using a freshly prepared oxidizer with high water content and using a DMAP catalyzed capping step after oxidation instead of (or in addition to) N-methylimidazole catalyzed capping before oxidation there is no need to acylate thymidine, cytosine and adenosine residues before oxidation. The guanidine modification problem (Eadie & Davidson, 1987, Nucleic Acids Res 15, 8333-49, which is hereby incorporated by reference), can be avoided in an oligonucleotide synthesizer, hardware and software, that efficiently performs a double capping protocol.

Depurination occurs at the acidic deprotection step. In commercial synthesizers, depurination is typically minimized by controlling the pH and reaction time. See Septak, 1996, Nucleic Acids Res 24, 3053-3058; and Paul & Royappa, 1996, Nucleic Acids Res, 24, 3048-3052, each which is hereby incorporated by reference in its entirety. An important parameter for adjusting the relative rates of different reactions is temperature, though this cannot be adjusted with current commercial synthesizer designs. Different dependencies of reaction rates on temperature were empirically described by Arrhenius in 1889 and subsequently theoretically validated by Eyring in 1935. According to transition state theory, the reaction constant (k) depends on temperature (T):

$$k = A \cdot e^{B/T}$$

Arrhenius equation

$$k = \frac{k_B T}{h} \cdot e^{\frac{\Delta S^{\#}}{R}} \cdot e^{-\frac{\Delta H^{\#}}{RT}}$$

Eyring equation

where,

A = Arrhenius constant;
B = reaction activation energy;
R = gas constant = [8.314 J / (mol·K)];
$\Delta S^{\#}$ = reaction activation entropy [$J \cdot mol^{-1} \cdot K^{-1}$];
$\Delta H^{\#}$ = reaction activation enthalpy [$kJ \cdot mol^{-1}$];
$k_B$ = Boltzmann's constant [$1.381 \cdot 10^{-23}$ $J \cdot K^{-1}$]; and
h = Plank constant [$6.626 \cdot 10^{-34}$ J · s].

The efficiency of adenosine detrylation relative to its depurination can be adjusted by altering the reaction temperature. The kinetic parameters $\Delta S^{\#}$ and $\Delta\#$ for other reactions can be determined by standard methods. An automated instrument with a controlled temperature deprotection block, for example controlled by a Peltier device, will allow control of the relative rates of the critical reactions. One example of such a device design is shown in Figure 51. One application of such a device is to reduce the formation of depurinated side-products during oligonucleotide detritylation. This reaction is performed below the room temperature. For this purpose, a container with a solution of dichloroacetic acid is cooled down by Peltier devices attached to the reaction chamber.

Accordingly, there is provided an oligonucleotide synthesizing apparatus comprising (i) a reaction cell for containing substrate supported seed nucleotides, (ii) a plurality of chemical supply reservoirs for containing certain predetermined bases, reagents and solvents to be used in an oligonucleotide synthesis process, (iii) a dispenser coupled to the plurality of chemical supply reservoirs and to the reaction cell for selectively dispensing one or more of the predetermined bases, reagents, and/or solvents at predetermined times and in predetermined controlled volumes, (iv) a processor for executing a plurality of subroutines corresponding to the sequential steps of an oligonucleotide synthesizing process; and (v) a temperature controller for controlling the temperature of the reaction cell in order to differentially affect the rate of two different reactions that occur in the reaction cell. In some embodiments, the temperature controller is a controlled temperature deprotection block. In some embodiments, this controlled temperature deprotection block is controlled by a Peltier device. In some embodiments, the dispenser comprises an open channel in fluid communication with the reaction cell and the oligonucleotide synthesizing apparatus further comprises a positive-pressure inert gas flow regulated by a stopcock, where the positive-pressure inert gas flow is configured to add chemicals through the open channel. In some embodiments, positive-pressure inert gas flow is an argon gas flow. Details of conventional nucleic acid synthesizers are found in Zelinka *et al.,* United States Patent No. 4,598,049, which is hereby incorporated by reference in its entirety.

### 5.5 ASSEMBLY OF OLIGONUCLEOTIDES INTO POLYNUCLEOTIDES

#### 5.5.1 COMBINED SYNTHESIZER AND CHEMISTRY IMPROVEMENTS

Polynucleotide synthesis typically comprises two steps. First, two or more oligonucleotides are synthesized chemically. These oligonucleotides are preferably between 5 and 200 nucleotides in length, more preferably between 10 and 100 nucleotides in length, even more preferably between 15 and 75 nucleotides in length. Second, these oligonucleotides are assembled in an enzyme-mediated process into polynucleotides. These polynucleotides are preferably longer than 100 nucleotides in length and more preferably longer than 200 nucleotides in length.

#### 5.5.2 POLYNUCLEOTIDE DESIGN

To assemble oligonucleotides into polynucleotides, the oligonucleotides are first annealed to one another, as shown in Figure 21. The annealing of each oligonucleotide to its two correct partners is important to ensure the subsequent formation of a polynucleotide with the correct sequence. The annealing of each oligonucleotide to its correct partners can be influenced by the design of the polynucleotide itself, the design of the oligonucleotides from which the polynucleotide will be assembled, and the reaction conditions and processes used for polynucleotide assembly. Methods for designing oligonucleotides, polynucleotides and choosing reaction conditions that improve the ease and fidelity of polynucleotide synthesis are aspects of the present invention.

Complementary nucleic acid sequences bind to one another, in part as a result of hydrogen bonding within a complementary base pair: two hydrogen bonds between a thymine and adenine, three hydrogen bonds between a guanine and cytosine. The different number of bonds within the two different complementary base pairs means that a thymine-adenine pair contributes less stability to a DNA duplex than a cytosine-guanine pair. As a result, the sequence of a polynucleotide affects the ease and fidelity with which that polynucleotide may be assembled from oligonucleotides.

Factors involving base composition affect the annealing temperatures of the oligonucleotides that will be used to assemble a polynucleotide. One such factor is the overall representation of each base; that is the fraction of nucleotides that are either cytosine or guanine. This is known as the GC content. A sequence with a higher GC content will tend to have a higher thermal stability than a sequence of the same length with a lower GC content. Another such factor is the uniformity of base representation, in other words, whether a part of the polynucleotide contains a high GC content while another part of the polynucleotide has a low GC content. In this case oligonucleotides for assembly of the part of the polynucleotide containing a high GC content would have a higher thermal stability than oligonucleotides for assembly of the part of the polynucleotide containing a low GC content.

The presence of repeated sequence elements can also affect the degree to which oligonucleotides anneal with one another in the polynucleotide assembly process. For example the set of oligonucleotides that are required to assemble a polynucleotide that contains a sequence of repeated nucleotides may contain two oligonucleotides containing this sequence and 2 oligonucleotides containing the reverse complement of this sequence, one being the correct annealing partner and one being the incorrect annealing partner. The longer this repeat sequence is, the higher its contribution to the stability of annealing of the oligonucleotide and its complement, and the greater the stability of annealing of the oligonucleotide with the incorrect annealing partner. The greater the stability of annealing of an oligonucleotide with an incorrect annealing partner, the greater the chances that it will anneal to this incorrect partner during the polynucleotide synthesis process, resulting in a decrease in fidelity of the polynucleotide synthesis process.

Because of the degeneracy of the genetic code, one polypeptide sequence may be encoded by many different polynucleotides. Some of these polynucleotides will be easier to synthesize in a high fidelity process, while others will be more difficult. When a polynucleotide is being designed and / or synthesized to encode a polypeptide, a polynucleotide sequence may therefore be chosen that facilitates the high fidelity synthesis of that polynucleotide, in addition to ensuring that the polynucleotide will possess the desired functional properties. Methods for choosing a polynucleotide sequence that fulfills functional as well as ease-of-synthesis criteria may be accomplished using computer programs (*e.g.*, software). The methods and the software for performing the methods are aspects of the present invention.

Most organisms use the same genetic code, that is, in general the same triplet of nucleotides (codon) specifies the same amino acid. Different organisms use these codons with different frequencies within their genes, however. For example different codon biases are found in humans, human viruses such as hepatitis A, hepatitis B, hepatitis C, human immunodeficiency virus (HIV), human papilloma virus (HPV), influenza, flaviviruses, lentiviruses, papovaviruses, human pathogens such as *Mycobacteria, Chlamydomonas, Candida, Plasmodium falciparum* (the causative agent of malaria), *Cryptosporidium, Leishmania* and other protozoa, model organisms such as *Tetrahymena,* and commonly used expression systems such as baculovirus, *Escherichia coli, Bacillus,* filamentous fungi, mammalian cell lines including COS cells and 3T3 cells, yeasts including *Saccharomyces cerevisiae,* plants including maize and cotton and model organisms for the study of disease and tests of the efficacies of DNA vaccines such as macaques, mice and rabbits. This difference in turn affects the ability of an organism to express a polypeptide that is encoded by a particular polynucleotide sequence. A polynucleotide that contains a large number of codons that are used rarely by an organism will generally express more poorly in that organism than one that does not. Polypeptide expression can be enhanced by using a polynucleotide whose distribution of codons matches the distribution found in the intended expression host organism. The distribution of codons used within the genes of an organism can be represented as a codon bias table. Examples of such tables are shown in Figures 22, 23 and 25. Such tables represent the average use of codons within many genes in an organism (Figures 23 and 25), or the average use of codons within many genes of a specific class (such as highly expressed genes, Figure 22) in an organism.

A codon bias table may be used in the design of a polynucleotide that encodes a specific polypeptide. The polynucleotide may be designed so that each of the 20 amino acids contained in the polypeptide is encoded in the polynucleotide by codons selected at a frequency represented in the codon bias table. For example, Tyr is encoded by the codons TAT and TAC. In highly expressed *E coli* genes TAT is used 35% of the time (its frequency is 0.35) and TAC is used 65% of the time (its frequency is 0.65), as shown in Figure 22. In a polynucleotide designed for expression in *E coli,* Tyr may therefore be encoded approximately 35% of the time with TAT and 65% of the time with TAC. Such a polynucleotide would have an E coli codon distribution for Tyr. The same process may be used for all of the amino acids in the polypeptide. Because a codon bias table contains average values compiled from information from many genes, it is not necessary to precisely match the values found in the codon bias tables in order to obtain a polypeptide that will express well in a host organism. It may be preferable to use the codon bias table to guide a probabilistic choice of amino acid. For example, in designing a polynucleotide to encode a polypeptide for expression in *E coli,* each time a Tyr is encountered, a selection method or computer program may be used that has a 35% chance of selecting TAT and a 65% chance of selecting TAC. On average, many polypeptides designed by such a method would contain TAT and TAC in the ratio of 0.35:0.65, although any individual polynucleotide may vary from this ratio and may still express well in the host. Similar methods may be used to select codons to encode the other amino acids from the polypeptide.

A second way in which codon bias tables may be used in the design of a polynucleotide that encodes a specific polypeptide is to identify and eliminate codons that are very rarely used in a specific host. For example in Figure 22 it can be seen that Arg is encoded by six possible codons: CGG, CGA, CGT, CGC, AGG and AGA. Of these, codons CGG, CGA, AGA and AGG each occur only about 1% of the time in highly expressed *E coli* genes, while CGT occurs 64% of the time and CGC 33% of the time. It may be advantageous to eliminate the four rarely used codons from the synthetic polynucleotide entirely. In this case only CGT and CGC would be used to encode Arg in the synthetic polynucleotide. Using a probabilistic selection method, CGT would then be selected 64/97 = 66% of the time, and CGC would be selected 33/97 = 34% of the time.

Threshold values for codons may be selected such that a codon that appears less frequently in a codon bias table than that threshold value are not used in a polynucleotide for expression in that host. Threshold values of 0.1 (10%), 0.09 (9%), 0.08 (8%), 0.07 (7%), 0.06 (6%), 0.05 (5%) and 0.04 (4%) can all be useful. Threshold values can be set using a method in which codons are selected probabilistically based upon a codon bias table, then codons whose frequency is below the threshold are discarded and another codon is chosen, again probabilistically. Alternatively a codon bias table may be precalculated with the frequency for a codon that appears below the threshold frequency being set to zero so that it is never selected by a probabilistic selection method.

Hybrid codon bias tables may be constructed for designing a polynucleotide encoding a polypeptide to be expressed in more than one expression system. One method of constructing such hybrid codon bias tables is to combine two or more starting codon bias tables from one or more organism. Figure 24 shows a hybrid codon bias table constructed from the codon bias tables shown in Figures 22 and 23. In one combination method, a threshold frequency is selected and any codons that fall below the threshold are eliminated from all of the starting codon bias tables. For the remaining codons there are several possible methods of processing the frequencies. An average of the frequencies in the starting bias tables may be obtained. Such an example for preparing a codon bias table is shown in Figure 24. Alternatively the higher of the values may be selected for each of the codons. Another possibility is to select the lower value. In all cases, the frequencies should be normalized so that the sum of the frequencies for all codons that encode one amino acid are equal to 1. By avoiding low frequency codons for multiple organisms, expression in all of those organisms will be improved, thereby increasing the general usefulness of the synthetic polynucleotide.

The incorporation of additional features into a designed polynucleotide may improve the usefulness of that polynucleotide. For example, it may be desirable to reduce the sequence identity between the synthetic polynucleotide and a naturally occurring polynucleotide that encodes a polypeptide or polypeptide fragment of the same amino acid sequence. Such a synthetic polynucleotide may function to produce its encoded polypeptide under conditions where the natural polynucleotide is inhibited by complementary oligonucleotides or polynucleotides, for example by antisense DNA or interfering RNA (RNAi). Conversely, it may be useful to increase the sequence identity between the synthetic polynucleotide and a naturally occurring polynucleotide to increase the frequency of recombination between the two under experimental conditions such as polynucleotide fragmentation and reassembly *in vitro* or *in vivo.* Methods and software for designing polynucleotides with maximized or minimized sequence identity to another polynucleotide sequence is an aspect of the invention.

Another feature that may improve the usefulness of a polynucleotide is the elimination or addition of sequences that serve as recognition and / or cleavage sites for restriction endonucleases. Such sequences may be useful for subsequent manipulations of the polynucleotide or subsequences of the polynucleotide such as subcloning or replacement of modules of the polynucleotide. Methods and software for designing polynucleotides with modified restriction endonuclease cleavage sites is an aspect of the invention.

It may also be advantageous to modify the restriction sites within a polynucleotide to effect subsequent recombinations between related polynucleotides. For example a polynucleotide may be designed to contain sites for one or more typeIIs restriction endonucleases at every place possible within a sequence, without changing the polypeptide sequence encoded by the polynucleotide. Type IIs restriction endonucleases cut outside their recognition sequence. Examples include AlwI, BbsI, BbvI, BpmI, BsaI, BseRI, BsgI, BsmAI, BsmBI, BsmFI, BspMI, BsrDI, EarI, FokI, HgaI, HphI, MboII, MnlI, PleI, SapI, SfaNI, BstF51, FauI. It is also possible to modify the polynucleotide sequence, without changing the polypeptide sequence encoded by the polynucleotide, so that each overhang resulting from digestion of the polynucleotide with the one or more typeIIs restriction endonuclease is unique. Such sites will preferably be between 10 and 500 bases apart, more preferably between 15 and 200 bases apart, even more preferably between 25 and 100 bases apart. Digestion of a polynucleotide with the one or more typeIIs restriction endonucleases, followed by ligation of the fragments will thus result in faithful reassembly of the original polynucleotide sequence. Design of two or more polynucleotide sequences to contain the same sets of unique overhangs following digestion with the one or more typeIIs restriction endonuclease will thus permit digestion of the two polynucleotides followed by ligation of all of the fragments to assemble chimeric polynucleotides. Methods and software for designing one or more polynucleotide to contain a frequency of typeIIs restriction sites to allow digestion and reassembly of fragments in the original order to create the original polynucleotide or chimeric polynucleotides is an aspect of the invention.

In addition to the functional criteria by which codons may be selected to encode a polypeptide, codons may be selected that contribute to the ease and fidelity of synthesis of the polynucleotide. Two factors of particular importance in designing a polynucleotide that can be easily and accurately assembled from oligonucleotides are an even distribution of GC content, and the reduction or elimination of repeated sequence elements.

A polynucleotide in which the GC content of the polynucleotide is evenly distributed, may be assembled from oligonucleotides with more uniform lengths and annealing temperatures than a polynucleotide in which the GC content is unevenly distributed. Both of these factors improve the ease and fidelity of subsequent assembly of oligonucleotides into polynucleotides. The uniformity of GC content may be assessed by selecting a "window" of contiguous nucleotides within the polynucleotide and determining the fraction of those nucleotides that are either G or C. A window may consist of 50 contiguous nucleotides, more preferably 40 contiguous nucleotides more preferably 35 contiguous nucleotides, more preferably 30 contiguous nucleotides and even more preferably 25 contiguous nucleotides. Within such a window the GC content of a designed polynucleotide is preferably less than 80% but more than 20%, more preferably it is less than 75% but more than 25%, more preferably it is less than 70% but more than 30% and even more preferably it is less than 65% but more than 35%.

The presence of repeated sequence elements within a polynucleotide will result in stretches of sequence identity in incorrect annealing partners. This in turn will result in a decrease in fidelity of assembly of the oligonucleotides and an increase in the frequency of internal deletions within the gene. The elimination or reduction of repeated sequence elements is thus an important component of a polynucleotide design process that seeks to improve speed and accuracy of synthesis. A repeated sequence element may be defined in terms of the length of a sequence of contiguous nucleotides within a polynucleotide, the frequency with which that sequence occurs within the polynucleotide. Preferably any sequence of 20 contiguous nucleotides within a polynucleotide will occur only once, more preferably any sequence of 18 contiguous nucleotides within a polynucleotide will occur only once, more preferably any sequence of 16 contiguous nucleotides within a polynucleotide will occur only once, more preferably any sequence of 14 contiguous nucleotides within a polynucleotide will occur only once, even more preferably any sequence of 12 contiguous nucleotides within a polynucleotide will occur only once.

The occurrence of sequences within a polynucleotide that differ by only a small number of nucleotides occur within the polynucleotide will also result in stretches of sequence identity in incorrect annealing partners. This in turn will result in a decrease in fidelity of assembly of the oligonucleotides and an increase in the frequency of internal deletions within the gene. The elimination or reduction of almost-repeated sequence elements is thus another important component of a polynucleotide design process that seeks to improve speed and accuracy of synthesis. Preferably no sequence of 35 contiguous nucleotides within a polynucleotide will occur a second time with three mismatched nucleotides, no sequence of 26 contiguous nucleotides within a polynucleotide will occur a second time with 2 mismatched nucleotides, no sequence of 16 contiguous nucleotides within a polynucleotide will occur a second time with 1 mismatched nucleotide.

Another method for reducing or eliminating repeated sequence elements that are likely to be problematic in the assembly of oligonucleotides into polynucleotides is to minimize the number of sub-sequences within the polynucleotide that will anneal to any of the other sub-sequences within the polynucleotide under the conditions that are to be used to assemble to polynucleotide. There are many ways to do this that are more or less computationally intensive.

Particularly useful methods for designing polynucleotides are those that integrate functional constraints such as the selection of codons that will express well in one or more chosen host systems, the elimination of unwanted restriction sites and the inclusion of desired restriction sites, with synthesis constraints such as the elimination of repeated sequence elements and the balancing of GC content throughout the sequence. Systematic methods for accomplishing such a design that are readily amenable to automation using computer programs are shown schematically in Figures 26, 27 and 28.

The first 50 codons are often the most important for getting good expression. If it is necessary to add codons with low frequencies in one or more of the starting codon bias tables to avoid additional constraints in the synthetic polynucleotide, these codons should preferably occur after the first 50 codons. Nucleotide regions that are likely to form secondary structures such as hairpins are also preferably avoided for 50 bases before the initiating ATG and within the first 50 codons, more preferably for 40 bases before the initiating ATG and within the first 40 codons and most preferably for 30 bases before the initiating ATG and within the first 30 codons.

### 5.5.3 OLIGONUCLEOTIDE DESIGN

When oligonucleotides are designed for assembly into polynucleotides, one factor that favors the annealing of oligonucleotides with their correct annealing partners is a difference between the annealing temperatures between intended annealing partners and the annealing temperatures between unintended annealing partners. Preferably the highest annealing temperature for any pair of unintended annealing partners will be at least 5°C lower than the lowest annealing temperature for any pair of correct annealing partners, more preferably this difference will be at least 8°C, more preferably it will be 11°C and even more preferably it will be at least 14°C. Such a difference helps to ensure that when the intended annealing partners anneal to one another during the assembly process, incorrect annealing partners are unable to anneal to one another. This increases the efficiency and fidelity of the polynucleotide synthesis process.

The annealing temperatures for all intended annealing partners within an oligonucleotide set that is to be assembled into a polynucleotide can affect the fidelity and efficiency of assembly. The optimal annealing temperature may vary as a result of the overall GC content of the polynucleotide. The lowest calculated annealing temperature for any pair of intended annealing partners within an oligonucleotide set to be assembled into a polynucleotide is preferably calculated to be 56°C, more preferably 58°C, more preferably 60°C and even more preferably 62°C.

The discrimination between intended and unintended annealing partners is further aided when oligonucleotides that are to be assembled into polynucleotides are designed such that the annealing temperatures between all intended oligonucleotide partners are approximately equal. Preferably the annealing temperatures between all of the intended annealing partners in a set of oligonucleotides for assembly into one polynucleotide will be within 10°C of each other, more preferably within 8°C of each other, more preferably within 6°C of each other, more preferably within 4°C of each other and even more preferably within 3°C of each other.

When oligonucleotides are designed for assembly into polynucleotides, it is also often desirable to have oligonucleotide lengths that are close to one another, as this helps to reduce the maximum oligonucleotide length required. This may be beneficial because shorter oligonucleotides can in general be synthesized more accurately than longer oligonucleotides. Preferably the maximum length of any oligonucleotide within a set of oligonucleotides designed to assemble into a polynucleotide is 75 bases, more preferably 70 bases, more preferably 65 bases, more preferably 60 bases and even more preferably 55 bases.

Suitable methods for designing oligonucleotides that are to be assembled into polynucleotides are those that consider all of these factors. Such methods are an aspect of the present invention. For example it is advantageous in the synthesis of polynucleotides with GC contents >60%, or polynucleotides containing regions of repeated sequence to increase the annealing temperature for oligonucleotides. Increased annealing temperatures will require greater oligonucleotide lengths

One example of a way in which this can be done, intended to illustrate but not to limit the invention, is shown schematically in Figures 29 to 31. Oligonucleotides can be designed to have a narrow range of annealing temperatures by dividing the polynucleotide into consecutive sections that are each calculated to have the same annealing temperatures to their complements.

One method for the initial division of a polynucleotide sequence into sub-sequences that are useful for subsequent oligonucleotide design is shown in Figure 13. First, an annealing temperature is selected. Then a first section of the polynucleotide is selected by sequential addition of consecutive bases until a sub-sequence is obtained whose annealing temperature to its intended complement exceeds the selected annealing temperature. A second section of the polynucleotide is selected by starting at the first nucleotide following the previous section and repeating the process. By continuing this process for the entire length of the polynucleotide, a set of sub-sequences can be obtained with a narrow range of annealing temperatures (a "constant Tm set" of sub-sequences). Other similar methods include those in which the process is initiated at the other end of the polynucleotide using the reverse complement sequence of the polynucleotide to produce a reverse set of "constant Tm" subsequences. In some cases it may also be desirable to create gaps in the polynucleotide sequence used to generate the "forward" or "reverse" set of "constant Tm" subsequences. For example, if a polynucleotide contains repetitive sequence elements, it may be preferable to omit a part or all of one or more of these repeat elements from the polynucleotide sequence used to calculate the "constant Tm set". Different sets of sub-sequences can be obtained by starting the process at different positions along the polynucleotide. Different sets of sub-sequences can be obtained by using different values for the annealing temperature. Even slight differences in annealing temperature can yield different sets of sub-sequences. For example annealing temperatures of 62°C, 62.1°C, 62.2°C, 62.3°C, 62.4°C and 62.5°C will yield different sets of sub-sequences. It is often of interest to produce many slightly different "constant Tm sets" of subsequences. These can then be combined to form oligonucleotides and then assessed for other properties that can influence assembly into polynucleotides.

A "constant Tm set" of polynucleotide sub-sequences can be converted into a set of oligonucleotides suitable for polynucleotide assembly in several ways. One method is represented schematically in Figure 30. In this method, a set of forward oligonucleotides is designed by combining the first and second "constant Tm" sub-sequences, then the next third and fourth and so on. A set of reverse olignucleotides is designed by combining the second and third "constant Tm" sub-sequences and obtaining the sequence of the reverse complement, then repeating the process with the fourth and fifth "constant Tm" sub-sequence and so on. Variations on this method include using a "constant Tm set" designed from the polynucleotide reverse complement sequence to design the reverse set of oligonucleotides, then associating this with the appropriate set of forward oligonucleotides. In another variation, oligonucleotides may be designed such that both strands of the polynucleotide are not completely covered. This may be particularly useful when the polynucleotide contains repetitive sequence elements, since inappropriate annealing of oligonucleotides to incorrect annealing partners is more likely if incorrect annealing partners contain longer or higher annealing temperature subsequences in common.

The computational resources required to design a "constant Tm set" of oligonucleotides are small. It is thus useful to produce many designs, each differing slightly from one another, but all constrained by the criterion of having a narrow range of annealing temperatures between correct annealing partners. These different sets of oligonucleotides with "constant Tm" can then be screened for other properties that also affect the efficiency and fidelity with which oligonucleotides assemble into polynucleotides. An example of such a set of screening criteria is shown in Figure 31.

Different criteria will be of different importance depending upon the physical method to be used to assemble the oligonucleotides. For example if the polymerase chain reaction is to be used, it is preferable to avoid an oligonucleotide that ends with a sequence that is repeated in an oligonucleotide other than the correct annealing partner. If ligation is used, it is preferable to ensure that no two oligonucleotides end with the same set of two, three or four bases. A method for designing oligonucleotides that are to be assembled by ligation is shown in Figure 32. method of designing a set of

There is also provided a method of designing a set of oligonucleotides for assembly into a polynucleotide. The method comprises identifying a first plurality of single-stranded oligonucleotides that collectively encode all or a portion of a first strand of the polynucleotide, where each respective single-stranded oligonucleotide in the first plurality of single-stranded oligonucleotides is characterized by an annealing temperature to its exact complement that is in a first predetermined annealing temperature range. A second plurality of single-stranded oligonucleotides is identified from the first plurality of single-stranded oligonucleotides, where a single-stranded oligonucleotide in the second plurality of single-stranded oligonucleotides is formed by joining an adjacent pair of oligonucleotides in the first plurality of single-stranded oligonucleotides. A third plurality of single-stranded oligonucleotides is identified that collectively encode all or a portion of a second strand of the polynucleotide, where each respective single-stranded oligonucleotide in the third plurality of single-stranded oligonucleotides is characterized by an annealing temperature to its exact complement that is in a second predetermined annealing temperature range. A fourth plurality of single-stranded oligonucleotides is identified from the third plurality of single-stranded oligonucleotides, where a single-stranded oligonucleotide in the fourth plurality of single-stranded oligonucleotides is formed by joining an adjacent pair of oligonucleotides in the third plurality of single-stranded oligonucleotides. The set of oligonucleotides comprises the second plurality of oligonucleotides and the fourth plurality of oligonucleotides. Next, a determination is made as to whether the set of oligonucleotides satisfies at least one assembly criterion, where (i) when the set of oligonucleotides satisfies the at least one assembly criterion, the set of oligonucleotides is selected, and (ii) when the set of oligonucleotides does not satisfy said at least one assembly criterion, the set of oligonucleotides is rejected and the aforementioned steps are repeated.

In some embodiments, a different first predetermined annealing temperature range and a different second predetermined annealing temperature range is used when the aforementioned steps are repeated. In some embodiments, the first predetermined annealing temperature range and the second predetermined annealing temperature range are the same. In some embodiments, the first predetermined annealing temperature range and the second predetermined annealing temperature range are different. In some embodiments, the first predetermined annealing temperature range and the second predetermined annealing temperature range is each between 45°C and 72°C. In some embodiments, the first predetermined annealing temperature range and the second predetermined annealing temperature range is each between 50°C and 65°C.

In some embodiments, the first predetermined annealing temperature range and the second predetermined annealing temperature range is each between 55°C and 62°C. In some embodiments, each single-stranded oligonucleotide in the second plurality of single-stranded oligonucleotides is formed by joining an adjacent pair of oligonucleotides in the first plurality of single-stranded oligonucleotides. In some embodiments, each single-stranded oligonucleotide in the fourth plurality of single-stranded oligonucleotides is formed by joining an adjacent pair of oligonucleotides in the third plurality of single-stranded oligonucleotides.

In some embodiments, the method further comprises (f) assembling the set of oligonucleotides by the polymerase chain reaction or ligase chain reaction with an annealing temperature that is a predetermined amount lower than the lowest annealing temperature of the first predetermined annealing temperature range, thereby forming an assembly mixture that comprises the polynucleotide. In some embodiments, the predetermined amount is 1°C or larger. In some embodiments, the method further comprises cloning the polynucleotide into a vector.

In some embodiments, the assembly mixture comprises a plurality of different polynucleotide molecules, and the method further comprises creating a plurality of heteroduplexes between different individual polynucleotide molecules within the plurality of different polynucleotide molecules in the assembly, treating the plurality of heteroduplexes with an agent that binds preferentially to mismatched sequences within a double-stranded DNA molecule, and using the agent to remove double-stranded DNA molecules containing mismatched sequences from the assembly mixture.

In some embodiments, the method further comprises amplifying the polynucleotide by the polymerase chain reaction. In some embodiments, the method further comprises cloning the polynucleotide into a vector. In some embodiments, the at least one assembly criterion comprises a requirement that the annealing temperature of each intended complementary pair of single-stranded oligonucleotides in the set of oligonucleotides falls within a third predetermined temperature range. In some embodiments, the third predetermined temperature range encompasses a total of 4°C or less. In some embodiments, the third predetermined temperature range encompasses a total of 3°C or less.

In some embodiments, the at least one assembly criterion comprises a requirement that the single-stranded oligonucleotide length of each oligonucleotide in the set of oligonucleotides is within a predetermined oligonucleotide length range. In some embodiments, the predetermined oligonucleotide length range is between 20 nucleotides and 70 nucleotides, or between 25 nucleotides and 65 nucleotides.

In some embodiments, the at least one assembly criterion comprises a requirement that the number of single-stranded oligonucleotides in the second plurality of single-stranded oligonucleotides matches the number of single-stranded oligonucleotides in the fourth plurality of single-stranded oligonucleotides. In some embodiments, the at least one assembly criterion comprises a requirement that the annealing temperature of each pair of single-stranded oligonucleotides in the set of oligonucleotides for assembly, whose annealing is not intended for said assembly, is below a predetermined temperature. In some embodiments, the predetermined temperature is the annealing temperature of a pair of oligonucleotides in the set of oligonucleotides whose annealing is intended for assembly of the polynucleotide. In some embodiments, this predetermined temperature is at least 10°C below, at least 15°C below, or at least 20°C below the annealing temperature of a pair of oligonucleotides in the set of oligonucleotides whose annealing is intended for assembly of said polynucleotide.

In some embodiments, the at least one assembly criterion comprises a requirement that a maximum length of a sequence that occurs more than once within the first strand of the polynucleotide and that is found at a terminus of any oligonucleotide in the set of oligonucleotides is less than a predetermined length. In some embodiments, the predetermined length is 10 nucleotides or greater, or 12 nucleotides or greater. In some embodiments, a pair of oligonucleotides in the set of oligonucleotides that are intended to be annealed to form the polynucleotide are not completely overlapping.

In some embodiments, a first single-stranded oligonucleotide has an n-mer overhang relative to a second single-stranded oligonucleotide in the set of oligonucleotides, and annealing of the first single-stranded oligonucleotide and the second oligonucleotide single-stranded oligonucleotide is intended for assembly of the polynucleotide, where n is between 1 and 40. In some embodiments, the at least one assembly criterion comprises a requirement that a predetermined length of a nucleotide sequence at a terminus of an oligonucleotide in the set of oligonucleotides is not found at either terminus of any other oligonucleotide in the set of oligonucleotides. In various embodiments, the predetermined length is 5 nucleotides, 4 nucleotides, or 3 nucleotides.

In some embodiments, the polynucleotide encodes a polypeptide, and the method further comprises (i) selecting, prior to the identifying step, an initial polynucleotide sequence for the polynucleotide that codes for the polypeptide, where a codon frequency in the initial polynucleotide sequence is determined by a codon bias table and (ii) modifying, prior to the identifying step, an initial codon choice in the initial, polynucleotide sequence for the polynucleotide in accordance with a design criterion, thereby constructing a final polynucleotide sequence for the polynucleotide that codes for the polypeptide. In some embodiments, the design criterion comprises one or more of:

(i) exclusion of a restriction site sequence in said initial polynucleotide sequence;
(ii) incorporation of a restriction site sequence in said initial polynucleotide sequence;
(iii) a designation of a target G+C content in the initial polynucleotide sequence;
(iv) an allowable length of a sub-sequence that can be exactly repeated within either strand of the initial polynucleotide sequence;
(v) an allowable annealing temperature of any sub-sequence to any other sub-sequence within either strand of the initial polynucleotide sequence;
(vi) exclusion of a hairpin turn in the initial polynucleotide sequence;
(vii) exclusion of a repeat element in the initial polynucleotide sequence;
(viii) exclusion of a ribosome binding site in the initial polynucleotide sequence;
(ix) exclusion of a polyadenylation signal in the initial polynucleotide sequence;
(x) exclusion of a splice site in the initial polynucleotide sequence;
(xi) exclusion of an open reading frame in each possible 5' reading frame in the initial polynucleotide sequence;
(xii) exclusion of a polynucleotide sequence that facilitates RNA degradation in the initial polynucleotide sequence;
(xiii) exclusion of an RNA polymerase termination signal in the initial polynucleotide sequence;
(xiv) exclusion of a transcriptional promoter in the initial polynucleotide sequence;
(xv) exclusion of an immunostimulatory sequence in the initial polynucleotide sequence;
(xvi) incorporation of an immunostimulatory sequence in the initial polynucleotide sequence;
(xvii) exclusion of an RNA methylation signal in the initial polynucleotide sequence;
(xviii) exclusion of a selenocysteine incorporation signal in the initial polynucleotide sequence;
(xix) exclusion of an RNA editing sequence in the initial polynucleotide sequence;
(xx) exclusion of an RNAi-targeted sequence in the initial polynucleotide sequence; and/or
(xxi) exclusion of an inverted repeat within the first 45 nucleotides encoding said synthetic polypeptide in the initial polynucleotide sequence.

In some embodiments, the design criterion comprises reduced sequence identity to a reference polynucleotide, and wherein modifying the initial codon choice in the initial polynucleotide in accordance with the design criterion comprises altering a codon choice in the initial polynucleotide sequence to reduce sequence identity to the reference polynucleotide. In some embodiments, the design criterion comprises increased sequence identity (*e.g.*, at least 0.05% or more identical, at least 1% or more identical, at least 2% or more identical, at least 3% or more identical, at least 4% or more identical) and modifying the initial codon choice in the initial polynucleotide in accordance with the design criterion comprises altering a codon choice in said initial polynucleotide sequence to increase sequence identity to the reference polynucleotide.

There is also provided a computer program product for use in conjunction with a computer system, the computer program product comprising a computer readable storage medium and a computer program mechanism embedded therein. In this aspect of the invention, the computer program mechanism comprises instructions for identifying a first plurality of single-stranded oligonucleotides that collectively encode all or a portion of a first strand of a polynucleotide, where each respective single-stranded oligonucleotide in the first plurality of single-stranded oligonucleotides is characterized by an annealing temperature to its exact complement that is in a first predetermined annealing temperature range. The computer program mechanism further comprises instructions for identifying a second plurality of single-stranded oligonucleotides from the first plurality of single-stranded oligonucleotides, where a single-stranded oligonucleotide in the second plurality of single-stranded oligonucleotides is formed by joining an adjacent pair of oligonucleotides in the first plurality of single-stranded oligonucleotides. The computer program mechanism further comprises instructions for identifying a third plurality of single-stranded oligonucleotides that collectively encode all or a portion of a second strand of the polynucleotide, where each respective single-stranded oligonucleotide in the third plurality of single-stranded oligonucleotides is characterized by an annealing temperature to its exact complement that is in a second predetermined annealing temperature range. The computer program mechanism further comprises instructions for identifying a fourth plurality of single-stranded oligonucleotides from the third plurality of single-stranded oligonucleotides, where a single-stranded oligonucleotide in the fourth plurality of single-stranded oligonucleotides is formed by joining an adjacent pair of oligonucleotides in the third plurality of single-stranded oligonucleotides. A set of oligonucleotides comprises the second plurality of oligonucleotides and the fourth plurality of oligonucleotides. Further, the computer program mechanism comprises instructions for determining whether the set of oligonucleotides satisfies at least one assembly criterion, where (i) when the set of oligonucleotides satisfies said at least one assembly criterion, the set of oligonucleotides is selected and (ii) when the set of oligonucleotides does not satisfy the at least one assembly criterion, the set of oligonucleotides is rejected and the aforementioned steps are repeated. In some embodiments, this process is stored in a computer system comprising a central processing unit and a memory, coupled to the central processing unit

### 5.5.4 OLIGONUCLEOTIDE ASSEMBLY CONDITIONS

Methods for assembling polynucleotides from oligonucleotides include ligation, the polymerase chain reaction, the ligase chain reaction and combinations thereof. These methods may all be used to construct synthetic polynucleotides from olignucleotides. See, for example, Hayden et al., 1988, DNA 7: 571-7.; Ciccarelli et al., 1991, Nucleic Acids Res 19: 6007-13.; Jayaraman et al., 1991, Proc Natl Acad Sci U S A 88: 4084-8; Jayaraman et al., 1992, Biotechniques 12: 392-8.; Graham et al., 1993, Nucleic Acids Res 21: 4923-8.; Kobayashi et al., 1997, Biotechniques 23: 500-3.; Au et al., 1998, Biochem Biophys Res Commun. 248: 200-203; Hoover et al., 2002, Nucleic Acids Res 30: e43, each of which is hereby incorporated by reference in its entirety. A suitable method for assembling any set of oligonucleotides depends upon the physical properties of the set of oligonucleotides. The optimal reaction conditions used to minimize incorporation of errors during assembly of the oligonucleotides depends upon the precise criteria used to design the oligonucleotides, and this interrelationship is an aspect of the present invention. Assembly methods that are optimized for assembling oligonucleotide sets designed according to the methods described here are another aspect of the invention.

Variables within a method for assembling oligonucleotides into a polynucleotide include the composition of the reaction buffer, the polymerase(s) used, the concentrations of oligonucleotides used and the thermal conditions of the reaction mixture.

The presence of dimethyl sulphoxide (DMSO), betain, trimethyl ammonium chloride and other agents that reduce the annealing temperature of nucleic acids may be included to improve the specificity of oligonucleotide annealing and the polymerization performance of the polymerase. These agents may also decrease the fidelity of the polymerase, however. To balance these activities, DMSO is preferably included in a reaction mix at 5% v/v, more preferably at 3% v/v. However, these agents should be omitted if the annealing temperature of the oligonucleotide set is lower than 60°C, more preferably if it is lower than 58°C, even more preferably if it is below 56°C. They should also be omitted if the GC content of the polynucleotide is below 46%, more preferably if it is below 44% and even more preferably if it is below 42%.

An important factor for correct assembly of oligonucleotides is the concentration of each oligonucleotide, and the total concentration of oligonucleotides. The total oligonucleotide concentration within an assembly reaction is preferably between 5 μM and 0.05 μM, more preferably between 2.5 μM and 0.1 μM, even more preferably between 1.5 μM and 0.25 μM. Within this reaction mixture, each oligonucleotide is preferably represented at an equimolar amount with all of the other oligonucleotides present.

The thermal conditions for the assembly of oligonucleotides into polynucleotides are also a critical factor in efficient and high fidelity polynucleotide synthesis. Of greatest importance is the alignment between the calculated annealing temperature for the correct annealing partners in an oligonucleotide set, and the annealing temperature used in the assembly reaction. Some examples of thermocycler programs of use in assembling polynucleotides form oligonucleotides are shown in Figures 32-36. The annealing temperature is preferably between 10°C below and 5°C above the lowest calculated annealing temperature between intended annealing partners within the oligonucleotide set, more preferably it is between 8°C below and 2°C above the lowest calculated annealing temperature between intended (correct) annealing partners within the oligonucleotide set, and even more preferably it is between 6°C below and 2°C below the lowest calculated annealing temperature between intended annealing partners within the oligonucleotide set.

An alternative strategy to the polymerase chain reaction for polynucleotide assembly is the use of thermostable ligases. An example of a thermocycle program for a typical ligation cycle reaction is shown in Figure 43. For a ligation-based assembly, the polynucleotide is preferably between 100 and 3,000 bases, more preferably between 150 and 2,000 bases, more preferably between 250 and 1,500 bases long.

Following the assembly of oligonucleotides into a polynucleotide, it is possible to amplify the full-length polynucleotide out of the mixture by PCR using a pair of amplification primers. Following the amplification of full-length polynucleotide, it is possible to reduce errors that are present in a subset of the polynucleotide population, for example those that were introduced in the oligonucleotide synthesis step, or in the polymerase chain reaction assembly or amplification steps. This can be done by using enzymes that recognize mismatched bases in double stranded DNA, for example T4 endonuclease VII and T7 endonuclease I (see, for example, Babon et al., 2003, Mol Biotechnol 23: 73-81, which is hereby incorporated by reference in its entirety). To create mismatches the mixture of polynucleotides in an assembly or amplification reaction is heated to a temperature that melts the DNA present (for example, to a temperature above 90°C), then cooled to a temperature that allows it to anneal at which the endonuclease enzyme is active (for example to a temperature below 50°C). The enzyme or mixture of enzymes that cleaves DNA at or near the site of a mismatched base, such as T4 endonuclease VII, T7 endonuclease I or a mixture thereof, is then added to the reaction and allowed to incubate and cleave the mismatched DNA.

Two alternative methods for DNA denaturation prior to endonuclease treatment are heat denaturation with ethylene glycol or alkali denaturation. To denature with ethylene glycol, the PCR product (1-5 μg) is added to a denaturation mix consisting of 10 mM Tris-Cl pH 7.5, 1 mM EDTA, 20% glycerol, and 20% ethylene glycol. The sample is heated at 95-100°C for 5 minutes, and then slowly cooled to room temperature. For alkali denaturation, the pcr product (1-5 μg) is suspended in 200 mM NaOH. The mix is incubated for 10 minutes at 37°C then placed on ice. 1.0 M HCl is added to a final concentration of 200 mM. A third alternative method for heteroduplex formations uses exonuclease. See, for example, Thomas et al., 2002, Biological Chemistry 383, 1459-1462, which is hereby incorporated by reference in its entirety. Prior to the use of exonuclease, two PCR reactions are performed. In the first, a 5N-phosphorylated primer is used along with a 3N-non-phosphorylated primer. In the second, a 3N-phosphorylated primer is employed along with a 5N-non-phosphorylated primer. Treatment of the resulting PCR products with exonuclease removes the phosphorylated strand. The two single strand polynucleotides are mixed, heat denatured, and annealed by slow cooling. Briefly, a typical reaction mix using exonuclease contains 67 mM glycine-KOH pH 9.4, 2.5 mM $MgCl_2$, 50 mg/ml BSA, 1-5 ug of PCR product, 5-10 U exonuclease. The reaction is incubated for 15 minutes to one hour at 37°C. PCR amplification using equimolar amounts of phosphorylated and non-phosphorylated primers may alternatively be performed to obviate the need for two separate PCR reactions.

Alternative enzymes capable of catalyzing DNA cleavage at mismatches in heteroduplex DNA include, the CEL I nuclease from celery, the Aspergillus SI nuclease, Endonuclease V from E. coli, and the MutHSL proteins from E. *coli.* See, for example, Smith and Modrich, 1997, Proc Natl Acad Sci 94, 6847-6850, which is hereby incorporated by reference in its entirety. Specificity and reaction rate of all these enzymes can be modulated by temperature of incubation and/or addition of DNA denaturants, such as formamide, ethylene glycol, and dimethyl sulfoxide. Using a mixture of two or more enzymes can additionally broaden specificity of mismatches cleaved.

For MutHSL-mediated removal of mutant sequences, a 20 μl mix consisting of Hepes pH 8.0, 50 mM KCL, 2.5 mM DTT, 125 μg/ml BSA, 5 mM ATP, 10 mM $MgCl_2$, and 1 μg of denatured and reannealed PCR product is incubed for eight minutes at 37°C. The reaction is initiated by adding 30 μl of a mix containing 5 μg of MutS, 12 μg of MutL, and 18 μg of MutH in 20 mM potassium phosphate pH 7.4, 50 mM KCL, 0.1 mM EDTA, 1 mM DTT, 1 mg/ml BSA. The reaction is incubated for 45 minutes at 37°C. The reactions can be supplemented with an additional 30 μl of the MutHSL mix, as well as 3 μl of a solution containing 500 mM Hepes pH 8.0, 200 mM KCL, 10 mM DTT, 20 mM ATP, and 40 mM $MgCl_2$. Incubation is continued at 37°C for 45 minutes. Supplementation and incubation is repeated. After final incubation, the reaction is stopped with the addition of EDTA to 10 mM. Standard molecular biology methods are used to concentrate, purify, and clone.

Chemical methods for mismatch cleavage can be used instead or in combination with endonucleases for removal of polynucleotides bearing undesirable mutations. These methods rely on the chemical modification of the mismatch by treatment of heteroduplex DNA with hydroxylamine and osmium tetroxide. See, for example, Cotton et al., 1998, Proc Natl Acad Sci 85, 4397-4401, which is hereby incorporated by reference in its entirety) or potassium permanganate and tetraethylammonium chloride. See, for example, Roberts et al., 1997, Nucl Acids Res 25, 3377-3378, which is hereby incorporated by reference in its entirety. This is followed by treatment with piperidine to cleave the DNA at the modified site. For example, 1-5 μg of denatured and reannealed PCR product in 6 μl of distilled water is added to 20 μl of a hydroxylamine solution, which is prepared by dissolving 1.39 g ofhydroxulamine hydrochloride in 1.6 ml of distilled water for an -2.5 M solution. The DNA-hydroxylamine solution is incubated for 2 hours at 37°C. The reaction is stopped by transferring the mixture to ice and adding 200 μl of a stop solution consisting of 0.3 M sodium acetate, 0.1 mM EDTA pH 5.2, and 25 μg/ml tRNA. The DNA is precipitated with ethanol, washed with 70% ethanol and dried. The pellet is suspended in 6 μl of distilled water and treated with 15 μl of 4% osmium tetroxide in a total volume of 24.5 μl containing 1 mM EDTA, 10 mM Tris-Cl pH 7.7, and 1.5% pyridine. The sample is incubated for 20-120 minutes at 37°C. The reaction is stopped and pelleted as described for hydroxylamine step. Chemical cleavage is achieved by incubating the DNA with piperidine. For this, 50 ml of 1 M piperidine is added directly to the pellet and incubated at 90°C for 30 minutes. The DNA is precipitated with ethanol and suspended in 20 μl Tris-Cl pH 8.8, Rnase A 0.5 mg/ml (70 U/mg) before purification and cloning. Immobilization of the starting DNA (denatured and reannealed) to a solid support, such as a silica solid support (Ultra-bind beads from MO BIO Laboratories, Inc.) allows the chemical methods for mismatch cleavage to be performed without ethanol precipitation between each step. See, for example, Bui et al. 2003, BMC Chemical Biology 3; http://www.biomedcentral.com/content/pdf/1472-6769-3-1.pdf, which is hereby incorporated by reference in its entirety.

Rhodium(III) complexes can be used for high-affinity mismatch recognition and photocleavage. See, for example, Junicke et al. 2003, Proc Natl Acad Sci 100: 3737-3742, which is hereby incorporated by reference in its entirety. Two such complexes are $[Rh(bpy)^2(chrysi)]^{3+}$ [chrysene-5,6-quinone diimine (chrysi)] and rac-$[Rh(bpy)_2phzi]^{3+}$ (bpy, 2,2'-bipyridine; phzi benzo[a]phenazine-5,6-quinone diimine). Binding is carried out in a mix containing 1-5 $\mu$g of heteroduplex DNA, 1-100 $\mu$M of the Rhodium(III) complex, 50 mM NaCl, 10 mM Tris-HCl, pH 8.5. The mix is irradiated for 15 minutes to one hour at wavelengths ranging from 300-600 nm.

Denaturing HPLC (dHPLC) on a sample of the amplified synthetic gene that has undergone one of the described treatments to form heteroduplexes can be used to separate heteroduplexes from homoduplexes (correct sequences). An example of a dHPLC system capable of performing this separation is the WAVE® system manufactured and sold by Transgenomic, Inc. (Omaha, NE). Using this system, the sample containing homoduplexes and heteroduplexes (if a mutation is present) is injected into the buffer flow path containing triethyammonium acetate (TEAA) and acetonitrile (ACN). In solution, the TEAA forms the positively charged triethylammonium ion (TEA+) that has both hydrophobic and hydrophilic ends. The DNASep® cartridge is located in the oven and contains beads that are hydrophobic. When the buffer passes through the cartridge the hydrophobic end of the TEA+ is attracted to the beads. The positively charged portion of the TEA+ forms an ionic bond with the negatively charged phosphate backbone of the DNA. The result is that the DNA fragments are held onto the cartridge by these bridging properties of the TEA+ ions. The fragment specific methods created by Navigator™ Software control both the temperature of the oven and the ACN gradient. The concentration of ACN increases over time based on this method. As the ACN concentration increases bridging capabilities of the TEA+ ions decrease and the DNA fragments are released from the cartridge. Heteroduplexes, with mismatched base pairs, elute off of the cartridge first followed by the homoduplexes. The homoduplex fraction is enriched for correct sequences and can be collected and cloned. Denaturing HPLC-mediated separation of heteroduplexes from homoduplexes is preferably performed on synthetic constructs <500 bp in length. Larger genes can be assembled from the collected homoduplexes using PCR SOEing (splicing by overlap extension) or type II restriction digest and ligation.

Following the assembly or amplification or mismatch digestion steps, the polynucleotide can be cloned into an appropriate vector, either by restriction digestion and ligation, TA cloning or recombinase-based cloning. Site-specific recombinase-based cloning is particularly advantageous because it requires a specific sequence to be present at each end of the polynucleotide. This provides a strong selection against partially assembled polynucleotides that lack one or both ends. Thus using recombinases for cloning eliminates any need for gel-purification of the polynucleotide prior to cloning, thus increasing the efficiency and fidelity of the process. Recombinase-based cloning of assembled synthetic oligonucleotides is thus an aspect of the invention. The efficiency of recombinase-based cloning also makes possible the assembly of polynucleotides using a ligation or ligase chain reaction strategy, and to omit the PCR amplification step.

### 5.5.5 DESIGN OF A POLYNUCLEOTIDE FOR SYNTHESIS IN MULTIPLE PARTS

In some cases it may not be possible to design and synthesize a polynucleotide in a single step. For example, the sequence may be too large, or it may be too repetitive to synthesize in a single unit. In this case, synthesis of the polynucleotide can be achieved by separately synthesizing two or more smaller polynucleotides and then enzymatically joining these, for example by restriction digestion and ligation, or splicing by overlap extension, Horton et al., 1989, Gene 77: 61-8, to form a single polynucleotide.

The division of the polynucleotide sequence into parts prior to synthesis can be performed manually or automatically using a computer. The most advantageous division of a sequence into parts will separate repeated sequence elements into different synthetic units, to reduce the possibility of incorrect oligonucleotide partner annealing. In one aspect of the invention, division of a polynucleotide into parts can be performed after the oligonucleotides have been designed and synthesized. The polynucleotide can then be assembled as two or more segments that can subsequently be joined for example by overlap extension. In another aspect of the invention, a polynucleotide can be divided into parts in conjunction with the design methods shown in Figures 26, 27 and 28. These methods are not computationally intensive, and can therefore be repeated many times using only a small amount of processor time. Examples of such a design process are shown in Figures 44 and 45. Processes that iterate polynucleotide design, oligonucleotide design and polypeptide or polynucleotide division allow many possible designs to be tested and one that fulfills multiple design criteria to be selected, thereby increasing the efficiency and fidelity of polynucleotide synthesis. These methods and computer programs that automate these processes are aspects of the invention.

Polynucleotides that are designed in parts must subsequently be joined to produce a single polynucleotide. This may be accomplished by adding sequences to the ends of polynucleotides containing recognition sites for restriction endonucleases. Particularly useful are the typeIIs restriction endonucleases that cut outside their recognition sequences. Adding these sites to the end of a polynucleotide sequence can allow two polynucleotides to be joined without the addition of any other sequence to the final polynucleotide. Figure 41 shows sequences that can be added to the 5' end of one polynucleotide and to the 3' end of another polynucleotide to produce two polynucleotide fragments that will produce a single designed sequence after digestion with the named restriction enzyme and ligation. Two or more fragments can be ligated simultaneously to form a single polynucleotide. The addition of these sequences can be automated. For example a two, three or four base sequence within a polynucleotide can be selected, either manually or automatically, and a computer program can then be used to add the desired ends to the 5' and 3' polynucleotide segments. In addition to adding sequences that facilitate subsequent joining of polynucleotide segments, it can be advantageous to add further sequences that facilitate recombinase-based cloning of the polynucleotides. This can also be accomplished using a computer program. Computer programs that automatically add type IIs restriction sites and recombinase cloning sites to the ends of polynucleotides aid in the efficient and high fidelity synthesis of polynucleotides and are an aspect of the invention.

### 5.5.6 VECTORS FOR SYNTHETIC POLYNUCLEOTIDES

Vectors that are amenable to the polynucleotide synthesis and joining processes include those that lack type IIs restriction endonuclease sites, and those that allow cloning using recombinases. Examples of such vector sequences are shown in Figures 46, 47 and 48. Polynucleotide fragments can be designed, synthesized and cloned into such a vector, then excised with one of many possible type IIs restriction enzymes without cutting the vector. Additional features that can be advantageous are replication origins that produce low copy number plasmids. These increase the stability of large segments of DNA. Such vectors increase the efficiency and fidelity of polynucleotide assembly and are an aspect of the invention.

There is also provided a method of designing a single designed polynucleotide having a sequence. In the method, the sequence is divided into a plurality of polynucleotide sub-sequences. A first restriction site is added to a 3' end of a first polynucleotide sub-sequence in the plurality of polynucleotide sub-sequences. A second restriction site is added to a 5' end of a second polynucleotide sub-sequence in the plurality of polynucleotide sub-sequences such that cleavage of the first restriction site and the second restriction site causes a terminal portion of the first polynucleotide sub-sequence to become complementary with a terminal portion of the second polynucleotide sub-sequence. For each respective sub-sequence in the plurality of polynucleotide subsequences a series of steps are performed. First, a first plurality of single-stranded oligonucleotides that collectively encode all or a portion of a first strand of the respective sub-sequence are identified, where each respective single-stranded oligonucleotide in the first plurality of single-stranded oligonucleotides is characterized by an annealing temperature to its exact complement that is in a first predetermined annealing temperature range. Second, a second plurality of single-stranded oligonucleotides is identified from the first plurality of single-stranded oligonucleotides, where a single-stranded oligonucleotide in the second plurality of single-stranded oligonucleotides is formed by joining an adjacent pair of oligonucleotides in the first plurality of single-stranded oligonucleotides. Third, a plurality of single-stranded oligonucleotides that collectively encode all or a portion of a second strand of the respective sub-sequence is identified, where each respective single-stranded oligonucleotide in the third plurality of single-stranded oligonucleotides is characterized by an annealing temperature to its exact complement that is in a second predetermined annealing temperature range. Fourth, a plurality of single-stranded oligonucleotides from the third plurality of single-stranded oligonucleotides is identified, where a single-stranded oligonucleotide in the fourth plurality of single-stranded oligonucleotides is formed by joining an adjacent pair of oligonucleotides in the third plurality of single-stranded oligonucleotides. Here, a set of oligonucleotides comprises the second plurality of oligonucleotides and the fourth plurality of oligonucleotides. Fifth, a determination is made as to whether the set of oligonucleotides satisfies at least one assembly criterion, where when the set of oligonucleotides satisfies the at least one assembly criterion, the set of oligonucleotides is selected, when the set of oligonucleotides does not satisfy the at least one assembly criterion, the set of oligonucleotides is rejected and steps one through five are repeated. The process is repeating when a set of oligonucleotides has not been selected for each respective sub-sequence in the plurality of polynucleotide sub-sequences.

In some embodiments, the first restriction site is for a restriction enzyme that cleaves outside its recognition sequence such as a typeIIs site. In some embodiments, the second restriction site is for a restriction enzyme that cleaves outside its recognition sequence such as a typeIIs site.

In some embodiments, method further comprises assembling the set of oligonucleotides for a respective sub-sequence in the plurality of polynucleotide subsequences using a polymerase chain reaction or a ligase chain reaction with an annealing temperature that is a predetermined amount lower than the lowest annealing temperature of any intended complementary pair of single-stranded oligonucleotides in the set of oligonucleotides, and, repeating this step for each respective sub-sequence in the plurality of polynucleotide sub-sequences.

In some embodiments, the method further comprises cloning each polynucleotide sub-sequence into a vector and obtaining each cloned polynucleotide sub-sequence from its vector and joining the sub-sequences to form the single designed polynucleotide.

There is also provided a computer program product for use in conjunction with a computer system, the computer program product comprising a computer readable storage medium and a computer program mechanism embedded therein, the computer program mechanism for designing a single designed polynucleotide having a sequence. The computer program mechanism comprises instructions for dividing the sequence into a plurality of polynucleotide sub-sequences and instructions for adding a first restriction site to a 3' end of a first polynucleotide sub-sequence in said plurality of polynucleotide sub-sequences. The computer program mechanism further comprises instructions for adding a second restriction site to a 5' end of a second polynucleotide sub-sequence in the plurality of polynucleotide sub-sequences, such that cleavage of the first restriction site and the second restriction site would cause a terminal portion of the first polynucleotide sub-sequence to become complementary with a terminal portion of the second polynucleotide sub-sequence. For each respective sub-sequence in the plurality of polynucleotide sub-sequences, the computer program product comprises (i) instructions for identifying a first plurality of single-stranded oligonucleotides that collectively encode all or a portion of a first strand of the respective sub-sequence, where each respective single-stranded oligonucleotide in the first plurality of single-stranded oligonucleotides is characterized by an annealing temperature to its exact complement that is in a first predetermined annealing temperature range, (ii) instructions for identifying a second plurality of single-stranded oligonucleotides from the first plurality of single-stranded oligonucleotides, where a single-stranded oligonucleotide in the second plurality of single-stranded oligonucleotides is formed by joining an adjacent pair of oligonucleotides in the first plurality of single-stranded oligonucleotides; (iii) instructions for identifying a third plurality of single-stranded oligonucleotides that collectively encode all or a portion of a second strand of said respective sub-sequence, where each respective single-stranded oligonucleotide in the third plurality of single-stranded oligonucleotides is characterized by an annealing temperature to its exact complement that is in a second predetermined annealing temperature range; (iv) instructions for identifying a fourth plurality of single-stranded oligonucleotides from the third plurality of single-stranded oligonucleotides, where a single-stranded oligonucleotide in the fourth plurality of single-stranded oligonucleotides is formed by joining an adjacent pair of oligonucleotides in the third plurality of single-stranded oligonucleotides; where a set of oligonucleotides comprises the second plurality of oligonucleotides and the fourth plurality of oligonucleotides; and (v) instructions for determining whether the set of oligonucleotides satisfies at least one assembly criterion, where, when the set of oligonucleotides satisfies the at least one assembly criterion, the set of oligonucleotides is selected; and when the set of oligonucleotides does not satisfy the at least one assembly criterion, the set of oligonucleotides is rejected and instructions (i) through (v) are repeated. The computer program product further includes instructions for repeating the aforementioned instructions when a set of oligonucleotides has not been selected for each respective sub-sequence in said plurality of polynucleotide sub-sequences. Some aspect of the present invention comprises a computer system for designing a single designed polynucleotide having a sequence, the computer system comprising a central processing unit and a memory, coupled to the central processing unit, where the memory stores the above identified computer program product.

There is also provided a method of cloning a polynucleotide, where the polynucleotide comprises i) a desired sequence, ii) a first restriction site at the 3' end of the desired sequence; iii) a second restriction site at the 5' end of the desired sequence; iv) a first recognition site that is recognized by a site-specific recombinase, where the first recognition site is outside the desired sequence and is in a 3' terminal portion of the polynucleotide; and v) a second recognition site sequence that is recognized by said site-specific recombinase, wherein the second recognition site is outside the desired sequence and is in a 5' terminal portion of the polynucleotide. The method comprises a) assembling the polynucleotide from a plurality of component oligonucleotides; and b) cloning the polynucleotide into a vector comprising a plurality of sites recognized by the site-specific recombinase, using a recombinase to effect the cloning.

In some embodiments, the vector does not comprise a recognition sequence for the first restriction site or the second restriction site. In some embodiments, a recognition sequence for the first restriction site is not in the desired sequence. In some embodiments, a recognition sequence for the second restriction site is not in the desired sequence.

In some embodiments, the method further comprises amplifying the nucleotide while the nucleotide is in the vector; and cleaving the polynucleotide from the vector using the first restriction site and the second restriction side, thereby deriving a polynucleotide having the desired sequence.

There is also provided a method of designing a polynucleotide that has a first oligonucleotide sequence, the method comprising (a) selecting an initial codon sequence that codes for the polypeptide, where a codon frequency in the initial codon sequence is determined by a codon bias table; and (b) modifying an initial codon choice in the initial codon sequence in accordance with a design criterion, thereby constructing a codon sequence that codes for the first oligonucleotide sequence. Then, a set of oligonucleotides is designed for assembly into a second oligonucleotide sequence, where the second oligonucleotide sequence encodes a contiguous portion of the first oligonucleotide sequence. Such a designing step (c) comprises: (i) identifying a first plurality of single-stranded oligonucleotides that collectively encode all or a portion of a first strand of said second oligonucleotide sequence, where each respective single-stranded oligonucleotide in the first plurality of single-stranded oligonucleotides is characterized by an annealing temperature to its exact complement that is in a first predetermined annealing temperature range; (ii) identifying a second plurality of single-stranded oligonucleotides from the first plurality of single-stranded oligonucleotides, where a single-stranded oligonucleotide in the second plurality of single-stranded oligonucleotides is formed by joining an adjacent pair of oligonucleotides in the first plurality of single-stranded oligonucleotides; and (iii) identifying a third plurality of single-stranded oligonucleotides that collectively encode all or a portion of a second strand of the second oligonucleotide sequence, where each respective single-stranded oligonucleotide in the third plurality of single-stranded oligonucleotides is characterized by an annealing temperature to its exact complement that is in a second predetermined annealing temperature range; (iv) identifying a fourth plurality of single-stranded oligonucleotides from the third plurality of single-stranded oligonucleotides, where a single-stranded oligonucleotide in the fourth plurality of single-stranded oligonucleotides is formed by joining an adjacent pair of oligonucleotides in the third plurality of single-stranded oligonucleotides. In the method, a set of oligonucleotides comprises the second plurality of oligonucleotides and the fourth plurality of oligonucleotides. A determination is made as to whether the set of oligonucleotides satisfies at least one assembly criterion, where when the set of oligonucleotides satisfies the at least one assembly criterion, the set of oligonucleotides is selected, and when the set of oligonucleotides does not satisfy the at least one assembly criterion, the set of oligonucleotides is rejected and the aforementioned steps are repeated.

In some embodiments a different first predetermined annealing temperature range and a different second predetermined annealing temperature range is used when steps i) through v) are repeated. In some embodiments the first predetermined annealing temperature range and the second predetermined annealing temperature range are the same. In some embodiments, the first predetermined annealing temperature range and the second predetermined annealing temperature range are different. In some embodiments, the first predetermined annealing temperature range and the second predetermined annealing temperature range is each between 45°C and 72°C, between 50°C and 65°C, or between 55°C and 62°C.

In some embodiments, each single-stranded oligonucleotide in the second plurality of single-stranded oligonucleotides is formed by joining an adjacent pair of oligonucleotides in the first plurality of single-stranded oligonucleotides. In some embodiments, each single-stranded oligonucleotide in the fourth plurality of single-stranded oligonucleotides is formed by joining an adjacent pair of oligonucleotides in the third plurality of single-stranded oligonucleotides.

In some embodiments, the method further comprises assembling the set of oligonucleotides by the polymerase chain reaction or ligase chain reaction with an annealing temperature that is a predetermined amount lower than the lowest annealing temperature of the first predetermined annealing temperature range, thereby forming an assembly mixture that comprises the polynucleotide. In some embodiments, the predetermined amount is 1 °C or larger.

In some embodiments, the method further comprises cloning the polynucleotide into a vector. In some embodiments, the assembly mixture comprises a plurality of different polynucleotide molecules, the method further comprising creating a plurality of heteroduplexes between different individual polynucleotide molecules within the plurality of different polynucleotide molecules in the assembly and then treating the plurality of heteroduplexes with an agent that binds preferentially to mismatched sequences within a double-stranded DNA molecule. In some embodiments, the agent is used to remove double-stranded DNA molecules containing mismatched sequences from the assembly mixture.

In some embodiments, the method further comprises amplifying the polynucleotide by the polymerase chain reaction. In some embodiments, the method further comprises cloning the polynucleotide into a vector. In some embodiments, the at least one assembly criterion comprises a requirement that the annealing temperature of each intended complementary pair of single-stranded oligonucleotides in the set of oligonucleotides falls within a third predetermined temperature range. In some embodiments, the third predetermined temperature range encompasses a total of 4°C or less. In some embodiments, the third predetermined temperature range encompasses a total of 3°C or less. In some embodiments, the at least one assembly criterion comprises a requirement that the single-stranded oligonucleotide length of each oligonucleotide in the set of oligonucleotides is within a predetermined oligonucleotide length range. In some embodiments, the predetermined oligonucleotide length range is between 20 nucleotides and 70 nucleotides, or between 25 nucleotides and 65 nucleotides.

In some embodiments, the at least one assembly criterion comprises a requirement that the number of single-stranded oligonucleotides in the second plurality of single-stranded oligonucleotides matches the number of single-stranded oligonucleotides in the fourth plurality of single-stranded oligonucleotides. In some embodiments, the at least one assembly criterion comprises a requirement that the annealing temperature of each pair of single-stranded oligonucleotides in the set of oligonucleotides for assembly, whose annealing is not intended for said assembly, is below a predetermined temperature. In some embodiments, the predetermined temperature is the annealing temperature of a pair of oligonucleotides in the set of oligonucleotides whose annealing is intended for assembly of the polynucleotide. In some embodiments, the predetermined temperature is 10°C below, 15°C below, or 20°C below the annealing temperature of a pair of oligonucleotides in the set of oligonucleotides whose annealing is intended for assembly of the polynucleotide. In some embodiments, the at least one assembly criterion comprises a requirement that a maximum length of a sequence that occurs more than once within the first strand of the polynucleotide and that is found at a terminus of any oligonucleotide in the set of oligonucleotides is less than a predetermined length. In some embodiments, the predetermined length is 10 nucleotides or greater, or 12 nucleotides or greater. In some embodiments, a pair of oligonucleotides in the set of oligonucleotides that are intended to be annealed to form the polynucleotide are not completely overlapping. In some embodiments, a first single-stranded oligonucleotide has an n-mer overhang relative to a second single-stranded oligonucleotide in the set of oligonucleotides, and annealing of the first single-stranded oligonucleotide and the second oligonucleotide single-stranded oligonucleotide is intended for assembly of the polynucleotide, where n is between 1 and 40.

In some embodiments, the at least one assembly criterion comprises a requirement that a predetermined length of a nucleotide sequence at a terminus of an oligonucleotide in the set of oligonucleotides is not found at either terminus of any other oligonucleotide in the set of oligonucleotides. In some embodiments, the predetermined length is 5 nucleotides, 4 nucleotides, or 3 nucleotides.

In some embodiments, the design criterion comprises one or more of:

(i) exclusion of a restriction site sequence in said initial polynucleotide sequence;
(ii) incorporation of a restriction site sequence in said initial polynucleotide sequence;
(iii) a designation of a target G+C content in the initial polynucleotide sequence;
(iv) an allowable length of a sub-sequence that can be exactly repeated within either strand of the initial polynucleotide sequence;
(v) an allowable annealing temperature of any sub-sequence to any other sub-sequence within either strand of the initial polynucleotide sequence;
(vi) exclusion of a hairpin turn in the initial polynucleotide sequence;
(vii) exclusion of a repeat element in the initial polynucleotide sequence;
(viii) exclusion of a ribosome binding site in the initial polynucleotide sequence;
(ix) exclusion of a polyadenylation signal in the initial polynucleotide sequence;
(x) exclusion of a splice site in the initial polynucleotide sequence;
(xi) exclusion of an open reading frame in each possible 5' reading frame in the initial polynucleotide sequence;
(xii) exclusion of a polynucleotide sequence that facilitates RNA degradation in the initial polynucleotide sequence;
(xiii) exclusion of an RNA polymerase termination signal in the initial polynucleotide sequence;
(xiv) exclusion of a transcriptional promoter in the initial polynucleotide sequence;
(xv) exclusion of an immunostimulatory sequence in the initial polynucleotide sequence;
(xvi) incorporation of an immunostimulatory sequence in the initial polynucleotide sequence;
(xvii) exclusion of an RNA methylation signal in the initial polynucleotide sequence;
(xviii) exclusion of a selenocysteine incorporation signal in the initial polynucleotide sequence;
(xix) exclusion of an RNA editing sequence in the initial polynucleotide sequence;
(xx) exclusion of an RNAi-targeted sequence in the initial polynucleotide sequence; and
(xxi) exclusion of an inverted repeat within the first 45 nucleotides encoding said polypeptide in the initial polynucleotide sequence.

In some embodiments, the design criterion comprises reduced sequence identity to a reference polynucleotide, and modifying the initial codon choice in the initial polynucleotide in accordance with the design criterion comprises altering a codon choice in the initial polynucleotide sequence to reduce sequence identity to the reference polynucleotide.

In some embodiments, the design criterion comprises increased sequence identity to a reference polynucleotide, and modifying the initial codon choice in the initial polynucleotide in accordance with the design criterion comprises altering a codon choice in the initial polynucleotide sequence to increase sequence identity to the reference polynucleotide.

In some embodiments, the method further comprise assembling the set of oligonucleotides by the polymerase chain reaction or ligase chain reaction with an annealing temperature that is a predetermined amount lower than the lowest annealing temperature of the first predetermined annealing temperature range, thereby forming an assembly mixture that comprises an oligonucleotide with the second oligonucleotide sequence. In some embodiments, the predetermined amount is 1°C or larger. In some embodiments, the method further comprises cloning the oligonucleotide with the second oligonucleotide sequence into a vector. In some embodiments, the assembly mixture comprises a plurality of different polynucleotide molecules, and the method further comprises creating a plurality of heteroduplexes between different individual polynucleotide molecules within the plurality of different polynucleotide molecules in the assembly and treating the plurality of heteroduplexes with an agent that binds preferentially to mismatched sequences within a double-stranded DNA molecule; and using the agent to remove double-stranded DNA molecules containing mismatched sequences from the assembly mixture. In some embodiments, the method further comprises amplifying the oligonucleotide with the second oligonucleotide sequence by the polymerase chain reaction.

In some embodiments, the method further comprises cloning the oligonucleotide with the second oligonucleotide sequence into a vector. The method further comprises repeating the selecting, modifying, and designing when repetition of steps i) through v) fails to identify a set of oligonucleotide that satisfies said at least one assembly criterion.

There is also provided a computer program product for use in conjunction with a computer system, the computer program product comprising a computer readable storage medium and a computer program mechanism embedded therein, the computer program mechanism for designing a polynucleotide that has a first oligonucleotide sequence. The computer program mechanism comprises a) instructions for selecting an initial codon sequence that codes for the polypeptide, where a codon frequency in the initial codon sequence is determined by a codon bias table; b) instructions for modifying an initial codon choice in the initial codon sequence in accordance with a design criterion, thereby constructing a codon sequence that codes for said first oligonucleotide sequence; c) instructions for designing a set of oligonucleotides for assembly into a second oligonucleotide sequence, where the second oligonucleotide sequence encodes a contiguous portion of the first oligonucleotide sequence, the designing step c) comprising: (i) instructions for identifying a first plurality of single-stranded oligonucleotides that collectively encode all or a portion of a first strand of the second oligonucleotide sequence, where each respective single-stranded oligonucleotide in the first plurality of single-stranded oligonucleotides is characterized by an annealing temperature to its exact complement that is in a first predetermined annealing temperature range; (ii) instructions for identifying a second plurality of single-stranded oligonucleotides from the first plurality of single-stranded oligonucleotides, where a single-stranded oligonucleotide in the second plurality of single-stranded oligonucleotides is formed by joining an adjacent pair of oligonucleotides in the first plurality of single-stranded oligonucleotides; (iii) instructions for identifying a third plurality of single-stranded oligonucleotides that collectively encode all or a portion of a second strand of the second oligonucleotide sequence, where each respective single-stranded oligonucleotide in the third plurality of single-stranded oligonucleotides is characterized by an annealing temperature to its exact complement that is in a second predetermined annealing temperature range; (iv) instructions for identifying a fourth plurality of single-stranded oligonucleotides from the third plurality of single-stranded oligonucleotides, where a single-stranded oligonucleotide in the fourth plurality of single-stranded oligonucleotides is formed by joining an adjacent pair of oligonucleotides in the third plurality of single-stranded oligonucleotides; where a set of oligonucleotides comprises the second plurality of oligonucleotides and the fourth plurality of oligonucleotides; and (v) instructions for determining whether the set of oligonucleotides satisfies at least one assembly criterion, where when the set of oligonucleotides satisfies the at least one assembly criterion, the set of oligonucleotides is selected; and when the set of oligonucleotides does not satisfy the at least one assembly criterion, the set of oligonucleotides is rejected and the aforementioned instructions are repeated. Still another aspect of the present invention provides a computer system

There is also provided a computer system for designing a polynucleotide that has a first oligonucleotide sequence, the computer system comprising a central processing unit and a memory, coupled to the central processing unit, the memory storing the aforementioned computer program product.

## 5.6 INTEGRATED DEVICES FOR POLYNUCLEOTIDE SYNTHESIS

The methods described here may be conveniently performed using a single device capable of performing one or more of the functions required for synthesis of a polynucleotide. A schematic representation of the functions performed by different modules of a polynucleotide synthesizing device is shown in Figure 49. In particular, Fig. 49 illustrates a system 10 that is operated in accordance with one embodiment of the present invention. System 10 comprises standard components including a central processing unit 22, and memory 36 for storing program modules and data structures, user input/output device 32, a network interface 20 for coupling computer 10 to other computers via a communication network 34, and one or more busses 30 that interconnect these components. User input/output device 32 comprises one or more user input/output components such as a mouse, display 26, and keyboard 28. In some embodiments, some of the program modules and data structures are stored in a permanent storage device 14 that is controlled by controller 12. In some embodiments, device 14 is a hard disk. System 10 further includes a power source 24 to power the aforementioned components.

Memory 36 comprises a number of modules and data structures that are used in accordance with the present invention. It will be appreciated that, at any one time during operation of the system, a portion of the modules and/or data structures stored in memory 36 is stored in random access memory while another portion of the modules and/or data structures is stored in non-volatile storage 14. In a typical embodiment, memory 36 comprises an operating system. The operating system comprises procedures for handling various basic system services and for performing hardware dependent tasks. Memory 36 further comprises a file system for file management. In some embodiments, the file system is a component of the operating system. Memory 36 and/or 14 also comprises the modules described below.

*Design module.* This is a primarily bioinformatic module that performs the following tasks. 1. Polynucleotide design (for example design of a polynucleotide to encode a specific polypeptide, reduction or elimination of repeat elements, design of two or more polynucleotides for synthesis and joining to form a single polynucleotide. Examples include computer programs that perform the processes shown in Figures 26, 27, 28, 44 and 45). 2. Oligonucleotide design (for example reduction or elimination of annealing regions in incorrect annealing partners, design of a "constant Tm" set. Examples include computer programs that perform the processes shown in Figures 29, 30, 31 and 42). 3. Select the assembly conditions appropriate for the designed oligonucleotide set (for example the annealing temperature, the number of cycles and time for each cycle, the use of polymerase or ligase-based assembly conditions. Examples include the conditions shown in Figures 32, 33, 34, 35, 36 and 43).

*Oligonucleotide Synthesis Module.* This module performs the physical process of oligonucleotide synthesis. The input to this module is a set of oligonucleotide sequences that is provided by the design module. The oligonucleotide synthesis module could be an outside oligonucleotide vendor that receives the sequence information electronically either directly form the design module, or via an intermediary such as an ordering system. The oligonucleotide synthesis module could also be an oligonucleotide synthesis machine that is physically or electronically linked to and instructed by the design module. The oligonucleotide synthesis module could synthesize oligonucleotides using standard phosphoramidite chemistry, or using the modifications described here.

*Synthesis module.* This module performs the physical process of assembling oligonucleotides into a polynucleotide. The synthesis module receives informational input from the design module, to set the parameters and conditions required for successful assembly of the oligonucleotides. It also receives physical input of oligonucleotides from the oligonucleotide synthesis module. The synthesis module is capable of performing variable temperature incubations required by polymerase chain reactions or ligase chain reactions in order to assemble the mixture of oligonucleotides into a polynucleotide. For example the synthesis module can include a thermocycler based on Peltier heating and cooling, or based on microfluidic flow past heating and cooling regions. The synthesis module also performs the tasks of amplifying the polynucleotide, if necessary, from the oligonucleotide assembly reaction. The synthesis module also performs the task of ligating or recombining the polynucleotide into an appropriate cloning vector.

*Transformation module.* This module performs the following tasks. 1. Transformation of the appropriate host with the polynucleotide ligated into a vector. 2. Separation and growth of individual transformants (*e.g.* flow-based separations, plating-based separations). 3. Selection and preparation of individual transformants for analysis.

*Analysis module.* This module performs the following tasks. 1. Determination of the sequence of each independent transformant. This can be done using a conventional sequencer using extension and termination reactions e.g. with dye terminators such as those recognized by Applied Biosystems Machines 3100, 3130 *etc.* Alternatively use of sequencing technologies developed for determining the sequence of polynucleotides whose sequence is already approximately known ("re-sequencing technologies") can also be used to more cheaply identify errors incorporated during polynucleotide synthesis. These include hybridization-based technologies. 2. Comparison of the determined sequence with the sequence that was designed. 3. Identification of transformants whose sequence matches the designed sequence.

The modules described above and depicted in Figure 49 can be physically distinct or combined into five or fewer devices. Computer programs to effect communication between the modules, as well as to perform the functions of each module, are an aspect of the invention.

## 6. EXAMPLES

The following examples are set forth so as to provide those of ordinary skill in the art with a complete description of how to make and use embodiments of the present invention, and are not intended to limit the scope of what is regarded as the invention.

### 6.1 INCREASED COUPLING EFFICIENCY ON A CONTROLLED PORE GLASS SUPPORT

The coupling efficiency of the modified protocol of Figure 13 is shown by a comparison between literature descriptions of high quality, low quality and gel purified oligonucleotides (Figure 14A-C), a commercially purchased oligonucleotide (Figure 14D) and oligonucleotides synthesized by the modified procedure (Figure 14E and F). The modified procedure described herein showed a coupling efficiency of 99.9%.

To measure the coupling efficiency the capping step was eliminated and multiple coupling cycles were performed as described in, for example, Matteucci and Caruthers, 1981, J Am Chem Soc 103, 3185-3191; Pon et al., 1985, Tetrahedron Lett. 26, 2525-2528; Adams et al., 1983, J Am Chem Soc 105, 661-663; McBride et al., 1986, J Am Chem Soc 108,2040-2048; Letsinger et al., 1984, Tetrahedron 40, 137-143; Hayakawa et al., 1990, J Am Chem Soc 112, 1691-1696; and Hayakawa & Kataoka, 1998, J Am Chem Soc 120, 12395-12401, which are hereby incorporated by reference in their entireties. The oligonucleotide synthesis products were analyzed by HPLC. Comparisons of the amounts of full-length and truncated products were used to calculate the coupling efficiencies of standard and modified procedures.

### Protocols for oligonucleotide synthesis on CPG and quartz rods: chain propagation steps.

A desoxythymidine modified quartz rod (4mm diameter) and/or 5 mg of dT-CPG 500 (Glen Research) were placed inside a 2mm filter funnel attached at the top to an argon line (2-3psi) and at the bottom to a waste line. When both rod and CPG were used, the rod was installed with its derivatized surface 0.5 mm above the CPG layer. CPG particles stuck to the wall were washed down with acetonitrile (1ml). After sedimentation of all glass particles, the system was purged with argon for one minute before addition of 200ul of capping reagent, prepared by mixing the equal amounts of stock solution A (1ml $Ac_2O$, 9ml DMA) and stock solution B (1.2g DMAP, 7.3ml DMA, 1.5ml 2,6-lutidine, was added to block all untritylated reactive groups for one minute followed by washes with acetonitrile (500μl), methanol (2x500μl), acetonitrile (2x500μl) and drying under argon flow for 1 minute. Detritylation with 15% dichloroacetic acid in methylene chloride (200μl) was performed for one minute. This reagent was removed by applying positive argon pressure, solid supports were washed with methyl cyanide (HPLC grade, 4x500μl) and dried for two minutes under argon flow. During this time 0.1M dimethoxytritylthymidine phosphoramidite solution in dry acetonitrile (100μl) was preactivated by mixing inside the Hamilton syringe (500μl) with 0.4M tetrazole solution in acetonitrile (100μl) with an argon bubble as an air-free mixer. After approximately 1.5 minutes of activation period, the phosphoramidite solution (200μl) was added to the dry solid support under argon: inert gas continued to flow on the top of the filter to prevent air from entering. After a one minute coupling step, reagents were washed away by acetonitrile (4x500μl) and argon was purged through the filter funnel for one minute. Oxidation was performed for one minute by addition of an aliquot (200μl) of 0.12M aqueous iodine stock solution that was prepared the same day. After completion of oxidation the supports were washed with acetonitrile (4x500μl), dried under argon for one minute and capped as described at the beginning of this paragraph. Following capping either the next synthesis cycle was repeated or the cleavage step was initiated.

**Protocols for oligonucleotide synthesis on CPG: Cleavage of oligos from CPG.**

Dried CPG was treated with 10M ammonia (100μl, N.F. grade, Baker) for two hours at room temperature. Ammonia solution was transferred into 1.5ml Eppendorf vial and solvents evaporated on SpeedVac SVC1000 (Savant). Diluted (1:100) aliquot (2ul) was analyzed by HPLC.

**Protocols for oligonucleotide synthesis on CPG and quartz rods: Determination of coupling efficiency.**

Samples obtained from CPG or rod (with or without PDE-II digestion in solution) were analyzed on an XTerra MS C18 column (3.5μm, 2.1x100mm, Waters) on a Waters HPLC. Four mobile phases were used for separation. Mobile phase A was 0.05M triethylamine acetate at pH=7, mobile phase B was mixture 12% of mobile phase D and 88% of mobile phase A, mobile phase C was 0.1% trifluoroacetic acid and mobile phase D was acetonitrile. The temperature was set at 60°C to prevent hybridization and increase mass-transaction between solid and mobile phases. Flow was 0.4 ml/min for optimal separation on 2.1mm column. Several gradients were followed one after another. During ten minutes of analysis of 5N-O unprotected oligos pool the mobile phase was change by curve #2 (Waters gradient curve specification) from 70% A and 30% B to 18% A and 82% B. After this column was stabilized for seven minutes by flushing with 100% A, dimethoxytrityl group was removed by solvent C for eigth minutes and column was prepared for analysis of "Trityl-OFF" fraction by stabilization washing with 70% A and 30% B for 10 min. The quality of sample adsorbed at the beginning of column was determined by ten minute gradient elution according to curve #2 until 18% A and 82% B was reached. Column was regenerated by streaming of acetonitrile for one minute and stabilized by 70% A and 30% B for twelve minutes to be prepared for the next injection.

**6.2 OLIGONUCLEOTIDE SYNTHESIS ON A NON-POROUS GLASS SUPPORT**

**Protocols for oligonucleotide synthesis on quartz rods: rod derivatization**

The end of a broken quartz rod (L=4cm, D=4 mm, Chemglass) was flattened by polishing on 220 mesh silicone carbide paper. After treatment with 50% w/v sodium hydroxide for ten minutes followed by concentrated nitric acid for five minutes rods were vacuum dried for five minutes, immersed in pyridine:trimethylchlorosilane (2:1) for one minute, washed with methanol and vacuum dried for one minute. The trimethylsilane layer was removed from the end of rod by polishing with 220 mesh carbide paper. Particles formed during the polishing process were blown out using dry air. The rod was inserted into a solution of 1% triethoxyaminopropylsilane in ethanol for one minute. Excess reagent was removed by washing with methanol. The rod was vacuum dried for one minute and treated with a mixture of 0.2 M 3'-succinyl protected nucleotide: 0.8M diisopropylethylamine (DIEA): 0.4M HBTU = 1:1:1 for 10 minutes. After washing with methanol and vacuum drying, the rod with immobilized nucleotide was ready for the nucleotide coupling procedure.

Maximal loading of approximately 1.3nmol /$cm^2$ was achieved on surfaces derivatized for 1-2 minutes (Figure 18A) and coupled for eight to ten minutes (Figure 18B). This loading density is almost ten times higher than previously reported for a glass support but almost ten times lower then described for plasma-activated polystyrene. See, Pon, 2000, Current protocols, UNIT 3.1 and 3.2, which is hereby incorporated by reference in its entirety. Without intending to be limited any particular theory, high loading capacity is attributed to rough micro-surface formed as a result of sandpaper polishing and 50% sodium hydroxide treatment (Figure 16G). Chain propagation steps were performed as for Example 6.1.

**Protocols for oligonucleotide synthesis on quartz rods: Cleavage of oligos from quartz rod.**

The dried rod was placed inside an autoclave (1gal 316SS Pressure Dispenser 130psi, 4355T68 McMaster) containing 50ml of 28% ammonia (technical grade, Lancaster). The temperature inside cleavage chamber was raised to 55°C and the pressure to 35 psi by heating on the water bath to 95°C. After one hour the ammonia gas was let out, the rod removed, and cleaved oligos collected into a Hamilton syringe (10μl) after applying drop of 0.05M phosphate buffer (10μl) to the end of the vertically positioned rod. The entire sample was analyzed by HPLC on narrow bore reverse phase column (2.1mm) with standard UV flow cell (8mm, 12ul). Determination of coupling efficiency was performed as for Example 6.1.

An initial test was performed of the quartz rod support by synthesizing a polythymidine 9mer following the standard procedure. The result was similar to those described by Seliger et al., 1989, J Chromatogr 476, 49-57; LeProust et al., 2001, Nucleic Acids Res 29, 2171-80, each of which is hereby incorporated by reference in its entirety (Figure 20A). When the modified procedure was used, a significant improvement in coupling efficiency, 98.9%, was obtained (Figure 20B), though the 99.9% efficiency obtained using CPG (Figure 20C) was not obtained. Grafted gelatinous polymer support on quartz rods will probably improve coupling efficiency further. See Pon, 2000, Current protocols, UNIT 3.1 and 3.2, which is hereby incorporated by reference in its entirety.

### 6.3 OLIGONUCLEOTIDE SYNTHESIS USING A NEW SYNTHESIZER DESIGN

The apparatus design shown in Figure 19 produced controlled synthetic conditions, suitable reproducibility of experiments and low chemical consumption. To compare the effectiveness of synthesis on CPG and non-porous glass supports an internal adapter was constructed that for the performance of both syntheses simultaneously under identical conditions.

The positive flow technique was used when designing a twelve-pin prototype (Figures 19B-D). A twelve-channel prototype of a 96-well synthesizer was built based on a round bottom 96-well reaction plate. Positive argon pressure created an acceptable level of inert atmosphere during reagent delivery and coupling steps (Figure 19E).

### 6.4 SYNTHESIS OF A 1574 BP GENE

In this example a 1574 BP gene was synthesize. The sequence was initially analyzed for GC content and the presence of repeats using a computer program:

Name=G00277 / Length=1574

Seq=TGCTGGGGAAAAGTAAACACACACAGGCGCACTCGAGAACAGATGAGTTCTTTGGA
CGAGGATGAAGAGGACTTCGAAATGCTGGACACGGAGAACCTCCAGTTTATGGGGAAGAA
GATGTTTGGCAAACAGGCCGGCGAAGACGAGAGTGATGATTTTGCTATAGGGGGTAGCAC
CCCGACCAATAAACTGAAATTTTATCCATATGCGAACAACAAATTGACAAGAGCTACGGG
GACCTTGAACCTGTCATTAAGTAATGCAGCTTTGTCAGAGGCTAACTCCAAATTTCTTGG
GAAAATTGAAGAAGAGGAAGAAGAGGAGGAAGAAGGCAAGGATGAGGAAAGCGTGGATGC
TCGTATTAAAAGGTGGTCTCCGTTCCATGAAAATGAAAGTGTTACTACTCCTATTGCAAA
AAGAGCTGCGGAAAAAACGAACAGTCCTATTGCTCTCAAACAATGGAACCAGCGATGGTT
TCCGAAAAATGATGCTCGCACTGAAAATACATCCTCATCCTCTTCATATAGCGTCGCTAA
ACCTAACCAATCAGCCTTTACGTCTTCGGGCCTCGTATCTAAAATGTCTATGGACACTTC
GTTATACCCTGCGAAATTGAGGATACCAGAAACACCAGTGAAAAAATCACCCTTAGTGGA
GGGAAGAGACCATAAGCATGTCCACCTTTCGAGTTCGAAAAATGCATCGTCTTCTCTAAG
TGTTTCCCCTTTAAATTTTGTTGAAGACAATAATTTACAAGAAGACCTTTTATTTTCAGA
TTCTCCGTCTTCGAAAGCTTTACCTTCCATCCATGTACCAACCATAGACGCATCCCCACT
GAGCGAGGCAAAATATCATGCACATGATCGTCACAATAACCAGACAAACATCCTGTCTCC
CACTAATAGCTTGGTTACCAACAGCTCTCCACAAACATTGCATTCTAACAAGTTCAAAAA
AATCAAAAGAGCAAGGAATTCGGTTATTTTGAAAAATAGAGAGCTAACAAACAGTTTACA
ACAATTCAAAGATGATTTATACGGCACGGACGAGAATTTCCCACCTCCAATCATAATATC
AAGTCATCATTCAACTAGAAAGAACCCTCAACCTTATCAATTTCGTGGACGCTATGACAA
TGACGCTGACGAAGAGATCTCCACTCCAACAAGACGAAAATCTATTATTGGGGCAGCATC
TCAAACACATAGAGAAAGCAGACCATTGTCACTCTCCTCTGCCATCGTGACAAACACAAC
AAGTGCAGAGACGCATTCCATATCTTCCACCGATTCTTCGCCGTTAAATTCCAAAAGGCG
TCTAATCTCTTCAAATAAGTTATCAGCAAATCCAGATTCCCATCTTTTCGAAAAATTTAC
GAATGTGCATTCCATTGGTAAAGGCCAGTTTTCCACGGTCTACCAGGTTACGTTTGCCCA
AACAAACAAAAAGTATGCAATCAAAGCCATTAAACCAAACAAATATAATTCCTTGAAACG
CATATTACTGGAAATTAAAATACTAAACGAGGTAACAAACCAAATTACCATGGATCAAGA
AGGGAAGGAATACATCAT (SEQ ID NO: 49)

A=550 T=376 G=296 C=352 GC%=0.41169
MAX REPEAT 11
MAX COMPLEMENT REPEAT 10
ALMOST REPEATS
304 17 GAAGAAGAGGAAGAAGA (SEQ ID NO: 50)
313 17 GAAGAAGAGGAGGAAGA (SEQ ID NO: 51)
316 16 GAAGAGGAGGAAGAAG (SEQ ID NO: 52)

One hundred and twenty different sets of "constant Tm" oligonucleotides were then designed using a process similar to that shown in Figure 30. Different sets were designed by starting at different positions within the polynucleotide, and by using different design annealing temperatures (Z was increased in 0.05°C increments from 60°C to 63°C). For each of these oligonucleotide sets the number of oligonucleotides (#), the lengths of amplification oligonucleotides at each end (Amp), the minimum and maximum annealing temperature of correct annealing pairs within the set (Tm), the minimum and maximum oligonucleotide length (Len), the maximum length of repeat sequence at the end of an oligonucleotide (MaxRep@Ends) and the initial set annealing temperature (TmCUT) were reported as follows.

| | | | | | | | |
|---|---|---|---|---|---|---|---|
| set1.txt | #Ol=73 | Amp[23,27] | ENDS[22-12] | Tm[60.0108-63.2891] | Len[35-55] | MaxRep@Ends:12 | TmCUT:60 |
| set2.txt | #Ol=73 | Amp[23,27] | ENDS[21-12] | Tm[60.0108-63.2891] | Len[35-55] | MaxRep@Ends:12 | TmCUT:60 |
| set3.txt | #Ol=73 | Amp[23,27] | ENDS[20-12] | Tm[60.0089-63.2891] | Len[36-55] | MaxRep@Ends:12 | TmCUT:60 |
| set4.txt | #Ol=73 | Amp[23,27] | ENDS[19-12] | Tm[60.0089-63.2891] | Len[36-55] | MaxRep@Ends:12 | TmCUT:60 |
| set5.txt | #Ol=73 | Amp[23,27] | ENDS[18-12] | Tm[60.0089-63.2891] | Len[36-55] | MaxRep@Ends:12 | TmCUT:60 |
| set6.txt | #Ol=73 | Amp[23,27] | ENDS[17-12] | Tm[60.0089-63.2891] | Len[36-55] | MaxRep@Ends:12 | TmCUT:60 |
| set7.txt | #Ol=73 | Amp[23,27] | ENDS[16-12] | Tm[60.0089-63.2891] | Len[36-55] | MaxRep@Ends:12 | TmCUT:60 |
| set8.txt | #Ol=73 | Amp[23,27] | ENDS[15-12] | Tm[60.0089-63.2891] | Len[36-55] | MaxRep@Ends:12 | TmCUT:60 |
| set9.txt | #Ol=73 | Amp[23,27] | ENDS[14-12] | Tm[60.0923-63.2891] | Len[36-55] | MaxRep@Ends:12 | TmCUT:60 |
| set10.txt | #Ol=73 | Amp[23,27] | ENDS[13-12] | Tm[60.0089-63.2891] | Len[36-55] | MaxRep@Ends:12 | TmCUT:60 |
| set11.txt | #Ol=73 | Amp[23,27] | ENDS[12-12] | Tm[60.0923-63.2891] | Len[36-55] | MaxRep@Ends:12 | TmCUT:60 |
| set12.txt | #Ol=73 | Amp[23,27] | ENDS[11-12] | Tm[60.0923-63.2891] | Len[36-55] | MaxRep@Ends:12 | TmCUT:60 |
| set13.txt | #Ol=73 | Amp[23,27] | ENDS[10-12] | Tm[60.0923-63.2891] | Len[36-55] | MaxRep@Ends:12 | TmCUT:60 |
| set14.txt | #Ol=73 | Amp[23,27] | ENDS[9-12] | Tm[60.0923-63.2891] | Len[36-55] | MaxRep@Ends:12 | TmCUT:60 |
| set15.txt | #Ol=73 | Amp[23,27] | ENDS[8-12] | Tm[60.0923-63.2891] | Len[36-55] | MaxRep@Ends:12 | TmCUT:60 |
| set16.txt | #Ol=74 | Amp[23,27] | ENDS[1-15] | Tm[60.0108-63.2891] | Len[35-55] | MaxRep@Ends:12 | TmCUT:60 |
| set17.txt | #Ol=74 | Amp[23,27] | ENDS[0-15] | Tm[60.0089-63.2891] | Len[36-55] | MaxRep@Ends:12 | TmCUT:60 |
| set18.txt | #Ol=72 | Amp[23,27] | ENDS[24-15] | Tm[60.0572-63.1624] | Len[35-57] | MaxRep@Ends:12 | TmCUT:60.05 |
| set19.txt | #Ol=72 | Amp[23,27] | ENDS[23-15] | Tm[60.0572-63.1624] | Len[35-57] | MaxRep@Ends:12 | TmCUT:60.05 |
| set20.txt | #Ol=73 | Amp[23,27] | ENDS[22-12] | Tm[60.0771-63.2891] | Len[35-55] | MaxRep@Ends:12 | TmCUT:60.05 |
| set21.txt | #Ol=73 | Amp[23,27] | ENDS[21-12] | Tm[60.0771-63.2891] | Len[35-55] | MaxRep@Ends:12 | TmCUT:60.05 |
| set22.txt | #Ol=73 | Amp[23,27] | ENDS[20-12] | Tm[60.0771-63.2891] | Len[36-55] | MaxRep@Ends:12 | TmCUT:60.05 |
| set23.txt | #Ol=73 | Amp[23,27] | ENDS[19-12] | Tm[60.0771-63.2891] | Len[36-55] | MaxRep@Ends:12 | TmCUT:60.05 |
| set24.txt | #Ol=73 | Amp[23,27] | ENDS[18-12] | Tm[60.0923-63.2891] | Len[36-55] | MaxRep@Ends:12 | TmCUT:60.05 |
| set25.txt | #Ol=73 | Amp[23,27] | ENDS[17-12] | Tm[60.0923-63.2891] | Len[36-55] | MaxRep@Ends:12 | TmCUT:60.05 |
| set26.txt | #Ol=73 | Amp[23,27] | ENDS[16-12] | Tm[60.0923-63.2891] | Len[36-55] | MaxRep@Ends:12 | TmCUT:60.05 |
| set27.txt | #Ol=73 | Amp[23,27] | ENDS[15-12] | Tm[60.0923-63.2891] | Len[36-55] | MaxRep@Ends:12 | TmCUT:60.05 |
| set28.txt | #Ol=73 | Amp[23,27] | ENDS[14-12] | Tm[60.0923-63.2891] | Len[36-55] | MaxRep@Ends:12 | TmCUT:60.05 |
| set29.txt | #Ol=73 | Amp[23,27] | ENDS[13-12] | Tm[60.0923-63.2891] | Len[36-55] | MaxRep@Ends:12 | TmCUT:60.05 |
| set30.txt | #Ol=73 | Amp[23,27] | ENDS[12-12] | Tm[60.0923-63.2891] | Len[36-55] | MaxRep@Ends:12 | TmCUT:60.05 |
| set31.txt | #Ol=73 | Amp[23,27] | ENDS[11-12] | Tm[60.0923-63.2891] | Len[36-55] | MaxRep@Ends:12 | TmCUT:60.05 |
| set32.txt | #Ol=73 | Amp[23,27] | ENDS[10-12] | Tm[60.0923-63.2891] | Len[36-55] | MaxRep@Ends:12 | TmCUT:60.05 |
| set33.txt | #Ol=73 | Amp[23,27] | ENDS[9-12] | Tm[60.0923-63.2891] | Len[36-55] | MaxRep@Ends:12 | TmCUT:60.05 |
| set34.txt | #Ol=73 | Amp[23,27] | ENDS[8-12] | Tm[60.0923-63.2891] | Len[36-55] | MaxRep@Ends:12 | TmCUT:60.05 |
| set35.txt | #Ol=74 | Amp[23,27] | ENDS[0-15] | Tm[60.0771-63.2891] | Len[36-55] | MaxRep@Ends:12 | TmCUT:60.05 |
| set36.txt | #Ol=72 | Amp[23,27] | ENDS[24-9] | Tm[60.1225-63.3229] | Len[35-58] | MaxRep@Ends:12 | TmCUT:60.1 |
| set37.txt | #Ol=72 | Amp[23,27] | ENDS[23-9] | Tm[60.1225-63.3229] | Len[35-58] | MaxRep@Ends:12 | TmCUT:60.1 |
| set38.txt | #Ol=73 | Amp[23,27] | ENDS[22-18] | Tm[60.1894-63.3229] | Len[35-58] | MaxRep@Ends:12 | TmCUT:60.1 |
| set39.txt | #Ol=73 | Amp[23,27] | ENDS[21-18] | Tm[60.1894-63.3229] | Len[35-58] | MaxRep@Ends:12 | TmCUT:60.1 |

| | | | | | | | |
|---|---|---|---|---|---|---|---|
| set40.txt | #O1=73 | Amp[23,27] | ENDS[20-18] | Tm[60.1894-63.3229] | Len[35-58] | MaxRep@Ends:12 | TmCUT:60.1 |
| set41.txt | #O1=73 | Amp[23,27] | ENDS[19-18] | Tm[60.1894-63.3229] | Len[35-58] | MaxRep@Ends:12 | TmCUT:60.1 |
| set42.txt | #O1=73 | Amp[23,27] | ENDS[18-18] | Tm[60.1143-63.3229] | Len[36-58] | MaxRep@Ends:12 | TmCUT:60.1 |
| set43.txt | #O1=73 | Amp[23,27] | ENDS[17-18] | Tm[60.1143-63.3229] | Len[36-58] | MaxRep@Ends:12 | TmCUT:60.1 |
| set44.txt | #O1=73 | Amp[23,27] | ENDS[16-18] | Tm[60.1143-63.3229] | Len[36-58] | MaxRep@Ends:12 | TmCUT:60.1 |
| set45.txt | #O1=73 | Amp[23,27] | ENDS[15-18] | Tm[60.1143-63.3229] | Len[36-58] | MaxRep@Ends:12 | TmCUT:60.1 |
| set46.txt | #O1=73 | Amp[23,27] | ENDS[14-18] | Tm[60.1143-63.3229] | Len[36-58] | MaxRep@Ends:12 | TmCUT:60.1 |
| set47.txt | #O1=73 | Amp[23,27] | ENDS[13-18] | Tm[60.1143-63.3229] | Len[36-58] | MaxRep@Ends:12 | TmCUT:60.1 |
| set48.txt | #O1=73 | Amp[23,27] | ENDS[12-18] | Tm[60.1894-63.3229] | Len[36-58] | MaxRep@Ends:12 | TmCUT:60.1 |
| set49.txt | #O1=73 | Amp[23,27] | ENDS[11-18] | Tm[60.1894-63.3229] | Len[36-58] | MaxRep@Ends:12 | TmCUT:60.1 |
| set50.txt | #O1=73 | Amp[23,27] | ENDS[10-18] | Tm[60.1894-63.3229] | Len[36-58] | MaxRep@Ends:12 | TmCUT:60.1 |
| set51.txt | #O1=73 | Amp[23,27] | ENDS[9-18] | Tm[60.1894-63.3229] | Len[36-58] | MaxRep@Ends:12 | TmCUT:60.1 |
| set52.txt | #O1=73 | Amp[23,27] | ENDS[8-18] | Tm[60.1894-63.3229] | Len[36-58] | MaxRep@Ends:12 | TmCUT:60.1 |
| set53.txt | #O1=72 | Amp[23,27] | ENDS[24-9] | Tm[60.2038-63.3229] | Len[35-58] | MaxRep@Ends:12 | TmCUT:60.15 |
| set54.txt | #O1=72 | Amp[23,27] | ENDS[23-9] | Tm[60.2038-63.3229] | Len[35-58] | MaxRep@Ends:12 | TmCUT:60.15 |
| set55.txt | #O1=73 | Amp[23,27] | ENDS[22-18] | Tm[60.1894-63.3229] | Len[35-58] | MaxRep@Ends:12 | TmCUT:60.15 |
| set56.txt | #O1=73 | Amp[23,27] | ENDS[21-18] | Tm[60.1894-63.3229] | Len[35-58] | MaxRep@Ends:12 | TmCUT:60.15 |
| set57.txt | #O1=73 | Amp[23,27] | ENDS[20-18] | Tm[60.1894-63.3229] | Len[35-58] | MaxRep@Ends:12 | TmCUT:60.15 |
| set58.txt | #O1=73 | Amp[23,27] | ENDS[19-18] | Tm[60.1894-63.3229] | Len[35-58] | MaxRep@Ends:12 | TmCUT:60.15 |
| set59.txt | #O1=73 | Amp[23,27] | ENDS[18-18] | Tm[60.1894-63.3229] | Len[35-58] | MaxRep@Ends:12 | TmCUT:60.15 |
| set60.txt | #O1=73 | Amp[23,27] | ENDS[17-18] | Tm[60.1894-63.3229] | Len[36-58] | MaxRep@Ends:12 | TmCUT:60.15 |
| set61.txt | #O1=73 | Amp[23,27] | ENDS[16-18] | Tm[60.1894-63.3229] | Len[36-58] | MaxRep@Ends:12 | TmCUT:60.15 |
| set62.txt | #O1=73 | Amp[23,27] | ENDS[15-18] | Tm[60.1894-63.3229] | Len[36-58] | MaxRep@Ends:12 | TmCUT:60.15 |
| set63.txt | #O1=73 | Amp[23,27] | ENDS[14-18] | Tm[60.1894-63.3229] | Len[36-58] | MaxRep@Ends:12 | TmCUT:60.15 |
| set64.txt | #O1=73 | Amp[23,27] | ENDS[13-18] | Tm[60.1894-63.3229] | Len[36-58] | MaxRep@Ends:12 | TmCUT:60.15 |
| set65.txt | #O1=73 | Amp[23,27] | ENDS[12-18] | Tm[60.1894-63.3229] | Len[36-58] | MaxRep@Ends:12 | TmCUT:60.15 |
| set66.txt | #O1=73 | Amp[23,27] | ENDS[11-18] | Tm[60.1894-63.3229] | Len[36-58] | MaxRep@Ends:12 | TmCUT:60.15 |
| set67.txt | #O1=73 | Amp[23,27] | ENDS[10-18] | Tm[60.1894-63.3229] | Len[36-58] | MaxRep@Ends:12 | TmCUT:60.15 |
| set68.txt | #O1=73 | Amp[23,27] | ENDS[9-18] | Tm[60.1894-63.3229] | Len[36-58] | MaxRep@Ends:12 | TmCUT:60.15 |
| set69.txt | #O1=73 | Amp[23,27] | ENDS[8-18] | Tm[60.1894-63.3229] | Len[36-58] | MaxRep@Ends:12 | TmCUT:60.15 |
| set70.txt | #O1=72 | Amp[23,27] | ENDS[24-9] | Tm[60.2038-63.3229] | Len[35-58] | MaxRep@Ends:12 | TmCUT:60.2 |
| set71.txt | #O1=72 | Amp[23,27] | ENDS[23-9] | Tm[60.2038-63.3229] | Len[35-58] | MaxRep@Ends:12 | TmCUT:60.2 |
| set72.txt | #O1=73 | Amp[23,27] | ENDS[22-18] | Tm[60.2038-63.2891] | Len[35-58] | MaxRep@Ends:12 | TmCUT:60.2 |
| set73.txt | #O1=73 | Amp[23,27] | ENDS[21-18] | Tm[60.2038-63.2891] | Len[35-58] | MaxRep@Ends:12 | TmCUT:60.2 |
| set74.txt | #O1=73 | Amp[23,27] | ENDS[20-18] | Tm[60.2038-63.2891] | Len[35-58] | MaxRep@Ends:12 | TmCUT:60.2 |
| set75.txt | #O1=73 | Amp[23,27] | ENDS[19-18] | Tm[60.2038-63.2891] | Len[35-58] | MaxRep@Ends:12 | TmCUT:60.2 |
| set76.txt | #O1=73 | Amp[23,27] | ENDS[18-18] | Tm[60.2038-63.2891] | Len[35-58] | MaxRep@Ends:12 | TmCUT:60.2 |
| set77.txt | #O1=73 | Amp[23,27] | ENDS[17-18] | Tm[60.2038-63.2891] | Len[35-58] | MaxRep@Ends:12 | TmCUT:60.2 |
| set78.txt | #O1=73 | Amp[23,27] | ENDS[16-18] | Tm[60.2038-63.2891] | Len[35-58] | MaxRep@Ends:12 | TmCUT:60.2 |

| | | | | | | | |
|---|---|---|---|---|---|---|---|
| set79.txt | #Ol=73 | Amp[23,27] | ENDS[15-18] | Tm[60.2038-63.2891] | Len[35-58] | MaxRep@Ends:12 | TmCUT:60.2 |
| set80.txt | #Ol=73 | Amp[23,27] | ENDS[14-18] | Tm[60.2038-63.2891] | Len[35-58] | MaxRep@Ends:12 | TmCUT:60.2 |
| set81.txt | #Ol=73 | Amp[23,27] | ENDS[13-18] | Tm[60.2038-63.2891] | Len[35-58] | MaxRep@Ends:12 | TmCUT:60.2 |
| set82.txt | #Ol=73 | Amp[23,27] | ENDS[12-18] | Tm[60.2038-63.2891] | Len[35-58] | MaxRep@Ends:12 | TmCUT:60.2 |
| set83.txt | #Ol=73 | Amp[23,27] | ENDS[11-18] | Tm[60.2038-63.2891] | Len[35-58] | MaxRep@Ends:12 | TmCUT:60.2 |
| set84.txt | #Ol=73 | Amp[23,27] | ENDS[10-18] | Tm[60.2038-63.2891] | Len[35-58] | MaxRep@Ends:12 | TmCUT:60.2 |
| set85.txt | #Ol=73 | Amp[23,27] | ENDS[9-18] | Tm[60.2038-63.2891] | Len[35-58] | MaxRep@Ends:12 | TmCUT:60.2 |
| set86.txt | #Ol=73 | Amp[23,27] | ENDS[8-18] | Tm[60.2038-63.2891] | Len[35-58] | MaxRep@Ends:12 | TmCUT:60.2 |
| set87.txt | #Ol=71 | Amp[23,27] | ENDS[24-2] | Tm[60.2825-63.3229] | Len[37-58] | MaxRep@Ends:12 | TmCUT:60.25 |
| set88.txt | #Ol=71 | Amp[23,27] | ENDS[23-2] | Tm[60.2825-63.3229] | Len[37-58] | MaxRep@Ends:12 | TmCUT:60.25 |
| set89.txt | #Ol=72 | Amp[23,27] | ENDS[22-22] | Tm[60.3016-63.2891] | Len[35-58] | MaxRep@Ends:12 | TmCUT:60.25 |
| set90.txt | #Ol=72 | Amp[23,27] | ENDS[21-22] | Tm[60.3016-63.2891] | Len[35-58] | MaxRep@Ends:12 | TmCUT:60.25 |
| set91.txt | #Ol=72 | Amp[23,27] | ENDS[20-22] | Tm[60.2574-63.2891] | Len[35-58] | MaxRep@Ends:12 | TmCUT:60.25 |
| set92.txt | #Ol=72 | Amp[23,27] | ENDS[19-22] | Tm[60.2574-63.2891] | Len[35-58] | MaxRep@Ends:12 | TmCUT:60.25 |
| set93.txt | #Ol=72 | Amp[23,27] | ENDS[18-22] | Tm[60.2574-63.2891] | Len[35-58] | MaxRep@Ends:12 | TmCUT:60.25 |
| set94.txt | #Ol=72 | Amp[23,27] | ENDS[17-22] | Tm[60.2574-63.2891] | Len[35-58] | MaxRep@Ends:12 | TmCUT:60.25 |
| set95.txt | #Ol=72 | Amp[23,27] | ENDS[16-22] | Tm[60.3016-63.2891] | Len[37-58] | MaxRep@Ends:12 | TmCUT:60.25 |
| set96.txt | #Ol=72 | Amp[23,27] | ENDS[15-22] | Tm[60.3016-63.2891] | Len[37-58] | MaxRep@Ends:12 | TmCUT:60.25 |
| set97.txt | #Ol=72 | Amp[23,27] | ENDS[14-22] | Tm[60.3016-63.2891] | Len[37-58] | MaxRep@Ends:12 | TmCUT:60.25 |
| set98.txt | #Ol=72 | Amp[23,27] | ENDS[13-22] | Tm[60.3016-63.2891] | Len[37-58] | MaxRep@Ends:12 | TmCUT:60.25 |
| set99.txt | #Ol=72 | Amp[23,27] | ENDS[12-22] | Tm[60.3016-63.4716] | Len[37-58] | MaxRep@Ends:12 | TmCUT:60.25 |
| set100.txt | #Ol=72 | Amp[23,27] | ENDS[11-22] | Tm[60.3016-63.4716] | Len[37-58] | MaxRep@Ends:12 | TmCUT:60.25 |
| set101.txt | #Ol=72 | Amp[23,27] | ENDS[10-22] | Tm[60.3016-63.4716] | Len[37-58] | MaxRep@Ends:12 | TmCUT:60.25 |
| set102.txt | #Ol=72 | Amp[23,27] | ENDS[9-22] | Tm[60.3016-63.4716] | Len[37-58] | MaxRep@Ends:12 | TmCUT:60.25 |
| set103.txt | #Ol=72 | Amp[23,27] | ENDS[8-22] | Tm[60.3016-63.4716] | Len[37-58] | MaxRep@Ends:12 | TmCUT:60.25 |
| set104.txt | #Ol=71 | Amp[23,27] | ENDS[24-2] | Tm[60.3055-63.1624] | Len[37-58] | MaxRep@Ends:12 | TmCUT:60.3 |
| set105.txt | #Ol=71 | Amp[23,27] | ENDS[23-2] | Tm[60.3055-63.1624] | Len[37-58] | MaxRep@Ends:12 | TmCUT:60.3 |
| set106.txt | #Ol=72 | Amp[23,27] | ENDS[22-22] | Tm[60.3016-63.2891] | Len[35-58] | MaxRep@Ends:12 | TmCUT:60.3 |
| set107.txt | #Ol=72 | Amp[23,27] | ENDS[21-22] | Tm[60.3016-63.2891] | Len[35-58] | MaxRep@Ends:12 | TmCUT:60.3 |
| set108.txt | #Ol=72 | Amp[23,27] | ENDS[20-22] | Tm[60.3016-63.2891] | Len[35-58] | MaxRep@Ends:12 | TmCUT:60.3 |
| set109.txt | #Ol=72 | Amp[23,27] | ENDS[19-22] | Tm[60.3016-63.2891] | Len[35-58] | MaxRep@Ends:12 | TmCUT:60.3 |
| set110.txt | #Ol=72 | Amp[23,27] | ENDS[18-22] | Tm[60.3016-63.2891] | Len[35-58] | MaxRep@Ends:12 | TmCUT:60.3 |
| set111.txt | #Ol=72 | Amp[23,27] | ENDS[17-22] | Tm[60.3016-63.2891] | Len[35-58] | MaxRep@Ends:12 | TmCUT:60.3 |
| set112.txt | #Ol=72 | Amp[23,27] | ENDS[16-22] | Tm[60.3016-63.2891] | Len[37-58] | MaxRep@Ends:12 | TmCUT:60.3 |
| set113.txt | #Ol=72 | Amp[23,27] | ENDS[15-22] | Tm[60.3016-63.2891] | Len[37-58] | MaxRep@Ends:12 | TmCUT:60.3 |
| set114.txt | #Ol=72 | Amp[23,27] | ENDS[14-22] | Tm[60.3016-63.2891] | Len[37-58] | MaxRep@Ends:12 | TmCUT:60.3 |
| set115.txt | #Ol=72 | Amp[23,27] | ENDS[13-22] | Tm[60.3016-63.2891] | Len[37-58] | MaxRep@Ends:12 | TmCUT:60.3 |
| set116.txt | #Ol=72 | Amp[23,27] | ENDS[12-22] | Tm[60.3016-63.4716] | Len[37-58] | MaxRep@Ends:12 | TmCUT:60.3 |
| set117.txt | #Ol=72 | Amp[23,27] | ENDS[11-22] | Tm[60.3016-63.4716] | Len[37-58] | MaxRep@Ends:12 | TmCUT:60.3 |

| | | | | | | | |
|---|---|---|---|---|---|---|---|
| set118.txt | #Ol=72 | Amp[23,27] | ENDS[10-22] | Tm[60.3016-63.4716] | Len[37-58] | MaxRep@Ends:12 | TmCUT:60.3 |
| set119.txt | #Ol=72 | Amp[23,27] | ENDS[9-22] | Tm[60.3016-63.4716] | Len[37-58] | MaxRep@Ends:12 | TmCUT:60.3 |
| set120.txt | #Ol=72 | Amp[23,27] | ENDS[8-22] | Tm[60.3016-63.4716] | Len[37-58] | MaxRep@Ends:12 | TmCUT:60.3 |

The oligonucleotide sets were then screened for an appropriate set using criteria similar to those shown in Figure 31. Set 107 was selected as having an even number of oligonucleotides (72), a narrow range of calculated annealing temperatures (60.301 to 63.289) and an acceptable maximum repeat at the end of any oligonucleotide (12).

For each oligonucleotide in set 107, the computer program reported its name, with F indicating an oligonucleotide in the forward (5' to 3') direction and R indicating a reverse complement oligonucleotide that runs in the 3' to 5' direction on the polynucleotide. The program also reported the bases or complementary bases, in the case of reverse oligonucleotides, of the polynucleotide that are represented by the oligonucleotide. The oligonucleotide set was also designed with a pair of amplification oligonucleotides, AF1 and AR1, which are to amplify the final product following synthesis.

All oligonucleotides except for AF1 and AR1 were adjusted to a concentration of 10 μM and an equal volume of each were mixed together to provide an oligonucleotide pool with a total oligonucleotide concentration of 10μM. This

pool was diluted 10-fold by adding 5 μl into a mixture of 5 μl 10x Herculase buffer (from Stratagene), 2.5 μl dNTPs (6 mM each of dATP, dCTP, dGTP and dTTP: the final concentration in the mixture is 300 μM each), 2.5 μl $MgSO_4$ (40 mM: the final concentration in the mix is 2 mM), 35 μl water and 0.5 μl Herculase polymerase (a mixture of Taq and Pfu thermostable DNA polymerases from Stratagene). A polynucleotide was synthesized from the mixture of oligonucleotides using the polymerase chain reaction by subjecting the mixture to the temperature steps shown in Figure 35, using an annealing temperature of 56°C.

After the synthesis step, the polynucleotide was amplified using a mix containing 1 x Herculase reaction buffer (supplied by Stratagene), 300 μM each of dATP, dCTP, dGTP and dTTP, 2 mM MgSO4, 0.5 μM oligonucleotide AF1, 0.5 μM oligonucleotide AR1, a 1/10 dilution (ie 5 μl in a 50 μl reaction) of the product of the synthesis reaction from the previous step and a 1/100 dilution of Herculase polymerase (a mixture of Taq and Pfu thermostable DNA polymerases from Stratagene). The product was amplified by subjecting the mixture to the following conditions: 96°C for 2 minutes, then 20 cycles of (96°C for 30 seconds, 56°C for 30 seconds, 72°C for 90 seconds). Finally an additional 1 μl of Taq DNA polymerase was added, and the mixture was heated to 72°C for 10 minutes. This step added an A residue to the 3' end of each strand of the polynucleotide, thereby facilitating its cloning into a TA cloning vector.

The gene was cloned by mixing 1 μl from the amplification reaction, 1 μl of water, 0.5 μl of pDRIVE vector (from Qiagen) and 2.5 μl of 2x ligation mix. After a 2 hour ligation, 1 μl of ligation mix was transformed into chemically competent *E. coli* TOP10 cells and plated onto LB agar plates supplemented with ampicillin and grown for 24 hours at 37°C. Four transformed colonies were picked into 3 ml liquid LB medium and grown for 24 hours at 37°C before plasmid was prepared from them. The sequences of the inserts cloned into the plasmids were determined by sequencing using an ABI 3730. One of the four plasmids contained an insert whose sequence was identical to the sequence designed.

### 6.5 DESIGNING A POLYNUCLEOTIDE TO ENCODE A POLYPEPTIDE

Many possible sequences encode any one polypeptide. It is thus often desirable to design more than one polynucleotide and then filter these sequences by discarding those that do not meet additional criteria.

In this example a polynucleotide was desired to encode the following polypeptide:

```
APAVEQRSEAAPLIEARGEMVANKYIVKFKEGSALSALDAAMEKISGKPDHVYKNVFSGF
AATLDENMVRVLRAHPDVEYIEQDAVVTINAAQTNAPWGLARISSTSPGTSTYYYDESAG
QGSCVYVIDTGIEASHPEFEGRAQMVKTYYYSSRDGNGHGTHCAGTVGSRTYGVAKKTQL
FGVKVLDDNGSGQYSTIIAGMDFVASDKNNRNCPKGVVASLSLGGGYSSSVNSAAARLQS
SGVMVAVAAGNNNADARNYSPASEPSVCTVGASDRYDRRSSFSNYGSVLDIFGPGTSILS
TWIGGSTRSISGTSMATPHVAGLAAYLMTLGKTTAASACRYIADTANKGDLSNIPFGTVN
LLAYNNYQA (SEQ ID NO: 53)
```

Polynucleotide sequences were designed using a computer program that selected codons based on their frequencies in an *E. coli* class II codon usage table shown in Figure 22. Any codon with a frequency of less than 0.1, the threshold frequency, was rejected.

The first polynucleotide design was as follows:

```
GCTCCGGCAGTTGAACAGCGTTCTGAAGCGGCGCCGCTGATCGAGGCGCGTGGTGAGATG
GTTGCTAACAAATACATTGTGAAATTCAAGGAGGGCTCTGCTCTGTCTGCACTGGACGCC
GCAATGGAAAAGATCAGCGGCAAGCCGGACCACGTGTACAAAAACGTGTTTTCCGGTTTC
GCCGCTACTCTGGATGAAAATATGGTTCGTGTTCTGCGTGCGCACCCGGATGTAGAATAT
ATCGAACAGGATGCAGTCGTAACCATCAATGCTGCTCAGACCAATGCGCCGTGGGGTCTG
GCACGTATTTCTTCTACCTCCCCGGGTACCAGCACCTATTATTACGACGAAAGCGCCGGC
CAGGGCTCTTGCGTTTACGTTATTGACACCGGCATCGAAGCTTCTCATCCAGAATTCGAG
GGTCGTGCGCAGATGGTGAAAACCTACTACTACTCCTCTCGCGATGGCAACGGTCATGGC
```

ACGCATTGCGCAGGCACGGTAGGCTCCCGTACGTACGGTGTTGCAAAAAAAACCCAGCTG
TTCGGCGTTAAAGTGCTGGACGATAACGGTTCTGGTCAGTACTCCACCATCATCGCAGGT
ATGGACTTCGTAGCGTCCGACAAAAACAACCGTAACTGTCCGAAAGGCGTCGTTGCGAGC
CTGAGCCTGGGTGGTGGCTATTCTTCCTCCGTGAACTCTGCGGCGGCCCGCCTGCAGAGC
TCTGGTGTAATGGTTGCAGTAGCCGCAGGCAACAACAACGCTGATGCACGTAACTACTCT
CCGGCTTCCGAACCATCTGTGTGTACCGTGGGTGCATCCGATCGTTACGACCGCCGTAGC
TCTTTTTCTAACTACGGCTCCGTGCTGGACATTTTCGGCCCGGGTACTTCTATTCTGTCT
ACTTGGATCGGCGGTTCTACCCGCAGCATCAGCGGTACTTCTATGGCGACCCCGCACGTG
GCAGGCCTGGCGGCTTATCTGATGACTCTGGGTAAAACCACCGCGGCGAGCGCGTGTCGT
TACATCGCGGATACTGCTAACAAAGGTGACCTGTCTAACATCCCTTTCGGTACCGTCAAC
CTGCTGGCATACAACAACTACCAAGCG (SEQ ID NO: 54)

The computer program also reported the following statistics for the polynucleotide:
Total bp = 1107
GC = 55.01%
A: 239 T: 259 G: 299 C:310 Codon Usage Report:

| | | | | | |
|---|---|---|---|---|---|
| A | GCG | 17 | T | ACG | 3 |
| A | GCA | 14 | T | ACA | 0 |
| A | GCT | 11 | T | ACT | 6 |
| A | GCC | 5 | T | ACC | 15 |
| R | AGG | 0 | W | TGG | 2 |
| R | AGA | 0 | Y | TAT | 5 |
| R | CGG | 0 | Y | TAC | 15 |
| R | CGA | 0 | V | GTG | 10 |
| R | CGT | 12 | V | GTA | 6 |
| R | CGC | 4 | V | GTT | 10 |
| N | AAT | 3 | V | GTC | 3 |
| N | AAC | 18 | * | TGA | 0 |
| D | GAT | 8 | * | TAG | 0 |
| D | GAC | 10 | * | TAA | 0 |
| C | TGT | 3 | | | |
| C | TGC | 2 | | | |
| Q | CAG | 8 | | | |
| Q | CAA | 1 | | | |
| E | GAG | 4 | | | |
| E | GAA | 10 | | | |
| G | GGG | 0 | | | |
| G | GGA | 0 | | | |
| G | GGT | 20 | | | |
| G | GGC | 17 | | | |
| H | CAT | 3 | | | |
| H | CAC | 3 | | | |
| I | ATA | 0 | | | |
| I | ATT | 5 | | | |
| I | ATC | 11 | | | |
| L | TTG | 0 | | | |

(continued)

| | | |
|---|---|---|
| L | TTA | 0 |
| L | CTG | 19 |
| L | CTA | 0 |
| L | CTT | 0 |
| L | CTC | 0 |
| K | AAG | 3 |
| K | AAA | 11 |
| M | ATG | 8 |
| F | TTT | 2 |
| F | TTC | 7 |
| P | CCG | 10 |
| P | CCA | 2 |
| P | CCT | 1 |
| P | CCC | 0 |
| S | TCT | 21 |
| S | TCC | 11 |
| S | TCA | 0 |
| S | TCG | 0 |
| S | AGT | 0 |
| S | AGC | 10 |

Repeats: None
Complementary Repeats (position1 position2 length sequence)

27, R38 12 AGCGGCGCCGCT (SEQ ID NO: 55)
507, R518 12 CCGTACGTACGG (SEQ I D NO: 56)

The first polynucleotide was rejected because it contained complementary repeats that could interfere with the assembly of oligonucleotides into a polynucleotide. A second polynucleotide was thus designed using the same probabilistic process. The second polynucleotide design was as follows:

```
GCTCCAGCGGTTGAACAGCGCAGCGAGGCCGCACCGCTGATCGAAGCCCGTGGTGAA
ATGGTGGCAAACAAATACATTGTCAAGTTCAAAGAAGGTTCCGCGCTGAGCGCTCTG
GATGCTGCAATGGAAAAAATCTCCGGTAAACCGGACCACGTATATAAAAATGTCTTT
TCTGGCTTCGCGGCTACTCTGGATGAGAACATGGTTCGTGTGCTGCGTGCGCATCCG
GATGTTGAATACATTGAACAGGACGCAGTTGTAACGATTAACGCTGCCCAAACTAAC
GCGCCATGGGGCCTGGCCCGCATTAGCTCCACCTCCCCAGGTACTTCCACTTATTAC
TACGACGAATCCGCAGGTCAGGGTTCCTGCGTATATGTTATCGACACCGGTATCGAA
GCGTCCCACCCGGAATTTGAGGGTCGTGCGCAAATGGTGAAGACCTACTACTACTCT
TCCCGTGACGGTAACGGTCACGGTACCCACTGTGCGGGTACTGTAGGTAGCCGTACC
TATGGTGTTGCCAAAAAAACCCAGCTGTTTGGCGTTAAAGTGCTGGATGATAATGGC
TCCGGTCAGTACTCCACCATCATCGCTGGCATGGACTTTGTCGCAAGCGACAAAAAC
AACCGCAACTGCCCGAAAGGTGTTGTGGCTTCTCTGTCCCTGGGTGGTGGCTATAGC
TCCTCTGTGAACTCTGCGGCAGCGCGTCTGCAATCCTCCGGCGTGATGGTCGCGGTT
GCCGCAGGTAACAACAACGCGGATGCGCGCAACTACTCTCCTGCATCCGAACCGTCC
GTTTGTACTGTTGGTGCGTCTGACCGTTACGACCGTCGTTCTTCTTTCTCCAACTAC
GGTTCTGTACTGGACATCTTCGGTCCTGGCACCTCCATCCTGTCTACGTGGATTGGC
GGTAGCACCCGTAGCATCTCTGGTACTAGCATGGCTACCCCGCACGTAGCAGGCCTG
GCGGCATATCTGATGACGCTGGGCAAGACTACCGCGGCTAGCGCTTGCCGTTACATC
GCGGATACCGCGAACAAAGGCGACCTGTCTAACATCCCGTTCGGCACCGTGAACCTG
CTGGCATACAACAACTATCAGGCG (SEQ ID NO: 57)
```

The computer program also reported the following statistics for the second polynucleotide:
Total bp = 1107
GC = 55.01 %
A: 241 T: 257 G: 294 C:315
Codon Usage Report:

| | | | | | | | | |
|---|---|---|---|---|---|---|---|---|
| A | GCG | 19 | G | GGT | 24 | S | TCT | 13 |
| A | GCA | 12 | G | GGC | 13 | S | TCC | 19 |
| A | GCT | 10 | H | CAT | 1 | S | TCA | 0 |
| A | GCC | 6 | H | CAC | 5 | S | TCG | 0 |
| R | AGG | 0 | I | ATA | 0 | S | AGT | 0 |
| R | AGA | 0 | I | ATT | 5 | S | AGC | 10 |
| R | CGG | 0 | I | ATC | 11 | T | ACG | 3 |
| R | CGA | 0 | L | TTG | 0 | T | ACA | 0 |
| R | CGT | 12 | L | TTA | 0 | T | ACT | 8 |
| R | CGC | 4 | L | CTG | 19 | T | ACC | 13 |
| N | AAT | 2 | L | CTA | 0 | W | TGG | 2 |
| N | AAC | 19 | L | CTT | 0 | Y | TAT | 7 |
| D | GAT | 7 | L | CTC | 0 | Y | TAC | 13 |
| D | GAC | 11 | K | AAG | 3 | V | GTG | 8 |
| C | TGT | 2 | K | AAA | 11 | V | GTA | 6 |
| C | TGC | 3 | M | ATG | 8 | V | GTT | 11 |
| Q | CAG | 6 | F | TTT | 4 | V | GTC | 4 |
| Q | CAA | 3 | F | TTC | 5 | * | TGA | 0 |
| E | GAG | 3 | P | CCG | 8 | * | TAG | 0 |
| E | GAA | 11 | P | CCA | 3 | * | TAA | 0 |
| G | GGG | 0 | P | CCT | 2 | | | |
| G | GGA | 0 | P | CCC | 0 | | | |

Repeats: None
Possible RNA stem loop structures:

459((weak): CCGTGACggtaacgGTCACGG (SEQ ID NO: 57)
607((weak): TTTGTCGcaagCGACAAA (SEQ ID NO: 58)

The sceond polynucleotide was rejected because it contained possible RNA stemloop structures that could interfere with the expression of the polynucleotide. A third polynucleotide was thus designed using the same probabilisitic process. The third polynucleotide design was as follows:

GCGCCGGCAGTAGAACAGCGTTCTGAAGCAGCACCGCTGATCGAAGCTCGCGGCG
AAATGGTAGCGAACAAATATATTGTAAAATTCAAAGAAGGCTCTGCACTGTCTGCGCTGG
ATGCTGCGATGGAGAAAATCTCTGGTAAACCGGATCACGTATACAAGAACGTTTTTTCTG
GCTTCGCTGCAACGCTGGATGAAAACATGGTGCGTGTACTGCGTGCGCACCCGGATGTGG
AGTACATCGAACAGGACGCAGTTGTGACCATCAACGCGGCGCAGACTAACGCTCCGTGGG
GCCTGGCTCGCATCTCTTCCACCTCCCCGGGCACTTCCACCTACTACTATGATGAGTCTG
CTGGTCAGGGTAGCTGTGTTTACGTTATCGATACGGGCATCGAAGCTTCCCACCCGGAAT
TCGAAGGCCGTGCGCAGATGGTGAAAACCTATTACTATTCTTCTCGTGATGGCAATGGCC

ACGGCACCCACTGCGCCGGCACCGTTGGTTCTCGCACCTACGGTGTGGCAAAGAAAACCC
AGCTGTTCGGTGTGAAGGTTCTGGACGATAACGGTTCCGGCCAGTACTCCACTATCATCG
CCGGCATGGACTTCGTTGCCTCCGACAAAAATAACCGTAATTGCCCGAAAGGTGTTGTTG
CTTCCCTGAGCCTGGGTGGCGGTTATTCCAGCTCTGTGAACTCTGCAGCCGCTCGCCTGC
AGTCCTCTGGCGTTATGGTAGCCGTCGCGGCTGGTAACAACAACGCGGATGCACGCAATT
ACTCCCCGGCCTCCGAACCTTCTGTCTGTACCGTTGGCGCTAGCGACCGTTATGATCGTC
GCTCTAGCTTCTCTAACTATGGTTCCGTACTGGATATCTTCGGCCCGGGTACCTCTATTC
TGTCCACTTGGATTGGCGGCTCTACCCGCTCTATCTCCGGTACCTCTATGGCCACGCCGC
ATGTCGCAGGCCTGGCAGCTTACCTGATGACTCTGGGTAAAACTACCGCGGCCTCCGCTT
GCCGCTACATTGCCGACACTGCTAACAAAGGCGACCTGAGCAACATTCCATTCGGCACCG
TTAACCTGCTGGCCTACAACAATTACCAGGCA (SEQ ID NO: 59)

The computer program also reported the following statistics for the third polynucleotide:
Total bp = 1107
GC = 55.28%
A: 233 T: 262 G: 287 C:325
Codon Usage Report:

| | | | | | | | | |
|---|---|---|---|---|---|---|---|---|
| A | GCG | 11 | H | CAT | 1 | S | AGT | 0 |
| A | GCA | 12 | H | CAC | 5 | S | AGC | 6 |
| A | GCT | 14 | I | ATA | 0 | T | ACG | 3 |
| A | GCC | 10 | I | ATT | 5 | T | ACA | 0 |
| R | AGG | 0 | I | ATC | 11 | T | ACT | 7 |
| R | AGA | 0 | L | TTG | 0 | T | ACC | 14 |
| R | CGG | 0 | L | TTA | 0 | W | TGG | 2 |
| R | CGA | 0 | L | CTG | 19 | Y | TAT | 7 |
| R | CGT | 8 | L | CTA | 0 | Y | TAC | 13 |
| R | CGC | 8 | L | CTT | 0 | V | GTG | 7 |
| N | AAT | 5 | L | CTC | 0 | V | GTA | 7 |
| N | AAC | 16 | K | AAG | 3 | V | GTT | 12 |
| D | GAT | 11 | K | AAA | 11 | V | GTC | 3 |
| D | GAC | 7 | M | ATG | 8 | * | TGA | 0 |
| C | TGT | 2 | F | TTT | 1 | * | TAG | 0 |
| C | TGC | 3 | F | TTC | 8 | * | TAA | 0 |
| Q | CAG | 9 | P | CCG | 11 | | | |
| Q | CAA | 0 | P | CCA | 1 | | | |
| E | GAG | 3 | P | CCT | 1 | | | |
| E | GAA | 11 | P | CCC | 0 | | | |
| G | GGG | 0 | S | TCT | 20 | | | |
| G | GGA | 0 | S | TCC | 16 | | | |
| G | GGT | 15 | S | TCA | 0 | | | |
| G | GGC | 22 | S | TCG | 0 | | | |

Repeats: None
Complementary Repeats: None
Possible RNA stem loop structures: None
The third polynucleotide design had no repeat sequence elements and no possible RNA secondary structure elements, so it was selected for synthesis. Three constant Tm sets of oligonucleotides were designed one for each of the three possible polynucleotide designs, using a calculated annealing temperature of 64.5°C. The computer program reported the following statistics for the three sets of oligonucleotides:

set 1 Tm[64.5335-67.6137] Len[33-55] MaxRep@Ends:11
set 2 Tm[64.5335-67.6137] Len[33-53] MaxRep@Ends:11
set 3 Tm[64.5312-66.8185] Len[32-51] MaxRep@Ends:11

Three criteria were then used to select the best design. The first criterion was whether there was less than a 3°C difference between the maximum and minimum calculated annealing temperatures for correct annealing partners within the set of oligonucleotides corresponding to the design. Only design three fulfilled this criterion. The second criterion was whether the maximum oligonucleotide length less than 55 bp. Designs two and three fulfilled this criterion. The third criterion was whether there were repeats greater than 12 bp at the ends of any oligonucleotides. Designs one, two and three fulfilled this criterion. From this calculation, design one was selected.

All oligonucleotides except for AF1 and AR1 were adjusted to a concentration of 10 μM and an equal volume of each were mixed together to provide an oligonucleotide pool with a total oligonucleotide concentration of 10 μM. This pool was diluted 10-fold by adding 5 μl into a mixture of 5 μl 10x Herculase buffer (from Stratagene), 2.5 μl DMSO, 2.5 μl dNTPs (6 mM each of dATP, dCTP, dGTP and dTTP: the final concentration in the mixture is 300 μM each), 2.5 μl $MgSO_4$ (40 mM: the final concentration in the mix is 2 mM), 32 μl water and 0.5 μl Herculase polymerase (a mixture of Taq and Pfu thermostable DNA polymerases from Stratagene). A polynucleotide was synthesized from the mixture of oligonucleotides using the polymerase chain reaction by subjecting the mixture to the temperature steps shown in Figure 35 using an annealing temperature of 58°C.

After the synthesis step, the polynucleotide was amplified using a mix containing 1x Herculase reaction buffer, supplied by Stratagene, 300 μM each of dATP, dCTP, dGTP and dTTP, 2 mM $MgSO_4$, 0.5 μM oligonucleotide AF1, 0.5 μM oligonucleotide AR1, a 1/10 dilution (*i.e.* 5 μl in a 50 μl reaction) of the product of the synthesis reaction from the previous step and a 1/100 dilution of Herculase polymerase (a mixture of Taq and Pfu thermostable DNA polymerases from Stratagene). The product was amplified by subjecting the mixture to the following conditions: 96°C for two minutes, then 20 cycles of (96°C for 30 seconds, 58°C for 30 seconds, 72°C for 90 seconds). The 1100 bp DNA product was then purified using a Qiagen PCR cleanup kit. The ends of the DNA were cleaved using Ncol and Sall restriction enzymes, the DNA was purified again using a Qiagen PCR cleanup kit and ligated into a vector that had been previously digested with NcoI and SalI. After a 4 hour ligation, 1 μl of ligation mix was transformed into chemically competent E coli TOP10 cells and plated onto LB agar plates supplemented with ampicillin and grown for 24 hours at 37°C. Four transformed colonies were picked into 3 ml liquid LB medium and grown for 24 hours at 37°C before plasmid was prepared from them. The sequences of the inserts cloned into the plasmids were determined by sequencing using an ABI 3730. Two of the four plasmids contained an insert whose sequence was identical to the sequence designed.

### 6.6 DESIGN AND SYNTHESIS OF A POLYNUCLEOTIDE ENCODING A REPETITIVE POLYPEPTIDE

Sometimes it may be impossible to completely avoid repetitive polynucleotide sequences when encoding a polypeptide and meeting codon bias criteria that are important for the function of the polypeptide. In such cases it may be desirable to divide the polynucleotide into two or more parts, or even to synthesize different parts of the polynucleotide by different methods.

In this example a polynucleotide was desired to encode the following polypeptide:

MAQHDEAQQNAFYQVLNMPNLNADQRNGFIQSLKDDPSQSANVLGEAQKLNDS
QAPKADAqQNNFNKDQQSAFYEILNMPNLNEAQRNGFIQSLKDDPSQSTNVLGE
AKKLNESQAPKaDNNFNKEQQNAFYEILNMPNLNEEQRNGFIQSLKDDPSQSANL
LSEAKKLNESQAPKaDNKFNKEQQNAFYEILHLPNLNEEQRNGFIQSLKDDPSQS
ANLLAEAKKLNDAQAPKaDNKFNKEQQNAFYEILHLPNLTEEQRNGFIQSLKDDP
SVSKEILAEAKKLNDAQAPKEEDNNKPGKEDNNKPGKEDNNKPGKEDNNKPGK
EDNNKPGKEDNNKPGKEDGNKPGKEDNKKPGKEDGNKPGKEDNKKPGKEDGN
KPGKEDGNKPGKEDGNGVHVVKPGDTVNDIAKANGTTADKIAADNK (SEQ ID
NO: 60)

This polynucleotide is repetitive. Such repetitions can be best visualized using a dot-plot. A dot-plot ofthis polypeptide sequence is shown in Figure 37. This dot-plot shows that the polypeptide consists of five repeats of approximately 58 amino acids, followed by a non-repeat stretch, fourteen repeats of eight amino acids and a second non-repeat stretch. Many polynucleotides were designed according to the process shown in Figure 27. However, none of these polynucleotides were free of repeated sequence elements. The sequence was thus broken down into 3 segments; part 1 contained the first three 58 amino acid repeats, part 2 contained the fourth and fifth 58 amino acid repeats, the first non-repeat stretch and the first two 8 amino acid repeats, and part 3 contained the remaining twelve 8 amino acid repeats and the second non-repeat region.

*Part 1.*

```
MAQHDEAQQNAFYQVLNMPNLNADQRNGFIQSLKDDPSQSANVLGEAQKLNDSQAPKADA
qQNNFNKDQQSAFYEILNMPNLNEAQRNGFIQSLKDDPSQSTNVLGEAKKLNESQAPKAD
NNFNKEQQNAFYEILNMPNLNEEQRNGFIQSLKDDPSQSANLLSEAKKLNESQAPK
(SEQ ID NO: 61)
```

*Part 2.*

```
ADNKFNKEQQNAFYEILHLPNLNEEQRNGFIQSLKDDPSQSANLLAEAKKLNDAQAPKaD
NKFNKEQQNAFYEILHLPNLTEEQRNGFIQSLKDDPSVSKEILAEAKKLNDAQAPKEEDN
NKPGKEDNNKPGKEDNN  (SEQ ID NO: 62)
```

*Part 3.*

```
KPGKEDNNKPGKEDNNKPGKEDNNKPGKEDGNKPGKEDNKKPGKEDGNKPGKEDNKKPGK
EDGNKPGKEDGNKPGKEDGNGVHVVKPGDTVNDIAKANGTTADKIAADNK  (SEQ ID
NO: 63)
```

Separate polynucleotides were then designed to encode each segment using a computer program to execute the scheme shown in Figure 26 with adjustable parameters set as follows. Step 02, the codon bias table selected was for *E. coli* classII codons, as shown in Figure 22. Step 03, a threshold frequency of 0.1 was selected. Step 07, N was set to 30, GC content limits were set between 30 and 70%. Step 08, M was set to 12, forbidden restriction sites were set to recognition sequences for BsaI, HindIII, KpnI, MluI, BamHI. Step 09, disallowed repeats were defined as a 14 base pair of sequence identical to a 14 base pair of sequence anywhere else in the polynucleotide. Step 11, X was set to 50. Step 12, Z was set to 7.

The resulting polynucleotides still contained repeated sequence elements, so the sequences were further modified using a computer program to execute the scheme shown in Figure 28 with adjustable parameters set as follows. Step 02, the codon bias table selected was for *E coli* classII codons, as shown in Figure 6. Step 03 a threshold frequency of 0.1 was selected. Step 04, the initial design was taken as the result of the sequence designed using the scheme of Figure 26. Step 05, N was set to 30, GC content limits were set between 30 and 70%. Step 06, forbidden restriction sites were set to recognition sequences for BsaI, HindIII, KpnI, MluI, BamHI. Step 07, P was set to 16, Y was set to 50°C. Step 09, X was set to 1,000.

Following this sequence modification process, polynucleotides for parts 1 and 2 were obtained that lacked repeats, as shown in Figures 38 and 39. However no polynucleotide lacking repeats was obtained for part 3, as shown in Figure 40, because of the extreme nature of the repeats, which were primarily composed of amino acids encoded by only two possible codons. Thus, while it was possible to synthesize polynucleotides for parts 1 and 2 using the polymerase chain reaction, such an assembly protocol was anticipated to be unsuccessful for part 3. An oligonucleotide set for the synthesis of part 3 was thus designed for a ligation-based assembly. To do this an iterative process was performed using the schemes shown in Figures 26, 28, 29, 30 and 42. First, a polynucleotide sequence was designed as for parts 1 and 2 using the processes shown in Figures 26 and 28 as described above. Second, a set of half-oligonucleotides was designed using the process shown in Figures 29 and 30 with adjustable parameters set as follows. Step 02, Z was set to 65°C. Third, the oligonucleotide boundaries were adjusted using the process shown in Figure 42 with adjustable parameters set as follows. Step 03, N was set to 3. Step 06, A was set to 60°C. Step 07, C was set to 20, D was set to 65. This sequence of design process steps was repeated until a polynucleotide sequence that could be encoded by oligonucleotides fulfilling the design criteria of minimum Tm>60°C, maximum oligonucleotide length <65bp, all oligonucleotides with unique trimers at their ends was obtained.

The sequences of the three polynucleotide sequences encoding the three parts of the polypeptide are shown below. The lower case sequence has been added to the 5' end of part 1 to add a KpnI and MluI site and to the 3' end of part 2 to add a BamHI and HindIII site for future manipulations of the sequence.

*Part 1.*

ggtaccccggtaacgcgtATGGCGCAACATGACGAAGCTCAGCAGAACGCTTTTTACCAGGTACTGAACATG
CCGAACCTGAACGCGGATCAGCGCAACGGTTTCATCCAGAGCCTGAAAGACGACCCTTCTCAGTCCGCAAAC
GTTCTGGGCGAGGCTCAGAAACTGAACGACAGCCAGGCCCCAAAAGCAGATGCTCAGCAAAATAACTTCAAC
AAGGACCAGCAGAGCGCATTCTACGAAATCCTGAACATGCCAAATCTGAACGAAGCTCAACGCAACGGCTTC
ATTCAGTCTCTGAAAGACGATCCGTCCCAGTCCACTAACGTTCTGGGTGAAGCTAAGAAGCTGAACGAATCC
CAGGCACCAAAAGCAGACAACAACTTCAACAAAGAGCAGCAGAACGCTTTCTATGAAATCTTGAACATGCCT
AACCTGAATGAAGAACAGCGTAACGGCTTCATCCAGTCTCTGAAGGACGACCCTAGCCAGTCTGCTAACCTG
CTGTCCGAAGCAAAAAAACTGAACGAGTCCCAGGCTCCAAAAGC (SEQ ID NO: 64)

Part 2.

GGATAACAAATTCAACAAGGAGCAGCAGAACGCATTCTACGAAATCCTGCACCTGCCGAACCTGAACGAAGA
ACAGCGTAACGGTTTCATCCAATCCCTGAAAGACGATCCTTCCCAGTCCGCAAATCTGCTGGCAGAAGCAAA
GAAACTGAACGACGCACAGGCACCGAAGGCTGACAACAAGTTCAACAAAGAGCAGCAGAATGCCTTCTACGA
GATTCTGCATCTGCCAAACCTGACTGAGGAGCAGCGCAACGGTTTCATTCAGTCCCTGAAGGACGACCCAAG
CGTCAGCAAGGAAATCCTGGCTGAGGCGAAAAAACTGAACGATGCACAGGCTCCGAAGGAAGAAGACAACAA
TAAACCTGGTAAAGAAGATAATAATAAGCCTGGCAAGGAAGATAACA. (SEQ ID NO: 65)

Part 3

ACAAGCCGGGCAAGGAGGACAACAATAAACCGGGCAAAGAGGATAATAACAAGCCTGGTAAGGAAGACAACA
ACAAACCAGGCAAAGAAGATGGCAACAAGCCGGGTAAGGAGGATAATAAAAAACCAGGCAAGGAAGACGGCA
ACAAACCTGGCAAGGAGGATAACAAAAAGCCAGGCAAGGAGGATGGTAATAAACCGGGCAAAGAAGACGGCA
ACAAGCCTGGTAAAGAAGACGGTAACGGTGTACACGTCGTTAAACCTGGTGACACCGTGAACGACATCGCTA
AGGCTAATGGCACCACGGCAGACAAGATTGCAGCGGACAATAAATTAGCTGATAAATAAggatccgcggaag
ctt (SEQ ID NO: 66)

These three segments were then designed to be independently cloned using recombinase-based cloning. To do this, restriction sites were added to the ends of the segments. A KpnI site (GGTACC) was added at the 5' end of the first segment. A HindIII site (AAGCTT) was added to the 3' end of the third segment. The joins between the first and second and second and third fragments were designed to use typeIIs restriction endonucleases; these enzymes cut outside their recognition sites, and can therefore be used to join sequences without introducing any changes or restriction sites into the final sequence. TypeIIs restriction sites can be added to a sequence to create a "sticky" overhang for ligation as shown in Figure 41. In this example, a BsaI site was added to the 3' end of Part1, the 5' and 3' ends of part 2 and the 5' end of part 3. The sites are underlined in the sequences shown below. The positioning of these sites, calculated using the scheme shown in Figure 41, creates a 4bp overhang GCGG at the 3' end of part 1 and the 5' end of part 2 and a 4bp overhang CAAC at the 3' end of part 2 and at the 5' end of part 3. After addition of the TypeIIs restriction site, an additional sequence was added to each end of each sequence to enable recombinase-based cloning into the vector pDONR221 (Invitrogen). The sequence
GGGGACAAGTTTGTACAAAAAAGCAGGCT (SEQ ID NO : 67) was added to the 5' end of each segment, and the sequence
ACCCAGCTTTCTTGTACAAAGTGGTCCCC (SEQ ID NO: 68) was added to the 3' end of each segment. These sequences are shown in italics on the sequences below.

*Part 1.*

*GGGGACAAGTTTGTACAAAAAAGCAGGCT*GGTACCCCGGTAACGCGTATGGCGCAACATGACGAAGCTCAGC
AGAACGCTTTTTACCAGGTACTGAACATGCCGAACCTGAACGCGGATCAGCGCAACGGTTTCATCCAGAGCC
TGAAAGACGACCCTTCTCAGTCCGCAAACGTTCTGGGCGAGGCTCAGAAACTGAACGACAGCCAGGCCCCAA

AAGCAGATGCTCAGCAAAATAACTTCAACAAGGACCAGCAGAGCGCATTCTACGAAATCCTGAACATGCCAA
ATCTGAACGAAGCTCAACGCAACGGCTTCATTCAGTCTCTGAAAGACGATCCGTCCCAGTCCACTAACGTTC
TGGGTGAAGCTAAGAAGCTGAACGAATCCCAGGCACCAAAAGCAGACAACAACTTCAACAAAGAGCAGCAGA
ACGCTTTCTATGAAATCTTGAACATGCCTAACCTGAATGAAGAACAGCGTAACGGCTTCATCCAGTCTCTGA
AGGACGACCCTAGCCAGTCTGCTAACCTGCTGTCCGAAGCAAAAAAACTGAACGAGTCCCAGGCTCCAAAAG
CGGAGAGACC*ACCCAGCTTTCTTGTACAAAGTGGTCCCC* (SEQ ID NO: 69)

*Part 2.*

*GGGGACAAGTTTGTACAAAAAAGCAGGCT*GGTCTCAGCGGATAACAAATTCAACAAGGAGCAGCAGAACGCA
TTCTACGAAATCCTGCACCTGCCGAACCTGAACGAAGAACAGCGTAACGGTTTCATCCAATCCCTGAAAGAC
GATCCTTCCCAGTCCGCAAATCTGCTGGCAGAAGCAAAGAAACTGAACGACGCACAGGCACCGAAGGCTGAC
AACAAGTTCAACAAAGAGCAGCAGAATGCCTTCTACGAGATTCTGCATCTGCCAAACCTGACTGAGGAGCAG
CGCAACGGTTTCATTCAGTCCCTGAAGGACGACCCAAGCGTCAGCAAGGAAATCCTGGCTGAGGCGAAAAAA
CTGAACGATGCACAGGCTCCGAAGGAAGAAGACAACAATAAACCTGGTAAAGAAGATAATAATAAGCCTGGC
AAGGAAGATAACAACAGAGACC*ACCCAGCTTTCTTGTACAAAGTGGTCCCC* (SEQ ID NO: 70)

*Part 3.*

*GGGGACAAGTTTGTACAAAAAAGCAGGCT*GGTCTCACAACAAGCCGGGCAAGGAGGACAACAATAAACCGGG
CAAAGAGGATAATAACAAGCCTGGTAAGGAAGACAACAACAAACCAGGCAAAGAAGATGGCAACAAGCCGGG
TAAGGAGGATAATAAAAAACCAGGCAAGGAAGACGGCAACAAACCTGGCAAGGAGGATAACAAAAAGCCAGG
CAAGGAGGATGGTAATAAACCGGGCAAAGAAGACGGCAACAAGCCTGGTAAAGAAGACGGTAACGGTGTACA
CGTCGTTAAACCTGGTGACACCGTGAACGACATCGCTAAGGCTAATGGCACCACGGCAGACAAGATTGCAGC
GGACAATAAATTAGCTGATAAATAAGGATCCGCGGAAGCTT*ACCCAGCTTTCTTGTACAAAGTGGTCCCC*
(SEQ ID NO: 71)

Constant Tm sets of oligonucleotides were then designed for the assembly of segments 1 and 2 using a computer program to execute the scheme shown in Figures 29-31. The adjustable parameters were set as follows. Figures 29 and 30. Step 02, Z was set to 62°C. Figure 31. Step 02 Y was set to 50°C, R was set to 14 bases. Step 06, A was set to 4°C. Step 07, C was set to 30 bases, D was set to 65 bases. Step 10, B was set to 15°C. Several oligonucleotides were assembled for each part (30 for part 1 and 24 for part 2).

Two separate synthesis reactions were used to assemble part 1 polynucleotide and the part 2 polynucleotide. For each segment, all oligonucleotides except for AF1 and AR1 were adjusted to a concentration of 10 μM and an equal volume of each were mixed together to provide an oligonucleotide pool with a total oligonucleotide concentration of 10 μM. This pool was diluted 10-fold by adding 5 μl into a mixture of 5 μl 10x Herculase buffer (from Stratagene), 2.5 μl dimethyl sulphoxide (DMSO), 2.5 μl dNTPs (6 mM each of dATP, dCTP, dGTP and dTTP: the final concentration in the mixture is 300 μM each), 2.5 μl $MgSO_4$ (40 mM: the final concentration in the mix is 2 mM), 32 μl water and 0.5 μl Herculase polymerase (a mixture of Taq and Pfu thermostable DNA polymerases from Stratagene). A polynucleotide was synthesized from the mixture of oligonucleotides using the polymerase chain reaction by subjecting the mixture to the temperature steps shown in Figure 32.

After the synthesis step, each polynucleotide segment was amplified using a mix containing 1 x Herculase reaction buffer (supplied by Stratagene), 300 μM each of dATP, dCTP, dGTP and dTTP, 2 mM $MgSO_4$, 0.5 μM oligonucleotide AF1, 0.5 μM oligonucleotide AR1, a 1/10 dilution (ie 5 μl in a 50 μl reaction) of the product of the synthesis reaction from the previous step and a 1/100 dilution of Herculase polymerase (a mixture of Taq and Pfu thermostable DNA polymerases from Stratagene). The product was amplified by subjecting the mixture to the following conditions: 96°C for two minutes, then 20 cycles of: 96°C for 30 seconds, 56°C for 30 seconds, and 72°C for 30 seconds. The PCR product was then cloned into Invitrogen vector pDONR221 by mixing 2 μl of PCR product, 2 μl (300 ng) of pDONR221 vector DNA, 4 μl of 5x clonase reaction buffer, 8 μl TE (10 mM Tris-Cl pH 7.5, 1 mM EDTA) and incubating for 60 minutes at 25°C. The reaction was stopped by addition of 2 μl proteinase K solution (2 mg/ml) and incubation at 37°C for ten minutes. Following this recombination, 1 μl of ligation mix was transformed into chemically competent E *coli* TOP 10 cells and plated onto LB agar plates supplemented with ampicillin and grown for 24 hours at 37°C. Four transformed colonies were picked into 3ml liquid LB medium and grown for 24 hours at 37°C before plasmid was prepared from them. The sequences of the inserts cloned into the plasmids were determined by sequencing using an ABI 3730. Three of the four plasmids for Part1 and two of the four plasmids for part 2 contained an insert whose sequence was identical to the sequence designed.

The set of oligonucleotides for assembly of part 3 were as follows:

>G00371C-F1 (with NO 5' phosphate) rescue
GGGGACAAGTTTGTACAAAAAAGCAGGCTGGTCTCACAACAAG (SEQ ID NO: 72)

>G00371C-F2 (with NO 5' phosphate)
GACAACAATAAACCGGGCAAAGAGGATAATAACAAGCCTGGTAAGGAAG (SEQ ID NO: 73)

>G00371C-F3 (with a 5' phosphate)
ACAACAACAAACCAGGCAAAGAAGATGGCAACAAGCCGGGTAAGGAGGATAATAAAAA (SEQ ID NO: 74)

>G00371C-F4 (with a 5' phosphate)
ACCAGGCAAGGAAGACGGCAACAAACCTGGCAAGGAGGATAACAAAAAGC (SEQ ID NO: 75)

>G00371C-F5 (with a 5' phosphate)
CAGGCAAGGAGGATGGTAATAAACCGGGCAAAGAAGACGGCAACAAGCCTGGTA (SEQ ID NO: 76)

>G00371C-F6 (with a 5' phosphate)
AAGAAGACGGTAACGGTGTACACGTCGTTAAACCTGGTGACACCGTGAA (SEQ ID NO: 77)

>G00371C-F7 (with a 5' phosphate)
CGACATCGCTAAGGCTAATGGCACCACGGCAGACAAGATTGCAGCGGACAATAAATTAGC TG (SEQ ID NO: 78)

>G00371C-F8 (with a 5' phosphate)
ATAAATAAGGATCCGCGGAAGCTTACCCAGCTTTCTTGTACAAAGTGGTCCCC (SEQ ID NO: 79)

>G00371C-R1 (with a 5' phosphate)
TTTGCCCGGTTTATTGTTGTCCTCCTTGCCCGGCTTGTTGTGAGACCAGCCTGCTTTTTT G (SEQ ID NO: 80)

>G00371C-R2 (with a 5' phosphate)
CTTCTTTGCCTGGTTTGTTGTTGTCTTCCTTACCAGGCTTGTTATTATCCTC (SEQ ID NO: 81)

>G00371C-R3 (with a 5' phosphate)
GGTTTGTTGCCGTCTTCCTTGCCTGGTTTTTTATTATCCTCCTTACCCGGCTTGTTGCCA T (SEQ ID NO: 82)

>G00371C-R4 (with a 5' phosphate)
TACCATCCTCCTTGCCTGGCTTTTTGTTATCCTCCTTGCCA (SEQ ID NO: 83)

>G00371C-R5 (with a 5' phosphate)
GTACACCGTTACCGTCTTCTTTACCAGGCTTGTTGCCGTCTTCTTTGCCCGGTTTAT (SEQ ID NO: 84)

>G00371C-R6 (with a 5' phosphate)
GTGGTGCCATTAGCCTTAGCGATGTCGTTCACGGTGTCACCAGGTTTAACGACGT (SEQ ID NO: 85)

>G00371C-R7 (with NO 5' phosphate)
TAAGCTTCCGCGGATCCTTATTTATCAGCTAATTTATTGTCCGCTGCAATCTTGTCTGCC (SEQ ID NO: 86)

>G00371C-R8 (with NO 5' phosphate) rescue
GGGGACCACTTTGTACAAGAAAGCTGGG (SEQ ID NO: 87)

All oligonucleotides except for F1 and R8 were adjusted to a concentration of 10 μM and an equal volume of each were mixed together to provide an oligonucleotide pool with a total oligonucleotide concentration of 10 μM. A mixture of 12 μl oligo pool, 32 μl water, 5 μl 10x buffer and 1 μl of thermostable DNA ligase (either Pfu ligase or Ampligase) was prepared. A polynucleotide was synthesized from the mixture of oligonucleotides using the polymerase chain reaction by subjecting the mixture to the temperature steps shown in Figure 43.

After the synthesis step, the polynucleotide segment was amplified using a mix containing 1 x Herculase reaction buffer (supplied by Stratagene), 300 μM each of dATP, dCTP, dGTP and dTTP, 2 mM MgSO4, 0.5 μM oligonucleotide AF1, 0.5 μM oligonucleotide AR1, a 1/10 dilution (ie 5 μl in a 50 μl reaction) of the product of the synthesis reaction from the previous step and a 1/100 dilution of Herculase polymerase (a mixture of Taq and Pfu thermostable DNA polymerases from Stratagene). The product was amplified by subjecting the mixture to the following conditions: 96°C for 2 minutes, then 20 cycles of (96°C for 30 seconds, 56°C for 30 seconds, 72°C for 30 seconds). The PCR product was then cloned into Invitrogen vector pDONR221 by mixing 2 μl of PCR product, 2 μl (300 ng) of pDONR221 vector DNA, 4 μl of 5x clonase reaction buffer, 8 μl TE (10 mM Tris-Cl pH 7.5, 1 mM EDTA) and incubating for 60 minutes at 25°C. The reaction was stopped by the addition of 2 μl proteinase K solution (2 mg/ml) and incubation at 37°C for ten minutes. Following this recombination, 1 μl of ligation mix was transformed into chemically competent E. *coli* TOP10 cells and plated onto LB agar plates supplemented with ampicillin and grown for 24 hours at 37°C. Four transformed colonies were picked into 3 ml liquid LB medium and grown for 24 hours at 37°C before plasmid was prepared from them. The sequences of the inserts cloned into the plasmids were determined by sequencing using an ABI 3730. One of the four plasmids for Part3 contained an insert whose sequence was identical to the sequence designed.

The inserts for the three parts were excised from pDONR221. Part 1 was excised by digestion with KpnI and BsaI. Part 2 was excised by digestion with BsaI. Part 3 was excised by digestion with BsaI and HindIII. Each fragment was purified on an agarose gel and equimolar amounts were combined with a vector (pDRIVE) that had been digested with HindIII and KpnI. After a two hour ligation, 1 μl of ligation mix was transformed into chemically competent E coli TOP 10 cells and plated onto LB agar plates supplemented with ampicillin and grown for 24 hours at 37°C. Four transformed colonies were picked into 3 ml liquid LB medium and grown for 24 hours at 37°C before plasmid was prepared from them. The sequences of the inserts cloned into the plasmids were determined by sequencing using an ABI 3730. Four of the plasmids contained an insert whose sequence was identical to the sequence designed as shown below:

```
ccggtaacgcgtATGGCGCAACATGACGAAGCTCAGCAGAACGCTTTTTACCAGGTACTGAACATGCCGAAC
CTGAACGCGGATCAGCGCAACGGTTTCATCCAGAGCCTGAAAGACGACCCTTCTCAGTCCGCAAACGTTCTG
GGCGAGGCTCAGAAACTGAACGACAGCCAGGCCCCAAAAGCAGATGCTCAGCAAAATAACTTCAACAAGGAC
CAGCAGAGCGCATTCTACGAAATCCTGAACATGCCAAATCTGAACGAAGCTCAACGCAACGGCTTCATTCAG

TCTCTGAAAGACGATCCGTCCCAGTCCACTAACGTTCTGGGTGAAGCTAAGAAGCTGAACGAATCCCAGGCA
CCAAAAGCAGACAACAACTTCAACAAAGAGCAGCAGAACGCTTTCTATGAAATCTTGAACATGCCTAACCTG
AATGAAGAACAGCGTAACGGCTTCATCCAGTCTCTGAAGGACGACCCTAGCCAGTCTGCTAACCTGCTGTCC
GAAGCAAAAAAACTGAACGAGTCCCAGGCTCCAAAAGCGGATAACAAATTCAACAAGGAGCAGCAGAACGCA
TTCTACGAAATCCTGCACCTGCCGAACCTGAACGAAGAACAGCGTAACGGTTTCATCCAATCCCTGAAAGAC
GATCCTTCCCAGTCCGCAAATCTGCTGGCAGAAGCAAAGAAACTGAACGACGCACAGGCACCGAAGGCTGAC
AACAAGTTCAACAAAGAGCAGCAGAATGCCTTCTACGAGATTCTGCATCTGCCAAACCTGACTGAGGAGCAG
CGCAACGGTTTCATTCAGTCCCTGAAGGACGACCCAAGCGTCAGCAAGGAAATCCTGGCTGAGGCGAAAAAA
CTGAACGATGCACAGGCTCCGAAGGAAGAAGACAACAATAAACCTGGTAAAGAAGATAATAATAAGCCTGGC
AAGGAAGATAACAACAAGCCGGGCAAGGAGGACAACAATAAACCGGGCAAAGAGGATAATAACAAGCCTGGT
AAGGAAGACAACAACAAACCAGGCAAAGAAGATGGCAACAAGCCGGGTAAGGAGGATAATAAAAAACCAGGC
AAGGAAGACGGCAACAAACCTGGCAAGGAGGATAACAAAAAGCCAGGCAAGGAGGATGGTAATAAACCGGGC
AAAGAAGACGGCAACAAGCCTGGTAAAGAAGACGGTAACGGTGTACACGTCGTTAAACCTGGTGACACCGTG
AACGACATCGCTAAGGCTAATGGCACCACGGCAGACAAGATTGCAGCGGACAATAAATTAGCTGATAAAtaa
ggatccgcgg  (SEQ ID NO: 88)
```

### 6.7 IMPROVING POLYNUCLEOTIDE SYNTHESIS FIDELITY USING T7 ENDONUCLEASE

In this example a polynucleotide was desired to encode the following polypeptide:

```
APAVEQRSEAAPLIEARGEMVANKYIVKFKEGSALSALDAAMEKISGKPDHVYKNVFSGFAATLDENMVRVL
RAHPDVEYIEQDAVVTINAAQTNAPWGLARISSTSPGTSTYYYDESAGQGSCVYVIDTGIEASHPEFEGRAQ
MVKTYYYSSRDGNGHGTHCAGTVGSRTYGVAKKTQLFGVKVLDDNGSGQYSTIIAGMDFVASDKNNRNCPKG
VVASLSLGGGYSSSVNSAAARLQSSGVMVAVAAGNNNADARNYSPASEPSVCTVGASDRYDRRSSFSNYGSV
LDIFAPGTSILSTWIGGSTRSISGTSMATPHVAGLAAYLMTLGKTTAASACRYIADTANKGDLSNIPFGTVN
LLAYNNYQA  (SEQ ID NO: 89)
```

The polynucleotide was designed as described in Example 6.2:

GCACCGGCCGTTGAACAGCGTTCTGAAGCAGCTCCTCTGATTGAGGCACGTGGTGAAATGGTAGCAAACAAG
TACATCGTGAAGTTCAAGGAGGGTTCTGCTCTGTCTGCTCTGGATGCTGCTATGGAAAAGATCTCTGGCAAG
CCTGATCACGTCTATAAGAACGTGTTCAGCGGTTTCGCAGCAACTCTGGACGAGAACATGGTCCGTGTACTG
CGTGCTCATCCAGACGTTGAATACATCGAACAGGACGCTGTGGTTACTATCAACGCGGCACAGACTAACGCA
CCTTGGGGTCTGGCACGTATTTCTTCTACTTCCCCGGGTACGTCTACTTACTACTACGACGAGTCTGCCGGT
CAAGGTTCTTGCGTTTACGTGATCGATACGGGCATCGAGGCTTCTCATCCTGAGTTTGAAGGCCGTGCACAA
ATGGTGAAGACCTACTACTACTCTTCCCGTGACGGTAATGGTCACGGTACTCATTGCGCAGGTACTGTTGGT
AGCCGTACCTACGGTGTTGCTAAGAAAACGCAACTGTTCGGCGTTAAAGTGCTGGACGACAACGGTTCTGGT
CAGTACTCCACCATTATCGCGGGTATGGATTTCGTAGCGAGCGATAAAAACAACCGCAACTGCCCGAAAGGT
GTTGTGGCTTCTCTGTCTCTGGGTGGTGGTTACTCCTCTTCTGTTAACAGCGCAGCTGCACGTCTGCAATCT
TCCGGTGTCATGGTCGCAGTAGCAGCTGGTAACAATAACGCTGATGCACGCAACTACTCTCCTGCTAGCGAG
CCTTCTGTTTGCACCGTGGGTGCATCTGATCGTTATGATCGTCGTAGCTCCTTCAGCAACTATGGTTCCGTC
CTGGATATCTTCGCGCCTGGTACTTCTATCCTGTCTACCTGGATTGGCGGTAGCACTCGTTCCATTTCCGGT
ACGAGCATGGCTACTCCACATGTTGCTGGTCTGGCAGCATACCTGATGACCCTGGGTAAGACCACTGCTGCA
TCCGCTTGTCGTTACATCGCGGATACTGCGAACAAAGGCGATCTGTCTAACATCCCGTTCGGCACCGTTAAT
CTGCTGGCATACAACAACTATCAGGCT (SEQ ID NO: 90)

An oligonucleotide set was designed as described in Example 6.2:oligo Name Sequence (5' to 3')

E189-AF1 TAACAGGAGGAATTAACCATGAAAAAACTG (SEQ ID NO: 91)
E189-AR1 TAATCTGTATCAGGCTGAAAATCTTCTCT (SEQ ID NO: 92)
E189-F1 TAACAGGAGGAATTAACCATGAAAAAACTGCTGTTC (SEQ ID NO: 93)
E189-F5 AAGTACATCGTGAAGTTCAAGGAGGGTTCTGCTCTGTCTGC (SEQ ID NO: 94)
E189-F9 CGTTGAATACATCGAACAGGACGCTGTGGTTACTATCAACGCG (SEQ ID NO: 95)
E189-F13 GGCATCGAGGCTTCTCATCCTGAGTTTGAAGGCCGTGC (SEQ ID NO: 96)
E189-F17 TTAAAGTGCTGGACGACAACGGTTCTGGTCAGTACTCCACC (SEQ ID NO: 97)
E189-F21 CGTCTGCAATCTTCCGGTGTCATGGTCGCAGTAGCAG (SEQ ID NO: 98)
E189-F29 CGTTACATCGCGGATACTGCGAACAAAGGCGATCTGTCTAACA (SEQ ID NO: 99)
E189-R1 CCACCAGCGGAATCGCGAACAGCAGTTTTTTCATGGTTAATT (SEQ ID NO: 100)
E189-R5 TTTTCCATAGCAGCATCCAGAGCAGACAGAGCAGAACCC (SEQ ID NO: 101)
E189-R9 AGGTGCGTTAGTCTGTGCCGCGTTGATAGTAACCACAGC (SEQ ID NO: 102)
E189-R13 GTAGTAGTAGGTCTTCACCATTTGTGCACGGCCTTCAAACTCAG (SEQ ID NO: 103)
E189-R17 CGAAATCCATACCCGCGATAATGGTGGAGTACTGACCAGAAC (SEQ ID NO: 104)
E189-R21 GCATCAGCGTTATTGTTACCAGCTGCTACTGCGACCATGAC (SEQ ID NO: 105)
E189-R25 ACGAGTGCTACCGCCAATCCAGGTAGACAGGATAGAAGTACC (SEQ ID NO: 106)
E189-R29 CGGTGCCGAACGGGATGTTAGACAGATCGCCTTTGTTC (SEQ ID NO: 107)
E189-F2 GCGATTCCGCTGGTGGTGCCGTTCTATAGCCATAGC (SEQ ID NO: 108)
E189-F6 TCTGGATGCTGCTATGGAAAAGATCTCTGGCAAGCCTGATC (SEQ ID NO: 109)
E189-F10 GCACAGACTAACGCACCTTGGGGTCTGGCACGTAT (SEQ ID NO: 110)
E189-F14 ACAAATGGTGAAGACCTACTACTACTCTTCCCGTGACGGTAATGG (SEQ ID NO: 111)
E189-F18 ATTATCGCGGGTATGGATTTCGTAGCGAGCGATAAAAACAACCG (SEQ ID NO: 112)
E189-F22CTGGTAACAATAACGCTGATGCACGCAACTACTCTCCTGCT (SEQ ID NO: 113)
E189-F26 ATTGGCGGTAGCACTCGTTCCATTTCCGGTACGAGCA (SEQ ID NO: 114)
E189-F30 TCCCGTTCGGCACCGTTAATCTGCTGGCATACAACAAC (SEQ ID NO: 115)
E189-R2 GGCCGGTGCCATGGTGCTATGGCTATAGAACGGCA (SEQ ID NO: 116)
E189-R6 GCTGAACACGTTCTTATAGACGTGATCAGGCTTGCCAGAGATC (SEQ ID NO: 117)
E189-R10 ACCCGGGGAAGTAGAAGAAATACGTGCCAGACCCCA (SEQ ID NO: 118)
E189-R14 GCGCAATGAGTACCGTGACCATTACCGTCACGGGAAGA (SEQ ID NO: 119)
E189-R18 ACACCTTTCGGGCAGTTGCGGTTGTTTTTATCGCTCGCTA (SEQ ID NO: 120)
E189-R22 GCAAACAGAAGGCTCGCTAGCAGGAGAGTAGTTGCGT (SEQ ID NO: 121)
E189-R26 GCAACATGTGGAGTAGCCATGCTCGTACCGGAAATGGA (SEQ ID NO: 122)
E189-R30 TGATGGTCGACAGCCTGATAGTTGTTGTATGCCAGCAGATTAA (SEQ ID NO: 123)
E189-F3 ACCATGGCACCGGCCGTTGAACAGCGTTCTGAAGC (SEQ ID NO: 124)

E189-F7 ACGTCTATAAGAACGTGTTCAGCGGTTTCGCAGCAACTCTGG (SEQ ID NO: 125)
E189-F11 TTCTTCTACTTCCCCGGGTACGTCTACTTACTACTACGACGA (SEQ ID NO: 126)
E189-F15 TCACGGTACTCATTGCGCAGGTACTGTTGGTAGCCGT (SEQ ID NO: 127)
E189-F19 CAACTGCCCGAAAGGTGTTGTGGCTTCTCTGTCTCTGG (SEQ ID NO: 128)
E189-F23 AGCGAGCCTTCTGTTTGCACCGTGGGTGCATCTGA (SEQ ID NO: 129)
E189-F27 TGGCTACTCCACATGTTGCTGGTCTGGCAGCATACCT (SEQ ID NO: 130)
E189-F31 TATCAGGCTGTCGACCATCATCATCATCATCATTGAGTTTAAACGG (SEQ ID NO: 131)
E189-R3 TGCCTCAATCAGAGGAGCTGCTTCAGAACGCTGTTCAAC (SEQ ID NO: 132)
E189-R7 ACACGGACCATGTTCTCGTCCAGAGTTGCTGCGAAACC (SEQ ID NO: 133)
E189-R11 GAACCTTGACCGGCAGACTCGTCGTAGTAGTAAGTAGACGT (SEQ ID NO: 134)
E189-R15 TTTCTTAGCAACACCGTAGGTACGGCTACCAACAGTACCT (SEQ ID NO: 135)
E189-R19 CAGAAGAGGAGTAACCACCACCCAGAGACAGAGAAGCCACA (SEQ ID NO: 136)
E189-R23 GAGCTACGACGATCATAACGATCAGATGCACCCACGGT (SEQ ID NO: 137)
E189-R27 TGGTCTTACCCAGGGTCATCAGGTATGCTGCCAGACCA (SEQ ID NO: 138)
E189-R31 AAAACAGCCAAGCTGGAGACCGTTTAAACTCAATGATGATGATGA (SEQ ID NO: 139)
E189-F4 AGCTCCTCTGATTGAGGCACGTGGTGAAATGGTAGCAAAC (SEQ ID NO: 140)
E189-F8 ACGAGAACATGGTCCGTGTACTGCGTGCTCATCCAGA (SEQ ID NO: 141)
E189-F12 GTCTGCCGGTCAAGGTTCTTGCGTTTACGTGATCGATACG (SEQ ID NO: 142)
E189-F16 ACCTACGGTGTTGCTAAGAAAACGCAACTGTTCGGCG (SEQ ID NO: 143)
E189-F20 GTGGTGGTTACTCCTCTTCTGTTAACAGCGCAGCTGCA (SEQ ID NO: 144)
E189-F24 TCGTTATGATCGTCGTAGCTCCTTCAGCAACTATGGTTCCGT (SEQ ID NO: 145)
E189-F28 GATGACCCTGGGTAAGACCACTGCTGCATCCGCTTGT (SEQ ID NO: 146)
E189-F32 TCTCCAGCTTGGCTGTTTTGGCGGATGAGAGAAGATTTTCA (SEQ ID NO: 147)
E189-F25 CCTGGATATCTTCGCGCCTGGTACTTCTATCCTGTCTACCTGG (SEQ ID NO: 148)
E189-R4 TCCTTGAACTTCACGATGTACTTGTTTGCTACCATTTCACCACG (SEQ ID NO: 149)
E189-R8 GTCCTGTTCGATGTATTCAACGTCTGGATGAGCACGCAGT (SEQ ID NO: 150)
E189-R12 GATGAGAAGCCTCGATGCCCGTATCGATCACGTAAACGCAA (SEQ ID NO: 151)
E189-R16 CGTTGTCGTCCAGCACTTTAACGCCGAACAGTTGCGT (SEQ ID NO: 152)
E189-R20 ACCGGAAGATTGCAGACGTGCAGCTGCGCTGTTAA (SEQ ID NO: 153)
E189-R28 GCAGTATCCGCGATGTAACGACAAGCGGATGCAGCAG (SEQ ID NO: 154)
E189-R32 TAATCTGTATCAGGCTGAAAATCTTCTCTCATCCGCC (SEQ ID NO: 155)
E189-R24 AGGCGCGAAGATATCCAGGACGGAACCATAGTTGCTGAAG (SEQ ID NO: 156)

All oligonucleotides except for AF1 and AR1 were adjusted to a concentration of 10 μM and an equal volume of each were mixed together to provide an oligonucleotide pool with a total oligonucleotide concentration of 10 μM. This pool was diluted 10-fold by adding 5 μl into a mixture of 5 μl 10x Herculase buffer (from Stratagene), 2.5 μl DMSO, 2.5 μl dNTPs (6 mM each of dATP, dCTP, dGTP and dTTP: the final concentration in the mixture is 300 μM each), 2.5 μl $MgSO_4$ (40 mM: the final concentration in the mix is 2 mM), 32 μl water and 0.5 μl Herculase polymerase (a mixture of Taq and Pfu thermostable DNA polymerases from Stratagene). A polynucleotide was synthesized from the mixture of oligonucleotides using the polymerase chain reaction by subjecting the mixture to the temperature steps shown in Figure 34 using an annealing temperature of 58°C.

After the synthesis step, the polynucleotide was amplified using a mix containing 1 x Herculase reaction buffer (supplied by Stratagene), 300 μM each of dATP, dCTP, dGTP and dTTP, 2 mM $MgSO_4$, 0.5 μM oligonucleotide AF1, 0.5 μM oligonucleotide AR1, a 1/10 dilution (ie 5 μl in a 50 μl reaction) of the product of the synthesis reaction from the previous step and a 1/100 dilution of Herculase polymerase (a mixture of Taq and Pfu thermostable DNA polymerases from Stratagene). The product was amplified by subjecting the mixture to the following conditions: 96°C for two minutes, then 20 cycles of (96°C for 30 seconds, 58°C for 30 seconds, 72°C for 90 seconds). The 1100 bp DNA product was then purified using a Qiagen PCR cleanup kit.

After purification, 2.5 μg of DNA was placed into a 100 μl reaction in 50 mM potassium acetate, 20 mM Tris-acetate, 10 mM magnesium acetate, 1 mM dithiothreitol pH 7.9. This mixture was heated to 94°C for three minutes, and then cooled to 75°C for five minutes. The tube was then cooled to 37°C, 3 μl of T7 endonuclease I (10U/ μl) was added, and the tube incubated at 37°C for 1 hour and 55°C for 1 hour. A control sample was treated in the same way, but the endonuclease was omitted. Following endonuclease digestion, the DNA was ethanol precipitated and resuspended before the ends of the DNA were cleaved using NcoI and SalI restriction enzymes. The DNA was purified again using a Qiagen PCR cleanup kit and ligated into a vector that had been previously digested with NcoI and SalI. After a 4-hour ligation, 1 μl of ligation mix was transformed into chemically competent E coli TOP10 cells and plated onto LB agar plates supplemented with ampicillin and grown for 24 hours at 37°C. A total of 48 colonies from the treated and untreated samples were subsequently analyzed for protease function. Twenty-four out of forty eight colonies from the treated sample were incorrect (50%), compared with thirty three of the forty eight colonies from the untreated sample (69%).

### 6.8 IMPROVING SYNTHETIC POLYNUCLEOTIDE CLONING FIDELTIY USING RECOMBINASE

In this example it was desired to synthesize the following polynucleotide:

```
CGGGGACAAGTTTGTACAAAAAAGCAGGCTGCTCTTCGCCTGCTGGCTGGTAATCGCCAGCAGGCCTTTTTA
TTTGGGGGAGAGGGAAGTCATGAAAAAACTAACCTTTGAAATTCGATCTCCACCACATCAGCTCTGAAGCAA

CGTAAAAAAACCCGCCCCGGCGGGTTTTTTTATACCCGTAGTATCCCCACTTATCTACAATAGCTGTCCTTA
ATTAAGGTTGAATAAATAAAAACAGCCGTTGCCAGAAAGAGGCACGGCTGTTTTTATTTTCTAGTGAGACCG
GGACCAGTTTATTAAGCGCCAGTGCTATGACGACCTTCTGCGCGCTCGTACTGTTCGACAATGGTGTAATCT
TCGTTGTGAGAAGTGATGTCCAGCTTGATGTCAGTTTTGTAAGCGCCCGGCAGTTGCACAGGTTTTTTTGCC
ATGTACGTAGTTTTTACCTCTGCGTCGTAGTGACCACCGTCCTTCAGCTTCAGGCGCATTTTAATTTCGCCC
TTCAGGGCACCATCTTCCGGGTACATACGCTCAGTGGACGCTTCCCAACCCATCGTCTTTTTCTGCATTACA
GGACCGTCAGACGGGAAGTTAGTACCGCGCAGCTTCACTTTGTAGATGAACTCGCCGTCTTGCAGGCTAGAG
TCTTGGGTCACAGTCACCACACCACCGTCCTCGAAGTTCATAACACGTTCCCATTTGAAACCTTCCGGGAAA
GACAGTTTCAGGTAATCCGGAATATCCGCCGGGTGTTTAACGTACGCCTTAGAGCCATACTGGAACTGAGGG
CTCAGAATATCCCATGCAAAAGGCAGTGGGCCACCTTTGGTCACTTTCAGTTTCGCGGTCTGAGTACCCTCG
TAAGGACGGCCTTCACCTTCACCCTCGATTTCAAATTCGTGGCCATTTACAGAGCCCTCCATACGCACTTTG
AAGCGCATGAACTCCTTGATTACATCTTCAGAGGAGGCCATTTTTTTTTCCTCCTTATTTTCTCAAGCCTAG
GTCTGTGTGAAATTGTTATCCGCTCACAATTGAATCTATCATAATTGTGAGCGCTCACAATTGTAAAGGTTA
GATCCGCTAATCTTATGGATAAAAATGCTATGTTCCCCCCGGGGGGATATCAACAGGAGTCCAAGCGACCGG
TGGTTGCATGTCTAGCTAGCTAGAACAGGACTAGTCCTGAGTAATAGTCAAAAGCCTCCGGTCGGAGGCTTT
TGACTTTCTGAAATGTAATCACACTGGCTCACCTTCGGGTGGGCCTTTCTGCGTTTATAAGAAGGAAAAAAG
CGGCCGCAAAAGGAAAAAATTATTCGTATAGCATACATTATACGAAGTTATAAGCTTACCCAGCTTTCTTGT
ACAAAGTGGTCCCC   (SEQ ID NO: 157)
```

A total of 64 oligonucleotides were designed and synthesized. No polynucleotide product was obtained when all oligonucleotides except for AF1 and AR1 were assembled in a single reaction. Instead the polynucleotide was divided into four segments, each consisting of sixteen oligonucleotides: segment 1 from 1-370, segment 2 from 347-691, segment 3 from 671-1028, segment 4 from 1004-1367. Each oligonucleotide was adjusted to a concentration of 10 μM and an equal volume of each was mixed together to provide four oligonucleotide pools, each with a total oligonucleotide concentration of 10 μM. The pools were oligonucleotides F1 to R8 (segment 1), F9 to R16 (segment 2), F17 to R24 (segment 3) and F25 to R32 (segment 4). The pools were diluted tenfold by adding 5 μl into a mixture of 5 μl 10x Herculase buffer (from Stratagene), 2.5 μl DMSO, 2.5 μl dNTPs (6 mM each of dATP, dCTP, dGTP and dTTP: the final concentration in the mixture is 300 μM each), 2.5 μl $MgSO_4$ (40 mM: the final concentration in the mix is 2 mM), 32 μl water and 0.5 μl Herculase polymerase (a mixture of Taq and Pfu thermostable DNA polymerases from Stratagene). Polynucleotides were synthesized from the mixture of oligonucleotides using the polymerase chain reaction by subjecting the mixture to the temperature steps shown in Figure 32 using an annealing temperature of 58°C.

After the synthesis step, the polynucleotide fragments were joined by overlap extension: 2 μl of each assembly reaction were mixed into an amplification reaction containing 1 x Herculase reaction buffer (supplied by Stratagene), 300 μM each of dATP, dCTP, dGTP and dTTP, 2 mM $MgSO_4$, 0.5 μM oligonucleotide AF1, 0.5 μM oligonucleotide AR1 and a 1/100 dilution of Herculase polymerase (a mixture of Taq and Pfu thermostable DNA polymerases from Stratagene). The product was amplified by subjecting the mixture to the following conditions: 96°C for two minutes, then 20 cycles of (96°C for thirty seconds, 58°C for thirty seconds, and 72°C for ninety seconds). Agarose gel analysis of the PCR product showed a ladder of sub-fragments and partially joined fragments.

The PCR product was cloned without purification into Invitrogen vector pDONR221 by mixing 2 μl of PCR product, 2 μl (300 ng) of pDONR221 vector DNA, four μl of 5x clonase reaction buffer, 8 μl TE (10 mM Tris-Cl pH 7.5, 1 mM EDTA) and incubating for sixty minutes at 25°C. The reaction was stopped by the addition of 2 μl proteinase K solution (2 mg/ml) and incubation at 37°C for ten minutes. Following this recombination, 1 μl of ligation mix was transformed into chemically competent E *coli* TOP10 cells and plated onto LB agar plates supplemented with ampicillin and grown for twenty-four hours at 37°C. Eight transformed colonies were picked into 3 ml liquid LB medium and grown for 24 hours at 37°C before plasmid was prepared from them. Eight out of eight of the inserts cloned into the plasmids were determined by restriction digestion to be the correct size. This efficiency in cloning full-length product resulted from the requirement of recombinase-based cloning for a specific sequence at each end. Thus only DNA fragments with the recombinase sites provided in primers AF1 and AR1 can be cloned. This is in contrast to TA or restriction cloning where smaller fragments containing the appropriate ends for cloning will be present in the mixture. Such small fragments tend to dominate cloning products, and can be reduced or eliminated only by gel purification. The recombinase cloning step can thus eliminate the requirement for gel purification, thereby increasing the efficiency and fidelity of polynucleotide synthesis.

## Claims

1. An isolated polynucleotide that encodes a polypeptide, the amino acid sequence of which consists of SEQ ID NO:60.

2. The isolated polynucleotide of claim 1, wherein the polynucleotide comprises codons selected for expression in a chosen host system.

3. The polynucleotide of claim 2, wherein the chosen host system is *Escherichia coli.*

4. A polynucleotide comprising the sequence of SEQ ID NO:88.

5. A vector comprising the polynucleotide of any of claims 1 to 4.

6. A plasmid comprising the nucleic acid molecule of any of claims 1 to 4.

7. An *Escherichia coli* cell comprising the vector of claim 5 or the expression vector of claim 6.

8. An *Escherichia coli* cell comprising the nucleic acid of any of claims 1 to 4.

9. An expression system comprising the nucleic acid of any of claims 1 to 4.

## Patentansprüche

1. Ein isoliertes Polynukleotid, das für ein Polypeptid kodiert, dessen Aminosäuresequenz aus der SEQ ID NO:60 besteht.

2. Das isolierte Polynukleotid nach Anspruch 1, wobei das Polynukleotid Codons umfasst, die für die Expression in einem ausgewählten Wirtssystem selektiert wurden.

3. Das Polynukleotid nach Anspruch 2, wobei das ausgewählte Wirtssystem *Escherichia coli* ist.

4. Ein Polynukleotid, das die Sequenz der SEQ ID NO:88 umfasst.

5. Ein Vektor, der das Polynukleotid nach irgendeinem der Ansprüche 1 bis 4 umfasst.

6. Ein Plasmid, das das Nukleinsäuremolekül nach irgendeinem der Ansprüche 1 bis 4 umfasst.

7. Eine *Escherichia coli* Zelle, die den Vektor nach Anspruch 5 oder den Expressionsvektor nach Anspruch 6 umfasst.

8. Eine *Escherichia coli* Zelle, die die Nukleinsäure nach irgendeinem der Ansprüche 1 bis 4 umfasst.

9. Ein Expressionssystem, das die Nukleinsäure nach irgendeinem der Ansprüche 1 bis 4 umfasst.

**Revendications**

1. Polynucléotide isolé qui code pour un polypeptide, dont la séquence d'acides aminés est constituée de SEQ ID NO: 60.

2. Polynucléotide isolé de la revendication 1, le polynucléotide comprenant des codons choisis pour l'expression dans un système hôte choisi.

3. Polynucléotide de la revendication 2, le système hôte choisi étant *Escherichia coli.*

4. Polynucléotide comprenant la séquence de SEQ ID NO: 88.

5. Vecteur comprenant le polynucléotide de l'une quelconque des revendications 1 à 4.

6. Plasmide comprenant la molécule d'acide nucléique de l'une quelconque des revendications 1 à 4.

7. Cellule *d'Escherichia coli* comprenant le vecteur de la revendication 5 ou le vecteur d'expression de la revendication 6.

8. Cellule *d'Escherichia coli* comprenant l'acide nucléique de l'une quelconque des revendications 1 à 4.

9. Système d'expression comprenant l'acide nucléique de l'une quelconque des revendications 1 à 4.

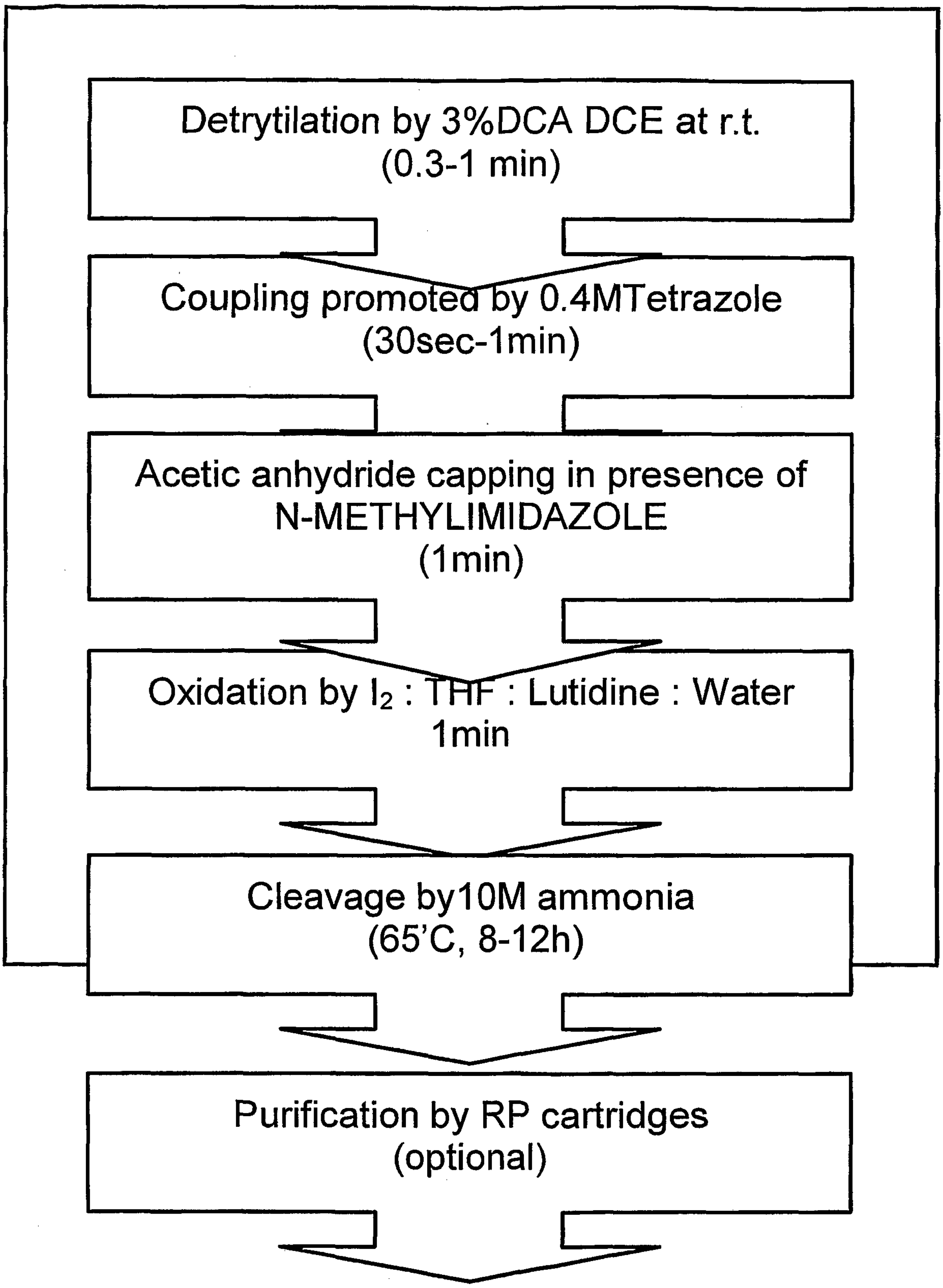


Figure 1
(Prior Art)

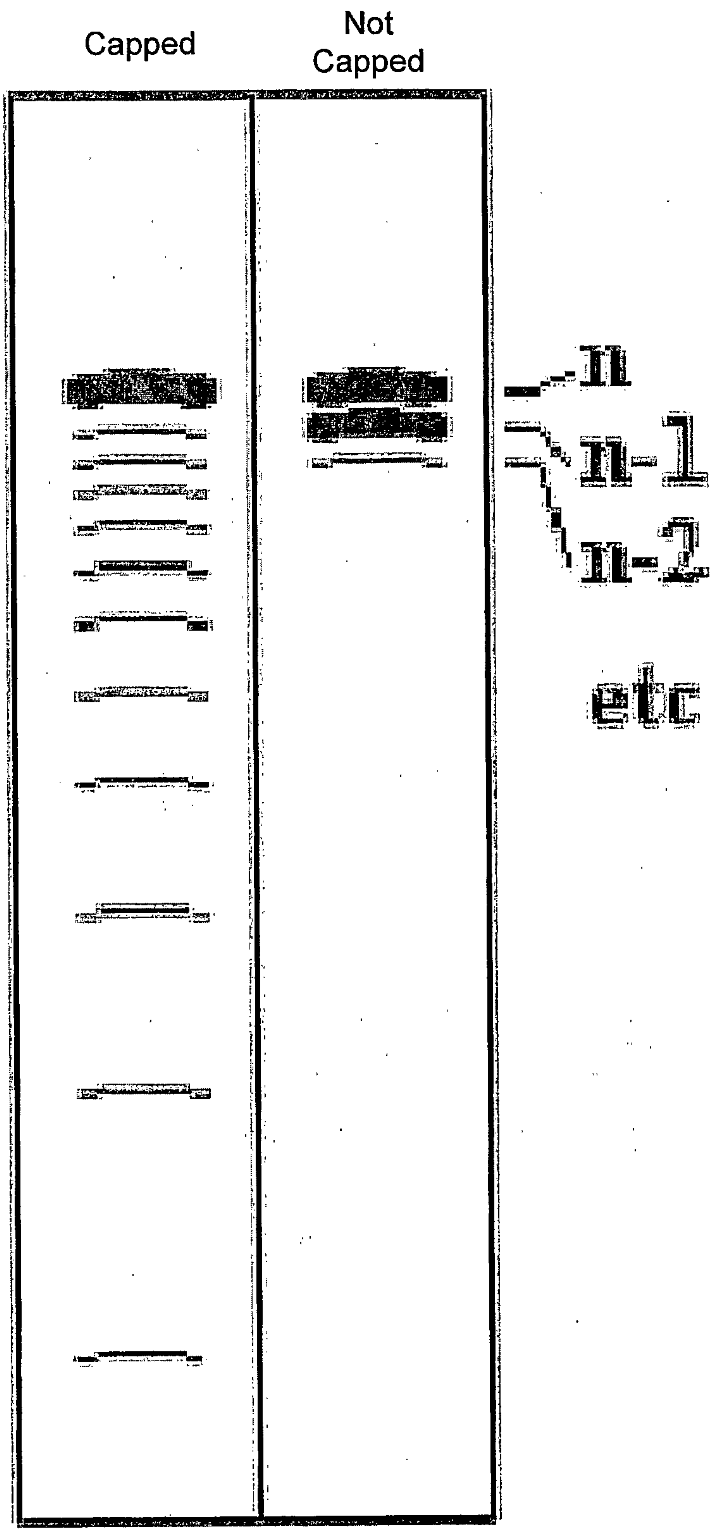


Figure 2A

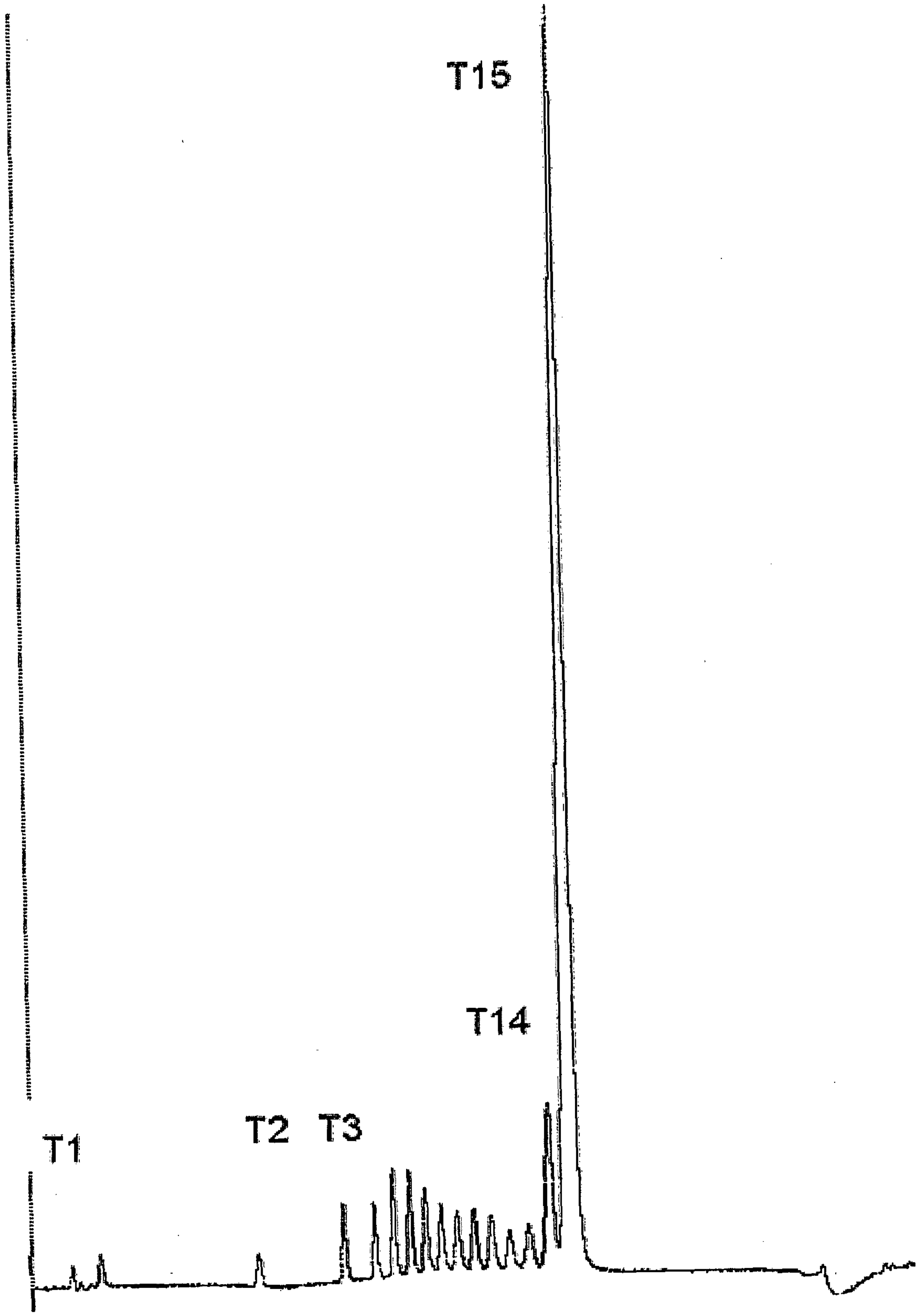


Figure 2B

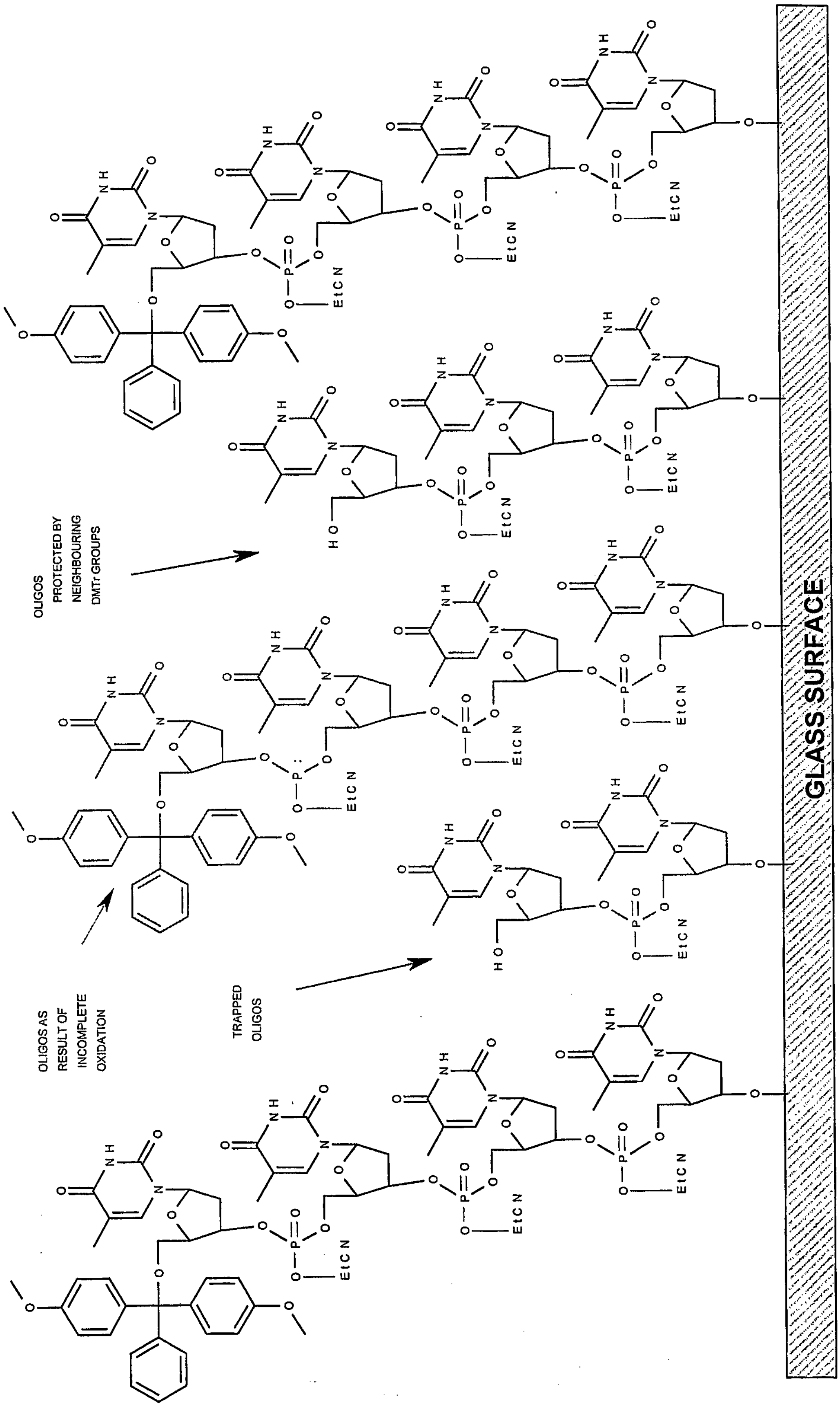
OLIGOS AS RESULT OF INCOMPLETE OXIDATION
TRAPPED OLIGOS
OLIGOS PROTECTED BY NEIGHBOURING DMTr GROUPS
GLASS SURFACE

Figure 2C

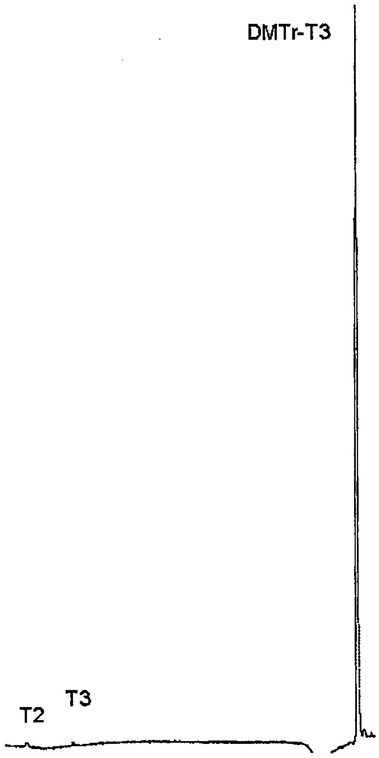

Figure 3A

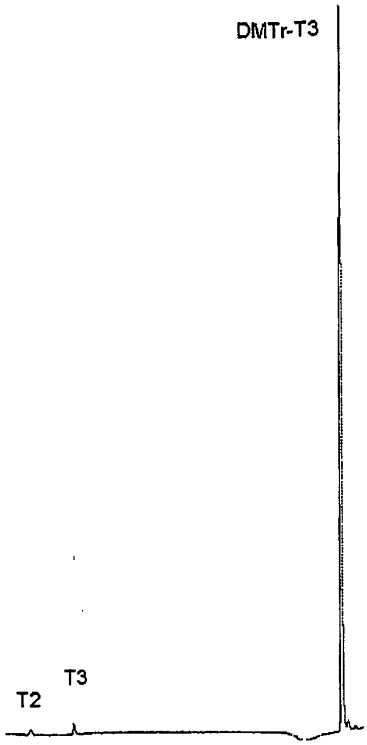

Figure 3B

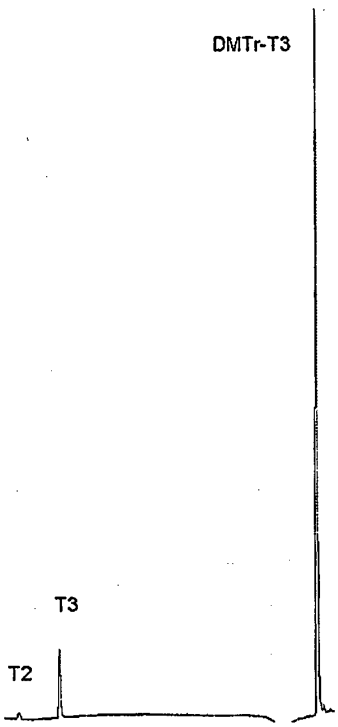

Figure 3C

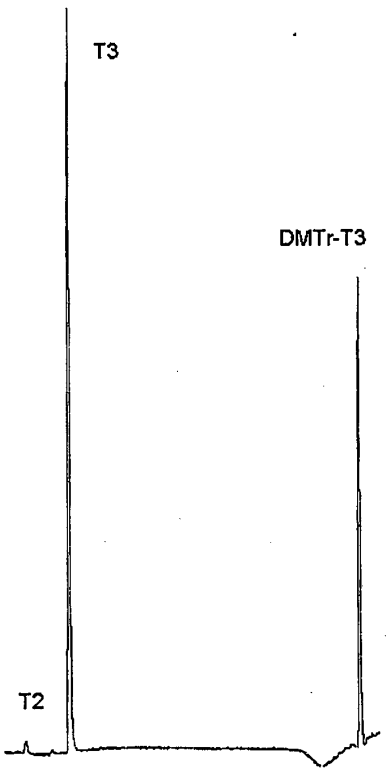

Figure 3D

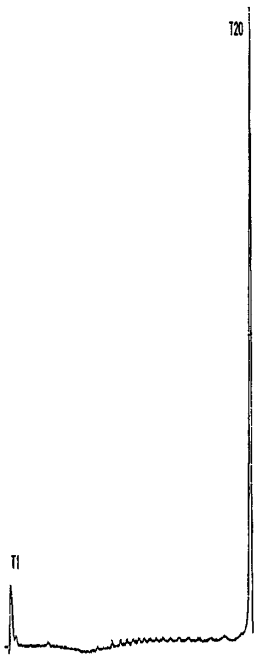


FIGURE 4A

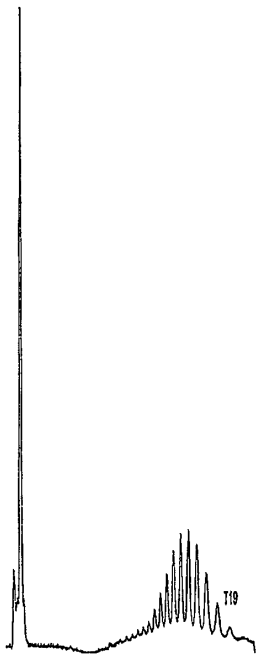


FIGURE 4B

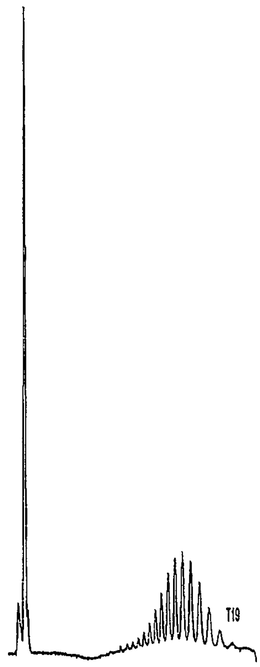


FIGURE 4C

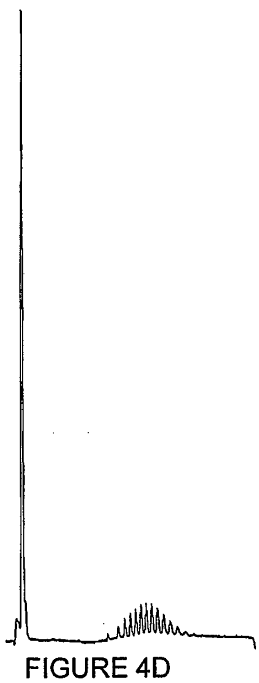

FIGURE 4D

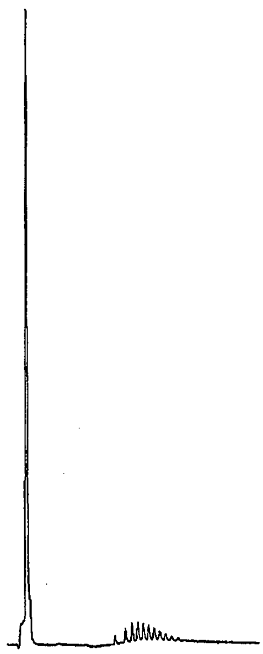

FIGURE 4E

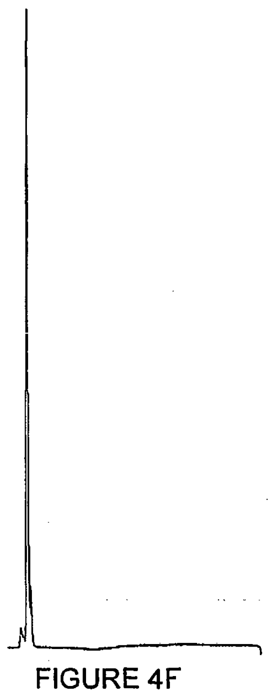

FIGURE 4F

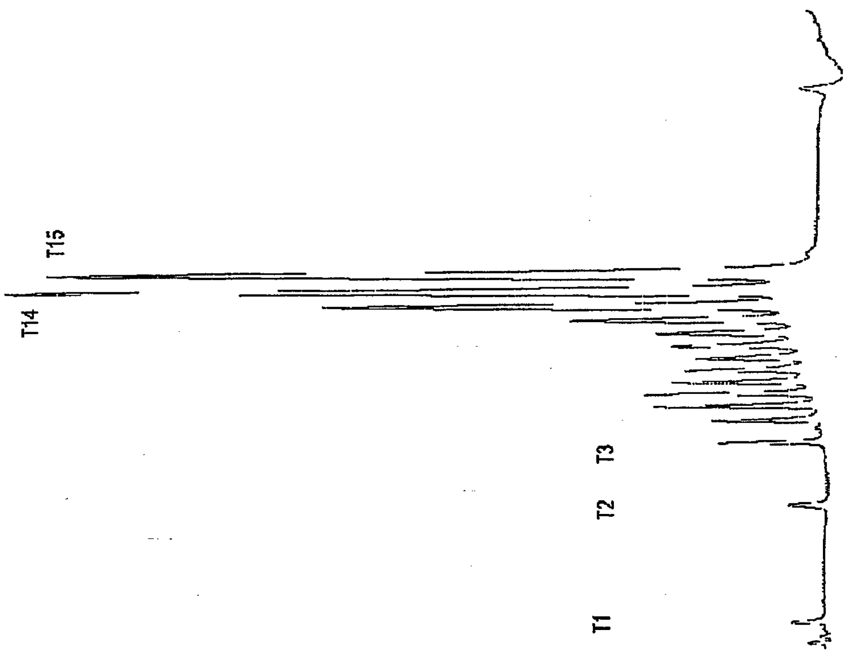


Figure 5A

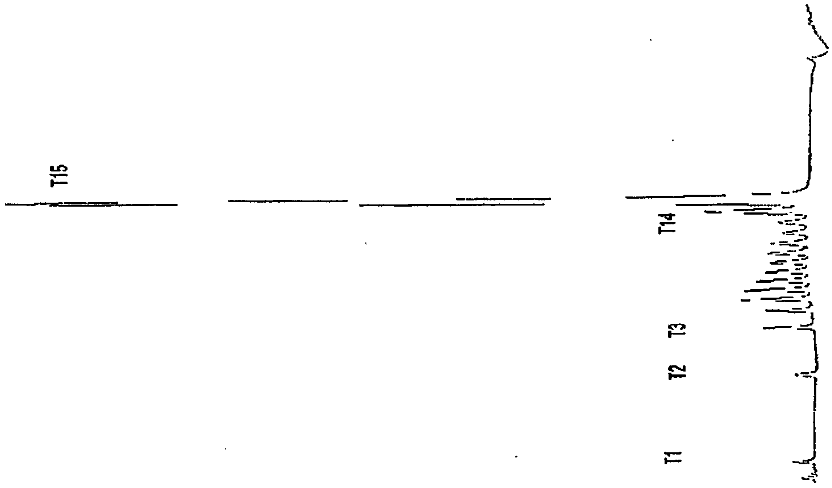


Figure 5B

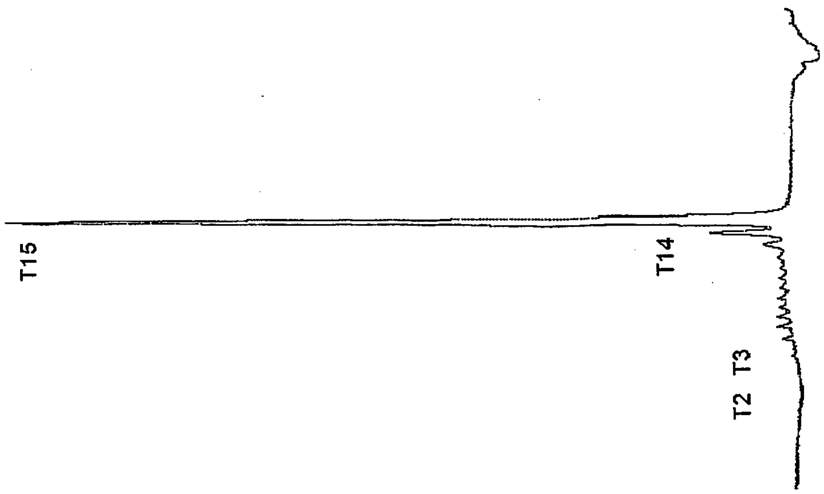


Figure 5C

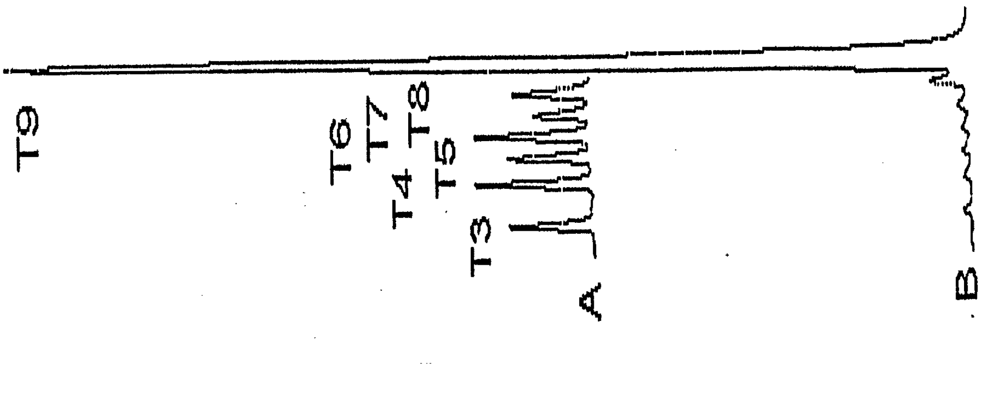

Figure 6A

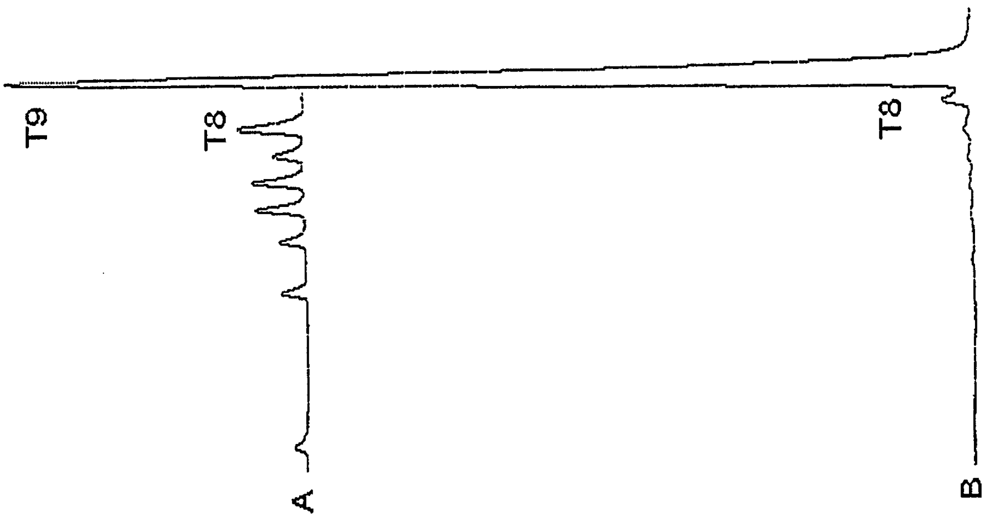

Figure 6B

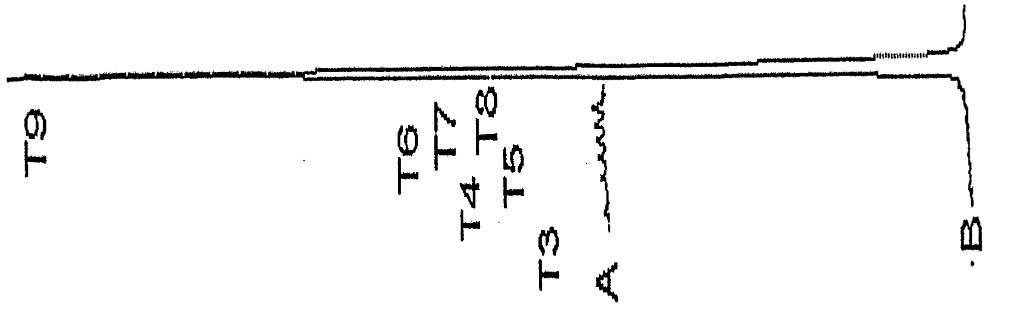

Figure 6C

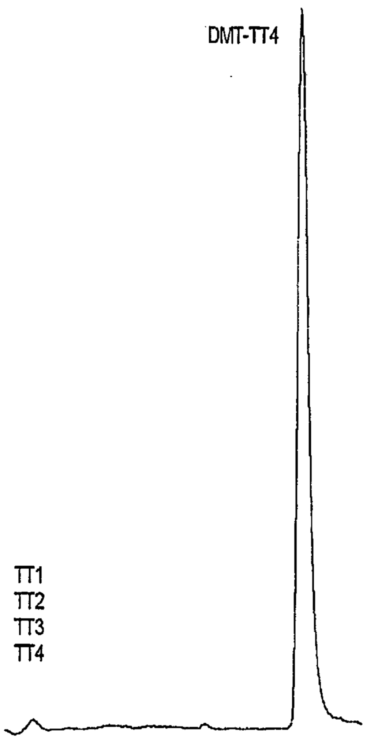


Figure 7A

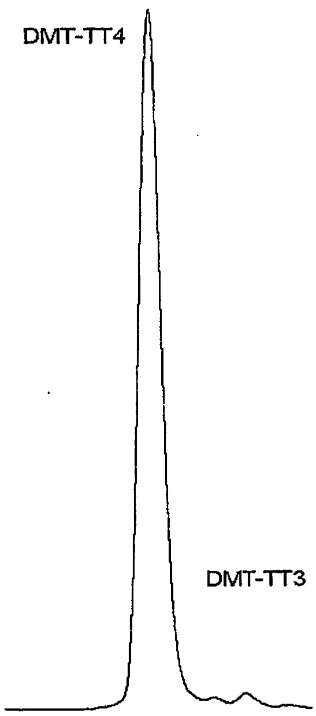


Figure 7B

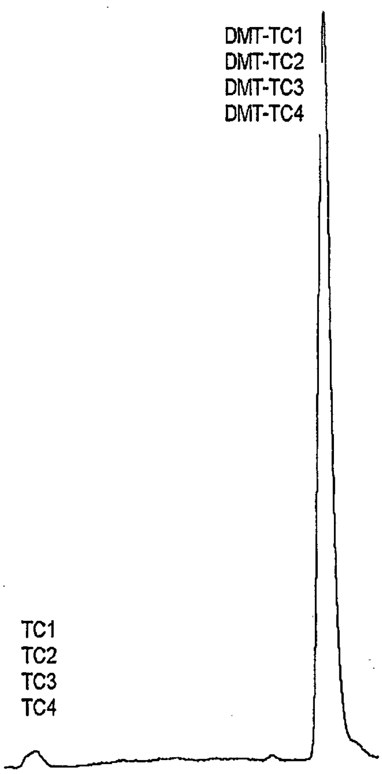


Figure 7C

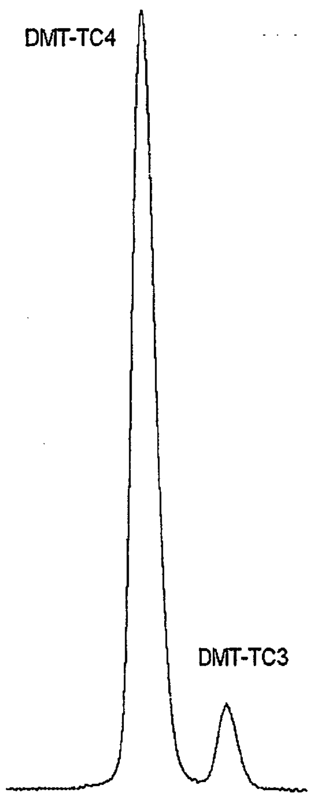


Figure 7D

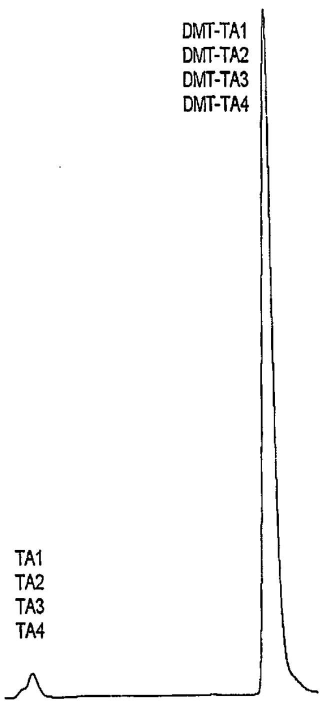


Figure 7E

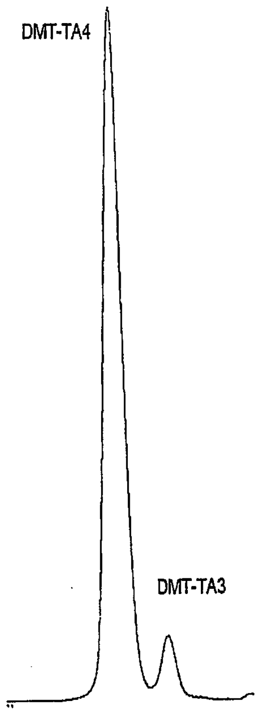


Figure 7F

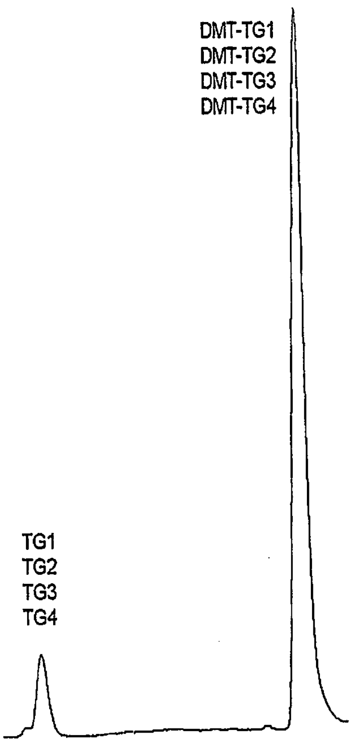


Figure 7G

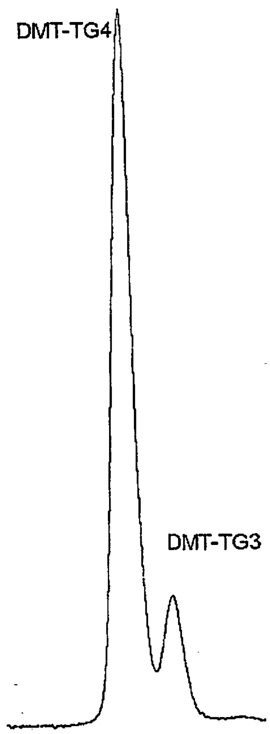


Figure 7H

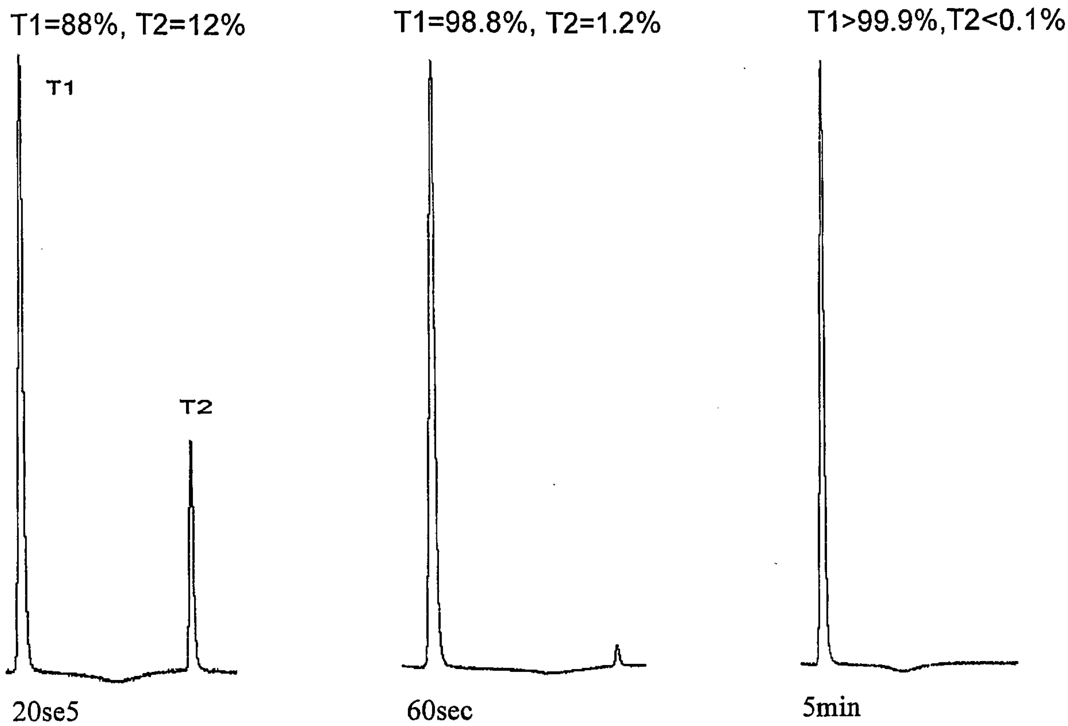


Figure 8A

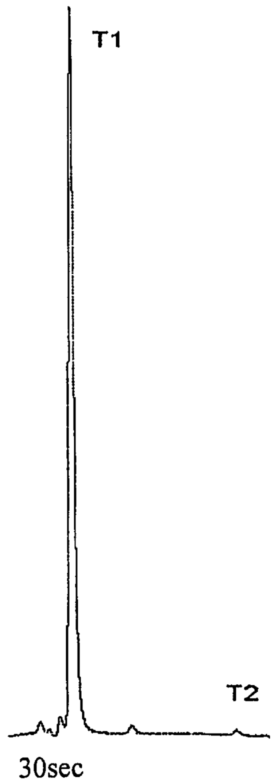


Figure 8B

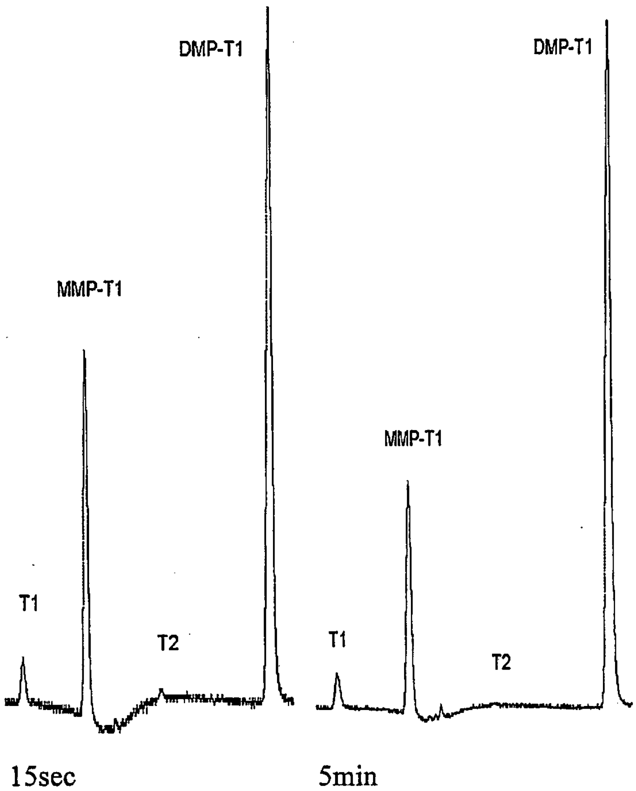


Figure 8C

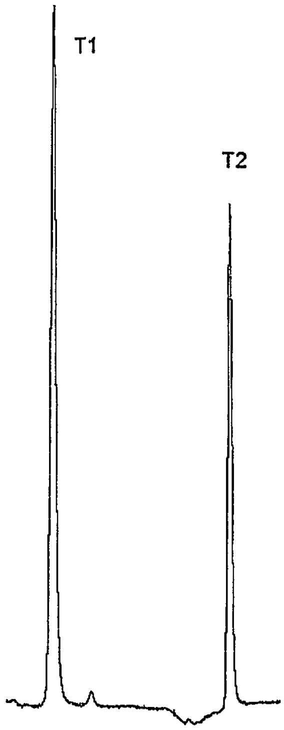


77.2%
99.1%
Figure 9A

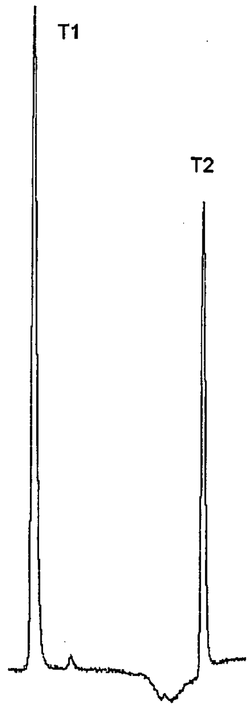


77.8%
99.2%
Figure 9B

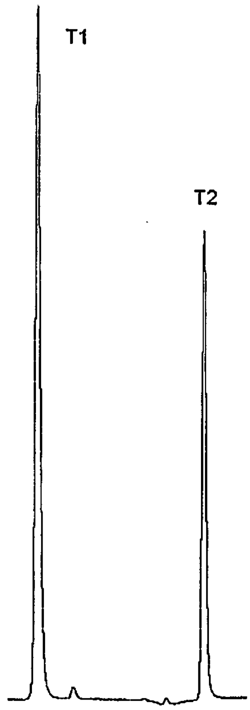


65.4% RCE (15 seconds)
97.1% RCE (1 minute)
Figure 9C

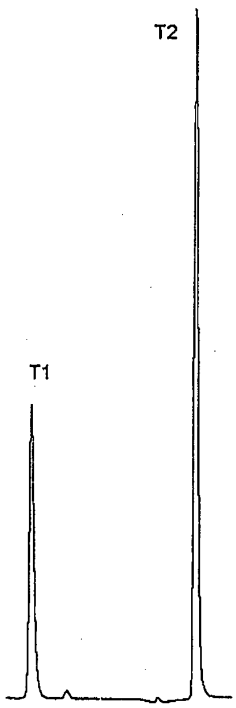


50.4%
93.8%
Figure 9D

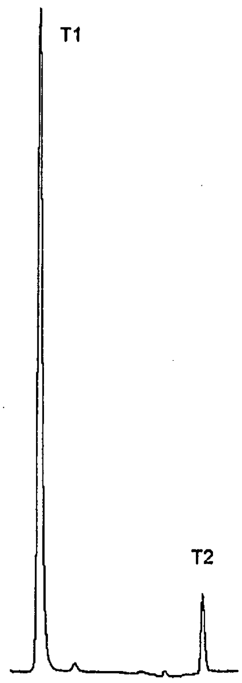


95.6%
100%
Figure 9E

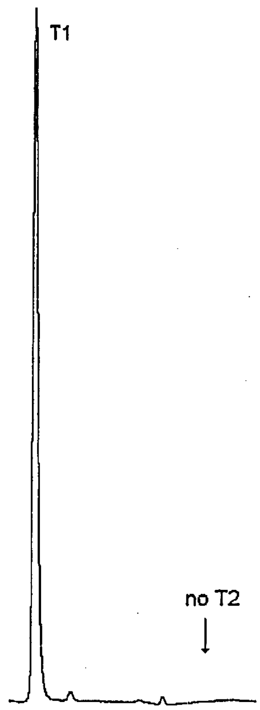


100% RCE (15 seconds)
100% RCE (1 minute)
Figure 9F

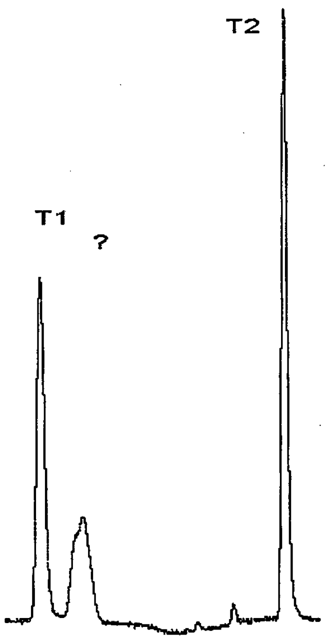

Figure 10A

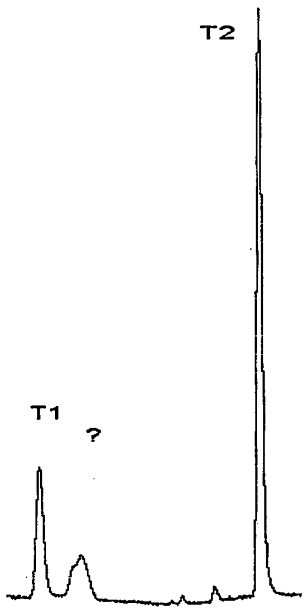

Figure 10B

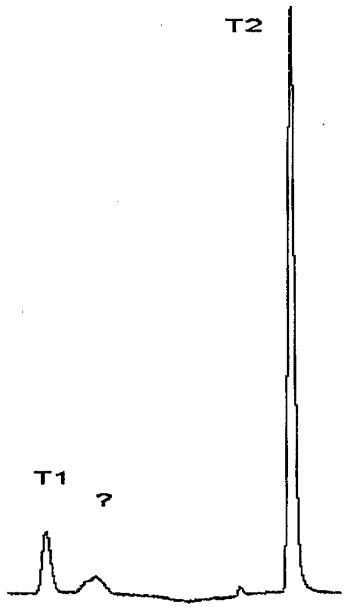

Figure 10C

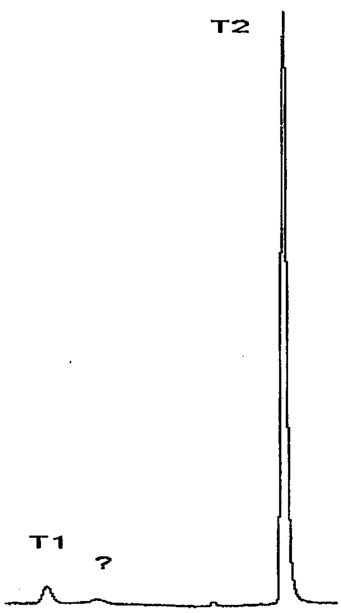

Figure 10D

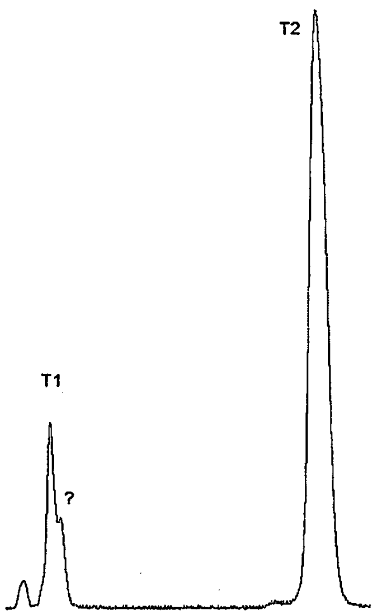

Figure 10E

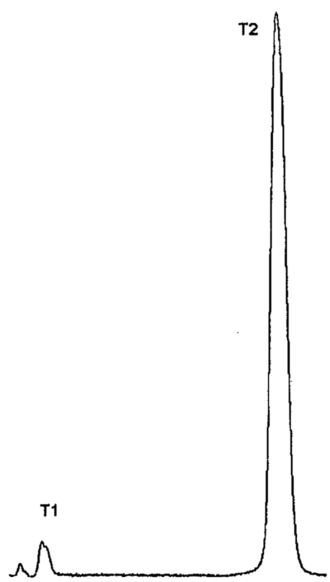

Figure 10F

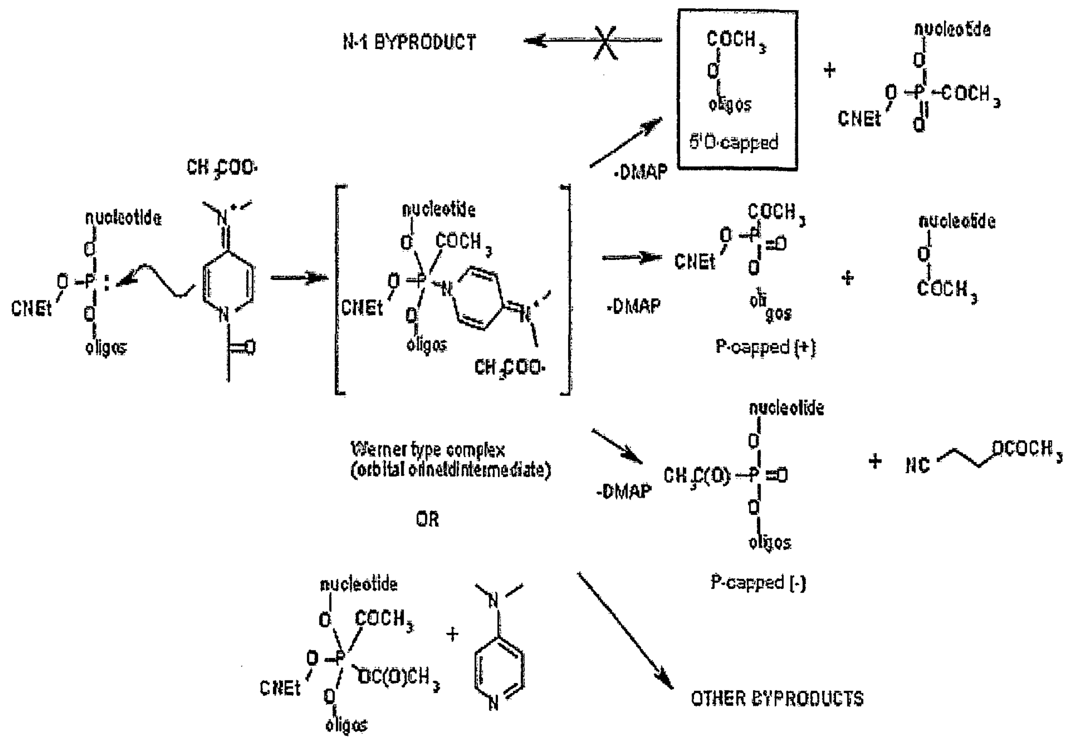


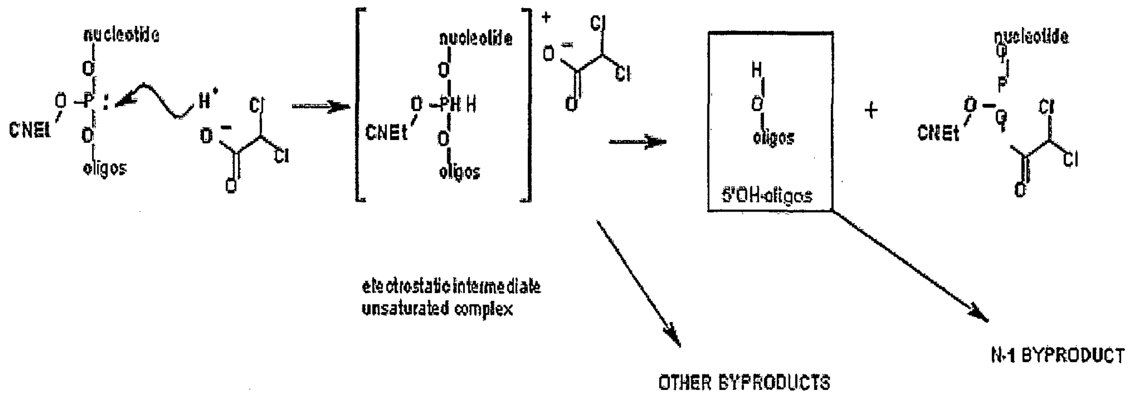


Figure 11
(Prior art)

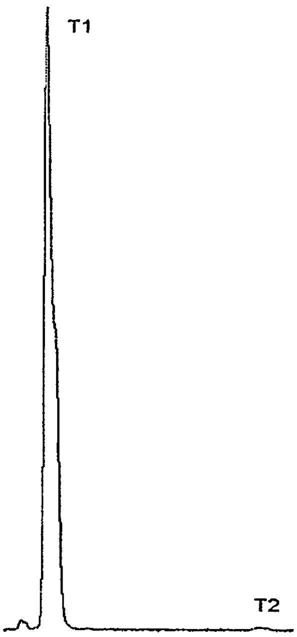

Figure 12A

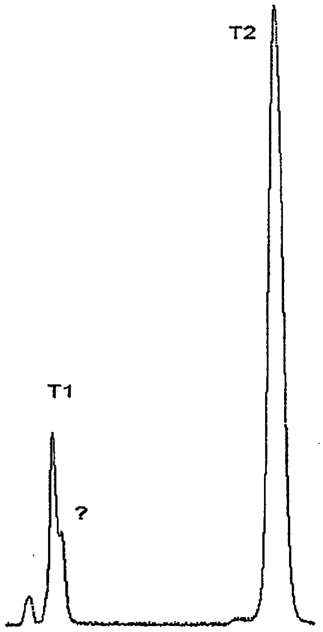

Figure 12B

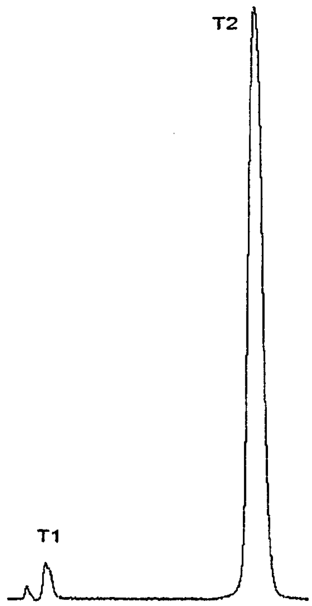

Figure 12C

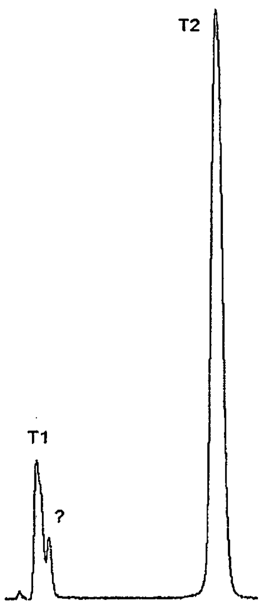

Figure 12D

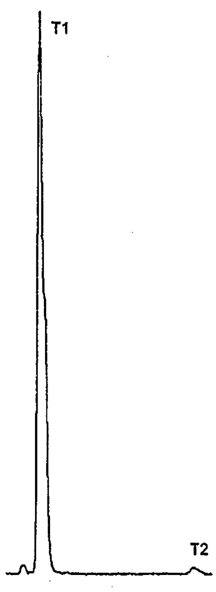

Figure 12E

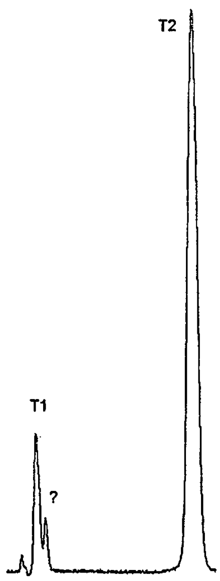

Figure 12F

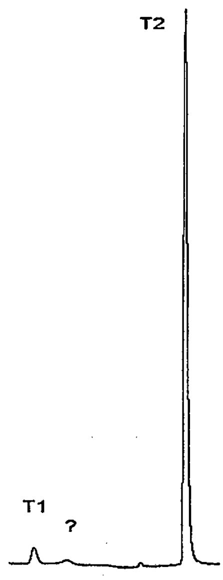

Figure 12G

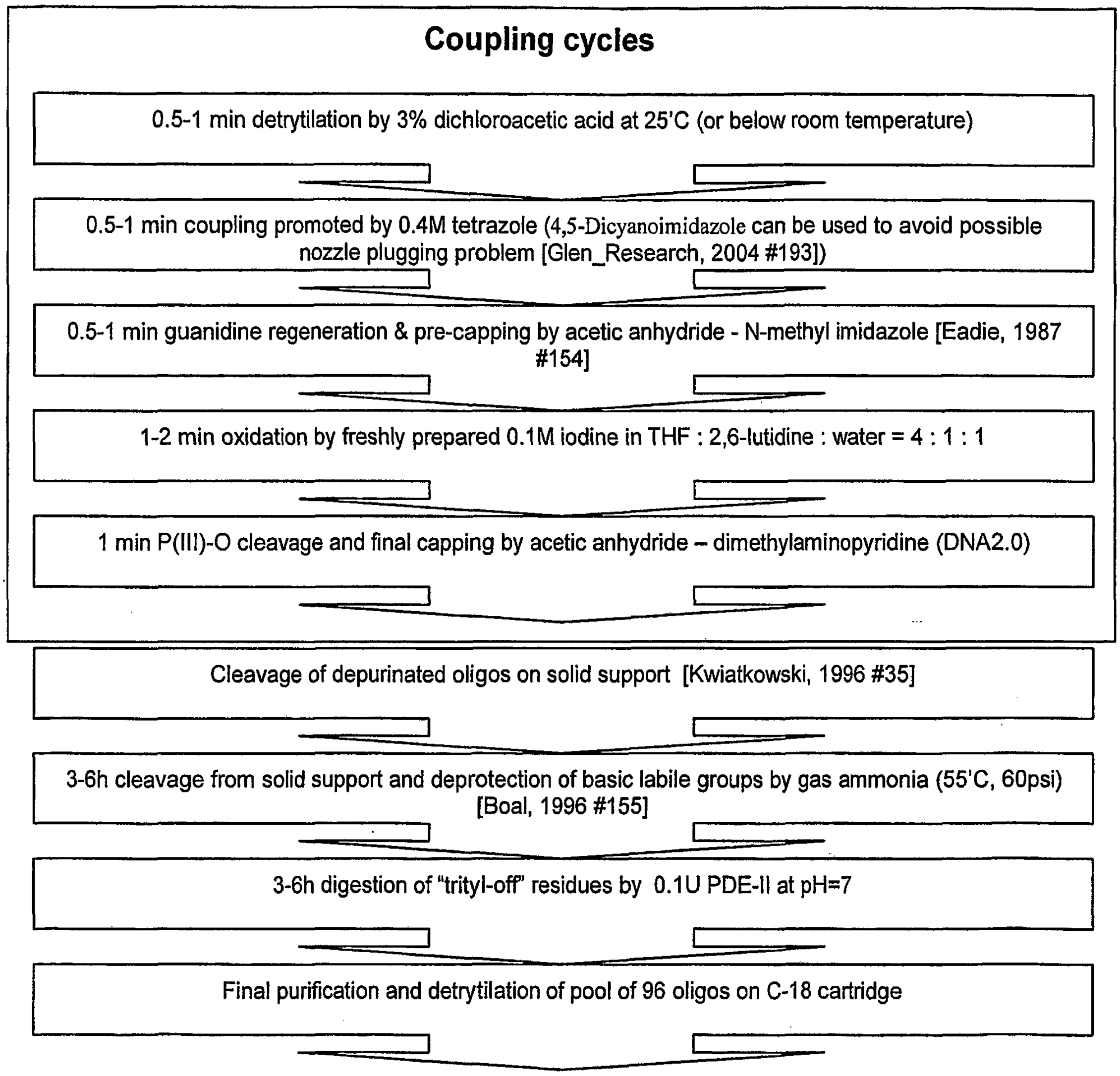
Coupling cycles
0.5-1 min detrytilation by 3% dichloroacetic acid at 25'C (or below room temperature)
0.5-1 min coupling promoted by 0.4M tetrazole (4,5-Dicyanoimidazole can be used to avoid possible nozzle plugging problem [Glen_Research, 2004 #193])
0.5-1 min guanidine regeneration & pre-capping by acetic anhydride - N-methyl imidazole [Eadie, 1987 #154]
1-2 min oxidation by freshly prepared 0.1M iodine in THF : 2,6-lutidine : water = 4 : 1 : 1
1 min P(III)-O cleavage and final capping by acetic anhydride – dimethylaminopyridine (DNA2.0)
Cleavage of depurinated oligos on solid support [Kwiatkowski, 1996 #35]
3-6h cleavage from solid support and deprotection of basic labile groups by gas ammonia (55'C, 60psi) [Boal, 1996 #155]
3-6h digestion of "trityl-off" residues by 0.1U PDE-II at pH=7
Final purification and detrytilation of pool of 96 oligos on C-18 cartridge

Figure 13

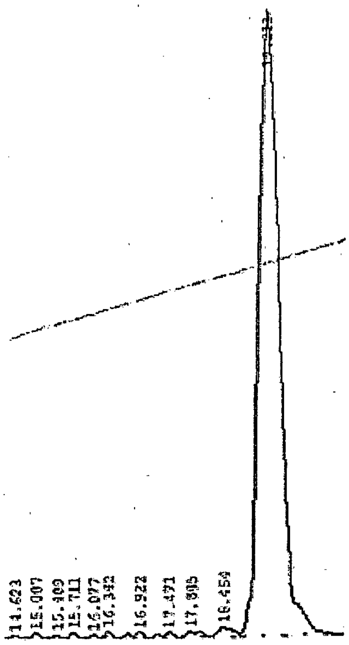


Figure 14A

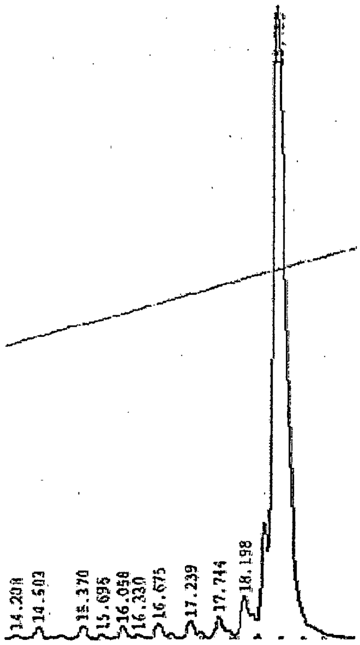


Figure 14B

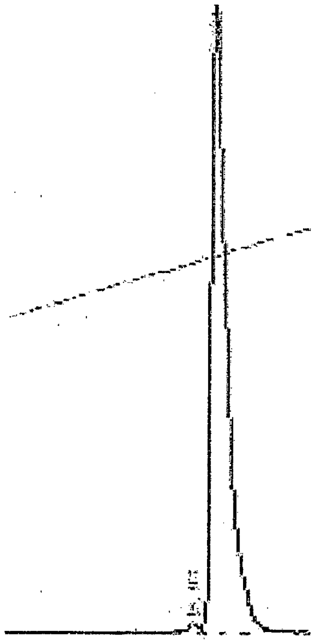

Figure 14C

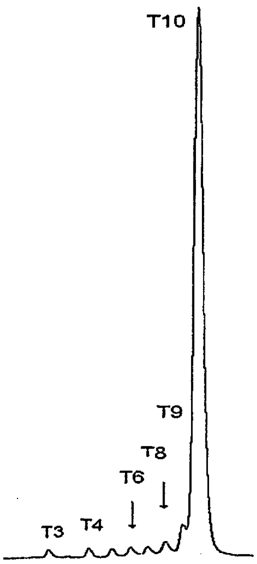


Figure 14D

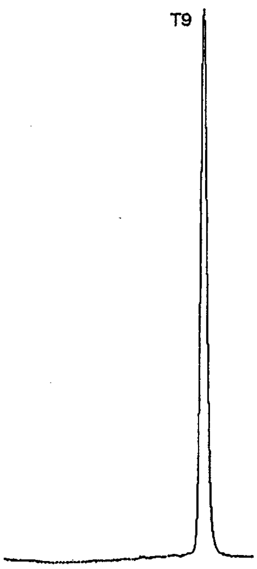


Figure 14E

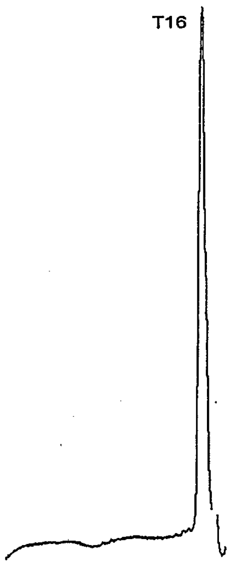


Figure 14F

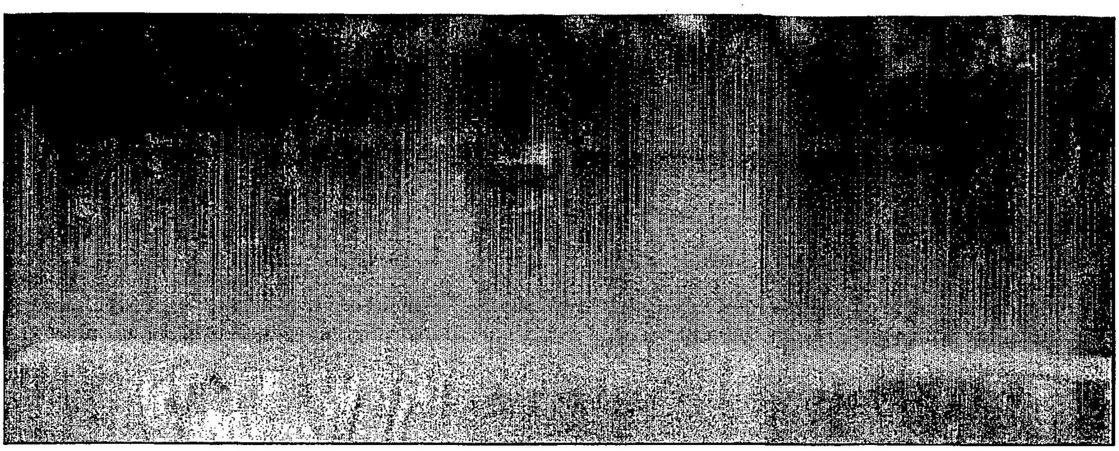

Figure 15A

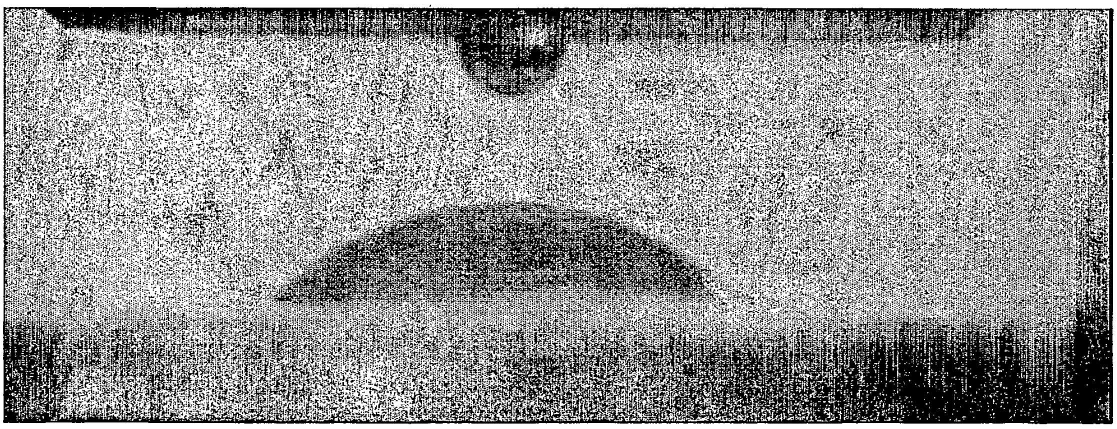

Figure 15B

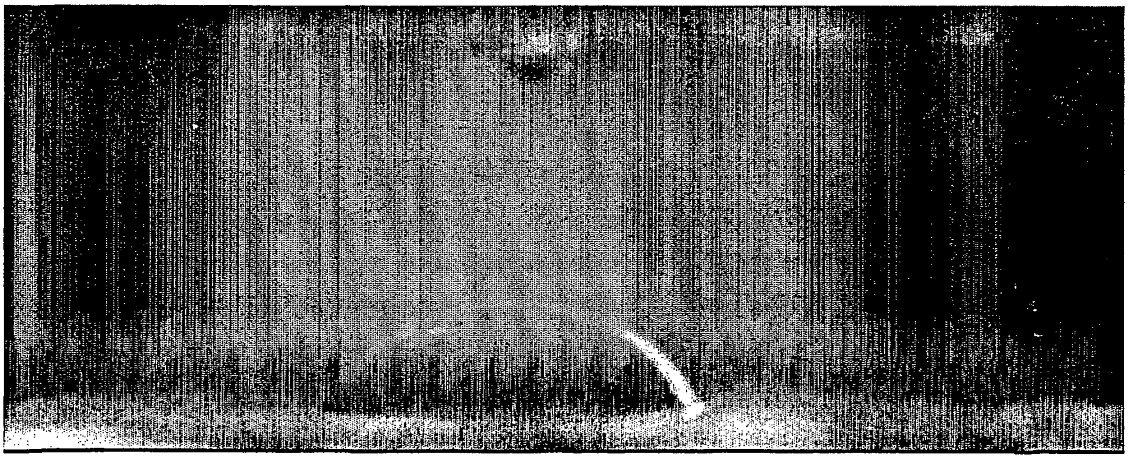

Figure 15C

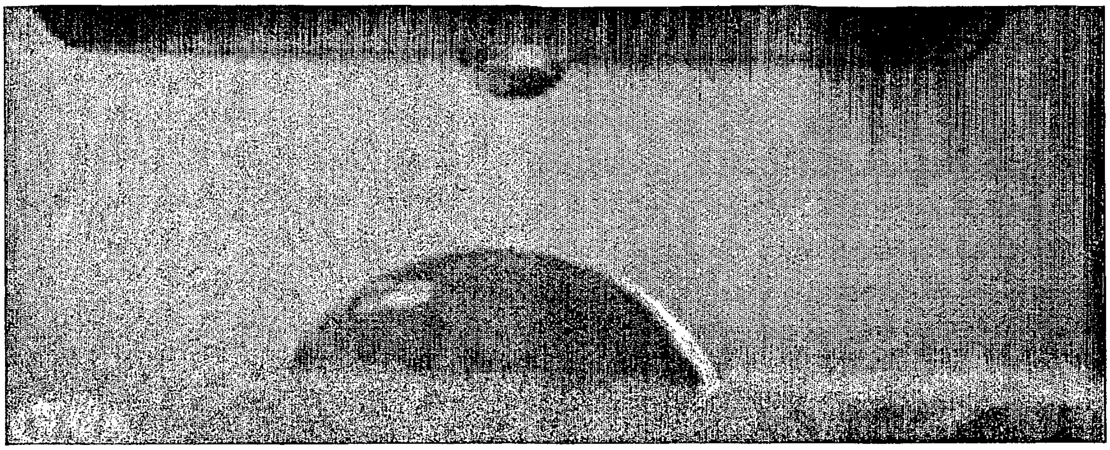

Figure 15D

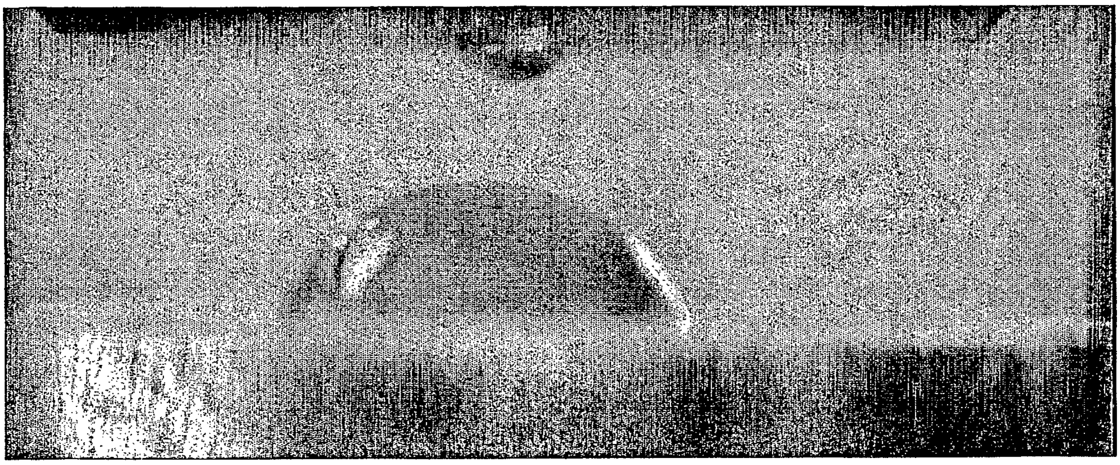

Figure 15E

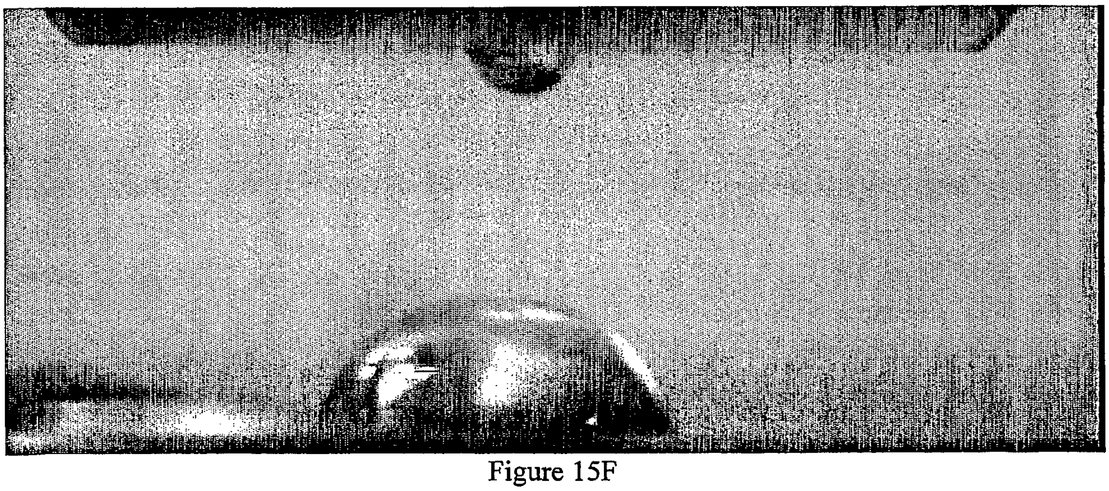

Figure 15F

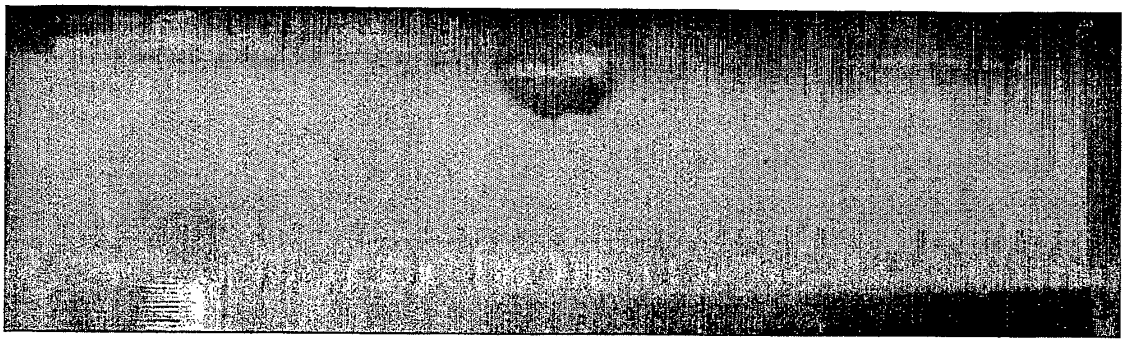

Figure 15G

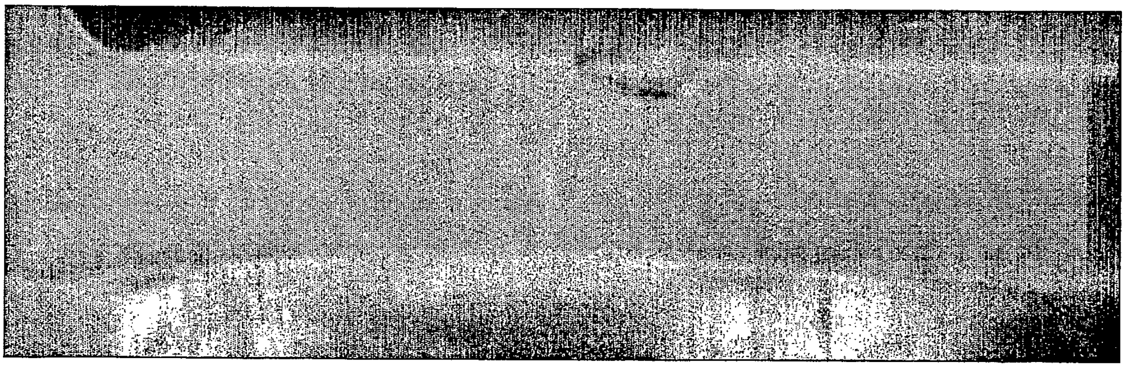

Figure 15H

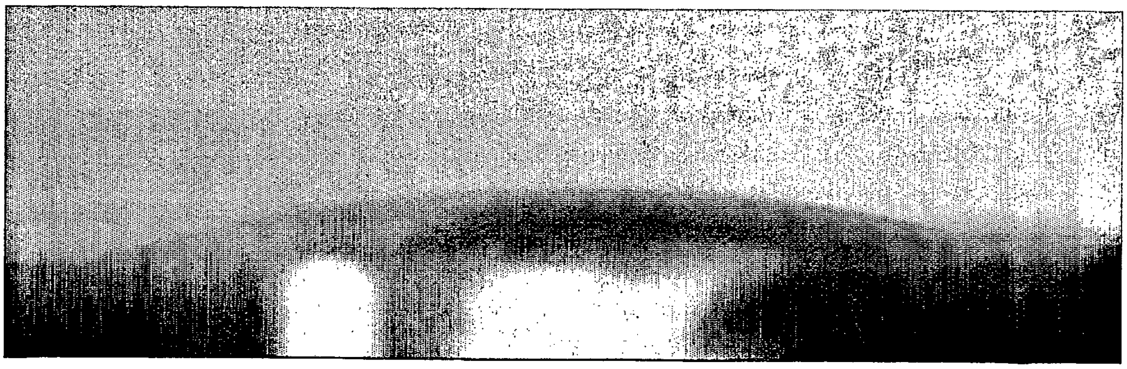

Figure 15I

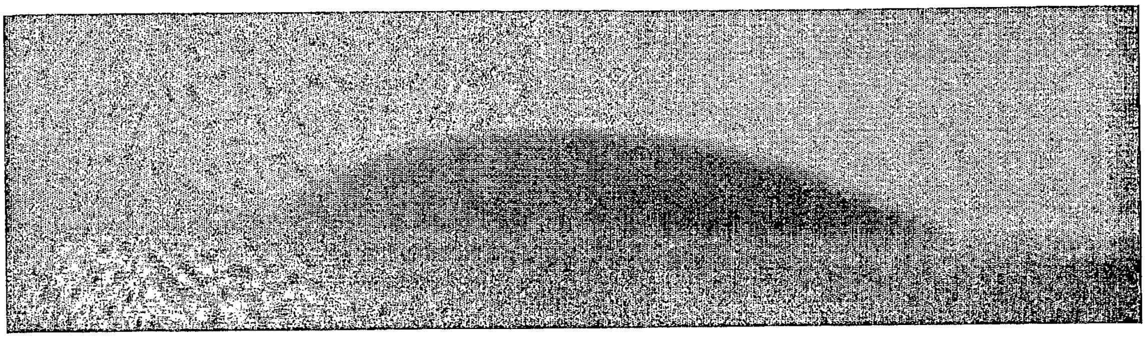

Figure 15J

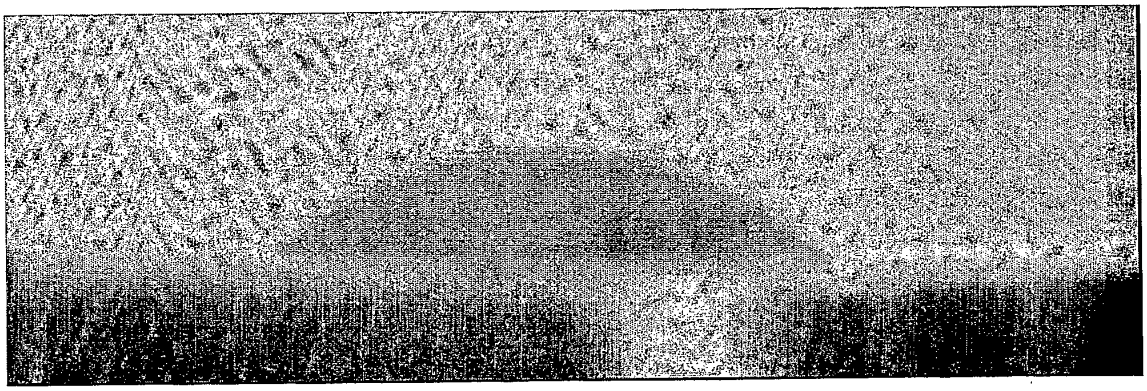

Figure 15K

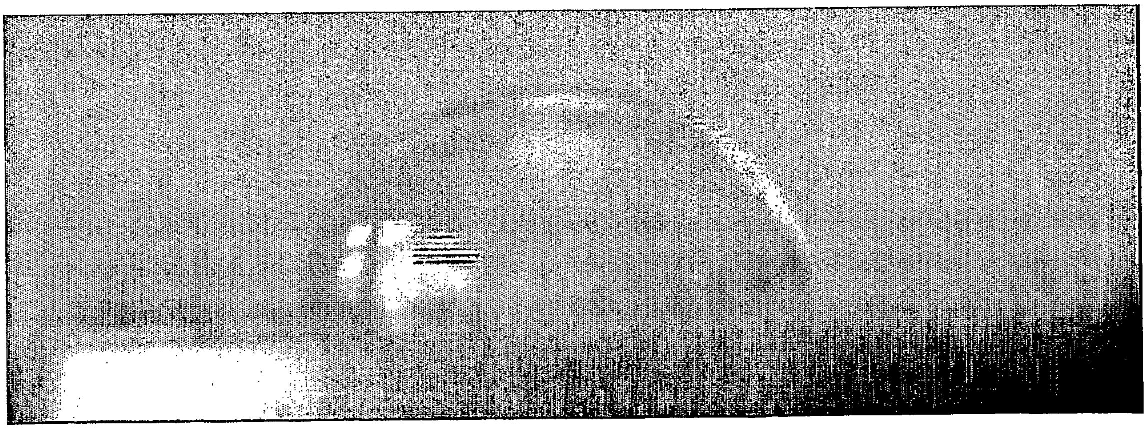

Figure 15L

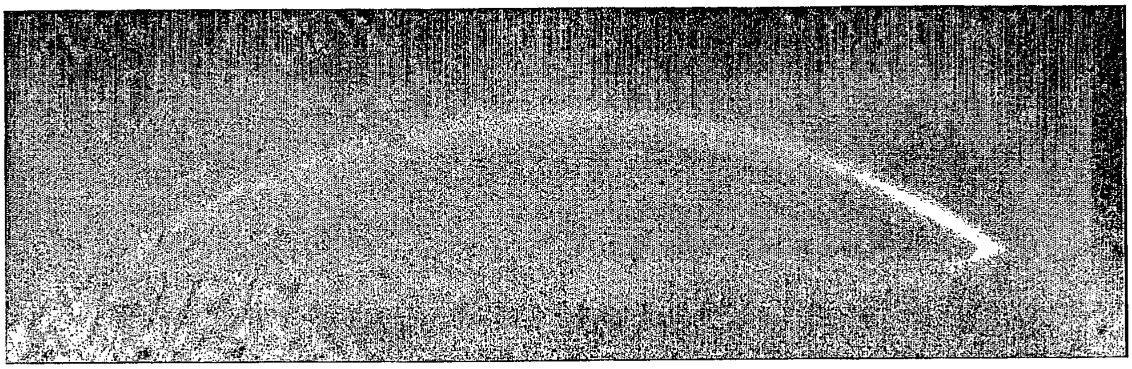

Figure 15M

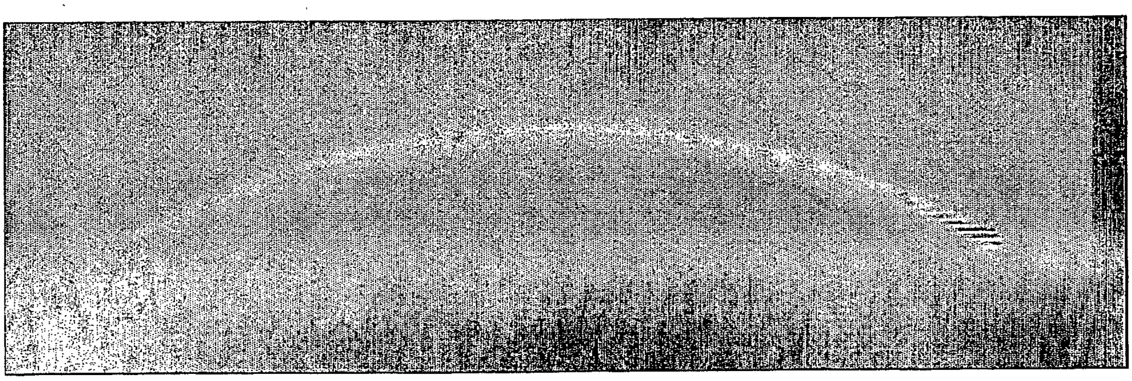

Figure 15N

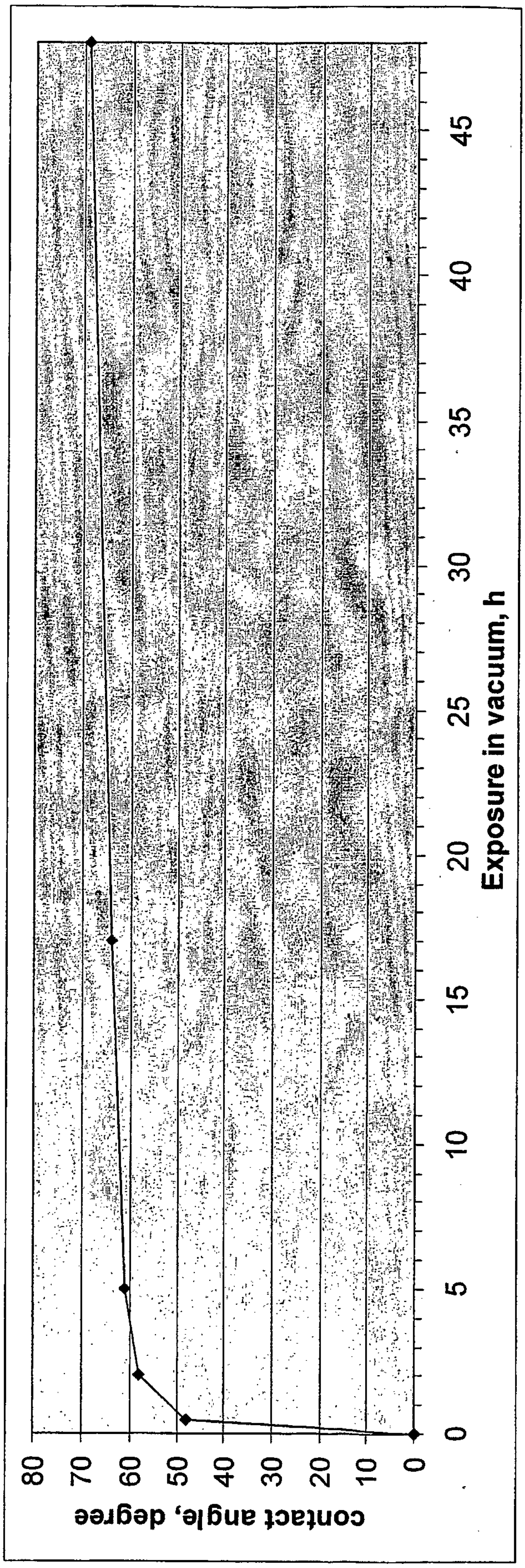
contact angle, degree
80
70
60
50
40
30
20
10
0
0
5
10
15
20
25
30
35
40
45
Exposure in vacuum, h

Figure 15O

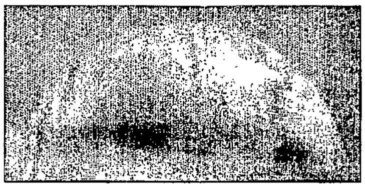

Figure 16A

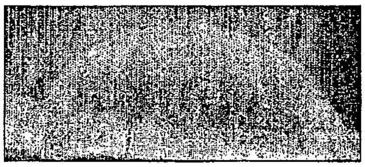

Figure 16B

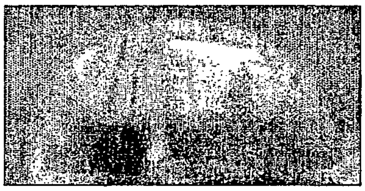

Figure 16C

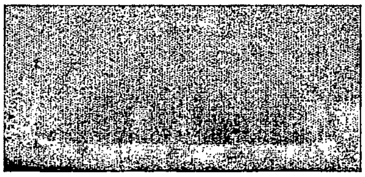

Figure 16D

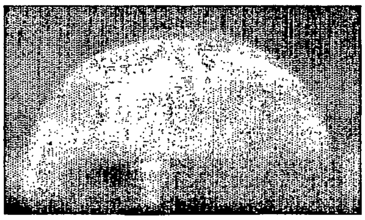

Figure 16E

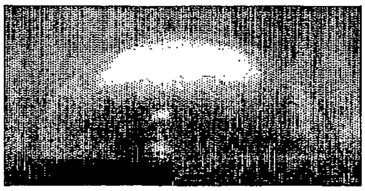

Figure 16F

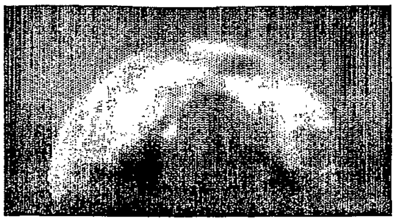

Figure 16G

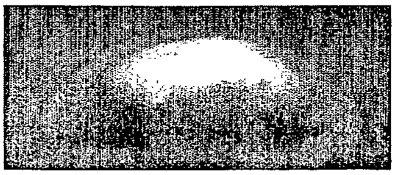

Figure 16H

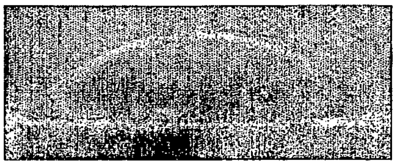

Figure 16I

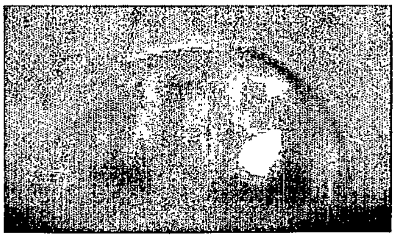

Figure 16J

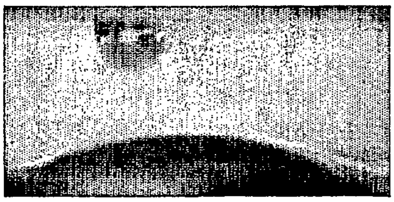

Figure 16K

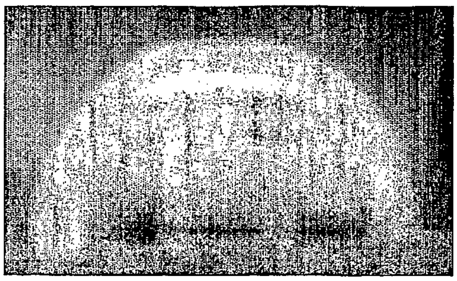

Figure 16L

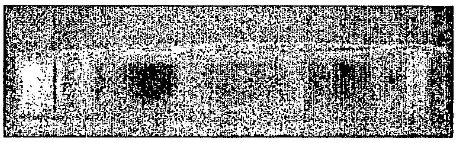

Figure 16M

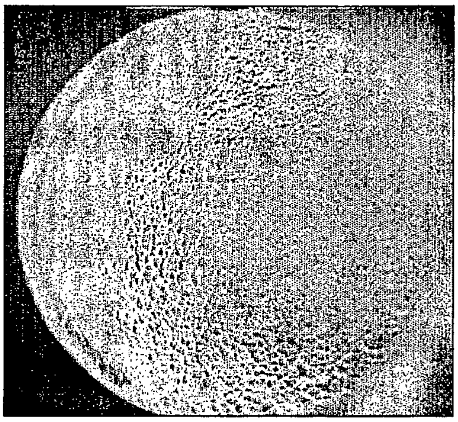

Figure 16N

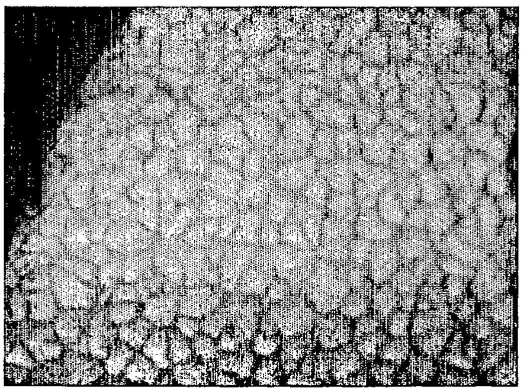

Figure 16O

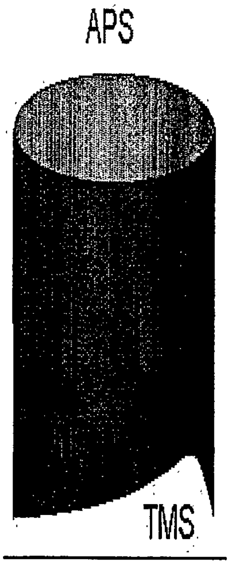


Figure 17A

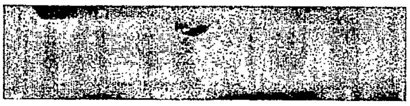

Figure 17B

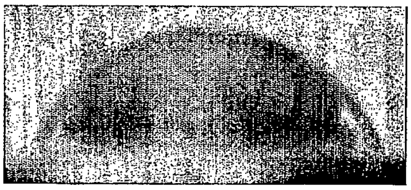

Figure 17C

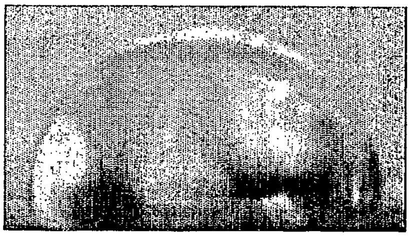

Figure 17D

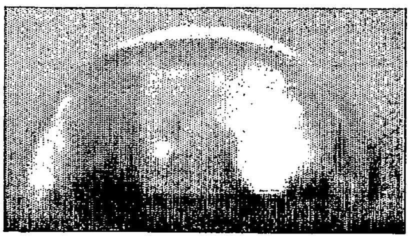

Figure 17E

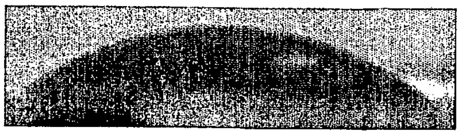

Figure 17F

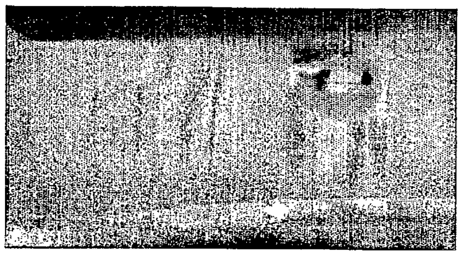

Figure 17G

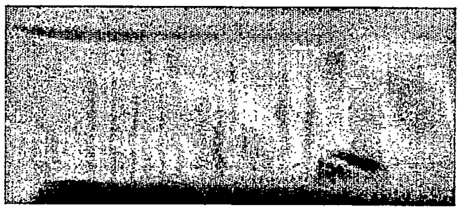

Figure 17H

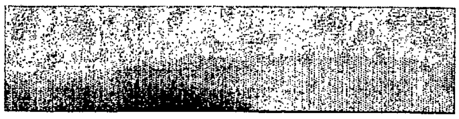

Figure 17I

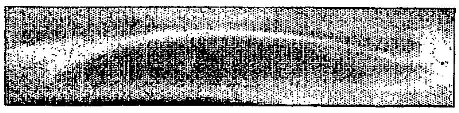

Figure 17J

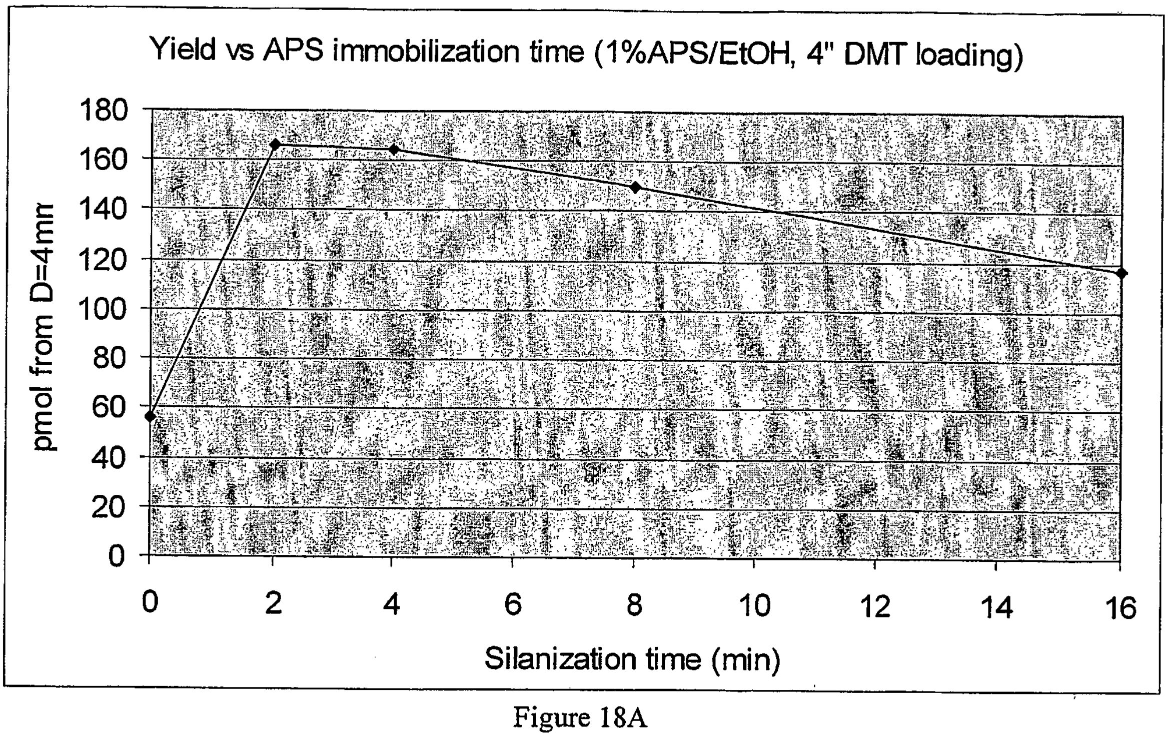


Figure 18A

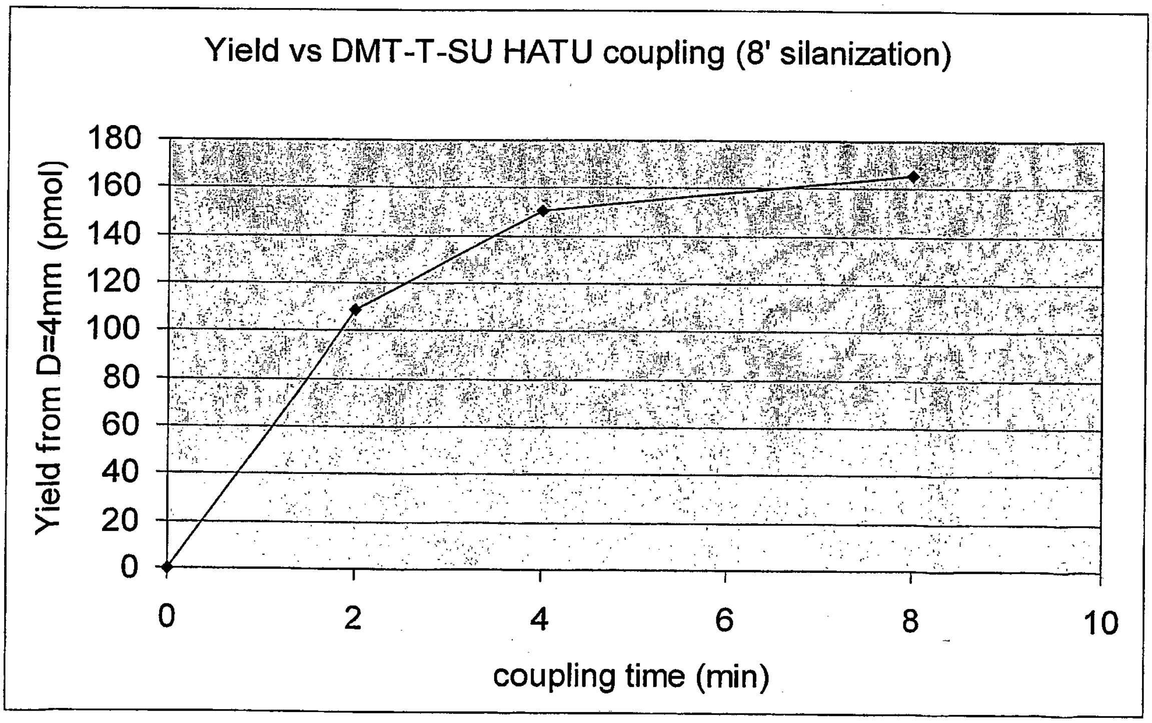


Figure 18B

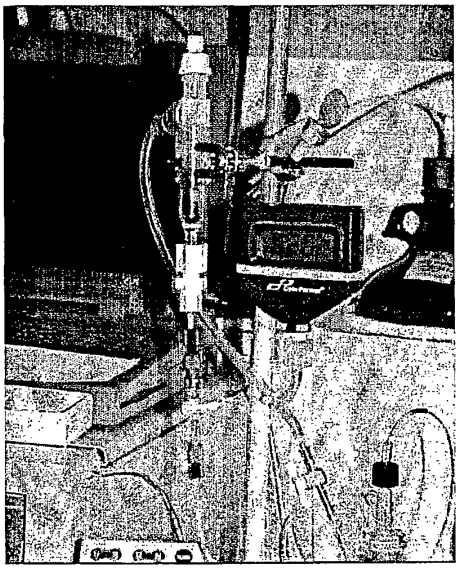

Figure 19A

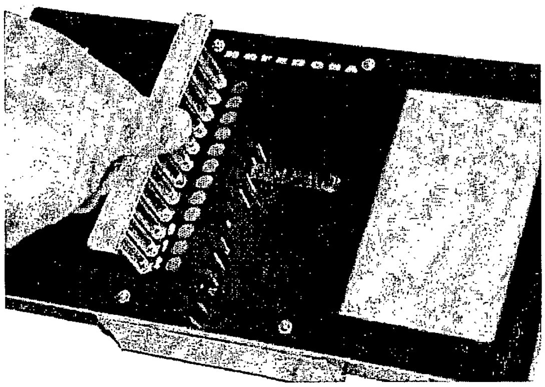

Figure 19B

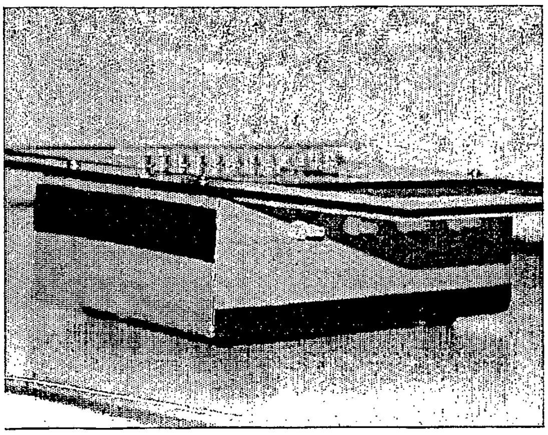

Figure 19C

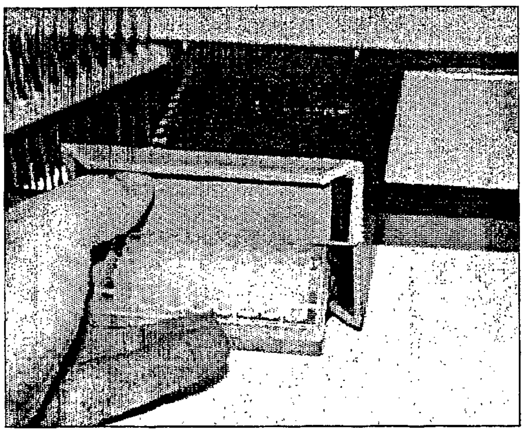

Figure 19D

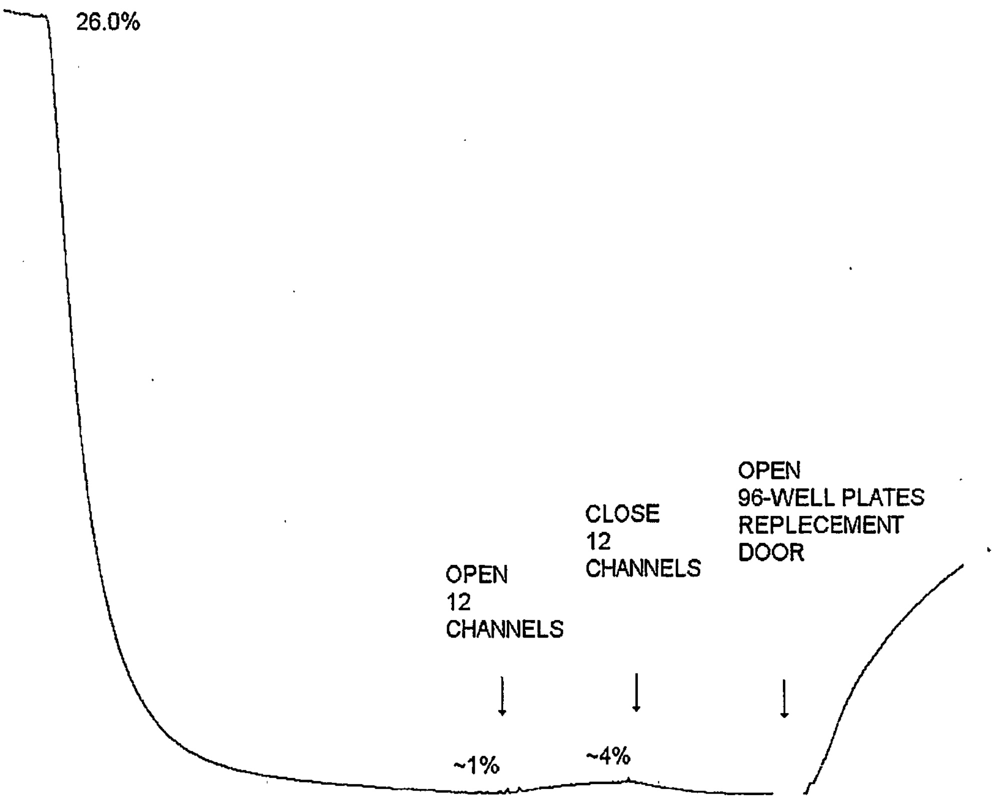


Figure 19E

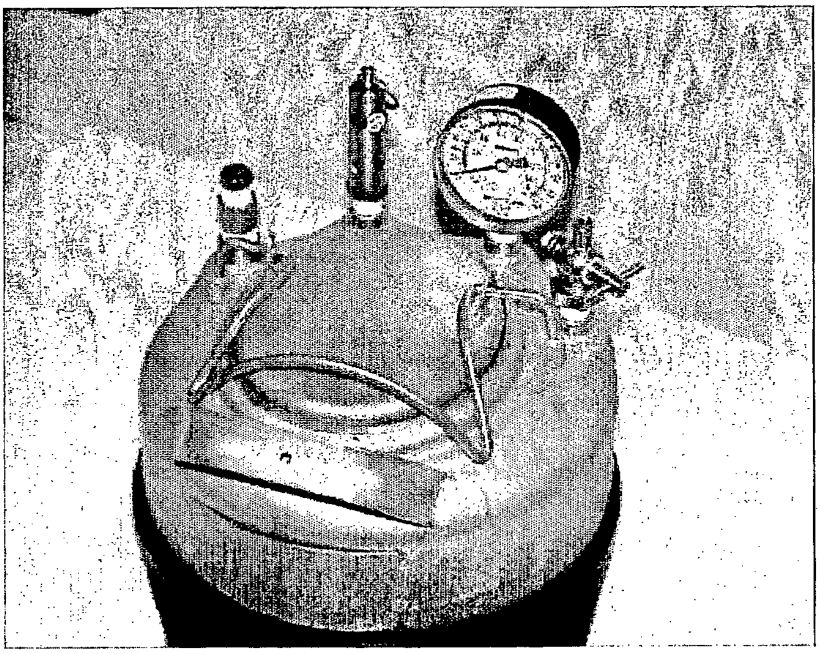

Figure 19F

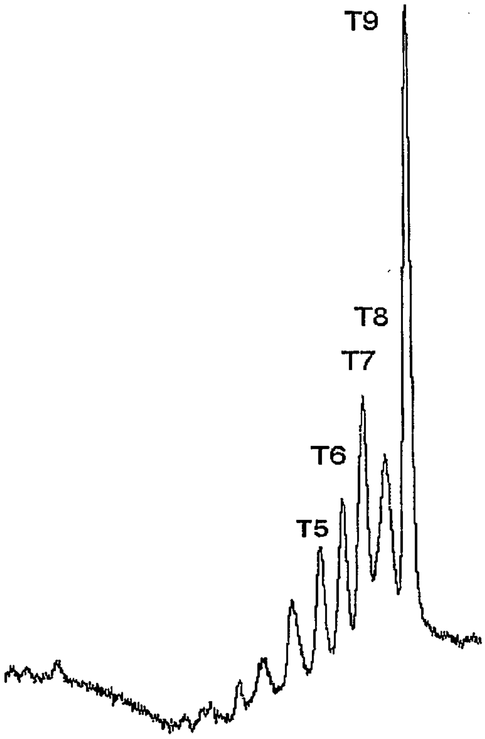


Figure 20A

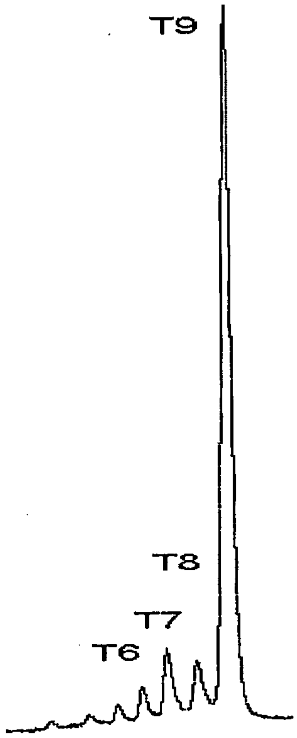


Figure 20B

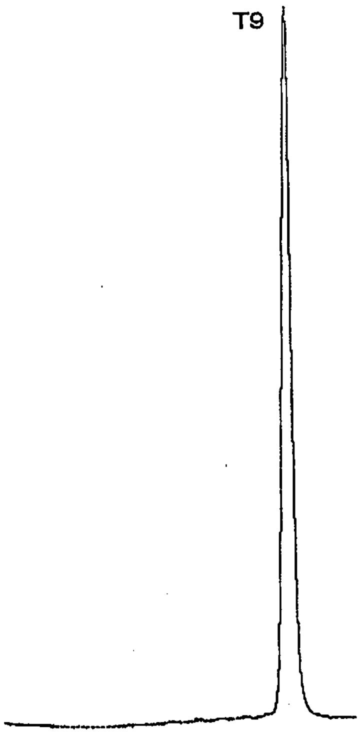


Figure 20C

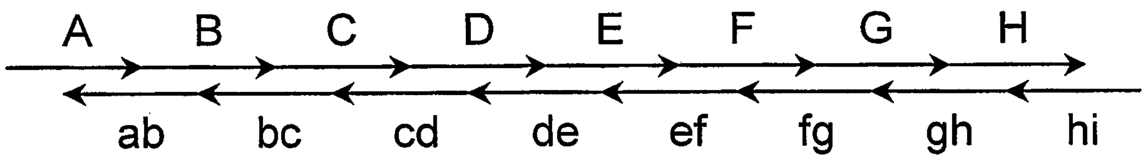


Figure 21

| Amino Acid | Codon | Frequency |
|---|---|---|
| Gly | GGG | 0.04 |
| Gly | GGA | 0.02 |
| Gly | GGT | 0.51 |
| Gly | GGC | 0.43 |
| Glu | GAG | 0.25 |
| Glu | GAA | 0.75 |
| Asp | GAT | 0.46 |
| Asp | GAC | 0.54 |
| Val | GTG | 0.27 |
| Val | GTA | 0.20 |
| Val | GTT | 0.40 |
| Val | GTC | 0.13 |
| Ala | GCG | 0.32 |
| Ala | GCA | 0.24 |
| Ala | GCT | 0.28 |
| Ala | GCC | 0.16 |
| Arg | AGG | 0.00 |
| Arg | AGA | 0.01 |
| Ser | AGT | 0.04 |
| Ser | AGC | 0.24 |
| Lys | AAG | 0.21 |
| Lys | AAA | 0.79 |
| Asn | AAT | 0.17 |
| Asn | AAC | 0.83 |
| Met | ATG | 1.00 |
| Ile | ATA | 0.01 |
| Ile | ATT | 0.33 |
| Ile | ATC | 0.66 |
| Thr | ACG | 0.13 |
| Thr | ACA | 0.04 |
| Thr | ACT | 0.29 |
| Thr | ACC | 0.54 |
| Trp | TGG | 1.00 |
| End | TGA | 0.29 |
| Cys | TGT | 0.39 |
| Cys | TGC | 0.61 |
| End | TAG | 0.08 |
| End | TAA | 0.63 |
| Tyr | TAT | 0.35 |
| Tyr | TAC | 0.65 |
| Leu | TTG | 0.05 |
| Leu | TTA | 0.03 |
| Phe | TTT | 0.29 |
| Phe | TTC | 0.71 |
| Ser | TCG | 0.07 |
| Ser | TCA | 0.05 |
| Ser | TCT | 0.33 |
| Ser | TCC | 0.27 |
| Arg | CGG | 0.01 |
| Arg | CGA | 0.01 |
| Arg | CGT | 0.64 |
| Arg | CGC | 0.33 |
| Gln | CAG | 0.81 |
| Gln | CAA | 0.19 |
| His | CAT | 0.30 |
| His | CAC | 0.70 |
| Leu | CTG | 0.77 |
| Leu | CTA | 0.01 |
| Leu | CTT | 0.06 |
| Leu | CTC | 0.08 |
| Pro | CCG | 0.72 |
| Pro | CCA | 0.15 |
| Pro | CCT | 0.11 |
| Pro | CCC | 0.02 |

# Figure 22

| Amino Acid | Codon | Frequency |
|---|---|---|
| Gly | GGG | 0.25 |
| Gly | GGA | 0.25 |
| Gly | GGT | 0.16 |
| Gly | GGC | 0.34 |
| Glu | GAG | 0.58 |
| Glu | GAA | 0.42 |
| Asp | GAT | 0.46 |
| Asp | GAC | 0.54 |
| Val | GTG | 0.46 |
| Val | GTA | 0.12 |
| Val | GTT | 0.18 |
| Val | GTC | 0.24 |
| Ala | GCG | 0.11 |
| Ala | GCA | 0.23 |
| Ala | GCT | 0.26 |
| Ala | GCC | 0.40 |
| Arg | AGG | 0.20 |
| Arg | AGA | 0.20 |
| Ser | AGT | 0.15 |
| Ser | AGC | 0.24 |
| Lys | AAG | 0.57 |
| Lys | AAA | 0.43 |
| Asn | AAT | 0.46 |
| Asn | AAC | 0.54 |
| Met | ATG | 1.00 |
| Ile | ATA | 0.17 |
| Ile | ATT | 0.35 |
| Ile | ATC | 0.48 |
| Thr | ACG | 0.11 |
| Thr | ACA | 0.28 |
| Thr | ACT | 0.24 |
| Thr | ACC | 0.36 |
| Trp | TGG | 1.00 |
| End | TGA | 0.54 |
| Cys | TGT | 0.45 |
| Cys | TGC | 0.55 |
| End | TAG | 0.20 |
| End | TAA | 0.26 |
| Tyr | TAT | 0.44 |
| Tyr | TAC | 0.56 |
| Leu | TTG | 0.13 |
| Leu | TTA | 0.07 |
| Phe | TTT | 0.45 |
| Phe | TTC | 0.55 |
| Ser | TCG | 0.06 |
| Ser | TCA | 0.15 |
| Ser | TCT | 0.18 |
| Ser | TCC | 0.22 |
| Arg | CGG | 0.21 |
| Arg | CGA | 0.11 |
| Arg | CGT | 0.08 |
| Arg | CGC | 0.19 |
| Gln | CAG | 0.74 |
| Gln | CAA | 0.26 |
| His | CAT | 0.41 |
| His | CAC | 0.59 |
| Leu | CTG | 0.40 |
| Leu | CTA | 0.08 |
| Leu | CTT | 0.13 |
| Leu | CTC | 0.20 |
| Pro | CCG | 0.11 |
| Pro | CCA | 0.27 |
| Pro | CCT | 0.28 |
| Pro | CCC | 0.33 |

Figure 23

| Codon | AmAcid | Frequency |
|---|---|---|
| AAA | Lys | 0.610000 |
| AAC | Asn | 0.685000 |
| AAG | Lys | 0.390000 |
| AAT | Asn | 0.315000 |
| ACA | Thr | 0.000000 |
| ACC | Thr | 0.540000 |
| ACG | Thr | 0.140000 |
| ACT | Thr | 0.320000 |
| AGA | Arg | 0.000000 |
| AGC | Ser | 0.270000 |
| AGG | Arg | 0.000000 |
| AGT | Ser | 0.000000 |
| ATA | Ile | 0.000000 |
| ATC | Ile | 0.630000 |
| ATG | Met | 1.000000 |
| ATT | Ile | 0.370000 |
| CAA | Gln | 0.225000 |
| CAC | His | 0.645000 |
| CAG | Gln | 0.775000 |
| CAT | His | 0.355000 |
| CCA | Pro | 0.260000 |
| CCC | Pro | 0.000000 |
| CCG | Pro | 0.510000 |
| CCT | Pro | 0.230000 |
| CGA | Arg | 0.000000 |
| CGC | Arg | 0.420000 |
| CGG | Arg | 0.000000 |
| CGT | Arg | 0.580000 |
| CTA | Leu | 0.000000 |
| CTC | Leu | 0.150000 |
| CTG | Leu | 0.640000 |
| CTT | Leu | 0.110000 |
| GAA | Glu | 0.585000 |
| GAC | Asp | 0.540000 |
| GAG | Glu | 0.415000 |
| GAT | Asp | 0.460000 |
| GCA | Ala | 0.235000 |
| GCC | Ala | 0.280000 |
| GCG | Ala | 0.215000 |
| GCT | Ala | 0.270000 |
| GGA | Gly | 0.000000 |
| GGC | Gly | 0.530000 |
| GGG | Gly | 0.000000 |
| GGT | Gly | 0.470000 |
| GTA | Val | 0.160000 |
| GTC | Val | 0.185000 |
| GTG | Val | 0.365000 |
| GTT | Val | 0.290000 |
| TAA | End | 0.445000 |
| TAC | Tyr | 0.605000 |
| TAG | End | 0.140000 |
| TAT | Tyr | 0.395000 |
| TCA | Ser | 0.110000 |
| TCC | Ser | 0.270000 |
| TCG | Ser | 0.070000 |
| TCT | Ser | 0.280000 |
| TGA | End | 0.415000 |
| TGC | Cys | 0.580000 |
| TGG | Trp | 1.000000 |
| TGT | Cys | 0.420000 |
| TTA | Leu | 0.000000 |
| TTC | Phe | 0.630000 |
| TTG | Leu | 0.100000 |
| TTT | Phe | 0.370000 |

Figure 24

| Amino Acid | Codon | Frequency |
|---|---|---|
| Gly | GGG | 0.23 |
| Gly | GGA | 0.26 |
| Gly | GGT | 0.18 |
| Gly | GGC | 0.33 |
| Glu | GAG | 0.60 |
| Glu | GAA | 0.40 |
| Asp | GAT | 0.44 |
| Asp | GAC | 0.56 |
| Val | GTG | 0.47 |
| Val | GTA | 0.12 |
| Val | GTT | 0.17 |
| Val | GTC | 0.25 |
| Ala | GCG | 0.10 |
| Ala | GCA | 0.23 |
| Ala | GCT | 0.29 |
| Ala | GCC | 0.38 |
| Arg | AGG | 0.21 |
| Arg | AGA | 0.21 |
| Ser | AGT | 0.15 |
| Ser | AGC | 0.24 |
| Lys | AAG | 0.61 |
| Lys | AAA | 0.39 |
| Asn | AAT | 0.43 |
| Asn | AAC | 0.57 |
| Met | ATG | 1.00 |
| Ile | ATA | 0.15 |
| Ile | ATT | 0.34 |
| Ile | ATC | 0.51 |
| Thr | ACG | 0.11 |
| Thr | ACA | 0.29 |
| Thr | ACT | 0.25 |
| Thr | ACC | 0.35 |
| Trp | TGG | 1.00 |
| End | TGA | 0.51 |
| Cys | TGT | 0.47 |
| Cys | TGC | 0.53 |
| End | TAG | 0.23 |
| End | TAA | 0.26 |
| Tyr | TAT | 0.42 |
| Tyr | TAC | 0.58 |
| Leu | TTG | 0.13 |
| Leu | TTA | 0.06 |
| Phe | TTT | 0.43 |
| Phe | TTC | 0.57 |
| Ser | TCG | 0.05 |
| Ser | TCA | 0.14 |
| Ser | TCT | 0.19 |
| Ser | TCC | 0.22 |
| Arg | CGG | 0.19 |
| Arg | CGA | 0.12 |
| Arg | CGT | 0.09 |
| Arg | CGC | 0.18 |
| Gln | CAG | 0.75 |
| Gln | CAA | 0.25 |
| His | CAT | 0.40 |
| His | CAC | 0.60 |
| Leu | CTG | 0.40 |
| Leu | CTA | 0.08 |
| Leu | CTT | 0.13 |
| Leu | CTC | 0.20 |
| Pro | CCG | 0.11 |
| Pro | CCA | 0.28 |
| Pro | CCT | 0.30 |
| Pro | CCC | 0.31 |

# Figure 25

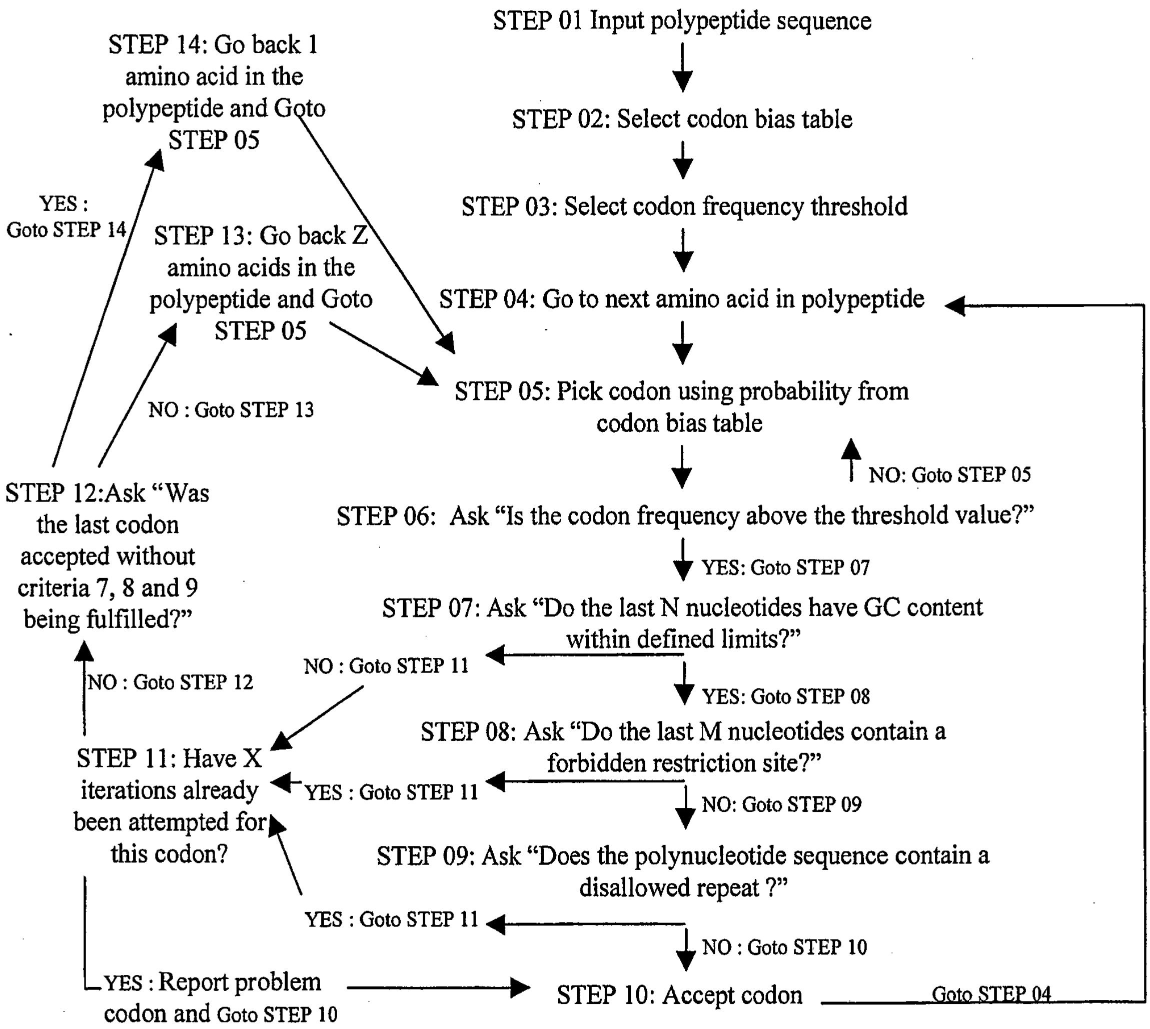
STEP 01 Input polypeptide sequence
STEP 02: Select codon bias table
STEP 03: Select codon frequency threshold
STEP 04: Go to next amino acid in polypeptide
STEP 05: Pick codon using probability from codon bias table
NO: Goto STEP 05
STEP 06: Ask "Is the codon frequency above the threshold value?"
YES: Goto STEP 07
STEP 07: Ask "Do the last N nucleotides have GC content within defined limits?"
NO : Goto STEP 11
YES: Goto STEP 08
STEP 08: Ask "Do the last M nucleotides contain a forbidden restriction site?"
YES : Goto STEP 11
NO: Goto STEP 09
STEP 09: Ask "Does the polynucleotide sequence contain a disallowed repeat ?"
YES : Goto STEP 11
NO : Goto STEP 10
STEP 10: Accept codon
Goto STEP 04
STEP 11: Have X iterations already been attempted for this codon?
YES : Report problem codon and Goto STEP 10
NO : Goto STEP 12
STEP 12:Ask "Was the last codon accepted without criteria 7, 8 and 9 being fulfilled?"
NO : Goto STEP 13
YES : Goto STEP 14
STEP 13: Go back Z amino acids in the polypeptide and Goto STEP 05
STEP 14: Go back 1 amino acid in the polypeptide and Goto STEP 05

Figure 26

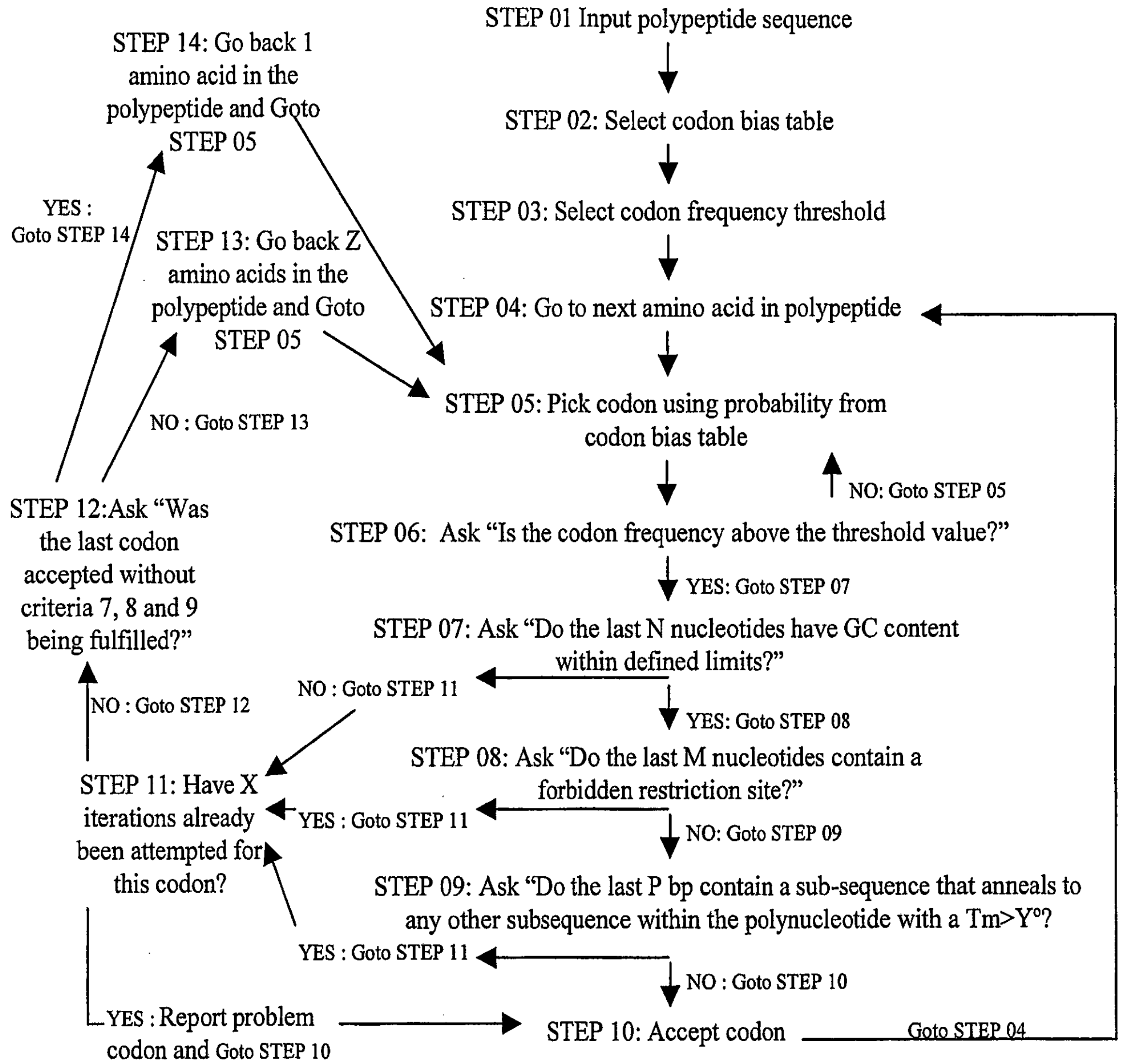
STEP 01 Input polypeptide sequence
STEP 02: Select codon bias table
STEP 03: Select codon frequency threshold
STEP 04: Go to next amino acid in polypeptide
STEP 05: Pick codon using probability from codon bias table
NO: Goto STEP 05
STEP 06: Ask "Is the codon frequency above the threshold value?"
YES: Goto STEP 07
STEP 07: Ask "Do the last N nucleotides have GC content within defined limits?"
YES: Goto STEP 08
STEP 08: Ask "Do the last M nucleotides contain a forbidden restriction site?"
NO: Goto STEP 09
STEP 09: Ask "Do the last P bp contain a sub-sequence that anneals to any other subsequence within the polynucleotide with a Tm>Y°?
NO : Goto STEP 10
STEP 10: Accept codon
Goto STEP 04
NO : Goto STEP 11
YES : Goto STEP 11
YES : Goto STEP 11
STEP 11: Have X iterations already been attempted for this codon?
YES : Report problem codon and Goto STEP 10
NO : Goto STEP 12
STEP 12:Ask "Was the last codon accepted without criteria 7, 8 and 9 being fulfilled?"
NO : Goto STEP 13
YES : Goto STEP 14
STEP 13: Go back Z amino acids in the polypeptide and Goto STEP 05
STEP 14: Go back 1 amino acid in the polypeptide and Goto STEP 05

Figure 27

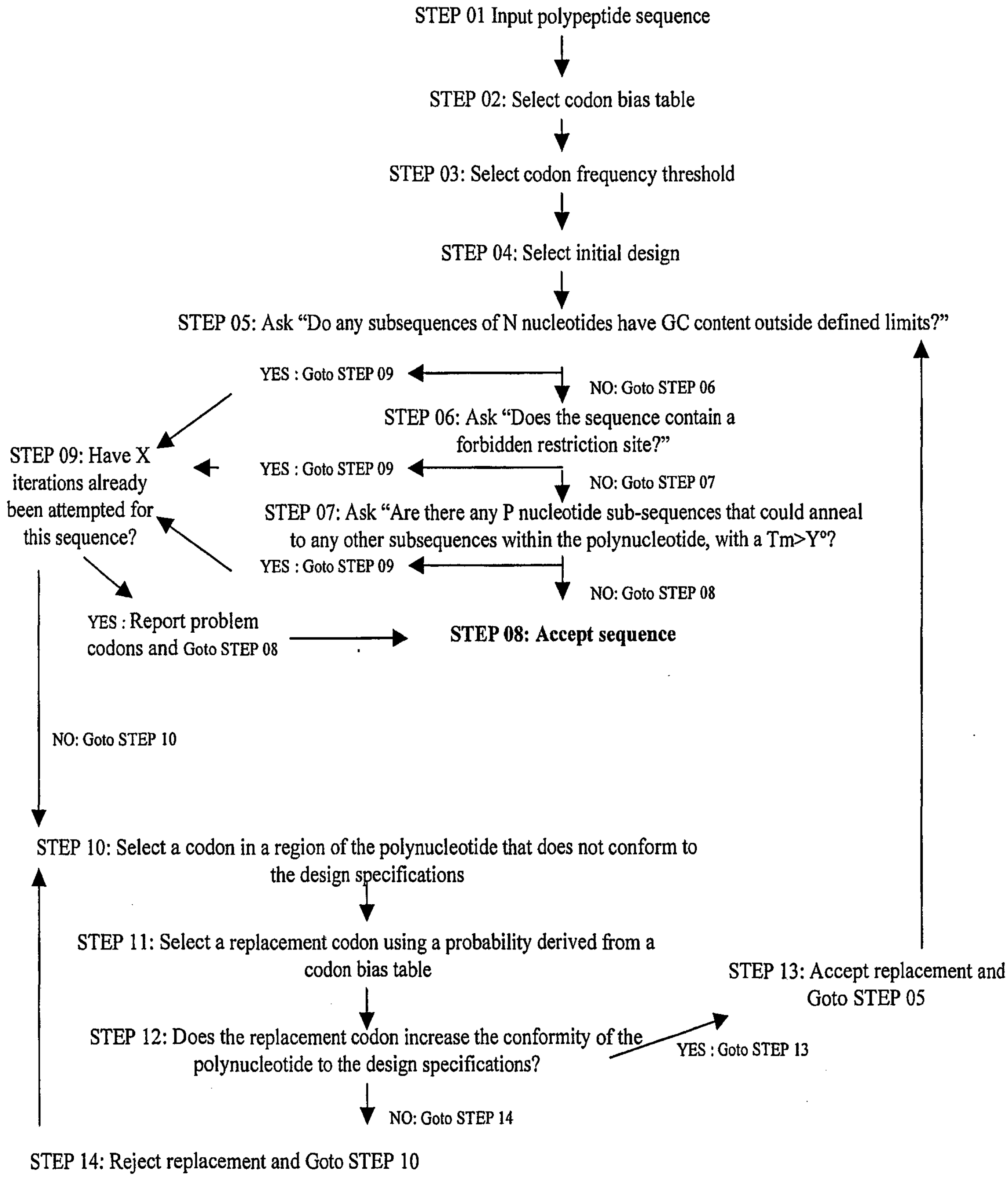
STEP 01 Input polypeptide sequence
STEP 02: Select codon bias table
STEP 03: Select codon frequency threshold
STEP 04: Select initial design
STEP 05: Ask "Do any subsequences of N nucleotides have GC content outside defined limits?"
YES : Goto STEP 09
NO: Goto STEP 06
STEP 06: Ask "Does the sequence contain a forbidden restriction site?"
YES : Goto STEP 09
NO: Goto STEP 07
STEP 07: Ask "Are there any P nucleotide sub-sequences that could anneal to any other subsequences within the polynucleotide, with a Tm>Y°?
YES : Goto STEP 09
NO: Goto STEP 08
STEP 09: Have X iterations already been attempted for this sequence?
YES : Report problem codons and Goto STEP 08
STEP 08: Accept sequence
NO: Goto STEP 10
STEP 10: Select a codon in a region of the polynucleotide that does not conform to the design specifications
STEP 11: Select a replacement codon using a probability derived from a codon bias table
STEP 12: Does the replacement codon increase the conformity of the polynucleotide to the design specifications?
YES : Goto STEP 13
STEP 13: Accept replacement and Goto STEP 05
NO: Goto STEP 14
STEP 14: Reject replacement and Goto STEP 10

Figure 28

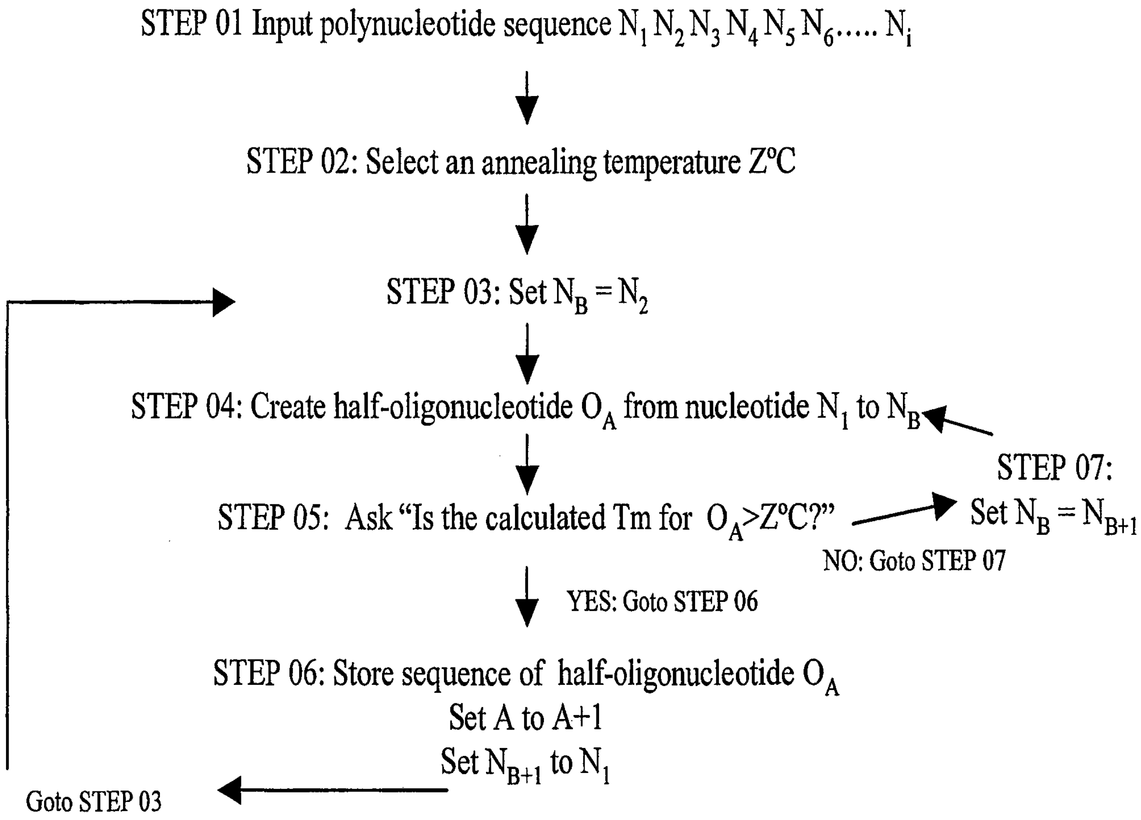
STEP 01 Input polynucleotide sequence N1 N2 N3 N4 N5 N6..... Ni
STEP 02: Select an annealing temperature Z°C
STEP 03: Set NB = N2
STEP 04: Create half-oligonucleotide OA from nucleotide N1 to NB
STEP 05: Ask "Is the calculated Tm for OA>Z°C?"
NO: Goto STEP 07
STEP 07:
Set NB = NB+1
YES: Goto STEP 06
STEP 06: Store sequence of half-oligonucleotide OA
Set A to A+1
Set NB+1 to N1
Goto STEP 03

Figure 29

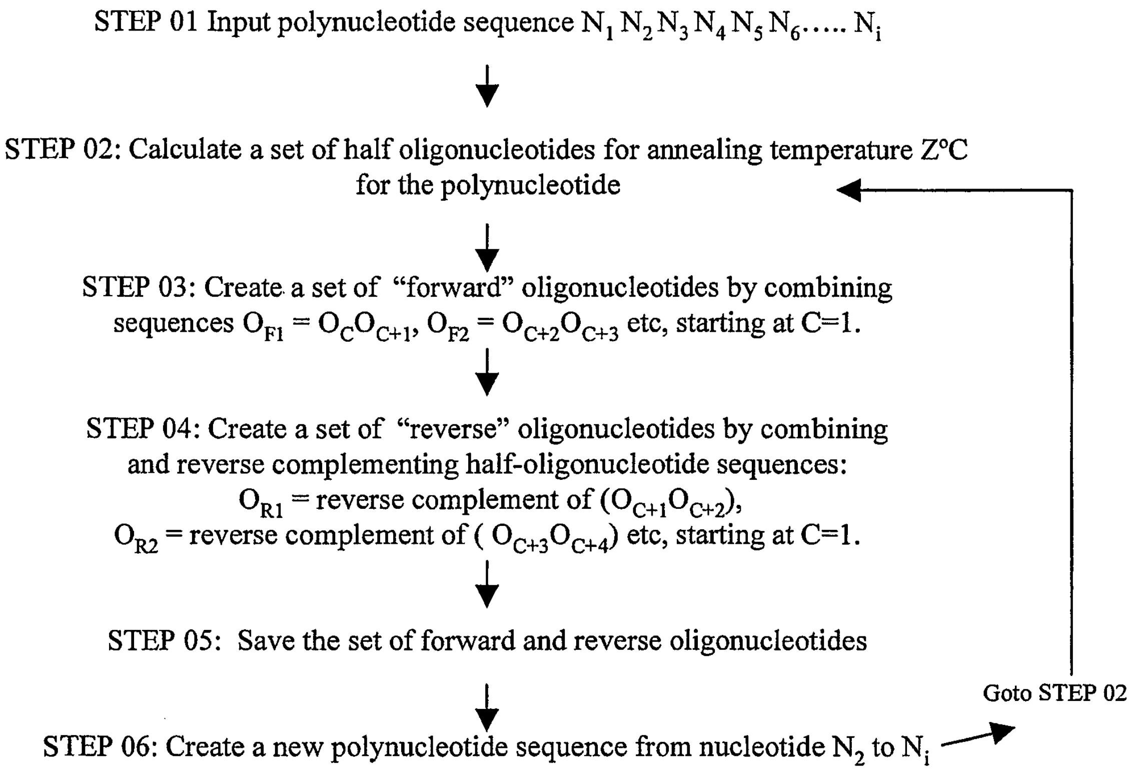
STEP 01 Input polynucleotide sequence N1 N2 N3 N4 N5 N6..... Ni
STEP 02: Calculate a set of half oligonucleotides for annealing temperature Z°C for the polynucleotide
STEP 03: Create a set of "forward" oligonucleotides by combining sequences OF1 = OCOC+1, OF2 = OC+2OC+3 etc, starting at C=1.
STEP 04: Create a set of "reverse" oligonucleotides by combining and reverse complementing half-oligonucleotide sequences:
OR1 = reverse complement of (OC+1OC+2),
OR2 = reverse complement of ( OC+3OC+4) etc, starting at C=1.
STEP 05: Save the set of forward and reverse oligonucleotides
Goto STEP 02
STEP 06: Create a new polynucleotide sequence from nucleotide N2 to Ni

Figure 30

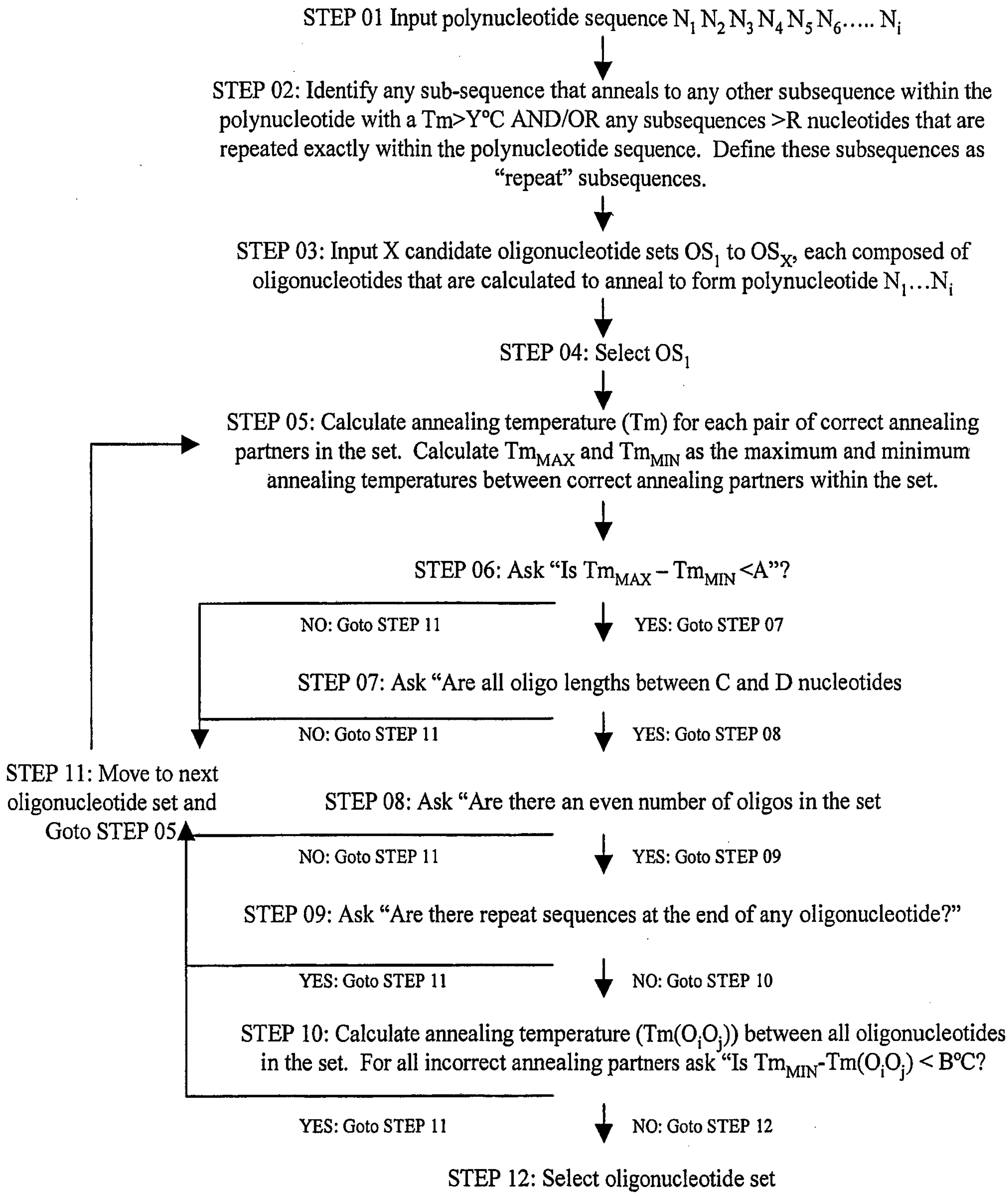
STEP 01 Input polynucleotide sequence $N_1 N_2 N_3 N_4 N_5 N_6 \ldots N_i$
STEP 02: Identify any sub-sequence that anneals to any other subsequence within the polynucleotide with a Tm>Y°C AND/OR any subsequences >R nucleotides that are repeated exactly within the polynucleotide sequence. Define these subsequences as "repeat" subsequences.
STEP 03: Input X candidate oligonucleotide sets $OS_1$ to $OS_X$, each composed of oligonucleotides that are calculated to anneal to form polynucleotide $N_1 \ldots N_i$
STEP 04: Select $OS_1$
STEP 05: Calculate annealing temperature (Tm) for each pair of correct annealing partners in the set. Calculate $Tm_{MAX}$ and $Tm_{MIN}$ as the maximum and minimum annealing temperatures between correct annealing partners within the set.
STEP 06: Ask "Is $Tm_{MAX} - Tm_{MIN} < A$"?
NO: Goto STEP 11
YES: Goto STEP 07
STEP 07: Ask "Are all oligo lengths between C and D nucleotides
NO: Goto STEP 11
YES: Goto STEP 08
STEP 11: Move to next oligonucleotide set and Goto STEP 05
STEP 08: Ask "Are there an even number of oligos in the set
NO: Goto STEP 11
YES: Goto STEP 09
STEP 09: Ask "Are there repeat sequences at the end of any oligonucleotide?"
YES: Goto STEP 11
NO: Goto STEP 10
STEP 10: Calculate annealing temperature ($Tm(O_iO_j)$) between all oligonucleotides in the set. For all incorrect annealing partners ask "Is $Tm_{MIN} - Tm(O_iO_j) < B°C$?
YES: Goto STEP 11
NO: Goto STEP 12
STEP 12: Select oligonucleotide set

Figure 31

| | | | |
|---|---|---|---|
| 1 | Denature | 96°C | 2min |
| 2 | Denature | 96°C | 30sec |
| 3 | Anneal | 40°C -72°C | 30sec |
| 4 | Extend | 72°C | 15sec |
| 5 | Goto | step 2 | 11 times |
| 6 | Denature | 96°C | 30sec |
| 7 | Anneal | 40°C -72°C | 30sec |
| 8 | Extend | 72°C | 30sec |
| 9 | Goto | step 6 | 11 times |
| 10 | Extend | 72°C | 1min |
| 11 | | 4°C | forever |
| 12 | End | | |

Figure 32

| | | | |
|---|---|---|---|
| 1 | Denature | 96°C | 2min |
| 2 | Denature | 96°C | 30sec |
| 3 | Anneal | 40°C -72°C | 30sec |
| 4 | Extend | 72°C | 15sec |
| 5 | Goto | step 2 | 7 times |
| 6 | Denature | 96°C | 30sec |
| 7 | Anneal | 40°C -72°C | 30sec |
| 8 | Extend | 72°C | 30sec |
| 9 | Goto | step 6 | 7 times |
| 10 | Denature | 96°C | 30sec |
| 11 | Anneal | 40°C -72°C | 30sec |
| 12 | Extend | 72°C | 45sec |
| 13 | Goto | step 10 | 7 times |
| 14 | Extend | 72°C | 1min |
| 15 | | 4°C | forever |
| 16 | End | | |

Figure 33

| | | | |
|---|---|---|---|
| 1 | Denature | 96°C | 2min |
| 2 | Denature | 96°C | 30sec |
| 3 | Anneal | 40°C -72°C | 30sec |
| 4 | Extend | 72°C | 15sec |
| 5 | Goto | step 2 | 7 times |
| 6 | Denature | 96°C | 30sec |
| 7 | Anneal | 40°C -72°C | 30sec |
| 8 | Extend | 72°C | 30sec |
| 9 | Goto | step 6 | 7 times |
| 10 | Denature | 96°C | 30sec |
| 11 | Anneal | 40°C -72°C | 30sec |
| 12 | Extend | 72°C | 45sec |
| 13 | Goto | step 10 | 7 times |
| 14 | Extend | 72°C | 1min |
| 15 | | 4°C | forever |
| 16 | End | | |

Figure 34

| | | | |
|---|---|---|---|
| 1 | Denature | 96°C | 2min |
| 2 | Denature | 96°C | 30sec |
| 3 | Anneal | 40°C -72°C | 30sec |
| 4 | Extend | 72°C | 15sec |
| 5 | Goto | step 2 | 3 times |
| 6 | Denature | 96°C | 30sec |
| 7 | Anneal | 40°C -72°C | 30sec |
| 8 | Extend | 72°C | 30sec |
| 9 | Goto | step 6 | 3 times |
| 10 | Denature | 96°C | 30sec |
| 11 | Anneal | 40°C -72°C | 30sec |
| 12 | Extend | 72°C | 45sec |
| 13 | Goto | step 10 | 3 times |
| 14 | Denature | 96°C | 30sec |
| 15 | Anneal | 40°C -72°C | 30sec |
| 16 | Extend | 72°C | 60sec |
| 17 | Goto | step 14 | 3 times |
| 18 | Denature | 96°C | 30sec |
| 19 | Anneal | 40°C -72°C | 30sec |
| 20 | Extend | 72°C | 75sec |
| 21 | Goto | step 18 | 3 times |
| 22 | Denature | 96°C | 30sec |
| 23 | Anneal | 40°C -72°C | 30sec |
| 24 | Extend | 72°C | 90sec |
| 25 | Goto | step 22 | 3 times |
| 26 | Extend | 72°C | 1min |
| 27 | | 4°C | forever |
| 28 | End | | |

# Figure 35

| | | | |
|---|---|---|---|
| 1 | Denature | 96°C | 2min |
| 2 | Denature | 96°C | 30sec |
| 3 | Anneal | 40°C -72°C | 30sec |
| 4 | Extend | 72°C | 15sec |
| 5 | Goto | step 2 | 2 times |
| 6 | Denature | 96°C | 30sec |
| 7 | Anneal | 40°C -72°C | 30sec |
| 8 | Extend | 72°C | 30sec |
| 9 | Goto | step 6 | 2 times |
| 10 | Denature | 96°C | 30sec |
| 11 | Anneal | 40°C -72°C | 30sec |
| 12 | Extend | 72°C | 45sec |
| 13 | Goto | step 10 | 2 times |
| 14 | Denature | 96°C | 30sec |
| 15 | Anneal | 40°C -72°C | 30sec |
| 16 | Extend | 72°C | 60sec |
| 17 | Goto | step 14 | 2 times |
| 18 | Denature | 96°C | 30sec |
| 19 | Anneal | 40°C -72°C | 30sec |
| 20 | Extend | 72°C | 75sec |
| 21 | Goto | step 18 | 2 times |
| 22 | Denature | 96°C | 30sec |
| 23 | Anneal | 40°C -72°C | 30sec |
| 24 | Extend | 72°C | 90sec |
| 25 | Goto | step 22 | 2 times |
| 26 | Denature | 96°C | 30sec |
| 27 | Anneal | 40°C -72°C | 30sec |
| 28 | Extend | 72°C | 105sec |
| 29 | Goto | step 26 | 2 times |
| 30 | Denature | 96°C | 30sec |
| 31 | Anneal | 40°C -72°C | 30sec |
| 32 | Extend | 72°C | 120sec |
| 33 | Goto | step 30 | 2 times |
| 34 | Extend | 72°C | 1min |
| 35 | | 4°C | forever |
| 36 | End | | |

Figure 36

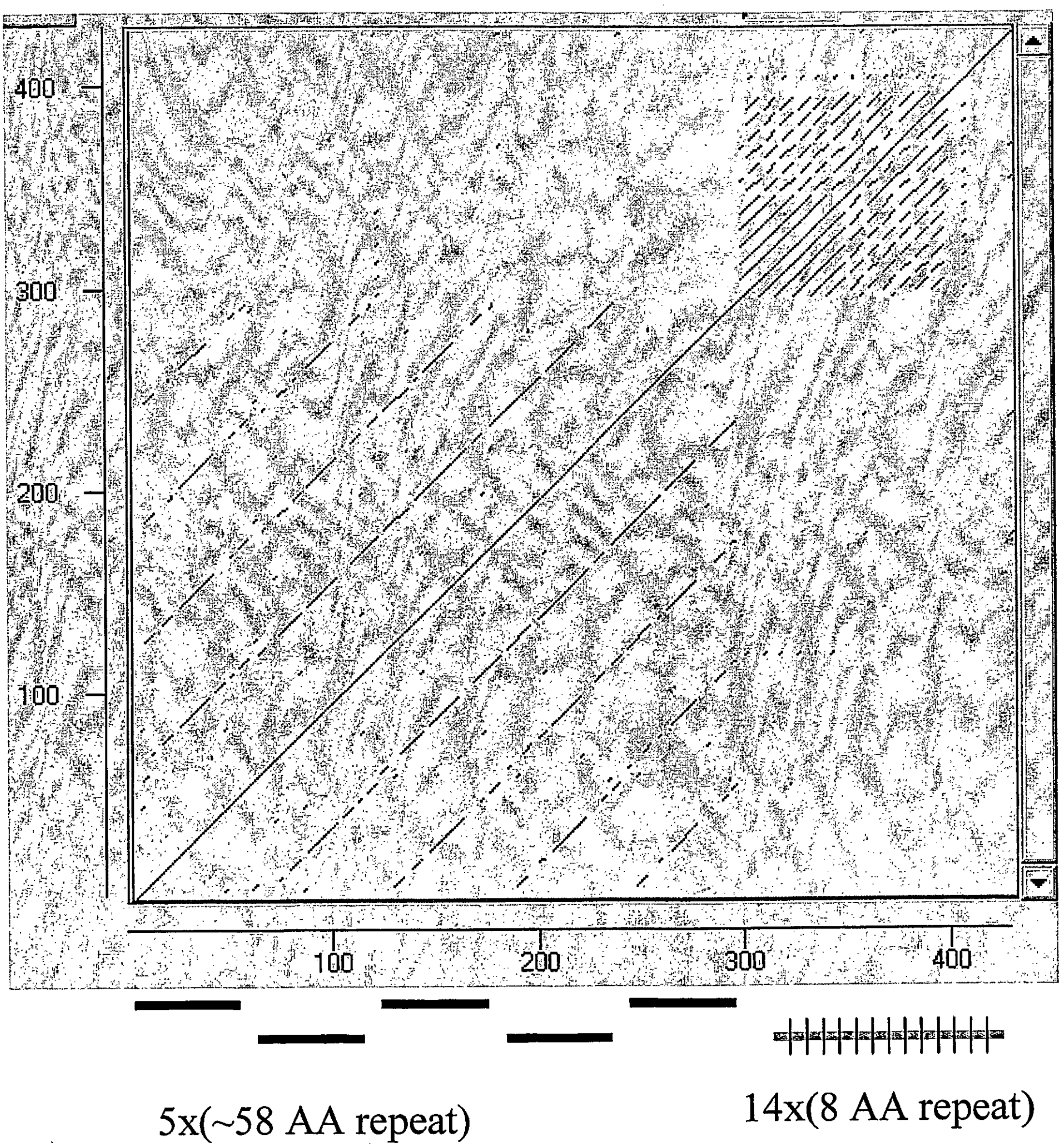


Figure 37

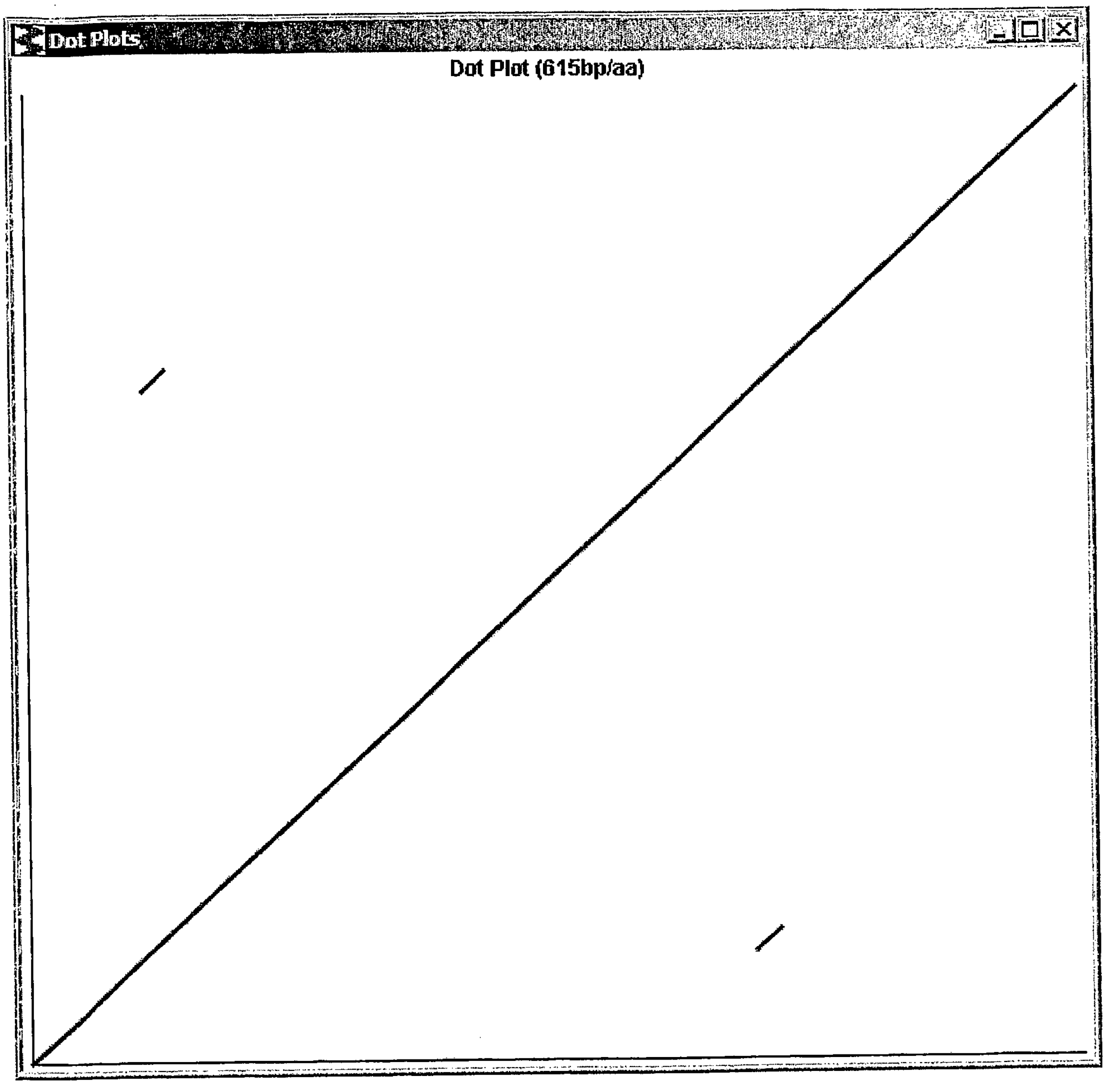


Figure 38

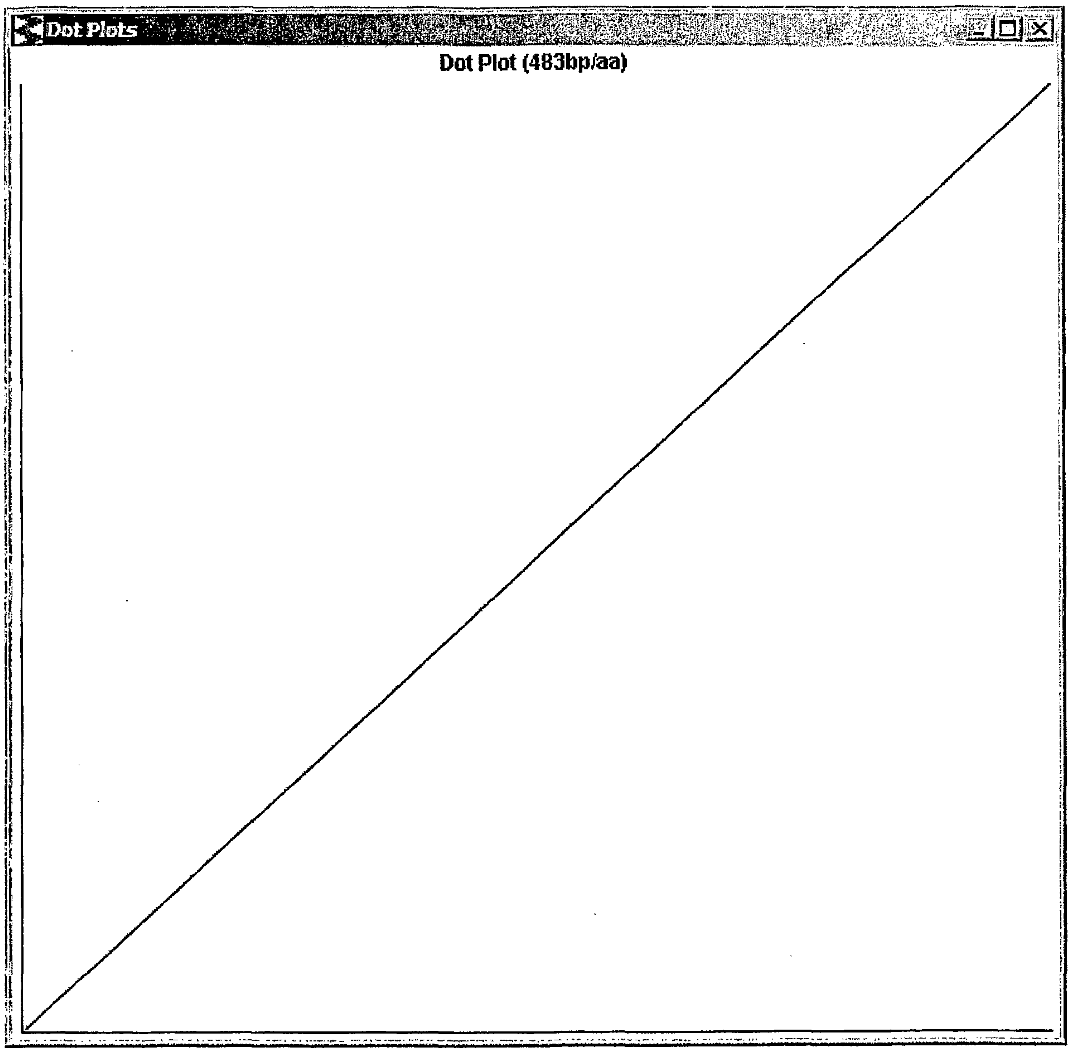


Figure 39

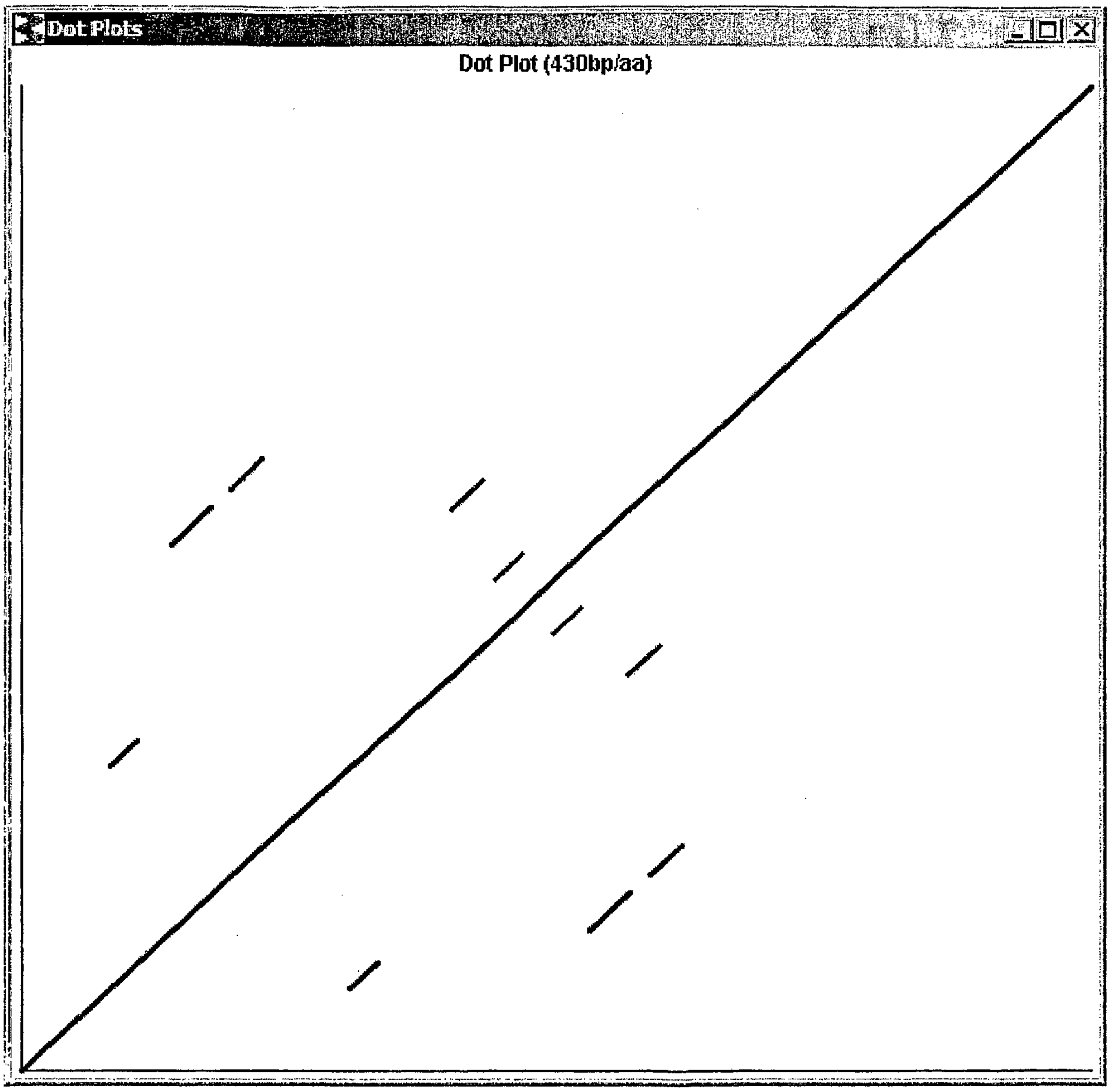


Figure 40

**3bp overlap, 5' overhang**

target sequence: 5'-$N_{-9}N_{-8}N_{-7}N_{-6}N_{-5}N_{-4}N_{-3}N_{-2}N_{-1}\mathbf{N_AN_BN_C}N_{+1}N_{+2}N_{+3}N_{+4}N_{+5}N_{+6}N_{+7}N_{+8}N_{+9}$-3'

7 base recognition site

***SapI:*** **GCTCTTC (5x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_AN_BN_C}N_{+1}$GAAGAGC-3' (SEQ ID NO: 1)
3' segment end: 5'-GCTCTTCN$_{-1}\mathbf{N_AN_BN_C}N_{+1}N_{+2}N_{+3}N_{+4}N_{+5}N_{+6}N_{+7}N_{+8}N_{+9}$-3' (SEQ ID NO: 2)

6 base recognition site

***EarI:*** **CTCTTC (2x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_AN_BN_C}N_{+1}$GAAGAG-3' (SEQ ID NO: 3)
3' segment end: 5'-CTCTTCN$_{-1}\mathbf{N_AN_BN_C}N_{+1}N_{+2}N_{+3}N_{+4}N_{+5}N_{+6}N_{+7}N_{+8}N_{+9}$-3' (SEQ ID NO: 4)

**4bp overlap, 5' overhang**

target sequence: 5'-$N_{-9}N_{-8}N_{-7}N_{-6}N_{-5}N_{-4}N_{-3}N_{-2}N_{-1}\mathbf{N_AN_BN_CN_D}N_{+1}N_{+2}N_{+3}N_{+4}N_{+5}N_{+6}N_{+7}N_{+8}N_{+9}$-3'

**6 base recognition site**

***BbsI:*** **GAAGAC (10x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_AN_BN_CN_D}N_{+1}N_{+2}$GTCTTC-3' (SEQ ID NO: 5)
3' segment end: 5'-GAAGACN$_{-2}N_{-1}\mathbf{N_AN_BN_CN_D}N_{+1}N_{+2}N_{+3}N_{+4}N_{+5}N_{+6}N_{+7}N_{+8}N_{+9}$-3' (SEQ ID NO: 6)

***BsaI:*** **GGTCTC (10x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_AN_BN_CN_D}N_{+1}$GAGACC-3' (SEQ ID NO: 7)
3' segment end: 5'-GGTCTCN$_{-1}\mathbf{N_AN_BN_CN_D}N_{+1}N_{+2}N_{+3}N_{+4}N_{+5}N_{+6}N_{+7}N_{+8}N_{+9}$-3' (SEQ ID NO: 8)

***BsmBI:*** **CGTCTC (2x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_AN_BN_CN_D}N_{+1}$GAGACG-3' (SEQ ID NO: 9)
3' segment end: 5'-CGTCTCN$_{-1}\mathbf{N_AN_BN_CN_D}N_{+1}N_{+2}N_{+3}N_{+4}N_{+5}N_{+6}N_{+7}N_{+8}N_{+9}$-3' (SEQ ID NO: 10)

***BspMI:*** **ACCTGC (5x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_AN_BN_CN_D}N_{+1}N_{+2}N_{+3}N_{+4}$GCAGGT-3' (SEQ ID NO: 11)
3' segment end: 5'-ACCTGCN$_{-4}N_{-3}N_{-2}N_{-1}\mathbf{N_AN_BN_CN_D}N_{+1}N_{+2}N_{+3}N_{+4}N_{+5}N_{+6}N_{+7}N_{+8}N_{+9}$-3' (SEQ ID NO: 12)

Figure 41A

**5 base recognition site**

***BbvI:*** **GCAGC (2x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_A N_B N_C N_D}N_{+1}N_{+2}N_{+3}N_{+4}N_{+5}N_{+6}N_{+7}N_{+8}$GCTGC-3' (SEQ ID NO: 13)

3' segment end: 5'-GCAGCN$_{-8}N_{-7}N_{-6}N_{-5}N_{-4}N_{-3}N_{-2}N_{-1}\mathbf{N_A N_B N_C N_D}N_{+1}N_{+2}N_{+3}N_{+4}N_{+5}N_{+6}N_{+7}N_{+8}N_{+9}$-3' (SEQ ID NO: 14)

***BsmAI:*** **GTCTC (5x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_A N_B N_C N_D}N_{+1}$GAGAC-3' (SEQ ID NO: 15)
3' segment end: 5'-GTCTCN$_{-1}\mathbf{N_A N_B N_C N_D}N_{+1}N_{+2}N_{+3}N_{+4}N_{+5}N_{+6}N_{+7}N_{+8}N_{+9}$-3' (SEQ ID NO: 16)

***BsmFI:*** **GGGAC (10x digestion, 95% ligation, 95% recut)**

5' segment end: 5'-$N_{-10}N_{-9}N_{-8}N_{-7}N_{-6}N_{-5}N_{-4}N_{-3}N_{-2}N_{-1}\mathbf{N_A N_B N_C N_D}N_{+1}N_{+2}N_{+3}N_{+4}N_{+5}N_{+6}N_{+7}N_{+8}N_{+9}N_{+10}$GTCCC-3' (SEQ ID NO: 17)

3' segment end: 5'-GGGACN$_{-10}N_{-9}N_{-8}N_{-7}N_{-6}N_{-5}N_{-4}N_{-3}N_{-2}N_{-1}\mathbf{N_A N_B N_C N_D}N_{+1}N_{+2}N_{+3}N_{+4}N_{+5}N_{+6}N_{+7}N_{+8}N_{+9}N_{+10}$-3'(SEQ ID NO: 18)

***BspBI***

5' segment end: 5'-$N_{-9}N_{-8}N_{-7}N_{-6}N_{-5}N_{-4}N_{-3}N_{-2}N_{-1}\mathbf{N_A N_B N_C N_D}N_{+1}$GAGACG-3' (SEQ ID NO: 19)
3' segment end: 5'-CGTCTCN$_{-1}\mathbf{N_A N_B N_C N_D}N_{+1}N_{+2}N_{+3}N_{+4}N_{+5}N_{+6}N_{+7}N_{+8}N_{+9}$-3' (SEQ ID NO: 20)

***FokI:*** **GGATG (5x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_A N_B N_C N_D}N_{+1}N_{+2}N_{+3}N_{+4}N_{+5}N_{+6}N_{+7}N_{+8}N_{+9}$CATCC-3' (SEQ ID NO: 21)

3' segment end: 5'-GGATGN$_{-9}N_{-8}N_{-7}N_{-6}N_{-5}N_{-4}N_{-3}N_{-2}N_{-1}\mathbf{N_A N_B N_C N_D}N_{+1}N_{+2}N_{+3}N_{+4}N_{+5}N_{+6}N_{+7}N_{+8}N_{+9}$-3' (SEQ ID NO: 22)

***SfaNI:*** **GCATC (2x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_A N_B N_C N_D}N_{+1}N_{+2}N_{+3}N_{+4}N_{+5}$GATGC-3' (SEQ ID NO: 23)
3' segment end: 5'-GCATCN$_{-5}N_{-4}N_{-3}N_{-2}N_{-1}\mathbf{N_A N_B N_C N_D}N_{+1}N_{+2}N_{+3}N_{+4}N_{+5}N_{+6}N_{+7}N_{+8}N_{+9}$-3' (SEQ ID NO: 24)

Figure 41B

**5bp overlap, 5' overhang**

target sequence: 5'-$N_{-9}N_{-8}N_{-7}N_{-6}N_{-5}N_{-4}N_{-3}N_{-2}N_{-1}\mathbf{N_A N_B N_C N_D N_E}N_{+1}N_{+2}N_{+3}N_{+4}N_{+5}N_{+6}N_{+7}N_{+8}N_{+9}$-3'

**5 base recognition site**

***HgaI:* GACGC (5x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_A N_B N_C N_D N_E}N_{+1}N_{+2}N_{+3}N_{+4}N_{+5}$GCGTC-3' (SEQ ID NO: 25)

3' segment end: 5'-GACGC$N_{-5}N_{-4}N_{-3}N_{-2}N_{-1}\mathbf{N_A N_B N_C N_D N_E}N_{+1}N_{+2}N_{+3}N_{+4}N_{+5}N_{+6}N_{+7}N_{+8}N_{+9}$-3' (SEQ ID NO: 26)

**3' OVERHANG**

**1bp overlap, 3' overhang**

target sequence: 5'-$N_{-9}N_{-8}N_{-7}N_{-6}N_{-5}N_{-4}N_{-3}N_{-2}N_{-1}\mathbf{N_A}N_{+1}N_{+2}N_{+3}N_{+4}N_{+5}N_{+6}N_{+7}N_{+8}N_{+9}$-3'

6 base recognition site

*BmrI:* ACTGGG (2x digestion, 75% ligation, 95% recut)

5' segment end: 5'-$N_{-10}N_{-9}N_{-8}N_{-7}N_{-6}N_{-5}N_{-4}N_{-3}N_{-2}N_{-1}\mathbf{N_A}N_{+1}N_{+2}N_{+3}N_{+4}$CCCAGT-3' (SEQ ID NO: 27)

3' segment end: 5'-ACTGGG$N_{-4}N_{-3}N_{-2}N_{-1}\mathbf{N_A}N_{+1}N_{+2}N_{+3}N_{+4}N_{+5}N_{+6}N_{+7}N_{+8}N_{+9}N_{+10}$-3' (SEQ ID NO: 28)

**2bp overlap, 3' overhang**

**6 base recognition site**

***BpmI:* CTGGAG (5x digestion, 95% ligation, 95% recut)**

5' segment end:
5'-$N_{-9}N_{-8}N_{-7}N_{-6}N_{-5}N_{-4}N_{-3}N_{-2}N_{-1}\mathbf{N_A N_B}N_{+1}N_{+2}N_{+3}N_{+4}N_{+5}N_{+6}N_{+7}N_{+8}N_{+9}N_{+10}N_{+11}N_{+12}N_{+13}N_{+14}$CTCCAG-3' (SEQ ID NO: 29)

3' segment end:
5'-CTGGAG$N_{-14}N_{-13}N_{-12}N_{-11}N_{-10}N_{-9}N_{-8}N_{-7}N_{-6}N_{-5}N_{-4}N_{-3}N_{-2}N_{-1}\mathbf{N_A N_B}N_{+1}N_{+2}N_{+3}N_{+4}N_{+5}N_{+6}N_{+7}N_{+8}N_{+9}N_{+10}$-3' (SEQ ID NO: 30)

***BseRI:* GAGGAG (4x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_A N_B}N_{+1}N_{+2}N_{+3}N_{+4}N_{+5}N_{+6}N_{+7}N_{+8}$CTCCAG-3' (SEQ ID NO: 31)

3' segment end: 5'-CTGGAG$N_{-8}N_{-7}N_{-6}N_{-5}N_{-4}N_{-3}N_{-2}N_{-1}\mathbf{N_A N_B}N_{+1}N_{+2}N_{+3}N_{+4}N_{+5}N_{+6}N_{+7}N_{+8}N_{+9}N_{+10}$-3' (SEQ ID NO: 32)

***BsmI:* GAATGC (10x digestion, 95% ligation, 95% recut) Note: $\mathbf{N_A N_B}$ must be CG**

5' segment end: 5'-$N_{-9}N_{-8}N_{-7}N_{-6}N_{-5}N_{-4}N_{-3}N_{-2}N_{-1}\mathbf{N_A}$GCATTC-3' (SEQ ID NO: 33)

3' segment end: 5'-GAATG**C**$\mathbf{N_B}N_{+1}N_{+2}N_{+3}N_{+4}N_{+5}N_{+6}N_{+7}N_{+8}N_{+9}N_{+10}$-3' (SEQ ID NO: 34)

***BsrI:* ACTGG (4x digestion, 95% ligation, 95% recut) Note: $\mathbf{N_A N_B}$ must be GC**

5' segment end: 5'-$N_{-9}N_{-8}N_{-7}N_{-6}N_{-5}N_{-4}N_{-3}N_{-2}N_{-1}\mathbf{N_A}$CCAGT-3' (SEQ ID NO: 35)

3' segment end: 5'-ACTG**G**$\mathbf{N_B}N_{+1}N_{+2}N_{+3}N_{+4}N_{+5}N_{+6}N_{+7}N_{+8}N_{+9}N_{+10}$-3' (SEQ ID NO: 36)

***BsrDI:* GCAATG (10x digestion, 95% ligation, 95% recut)**

5' segment end: 5'-$N_{-9}N_{-8}N_{-7}N_{-6}N_{-5}N_{-4}N_{-3}N_{-2}N_{-1}\mathbf{N_A N_B}$CATTGC-3' (SEQ ID NO: 37)

3' segment end: 5'-GCAATG$\mathbf{N_A N_B}N_{+1}N_{+2}N_{+3}N_{+4}N_{+5}N_{+6}N_{+7}N_{+8}N_{+9}N_{+10}$-3' (SEQ ID NO: 38)

# Figure 41C

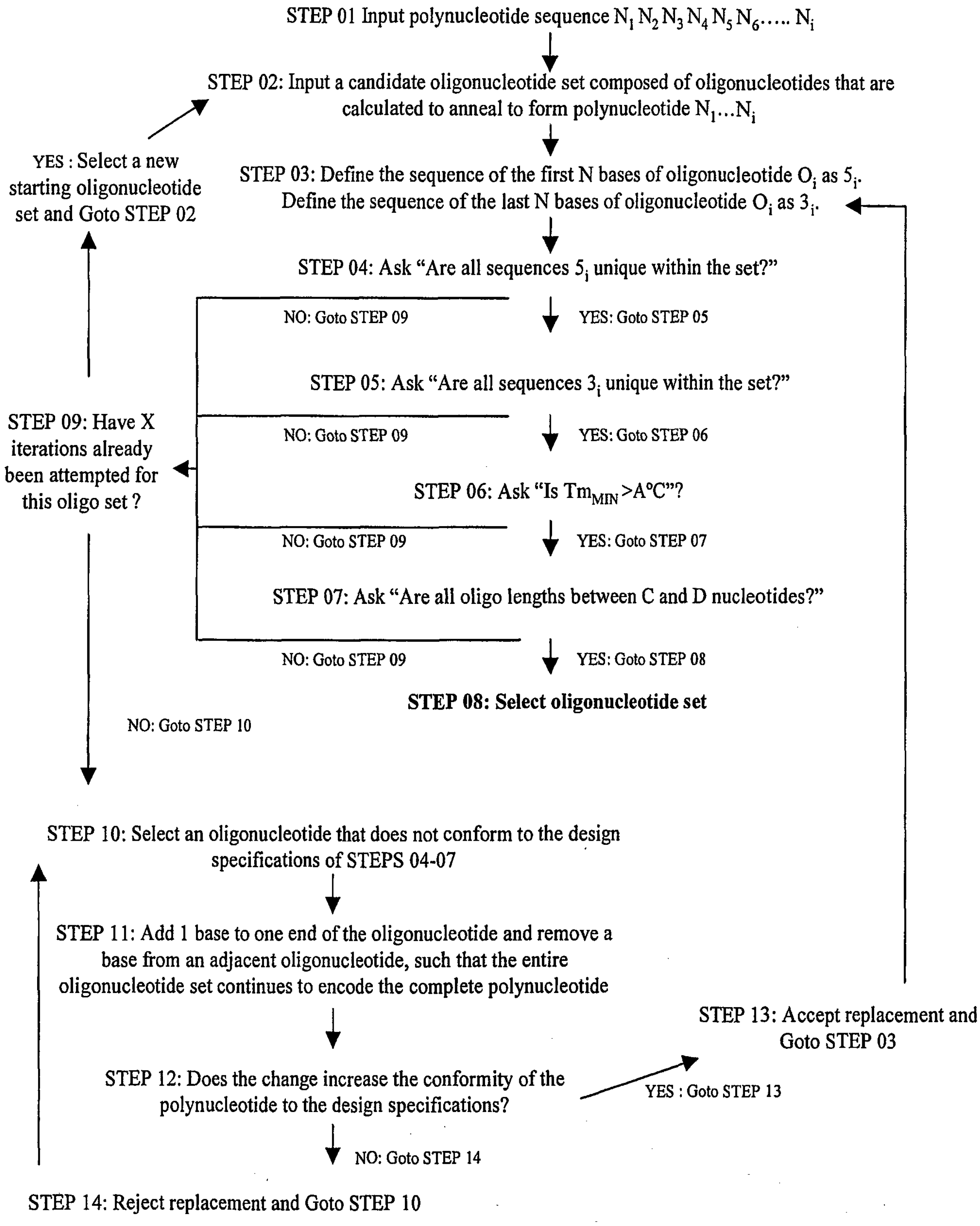
STEP 01 Input polynucleotide sequence $N_1 N_2 N_3 N_4 N_5 N_6 \ldots N_i$
STEP 02: Input a candidate oligonucleotide set composed of oligonucleotides that are calculated to anneal to form polynucleotide $N_1 \ldots N_i$
YES : Select a new starting oligonucleotide set and Goto STEP 02
STEP 03: Define the sequence of the first N bases of oligonucleotide $O_i$ as $5_i$. Define the sequence of the last N bases of oligonucleotide $O_i$ as $3_i$.
STEP 04: Ask "Are all sequences $5_i$ unique within the set?"
NO: Goto STEP 09
YES: Goto STEP 05
STEP 05: Ask "Are all sequences $3_i$ unique within the set?"
STEP 09: Have X iterations already been attempted for this oligo set ?
NO: Goto STEP 09
YES: Goto STEP 06
STEP 06: Ask "Is $Tm_{MIN}$ >A°C"?
NO: Goto STEP 09
YES: Goto STEP 07
STEP 07: Ask "Are all oligo lengths between C and D nucleotides?"
NO: Goto STEP 09
YES: Goto STEP 08
STEP 08: Select oligonucleotide set
NO: Goto STEP 10
STEP 10: Select an oligonucleotide that does not conform to the design specifications of STEPS 04-07
STEP 11: Add 1 base to one end of the oligonucleotide and remove a base from an adjacent oligonucleotide, such that the entire oligonucleotide set continues to encode the complete polynucleotide
STEP 13: Accept replacement and Goto STEP 03
STEP 12: Does the change increase the conformity of the polynucleotide to the design specifications?
YES : Goto STEP 13
NO: Goto STEP 14
STEP 14: Reject replacement and Goto STEP 10

Figure 42

| | | | |
|---|---|---|---|
| 1 | Denature | 95°C | 30sec |
| 2 | Denature | 95°C | 30sec |
| 3 | Anneal | 48°C -70°C | 30sec |
| 4 | Extend | 70°C | 60sec |
| 5 | Goto | step 2 | 39 times |
| 6 | | 4°C | forever |
| 7 | End | | |

Figure 43

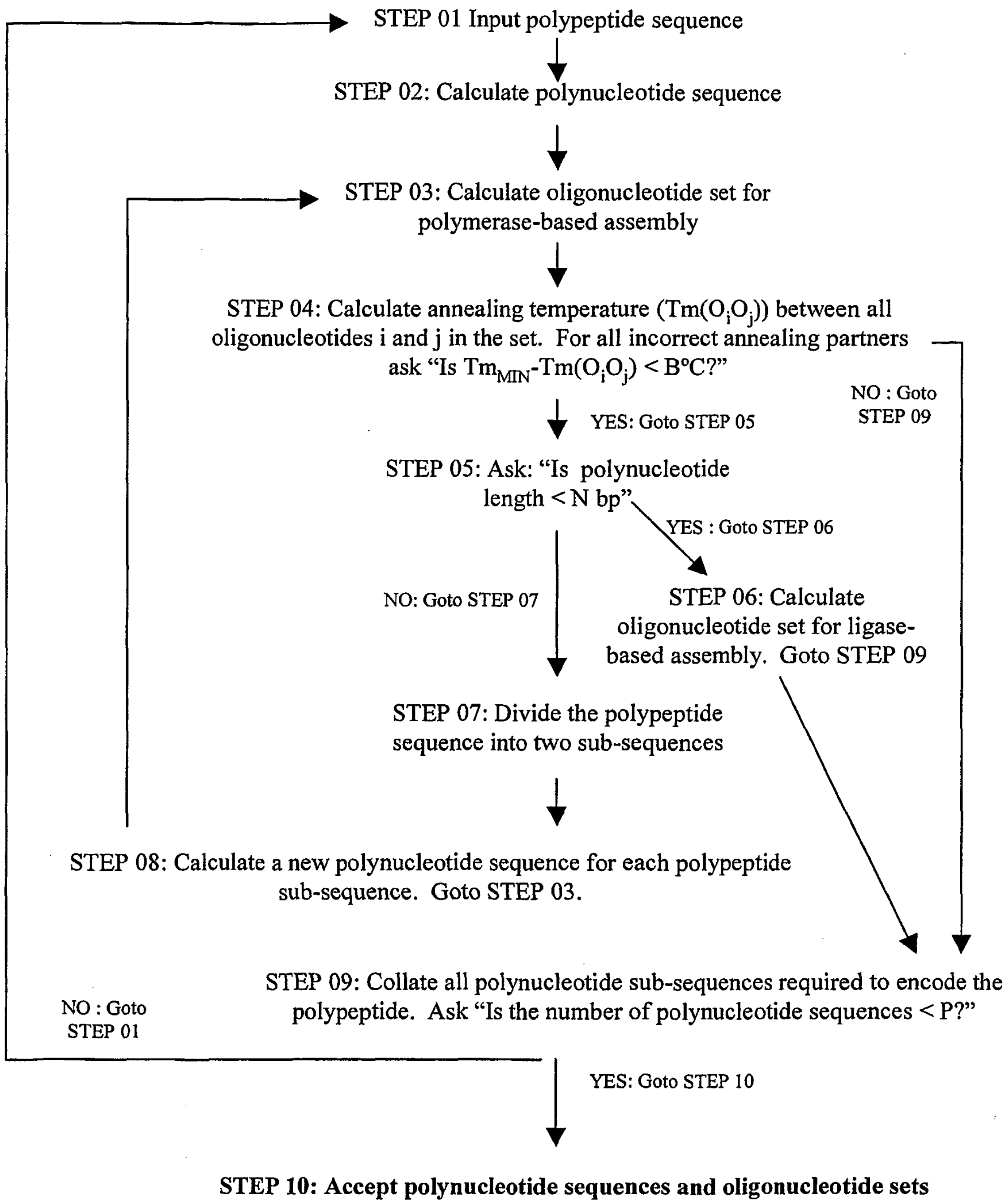
STEP 01 Input polypeptide sequence
STEP 02: Calculate polynucleotide sequence
STEP 03: Calculate oligonucleotide set for polymerase-based assembly
STEP 04: Calculate annealing temperature ($Tm(O_iO_j)$) between all oligonucleotides i and j in the set. For all incorrect annealing partners ask "Is $Tm_{MIN}$-$Tm(O_iO_j)$ < B°C?"
NO : Goto STEP 09
YES: Goto STEP 05
STEP 05: Ask: "Is polynucleotide length < N bp"
YES : Goto STEP 06
NO: Goto STEP 07
STEP 06: Calculate oligonucleotide set for ligase-based assembly. Goto STEP 09
STEP 07: Divide the polypeptide sequence into two sub-sequences
STEP 08: Calculate a new polynucleotide sequence for each polypeptide sub-sequence. Goto STEP 03.
STEP 09: Collate all polynucleotide sub-sequences required to encode the polypeptide. Ask "Is the number of polynucleotide sequences < P?"
NO : Goto STEP 01
YES: Goto STEP 10
STEP 10: Accept polynucleotide sequences and oligonucleotide sets

Figure 44

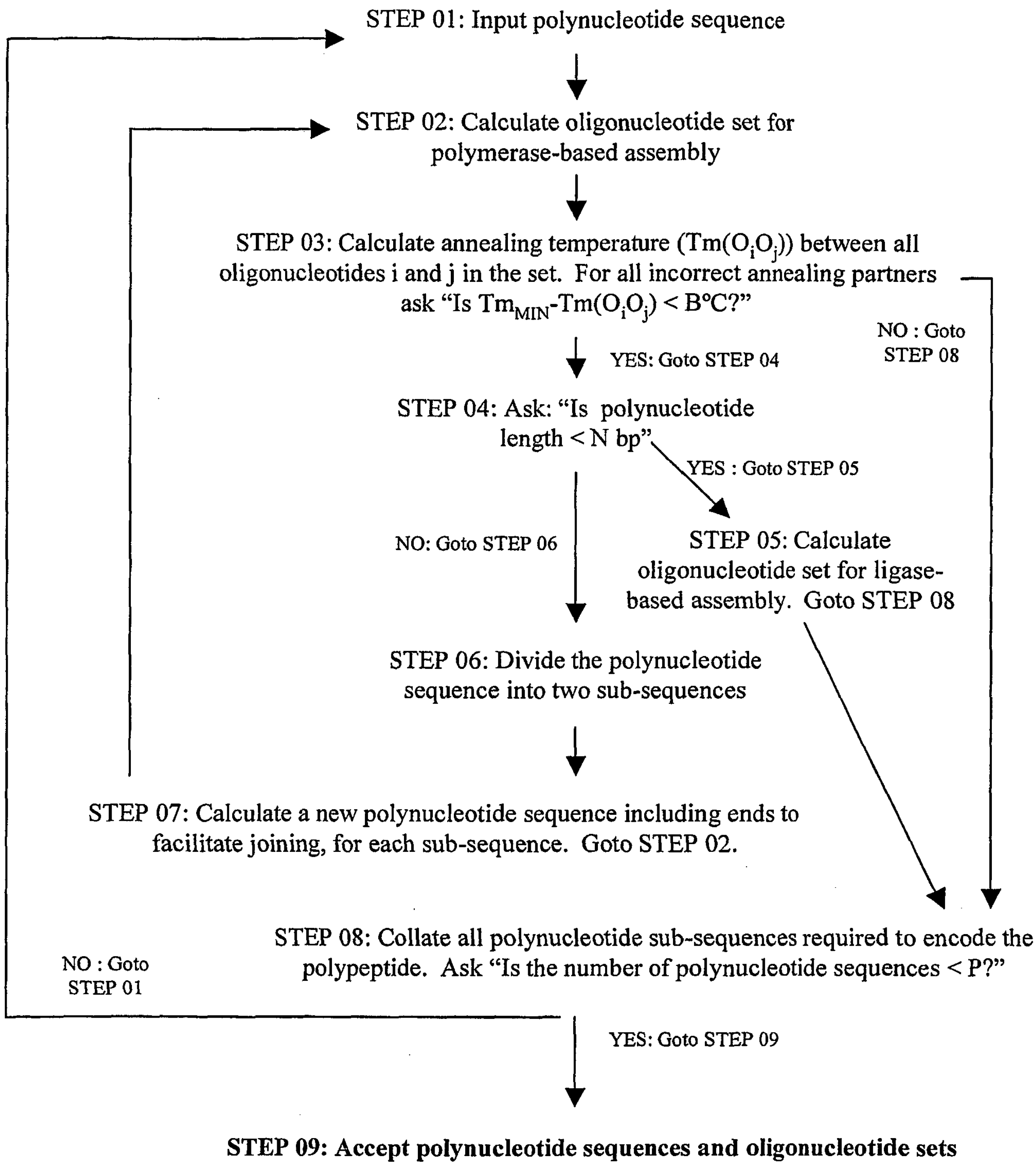
STEP 01: Input polynucleotide sequence
STEP 02: Calculate oligonucleotide set for polymerase-based assembly
STEP 03: Calculate annealing temperature ($Tm(O_iO_j)$) between all oligonucleotides i and j in the set. For all incorrect annealing partners ask "Is $Tm_{MIN}$-$Tm(O_iO_j)$ < B°C?"
NO : Goto STEP 08
YES: Goto STEP 04
STEP 04: Ask: "Is polynucleotide length < N bp"
YES : Goto STEP 05
NO: Goto STEP 06
STEP 05: Calculate oligonucleotide set for ligase-based assembly. Goto STEP 08
STEP 06: Divide the polynucleotide sequence into two sub-sequences
STEP 07: Calculate a new polynucleotide sequence including ends to facilitate joining, for each sub-sequence. Goto STEP 02.
STEP 08: Collate all polynucleotide sub-sequences required to encode the polypeptide. Ask "Is the number of polynucleotide sequences < P?"
NO : Goto STEP 01
YES: Goto STEP 09
STEP 09: Accept polynucleotide sequences and oligonucleotide sets

Figure 45

gaggaagcggaaggcgagagtagggaactgccaggcatcaaactaagcagaaggcccctgacggatggcc
tttttgcgtttctacaaactctttctgtgttgtaaaacgacggccagtcttaagctcgggcctcaaataa
tgattttagatatcgccatccagctgatattccctatagtgcatggtcatagctgtttcctggcagctct
ggcccgtgtctcaaaatctctgatgttacattgtacaagataaaataatatcatcatgaacaataaaact
gtctgcttacataaacagtaatacaaggggtgttatgagccatattcaacgggaaacgtcgaggccgcga
ttaaattccaacatggatgctgatttatatgggtataaatgggctcgcgataatgtcgggcaatcaggtg
cgacaatctatcgcttgtatgggaagcccgatgcgccagagttgtttctgaaacatggcaaaggtagcgt
tgccaatgatgttacagatgagatggtcagactaaactggctgacggaatttatgccacttccgaccatc
aagcattttatccgtactcctgatgatgcatggttactcaccactgcgatccccggaaaaacagcgttcc
aggtattagaagaatatcctgattcaggtgaaaatattgttgatgcgctggcagtgttcctgcgccggtt
gcactcgattcctgtttgtaattgtccttttaacagcgatcgcgtatttcgcctcgctcaggcgcaatca
cgaatgaataacggtttggttgatgcgagtgattttgatgacgagcgtaatggctggcctgttgaacaag
tctggaaagaaatgcataaacttttgccattctcaccggattcagtcgtcactcatggtgatttctcact
tgataaccttatttttgacgaggggaaattaataggttgtattgatgttggacgagtcggaatcgcagac
cgataccaggatcttgccatcctatggaactgcctcggtgagttttctccttcattacagaaacggcttt
ttcaaaaatatggtattgataatcctgatatgaataaattgcagtttcatttgatgctcgatgagttttt
ctaatcagaattggttaattggttgtaacactggcagagcattacgctgacttgacgggacggcgcaagc
tcatgaccaaaatcccttaacgtgagttacgcgcgcgtcgttccactgagcgtcagaccccgtagaaaag
atcaaaggatcttcttgagatcctttttttctgcgcgtaatctgctgcttgcaaacaaaaaaaccaccgc
taccagcggtggtttgtttgccggatcaagagctaccaactctttttccgaaggtaactggcttcagcag
agcgcagataccaaatactgttcttctagtgtagccgtagttagcccaccacttcaagaactctgtagca
ccgcctacatacctcgctctgctaatcctgttaccagtggctgctgccagtggcgataagtcgtgtctta
ccgggttggactcaagacgatagttaccggataaggcgcagcggtcgggctgaacggggggttcgtgcac
acagcccagcttggagcgaacgacctacaccgaactgagatacctacagcgtgagctatgagaaagcgcc
acgcttcccgaagggagaaaggcggacaggtatccggtaagcggcagggtcggaacaggagagcgcacga
gggagcttccagggggaaacgcctggtatctttatagtcctgtcgggtttcgccacctctgacttgagcg
tcgatttttgtgatgctcgtcaggggggcggagcctatggaaaaacgccagcaacgcggcctttttacgg
ttcctggccttttgctggccttttgctcacatgttctttcctgcgttatcccctgattctgtggataacc
gtattaccgcctttgagtgagctgataccgctcgccgcagccgaacgaccgagcgcagcgagtcagtgag
c (SEQ ID NO: 39)

Figure 46

gaggaagcggaaggcgagagtagggaactgccaggcatcaaactaagcagaaggcccctgacggatggcc
tttttgcgtttctacaaactctttctgtgttgtaaaacgacggccagtcttaagctcgggccccaaataa
tgattttattttgactgatagtgacctgttcgttgcaacacattgatgagcaatgcttttttataatgcc
aactttgtacaaaaaagctgaacgagaaacgtaaaatgatataaatatcaatatattaaattagattttg
cataaaaaacagactacataatactgtaaaacacaacatatccagtcactatgaatcaactacttagatg
gtattagtgacctgtagtcgaccgacagccttccaaatgttcttcgggtgatgctgccaacttagtcgac
cgacagccttccaaatgttcttctcaaacggaatcgtcgtatccagcctactcgctattgtcctcaatgc
cgtattaaatcataaaaagaaataagaaaaagaggtgcgagcctcttttttgtgtgacaaaataaaaaca
tctacctattcatatacgctagtgtcatagtcctgaaaatcatctgcatcaagaacaatttcacaactct
tatacttttctcttacaagtcgttcggcttcatctggattttcagcctctatacttactaaacgtgataa
agtttctgtaatttctactgtatcgacctgcagactggctgtgtataagggagcctgacatttatattcc
ccagaacatcaggttaatggcgtttttgatgtcattttcgcggtggctgagatcagccacttcttccccg
ataacggagaccggcacactggccatatcggtggtcatcatgcgccagctttcatccccgatatgcacca
ccgggtaaagttcacgggagactttatctgacagcagacgtgcactggccagggggatcaccatccgtcg
cccgggcgtgtcaataatatcactctgtacatccacaaacagacgataacggctctctcttttataggtg
taaaccttaaactgcatttcaccagcccctgttctcgtcagcaaaagagccgttcatttcaataaaccgg
gcgacctcagccatcccttcctgattttccgctttccagcgttcggcacgcagacgacgggcttcattct
gcatggttgtgcttaccagaccggagatattgacatcatatatgccttgagcaactgatagctgtcgctg
tcaactgtcactgtaatacgctgcttcatagcatacctctttttgacatacttcgggtatacatatcagt
atatattcttataccgcaaaaatcagcgcgcaaatacgcatactgttatctggcttttagtaagccggat
ccacgcggcgtttacgccccgccctgccactcatcgcagtactgttgtaattcattaagcattctgccga
catggaagccatcacagacggcatgatgaacctgaatcgccagcggcatcagcaccttgtcgccttgcgt
ataatatttgcccatggtgaaaacgggggcgaagaagttgtccatattggccacgtttaaatcaaaactg
gtgaaactcacccagggattggctgagacgaaaaacatattctcaataaaccctttagggaaataggcca
ggttttcaccgtaacacgccacatcttgcgaatatatgtgtagaaactgccggaaatcgtcgtggtattc
actccagagcgatgaaaacgtttcagtttgctcatggaaaacggtgtaacaagggtgaacactatcccat
atcaccagctcaccgtctttcattgccatacggaattccggatgagcattcatcaggcgggcaagaatgt
gaataaaggccggataaaacttgtgcttatttttctttacggtctttaaaaaggccgtaatatccagctg
aacggtctggttataggtacattgagcaactgactgaaatgcctcaaaatgttctttacgatgccattgg
gatatatcaacggtggtatatccagtgatttttttctccattttagcttccttagctcctgaaaatctcg
ataactcaaaaaatacgcccggtagtgatcttatttcattatggtgaaagttggaacctcttacgtgccg
atcaacgtctcattttcgccaaaagttggcccagggcttcccggtatcaacagggacaccaggatttatt
tattctgcgaagtgatcttccgtcacaggtatttattcggcgcaaagtgcgtcgggtgatgctgccaact
tagtcgactacaggtcactaataccatctaagtagttgattcatagtgactggatatgttgtgttttaca
gtattatgtagtctgtttttttatgcaaaatctaatttaatatattgatatttatatcattttacgtttct
cgttcagctttcttgtacaaagttggcattataagaaagcattgcttatcaatttgttgcaacgaacagg
tcactatcagtcaaaataaaatcattatttgccatccagctgatatcccctataggtcatagctgtttcc
tggcagctctggcccgtgtctcaaaatctctgatgttacattgtacaagataaaataatatcatcatgaa
caataaaactgtctgcttacataaacagtaatacaaggggtgttatgagccatattcaacgggaaacgtc
gaggccgcgattaaattccaacatggatgctgatttatatgggtataaatgggctcgcgataatgtcggg
caatcaggtgcgacaatctatcgcttgtatgggaagcccgatgcgccagagttgtttctgaaacatggca
aaggtagcgttgccaatgatgttacagatgagatggtcagactaaactggctgacggaatttatgccact
tccgaccatcaagcattttatccgtactcctgatgatgcatggttactcaccactgcgatccccggaaaa
acagcgttccaggtattagaagaatatcctgattcaggtgaaaatattgttgatgcgctggcagtgttcc
tgcgccggttgcactcgattcctgtttgtaattgtccttttaacagcgatcgcgtatttcgcctcgctca
ggcgcaatcacgaatgaataacggtttggttgatgcgagtgattttgatgacgagcgtaatggctggcct
gttgaacaagtctggaaagaaatgcataaacttttgccattctcaccggattcagtcgtcactcatggtg
atttctcacttgataaccttatttttgacgaggggaaattaataggttgtattgatgttggacgagtcgg
aatcgcagaccgataccaggatcttgccatcctatggaactgcctcggtgagttttctccttcattacag
aaacggctttttcaaaaatatggtattgataatcctgatatgaataaattgcagtttcatttgatgctcg
atgagtttttctaatcagaattggttaattggttgtaacactggcagagcattacgctgacttgacgga
cggcgcaagctcatgaccaaaatcccttaacgtgagttacgcgcgtcgttccactgagcgtcagaccc
cgtagaaaagatcaaaggatcttcttgagatcctttttttctgcgcgtaatctgctgcttgcaaacaaaa
aaaccaccgctaccagcggtggtttgtttgccggatcaagagctaccaactctttttccgaaggtaactg
gcttcagcagagcgcagataccaaatactgttcttctagtgtagccgtagttagcccaccacttcaagaa
ctctgtagcaccgcctacatacctcgctctgctaatcctgttaccagtggctgctgccagtggcgataag
tcgtgtcttaccgggttggactcaagacgatagttaccggataaggcgcagcggtcgggctgaacggggg
gttcgtgcacacagcccagcttggagcgaacgacctacaccgaactgagatacctacagcgtgagctatg
agaaagcgccacgcttcccgaagggagaaaggcggacaggtatccggtaagcggcagggtcggaacagga
gagcgcacgagggagcttccagggggaaacgcctggtatctttatagtcctgtcgggtttcgccacctcg
acttgagcgtcgatttttgtgatgctcgtcaggggggcggagcctatggaaaaacgccagcaacgcggcc
tttttacggttcctggccttttgctggccttttgctcacatgttctttcctgcgttatcccctgattctg
tggataaccgtattaccgcctttgagtgagctgataccgctcgccgcagccgaacgaccgagcgcagcga
gtcagtgagc (SEQ ID NO: 40)

# Figure 47

gaggaagcggaaggcgagagtagggaactgccaggcatcaaactaagcagaaggcccctgacggatggcc
tttttgcgtttctacaaactctttctgtgttgtaaaacgacggccagtcttaagctcgggcctcaaataa
tgattttagattaacggtctccttttctcgagcggataaatgtgagcggataacattgacattgtgagcg
gataacaagatactgagcacatcagcaggacgcactgaccgcgggatcccggtgcagaaaataaggagga
aaaaaaaatgagcaaaggtgaagaactgttcaccggcgttgtgccaattctggttgagctggatggtgac
gtgaatggccacaaattttccgtgtctggtgaaggcgagggtgatgctacttatggcaaactgactctga
aactgatctgtaccaccggcaaactgcctgttccgtggccaactctggtcactactctgggttacggcct
gatgtgttttgcgcgttacccggatcacatgaaacagcatgacttcttcaaatctgccatgccggaaggc
tatgtccaagaacgtacgatctttttcaaggacgacggcaactataaaacccgtgccgaagttaaattcg
agggtgacaccctggttaaccgcatcgaactgaaaggcattgacttcaaagaggacggcaacattctggg
tcacaagctggaatacaactacaactcccacaacgtttacattactgctgacaagcagaaaaacggcatc
aaagcaaacttcaagatccgtcacaacattgaagatggtggcgtacagctggcagatcactaccagcaga
acactccaatcggtgatggcccagtactgctgccagataaccattacctgtcctaccagagcaaactgtc
taaagacccgaacgaaaaacgtgaccacatggtactgctggaatttgttaccgcggcaggcattacccac
ggtatggacgaactgtataaataaccccagagaccgttaatcgccatccagctgatattccctatagtgc
atggtcatagctgtttcctggcagctctggcccgtgtctcaaaatctctgatgttacattgtacaagata
aaataatatcatcatgaacaataaaactgtctgcttacataaacagtaatacaaggggtgttatgagcca
tattcaacgggaaacgtcgaggccgcgattaaattccaacatggatgctgatttatatgggtataaatgg
gctcgcgataatgtcgggcaatcaggtgcgacaatctatcgcttgtatgggaagcccgatgcgccagagt
tgtttctgaaacatggcaaaggtagcgttgccaatgatgttacagatgagatggtcagactaaactggct
gacggaatttatgccacttccgaccatcaagcattttatccgtactcctgatgatgcatggttactcacc
actgcgatccccggaaaaacagcgttccaggtattagaagaatatcctgattcaggtgaaaatattgttg
atgcgctggcagtgttcctgcgccggttgcactcgattcctgtttgtaattgtccttttaacagcgatcg
cgtatttcgcctcgctcaggcgcaatcacgaatgaataacggtttggttgatgcgagtgattttgatgac
gagcgtaatggctggcctgttgaacaagtctggaaagaaatgcataaacttttgccattctcaccggatt
cagtcgtcactcatggtgatttctcacttgataaccttatttttgacgaggggaaattaataggttgtat
tgatgttggacgagtcggaatcgcagaccgataccaggatcttgccatcctatggaactgcctcggtgag
ttttctccttcattacagaaacggctttttcaaaaatatggtattgataatcctgatatgaataaattgc
agtttcatttgatgctcgatgagtttttctaatcagaattggttaattggttgtaacactggcagagcat
tacgctgacttgacgggacggcgcaagctcatgaccaaaatcccttaacgtgagttacgcgcgcgtcgtt
ccactgagcgtcagaccccgtagaaaagatcaaaggatcttcttgagatcctttttttctgcgcgtaatc
tgctgcttgcaaacaaaaaaaccaccgctaccagcggtggtttgtttgccggatcaagagctaccaactc
tttttccgaaggtaactggcttcagcagagcgcagataccaaatactgttcttctagtgtagccgtagtt
agcccaccacttcaagaactctgtagcaccgcctacatacctcgctctgctaatcctgttaccagtggct
gctgccagtggcgataagtcgtgtcttaccgggttggactcaagacgatagttaccggataaggcgcagc
ggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagc
ggcagggtcggaacaggagagcgcacgagggagcttccagggggaaacgcctggtatctttatagtcctg
tcgggtttcgccacctctgacttgagcgtcgatttttgtgatgctcgtcaggggggcggagcctatggaa
aaacgccagcaacgcggcctttttacggttcctggccttttgctggccttttgctcacatgttctttcct
gcgttatcccctgattctgtggataaccgtattaccgcctttgagtgagctgataccgctcgccgcagcc
gaacgaccgagcgcagcgagtcagtgagc  (SEQ ID NO: 43)

# Figure 48

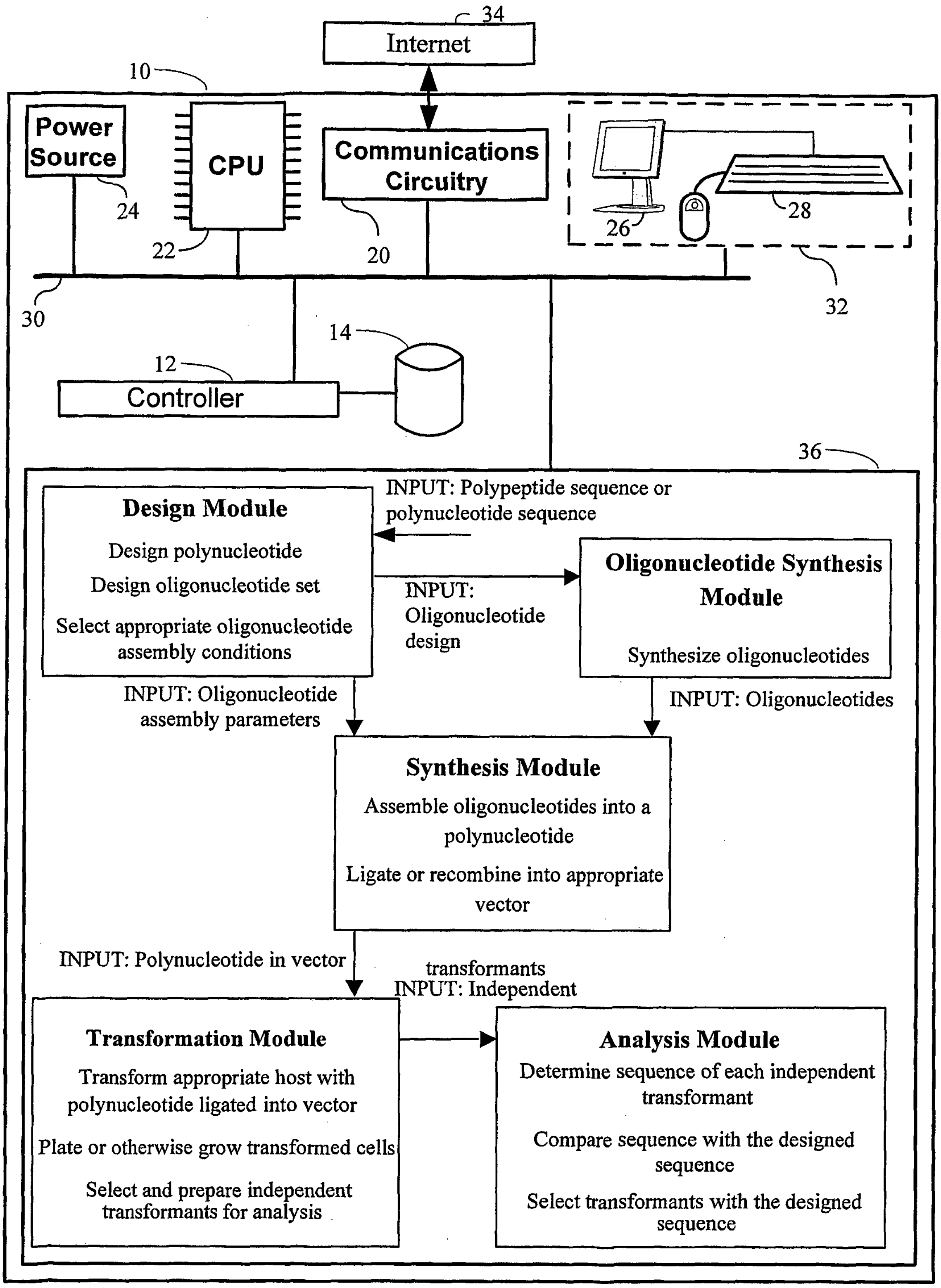
34
Internet
10
Power Source
24
CPU
22
Communications Circuitry
20
26
28
32
30
14
12
Controller
36
Design Module
Design polynucleotide
Design oligonucleotide set
Select appropriate oligonucleotide assembly conditions
INPUT: Polypeptide sequence or polynucleotide sequence
INPUT: Oligonucleotide design
Oligonucleotide Synthesis Module
Synthesize oligonucleotides
INPUT: Oligonucleotide assembly parameters
INPUT: Oligonucleotides
Synthesis Module
Assemble oligonucleotides into a polynucleotide
Ligate or recombine into appropriate vector
INPUT: Polynucleotide in vector
INPUT: Independent transformants
Transformation Module
Transform appropriate host with polynucleotide ligated into vector
Plate or otherwise grow transformed cells
Select and prepare independent transformants for analysis
Analysis Module
Determine sequence of each independent transformant
Compare sequence with the designed sequence
Select transformants with the designed sequence

Figure 49

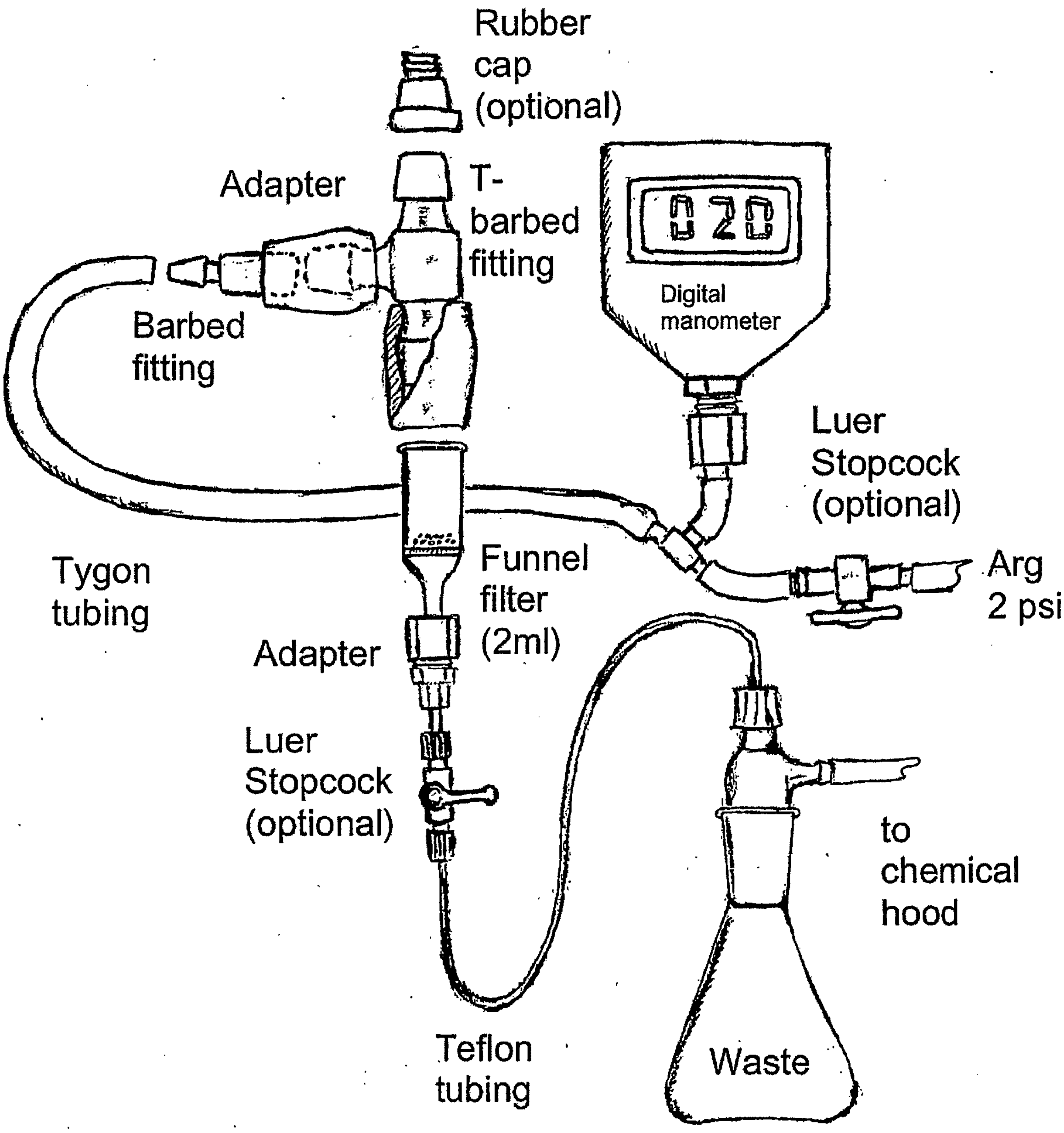
Rubber
cap
(optional)
Adapter
T-
barbed
fitting
020
Digital
manometer
Barbed
fitting
Luer
Stopcock
(optional)
Tygon
tubing
Funnel
filter
(2ml)
Arg
2 psi
Adapter
Luer
Stopcock
(optional)
to
chemical
hood
Teflon
tubing
Waste

Figure 50

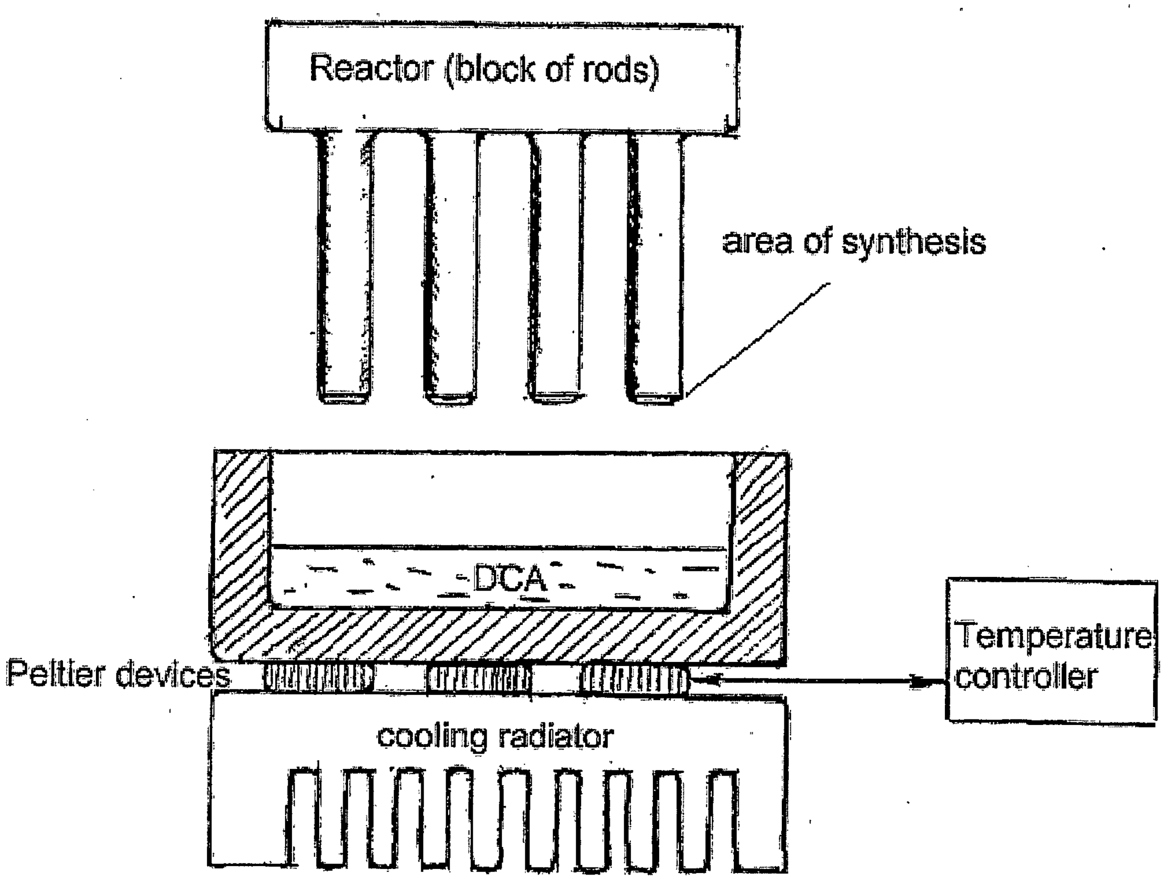
Reactor (block of rods)
area of synthesis
DCA
Peltier devices
Temperature controller
cooling radiator

Figure 51

CYP52A17

1 atgatcg***<u>agcagctgc</u>***tggaatactggtacgtggttgtgcctgttctgtatattatcaaa
61 cagctgctggcgtacactaaaacgcgtgtcctgatgaagaaactgggcgcagcgccggtg
121 actaacaaactgtacgataacgctttcggcatcgtaaatggttggaaagccctgcagttt
181 aagaaagagggtcgtgcgcaagaatataacgactataaattcgatcattctaagaacccg
241 agcgtgggtacttatgtgtctatcctgttcggtactcgcatcgtggtaactaaagaccca
301 gaaaacatcaaagcaatcctggcgacgcaattcggcgacttctctctgggtaaacgtcac
361 acgctgttcaaacctctgctgggcgatggcattttcaccctggatggtgaaggttggaaa
421 cattcccgtgcgatgctgcgtccgcagtttgcgcgtgaacaggttgcgcacgttacgtct
481 ctggagccgcacttccagctgctgaagaaacatatcctgaaacacaaaggcgagtatttc
541 gatatccaggagctgttcttccgtttcaccgtagattccgctaccgaatttctgttcggt
601 gaatctgttcatagcctgaaagatgaaagcatcggcatcaaccaggatgacatcgacttc
661 gctggtcgcaaggatttcgcagaatccttcaataaagctcaggaatatctggcgatccgt
721 actctggtgcaaactttctattggctggttaacaataaagagtttcgcgactgtaccaaa
781 tccgttcataaattcactaactactacgttcagaaagctctggatgcatccccggaagaa
841 ctggaaaagcagtccggttacgttttcctgtacgaactggtgaaacagactcgtgacccg
901 aacgtcctgcgtgaccagtctctgaacatcctgctggccggccgtgacactaccgctggc
961 ctgctgtccttcgcggtcttcgagctggcccgtcatccggaaatctgggccaaactgcgt
1021 gaagaaatcgaacagcaattcggcctgggtgaggactcccgtgttgaagaaatcactttc
1081 gaatctctgaaacgttgcgaatatctgaaagcattcctgaacgaaacgctgcgtatctac
1141 ccgtccgttccgcgcaacttccgcattgctaccaagaacacgaccctgccgcgtggcggt
1201 ggcagcgacggcacctctccgatcctgattcaaaagggtgaagcagtatcctacggtatt
1261 aactccacccacctggacccggtatactacggtccggacgcggcagaatttcgtccagag
1321 cgctggtttgaaccgtctaccaagaagctgggttgggcttatctgccgttcaacggcggc
1381 cctcgtatctgtctgggtcagcagtttgccctgaccgaggcaggctacgttctggttcgc
1441 ctggtccaagaattttctcacgtacgtagcgacccggacgaagtttacccgccgaagcgc
1501 ctgaccaacctgactatgtgcctgcaagatggcgctatcgtcaaatttgattaataa
(SEQ ID NO: 47)

CYP52A No Palindrome

1 atgatcga*<u>a</u>*ca*<u>a</u>*ctgctggaatactggtacgtggttgtgcctgttctgtatattatcaaa
61 cagctgctggcgtacactaaaacgcgtgtcctgatgaagaaactgggcgcagcgccggtg
121 actaacaaactgtacgataacgctttcggcatcgtaaatggttggaaagccctgcagttt
181 aagaaagagggtcgtgcgcaagaatataacgactataaattcgatcattctaagaacccg
241 agcgtgggtacttatgtgtctatcctgttcggtactcgcatcgtggtaactaaagaccca
301 gaaaacatcaaagcaatcctggcgacgcaattcggcgacttctctctgggtaaacgtcac
361 acgctgttcaaacctctgctgggcgatggcattttcaccctggatggtgaaggttggaaa
421 cattcccgtgcgatgctgcgtccgcagtttgcgcgtgaacaggttgcgcacgttacgtct
481 ctggagccgcacttccagctgctgaagaaacatatcctgaaacacaaaggcgagtatttc
541 gatatccaggagctgttcttccgtttcaccgtagattccgctaccgaatttctgttcggt
601 gaatctgttcatagcctgaaagatgaaagcatcggcatcaaccaggatgacatcgacttc
661 gctggtcgcaaggatttcgcagaatccttcaataaagctcaggaatatctggcgatccgt
721 actctggtgcaaactttctattggctggttaacaataaagagtttcgcgactgtaccaaa
781 tccgttcataaattcactaactactacgttcagaaagctctggatgcatccccggaagaa
841 ctggaaaagcagtccggttacgttttcctgtacgaactggtgaaacagactcgtgacccg
901 aacgtcctgcgtgaccagtctctgaacatcctgctggccggccgtgacactaccgctggc
961 ctgctgtccttcgcggtcttcgagctggcccgtcatccggaaatctgggccaaactgcgt
1021 gaagaaatcgaacagcaattcggcctgggtgaggactcccgtgttgaagaaatcactttc
1081 gaatctctgaaacgttgcgaatatctgaaagcattcctgaacgaaacgctgcgtatctac
1141 ccgtccgttccgcgcaacttccgcattgctaccaagaacacgaccctgccgcgtggcggt
1201 ggcagcgacggcacctctccgatcctgattcaaaagggtgaagcagtatcctacggtatt
1261 aactccacccacctggacccggtatactacggtccggacgcggcagaatttcgtccagag
1321 cgctggtttgaaccgtctaccaagaagctgggttgggcttatctgccgttcaacggcggc
1381 cctcgtatctgtctgggtcagcagtttgccctgaccgaggcaggctacgttctggttcgc
1441 ctggtccaagaattttctcacgtacgtagcgacccggacgaagtttacccgccgaagcgc
1501 ctgaccaacctgactatgtgcctgcaagatggcgctatcgtcaaatttgattaataa
SEQ ID NO: 48)

Figure 52

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5681702 A, Collins
- US 5780610 A, Collins
- US 4598049 A
- EP 05780055 A
- US 2005015593 W
- US 60666909 B
- US 60567460 B


### Non-patent literature cited in the description


- **HAYDEN et al.** *DNA,* 1988, vol. 7, 571-7
- **CICCARELLI et al.** *Nucleic Acids Res,* 1991, vol. 19, 6007-13
- **JAYARAMAN et al.** *Proc Natl Acad Sci U S A,* 1991, vol. 88, 4084-8
- **JAYARAMAN et al.** *Biotechniques,* 1992, vol. 12, 392-8
- **GRAHAM et al.** *Nucleic Acids Res,* 1993, vol. 21, 4923-8
- **KOBAYASHI et al.** *Biotechniques,* 1997, vol. 23, 500-3
- **AU et al.** *Biochem Biophys Res Commun.,* 1998, vol. 248, 200-203
- **HOOVER et al.** *Nucleic Acids Res,* 2002, vol. 30, e43
- **SHUTTLEWORTH et al.** *Gene,* 1987, vol. 58, 283-295
- **JINE et al.** *Journal of Biotechnology,* 1992, vol. 22, 271-282
- **GAIT.** *Practical approach series,* 1984, vol. xiii, 217
- **MATTEUCCI ; CARUTHERS.** *J Am Chem Soc.,* 1981, vol. 103, 3185-3191
- **PON et al.** *Tetrahedron Lett.,* 1985, vol. 26, 2525-2528
- **ADAMS et al.** *J Am Chem Soc,* 1983, vol. 105, 661-663
- **MCBRIDE et al.** *J Am Chem Soc,* 1986, vol. 108, 2040-2048
- **LETSINGER et al.** *Tetrahedron,* 1984, vol. 40, 137-143
- **HAYAKAWA et al.** *J Am Chem Soc,* 1990, vol. 112, 1691-1696
- **HAYAKAWA ; KATAOKA.** *J Am Chem Soc,* vol. 120, 12395-12401
- **GAIT.** *Practical Approach Series,* 1984, vol. xiii, 217
- **PON.** *Nucleic Acids Res,* 1987, vol. 15, 7203
- **HAYAKAWA et al.** *Tetrahedron Lett,* 1986, vol. 27, 4191-4194
- **PADIYA ; SALUNKHE.** *J Chem Research,* 1998, 804
- **EADIE ; DAVIDSON.** *Nucleic Acids Res,* 1987, vol. 15, 8333-49
- **BOAL et al.** *Nucleic Acids Res,* 1996, vol. 24, 3115
- **KWIATKOWSKI et al.** *Nucleic Acids Res,* 1996, vol. 24, 4632-4638
- **SINGLETON et al.** Dictionary of Microbiology and Molecular Biology. John Wiley and Sons, 1994
- **HALE ; MARHAM.** The Harper Collins Dictionary of Biology. Harper Perennial, 1991
- **SWITZER et al.** *Biochemistry,* 1993, vol. 32, 10489-10496
- **TOR et al.** *J. Am. Chem. Soc.,* 1993, vol. 115, 4461-4467
- **MANTSCH et al.** *Biochem.,* 1993, vol. 14, 5593-5601
- **PICCIRILLI et al.** *Nature,* 1990, vol. 343, 33-37
- **LEACH et al.** *J. Am. Chem. Soc.,* 1992, vol. 114, 3675-3683
- Gait's practical handbook. **GAIT.** Practical approach series. 1984, vol. xiii, 217
- **BEAUCAGE ; RADHAKRISHNAN.** *Tetrahedron,* 1992, vol. 48, 2223-2311
- **URDEA ; HORN.** *Tetrahedron Lett,* 1986, vol. 27, 2933-2936
- **BOLLEN et al.** *Critical Reviews in Biochemistry and Molecular Biology,* 2000, vol. 35, 393-432
- **MATTEUCCI ; CARUTHERS.** *J Am Chem Soc,* 1981, vol. 103, 3185-3191
- **CHAIX et al.** *Tetrahedron Lett,* 1989, vol. 30, 71-74
- **YU et al.** *Tetrahedron Lett,* 1994, vol. 34, 8565-8568
- **PON et al.** *Nucleic Acids Res,* 1985, vol. 13, 6447-65
- **PON et al.** *Nucleic Acids Res,* 1986, vol. 14, 6453-70

- **LETSINGER et al.** *J Am Chem Soc,* 1975, 3278-3279
- **PETRI et al.** *Langmuir,* 1999, vol. 15, 4520-4523
- **ALLENMARK.** analytical chemistry. Ellis Horwood series, 1988, 224
- **BOAL et al.** *Nucleic Acids Res,* 1996, vol. 24, 3115-7
- **CHU et al.** *Electrophoresis,* 1992, vol. 13, 105-14
- **ITO et al.** *Nucleic Acids Res,* 1982, vol. 10, 1755
- **SEPTAK.** *Nucleic Acids Res,* 1996, vol. 24, 3053-3058
- **PAUL ; ROYAPPA.** *Nucleic Acids Res,* 1996, vol. 24, 3048-3052
- **BABON et al.** *Mol Biotechnol,* 2003, vol. 23, 73-81
- **THOMAS et al.** *Biological Chemistry,* 2002, vol. 383, 1459-1462
- **SMITH ; MODRICH.** *Proc Natl Acad Sci,* 1997, vol. 94, 6847-6850
- **COTTON et al.** *Proc Natl Acad Sci,* 1998, vol. 85, 4397-4401
- **ROBERTS et al.** *Nucl Acids Res,* 1997, vol. 25, 3377-3378
- **BUI et al.** *BMC Chemical Biology,* 2003, vol. 3, http://www.biomedcentral.com/content/pdf/1472-6769-3-1.pdf
- **JUNICKE et al.** *Proc Natl Acad Sci,* 2003, vol. 100, 3737-3742
- **HORTON et al.** *Gene,* 1989, vol. 77, 61-8
- **HAYAKAWA ; KATAOKA.** *J Am Chem Soc,* 1990, vol. 120, 12395-12401
- **SELIGER et al.** *J Chromatogr,* 1989, vol. 476, 49-57
- **LEPROUST et al.** *Nucleic Acids Res,* 2001, vol. 29, 2171-80
- **PON.** *Current protocols, UNIT 3.1 and 3.2*